# EUROPEAN PATENT APPLICATION

(11) **EP 4 461 822 A1**
(43) Date of publication of application: **13.11.2024**
(21) Application number: 23737452.5
(22) Date of filing: 06.01.2023
(51) Int. Cl.: C12Q 1/6869, C12N 15/10, C12N 9/22, C12N 15/113

(54) **METHOD FOR PREDICTING POSSIBLE OFF-TARGETS IN GENE EDITING PROCESS**

(30) Priority: 07.01.2022 KR 20220003002; 25.11.2022 KR 20220160591
(71) Applicant: Toolgen Incorporated, Seoul 07789 (KR)
(72) Inventor: LEE, Jeongjoon, Seongnam-si, Gyeonggi-do 13532 (KR); KWON, Jeonghun, Seoul 08279 (KR); KIM, Minyoung, Seoul 07246 (KR); JO, Anna, Anyang-si, Gyeonggi-do 14075 (KR); KIM, Ungi, Seoul 08767 (KR); KIM, Youngho, Yongin-si, Gyeonggi-do 16839 (KR)
(74) Representative: dompatent von Kreisler Selting Werner - Partnerschaft von Patent- und Rechtsanwälten mbB
(86) International application number: PCT/KR2023/000332
(87) International publication number: WO 2023/132704

(57) **Abstract**

The present application relates to a method of predicting possible off-targets in a gene editing process (for example, genome editing process) using a gene editing system.

## Description

### [Technical Field]

The present application relates to a method of predicting possible off-targets in a gene editing process. The gene editing process may be a genomic DNA editing process, for example, using a CRISPR/Cas gene editing system.

### [Background Art]

Since 2005, investigational new drug (IND) applications have been submitted for various gene editors (e.g., zinc finger nuclease-based, TALEN-based, and CRISPR nuclease-based gene editors) (refer to the document [Mullard, Asher. "Gene-editing pipeline takes off." Nature Reviews Drug Discovery 19.6 (2020): 367-373.]). Unlike other drugs such as chemicals or antibodies, which are generally associated with reversible side effects, the effects of genome editing drugs are permanent. That is, the identification of off-target sites throughout the whole genome is particularly important for genome-editing drugs because an effect that frequently occurs at an undesirable location (i.e., an off-target effect) in a genome editing process causes important safety concerns. In order to confirm information about an off-target effect that can occur in the genome editing process, many researchers have developed various methods for predicting off-target effects in the whole genome through various approaches.

However, the currently developed off-target prediction tools (systems) have limitations. For example, cell-based methods have problems such as missing sometimes a bona-fide off-target site. On the other hand, in vitro and in silico methods have problems, such as showing too many false-positive data points.

### [Disclosure]

### [Technical Problem]

There may be off-target issues in a genome editing process using a gene editing tools (e.g., a CRISPR/Cas gene editing system). Such off-target effects cause strong side effects. An embodiment of the present application provides a method of predicting off-targets occurring in the genome editing process.

### [Technical Solution]

The present application provides a method of predicting off-targets occurring in a gene editing process using a gene editing system.

One embodiment of the present application provides a method of identifying information about off-targets that occur in a genome editing process using a CRISPR/Cas genome editing system, the method comprising:
(i) preparing a starting composition comprising a Cas protein, a guide RNA, and a cell;
(ii) obtaining a composition to be analyzed through physically disrupting the cell, wherein, through the physically disrupting the cell, a genomic DNA contacts with a Cas/gRNA complex formed by the Cas protein and the guide RNA, whereby the genomic DNA is cleaved at one or more cleavage sites; and
(iii) obtaining information about the cleavage site(s) through analyzing the composition to be analyzed.

In a specific embodiment, the physically disrupting the cell may comprise passing the cell through a filter having pores, wherein an average diameter of the pores in the filter is smaller than the size of the cells.

In a specific embodiment, a force that causes the cells to pass through the filter may be pressure.

In a specific embodiment, the average diameter of the pores in the filter may be 5 to 15 µm.

In a specific embodiment, the physically disrupting the first cell may be achieved using an extruder comprising a filter having pores.

In a specific embodiment, an average diameter of the pores in the filter comprised in the extruder may be smaller than a size of the cell.

In a specific embodiment, the average diameter of the pores in the filter may be 5 to 15 µm.

In a specific embodiment, information about the cleavage site(s) may comprise one or more of the following:
a location on genomic DNA of each of the one or more cleavage site(s);
a cleavage score for each of the one or more cleavage site(s); and
the number of the cleavage site(s).

In a specific embodiment, the method may further comprise:
(iv) identifying information about the off-target candidate(s) from information about the cleavage site(s) obtained from (iii).

In a specific embodiment, the information on the off-target candidates may comprise one or more of the following:
a location on genomic DNA of each of the one or more off-target candidates;
an off-target prediction score for each of the one or more off-target candidates; and
the number of the predicted off-target candidates.

In a specific embodiment, analyzing the composition to be analyzed may comprise: analyzing cleaved genomic DNA comprised in the composition to be analyzed through sequencing.

In a specific embodiment, analyzing the composition to be analyzed may comprise: analyzing the cleaved genomic DNA comprised in the composition to be analyzed through a PCR-based method.

In a specific embodiment, a membrane structure of the cell, comprising a cell membrane, may be disrupted through the physically disrupting the cell, whereby an environment in which the Cas/gRNA complex can be brought into a contact with the genomic DNA derived from the cell is prepared.

In a specific embodiment, a membrane structure of the cell, comprising a nuclear membrane, may be disrupted through physically disrupting the cell, whereby an environment in which the Cas/gRNA complex can be brought into a contact with the genomic DNA derived from the cell is prepared.

In a specific embodiment, the method may further comprise:
identifying a predetermined CRISPR/Cas genome editing system, wherein the identifying of the predetermined CRISPR/Cas genome editing system is performed prior to (i).

In a specific embodiment, here, the predetermined CRISPR/Cas genome editing system comprises the use of predetermined guide RNA having a predetermined guide sequence, wherein the predetermined guide sequence may be the same as the guide sequence of the guide RNA.

In a specific embodiment, here, the predetermined CRISPR/Cas genome editing system comprises the use of predetermined cells, wherein the predetermined cells may be the same as the cell.

In a specific embodiment, the composition to be analyzed may comprise cleaved genomic DNA in which the genomic DNA derived from the physically disrupted cell is cleaved by the Cas/gRNA complex.

In a specific embodiment, the concentration of the Cas protein comprised in the starting composition may be 4000 nM or more and 6000 nM or less.

In a specific embodiment, the concentration of the guide RNA comprised in the starting composition may be 4000 nM or more and 6000 nM or less.

In a specific embodiment, the concentration of the Cas/gRNA complex comprised in the starting composition may be 4000 nM or more and 6000 nM or less.

In a specific embodiment, the concentration of the cells comprised in the starting composition may be 1×10⁷ cells/mL.

In a specific embodiment, the obtaining of the composition to be analyzed may further comprise:
incubating a composition obtained through the disrupting the cell.

In a specific embodiment, the obtaining of the composition to be analyzed may further comprise:
removing RNA from the composition obtained through the disrupting the cell.

In a specific embodiment, the obtaining of the composition to be analyzed may further comprise:
purifying DNA from the composition obtained through the disrupting the cell.

One embodiment of the present application provides a method of identifying information about off-targets that occur in a genome editing process using a CRISPR/Cas genome editing system, which comprises:
(i) loading a starting composition comprising a Cas protein, a guide RNA, and a cell into a first container of an extruder;
(ii) performing an extrusion process comprising the following step using the extruder to obtain a composition to be analyzed:
   (a) applying pressure to the first container to move components of the starting composition from the first container of the extruder to a second container of the extruder,

   wherein the components of the starting composition pass through a filter having pores, located between the first container and the second container of the extruder, by applied pressure, and thus the mixture settles in the second container, and
   wherein the cell, which is the component larger in size than the diameter of a pore of the filter, is disrupted and pass through the pores of the filter, by the applied pressure,
   wherein an environment in which genomic DNA can contact with the Cas protein and the guide RNA is created by physically disrupting the cell,
   whereby the genomic DNA contacts with the Cas/gRNA complex
   thereby contacting the genomic DNA with the Cas/gRNA complex,
   whereby the genomic DNA is cleaved at one or more cleavage sites; and
(iii) analyzing the composition to be analyzed to obtain information about the cleavage sites.

In a specific embodiment, the pressure applied to the first container is generated through a process of pushing a piston designed for applying the pressure to the first container in the direction of the first container and the filter.

One embodiment of the present application provides a method of identifying information about off-targets that occur in a genome editing process using a CRISPR/Cas genome editing system, which comprises:
(i) loading a starting composition comprising a Cas protein, a guide RNA, and a cell into a first container of an extruder;
(ii) performing an extrusion process comprising the following steps using the extruder to obtain a composition to be analyzed:
   (a) applying pressure to the first container to move components of the starting composition from the first container of the extruder to a second container of the extruder, wherein the components of the starting composition pass through a filter having pores, located between the first container and the second container of the extruder, by the applied pressure, and thus the mixture settles in the second container,
   (b) applying pressure to the second container to move components of the mixture comprised in the second container from the second container to the first container,
      wherein the components of the mixture comprised in the second container pass through the filter having pores, located between the first container and the second container, by the applied pressure and thus move from the second container to the first container, thereby settling the mixture moved from the second container through the filter by the pressure in the first container, and
   (c) repeatedly performing (a) and (b) a predetermined number of times,
      wherein the predetermined number of times is counted in 0.5 increments, and 0.5 indicates a performance of a single process of (a) or (b), and
      wherein the cell, which is larger than the diameter of pores of the filter, is disrupted by passing through the pores of the filter by the applied pressure,
      wherein physically disrupting the cell, an environment where the genomic DNA can come into contact with the Cas protein and the guide RNA,
      whereby the genomic DNA contacts with a Cas/gRNA complex,
      whereby the genomic DNA is cleaved at one or more cleavage sites; and
(iii) analyzing the composition to be analyzed to obtain information about the cleavage sites.

In a specific embodiment, the pressure applied to the first container is generated by pushing a piston designed for applying the pressure to the first container in a direction of the first container and the filter, and the pressure applied to the second container is generated by pushing a piston designed for applying the pressure to the second container in a direction of the second container and the filter.

### [Advantageous Effects]

The present application provides a method of predicting off-targets that can occur in a gene (e.g., genome) editing process. The present application provides a method of identifying candidates of off-targets that can occur in a genome editing process. The present application provides a method of predicting off-targets, which can be performed more simply. The method of predicting off-targets of the present application has the advantages of an in vitro-based off-target prediction method and the advantages of cell-based off-target prediction method. The off-target prediction method of the present application shows a smaller false-positive rate. The off-target prediction method of the present application shows a smaller miss rate. That is, when the off-target prediction method of the present application is used, off-targets that can occur in the genome editing process can be easily and accurately predicted.

### [Description of Drawings]

FIG. 1 shows three categories (cell-based, in vitro, and in silico) of off-target prediction methods.
FIG. 2 is a schematic diagram illustrating a method of predicting off-targets, provided according to one embodiment of the present application.
FIG. 3 shows the comparison results for off-target candidates predicted through different off-target prediction methods (Digenome-seq, Extru-seq, GUIDE-seq, and in silico). Comparative experiments for the off-target prediction systems were performed using sgRNA targeting human *PCSK9* and sgRNA targeting human *Albumin,* respectively.
FIG. 4 shows the comparison results for off-target candidates predicted through different off-target prediction methods (Digenome-seq, Extru-seq, GUIDE-seq, and in silico). Comparative experiments for the off-target prediction systems were performed using sgRNA targeting mouse *PCSK9* and sgRNA targeting mouse *Albumin,* respectively.
FIG. 5 shows the validation rate of top off-target sites predicted by an in silico method, GUIDE-seq, Digenome-seq, and Extru-seq. The results for sgRNA targeting human *PCSK9,* sgRNA targeting human *Albumin,* sgRNA targeting mouse *PCSK9,* and sgRNA targeting mouse *Albumin* are shown.
FIG. 6 shows the comparison results for off-target candidates predicted through different off-target prediction methods (Digenome-seq, Extru-seq, GUIDE-seq, and DIG-seq). Comparative experiments for the off-target prediction systems were performed using sgRNA targeting *FANCF* and sgRNA targeting *VEGFA,* respectively.
FIG. 7 shows the comparison results for off-target candidates predicted through different off-target prediction methods (Digenome-seq, Extru-seq, GUIDE-seq, and DIG-seq). Comparative experiments for the off-target prediction systems were performed using sgRNA targeting *HBB.*
FIG. 8 shows the validation rate of top off-target sites predicted by DIG-seq, GUIDE-seq, Digenome-seq, and Extru-seq. The results for sgRNA targeting *FANCF,* sgRNA targeting *VEGFA*, and sgRNA targeting *HBB* are shown.
FIG. 9 shows the comparison results for different off-target prediction methods, analyzed through the intersection of Venn diagrams (FIGS. 3 and 4, and FIGS. 6 and 7), respectively.
FIG. 10 shows the comparison of the validation results and the results for off-targets predicted by GUIDE-seq and Extru-seq. FIG. 10A shows the result related to sgRNA targeting human *PCSK9.* FIG. 10B shows the result related to sgRNA targeting human *Albumin.*
FIG. 11 shows the comparison of validation results and the results for off-targets predicted by GUIDE-seq and Extru-seq. FIG. 11C shows the result related to sgRNA targeting mouse *PCSK9.* FIG. 11D shows the result related to sgRNA targeting mouse *Albumin.*
FIG. 12 shows the comparison of the validation results and the results for off-targets predicted by GUIDE-seq and Extru-seq. FIG. 12E shows the result related to sgRNA targeting human *FANCF.* FIG. 12F shows the result related to sgRNA targeting human *VEGFA.*
FIG. 13 shows the comparison of the validation results and the results for off-targets predicted by GUIDE-seq and Extru-seq. FIG. 12G shows the result related to sgRNA targeting human *HBB.*
FIG. 14 shows the miss rate for the GUIDE-seq and Extru-seq off-target prediction methods, calculated based on the validation results.
FIG. 15 shows the distribution of the number of mismatches for off-targets missed by GUIDE-seq, confirmed based on the validation results.
FIG. 16 shows ROC curves according to each off-target prediction method. (a) of FIG.16 illustrates the results related to sgRNA targeting human *PCSK9.* (b) of FIG.16 illustrates the results related to sgRNA targeting human *Albumin.*
FIG. 17 shows ROC curves according to each off-target prediction method. (c) of FIG. 17 illustrates the results related to sgRNA targeting mouse *PCSK9.* (d) of FIG. 17 illustrates the results related to sgRNA targeting mouse *Albumin.*
FIG. 18 shows ROC curves according to each off-target prediction method. (e) of FIG. 18 illustrates the results related to sgRNA targeting human *FANCF.* (f) of FIG. 18 illustrates the results related to sgRNA targeting human *VEGFA.* (g) of FIG. 18 illustrates the results related to sgRNA targeting human *HBB.*
FIG. 19 shows AUC calculated using the ROC curve data of FIGS. 16 to 18. AUC was calculated for each of GUIDE-seq, Digenome-seq, Extru-seq, CROP, CFD, and DIG-seq.
FIGS. 20 and 21 show the results and experimental conditions of experiments performed to find optimization conditions for the average pore size of a filter, a Cas9 RNP concentration of a mixture, and the number of cells in Extru-seq.
FIGS. 22 and 23 show cleavage rates for on- and off-target sites recognized by sgRNA targeting the human *PCSK9* site, measured by quantitative PCR (qPCR). FIGS. 22 and 23 are results obtained through Extru-seq.
FIGS. 24 to 30 show WGS data of Extru-seq analyzed using IGV to identify a cleavage pattern.
FIG. 31 shows the cleavage rate of 7 on-target sites of each target, obtained through qPCR and manual calculation based on the IGV analysis of WGS data.
FIG. 32 shows the results of dip-sequencing for non-cleaved groups, performed to confirm the degree of NHEJ generation after the extrusion process of Extru-seq.
FIG. 33 shows the results for a cleavage rate according to SCR7 treatment, performed to confirm the degree ofNHEJ generation after the extrusion process of Extru-seq.
FIGS. 34 to 41 show the sequence read results for off-target candidates predicted through GUIDE-seq. Specifically, FIGS. 34 and 35 are the sequence read results for GUIDE-seq obtained from HEK293T using sgRNA targeting *PCSK9.* FIGS. 36 and 37 are the sequence read results for GUIDE-seq obtained from HEK293 T using sgRNA targeting *Albumin.* FIGS. 38 and 39 are the sequence read results for GUIDE-seq obtained from NIH-3T3 using sgRNA targeting *PCSK9.* FIGS. 40 and 41 are the sequence read results for GUIDE-seq obtained from NIH-3T3 using sgRNA targeting *Albumin.*

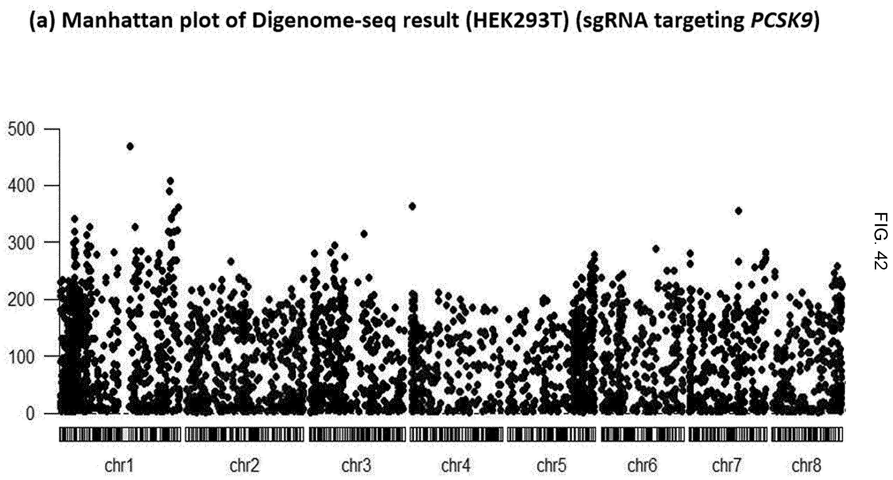

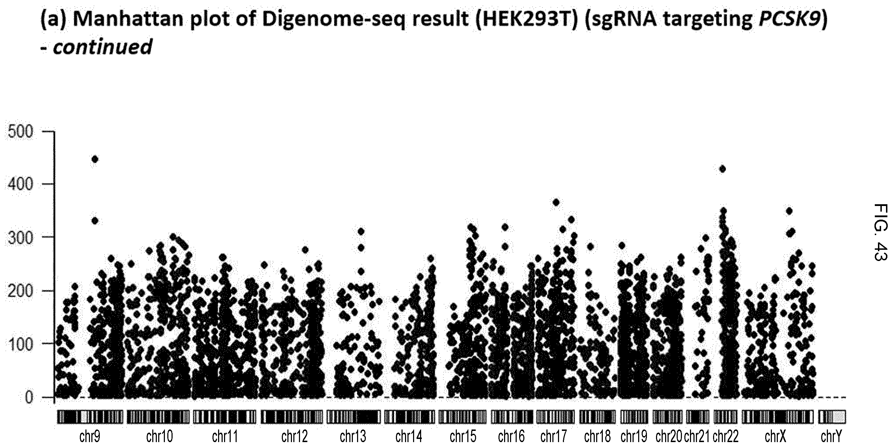

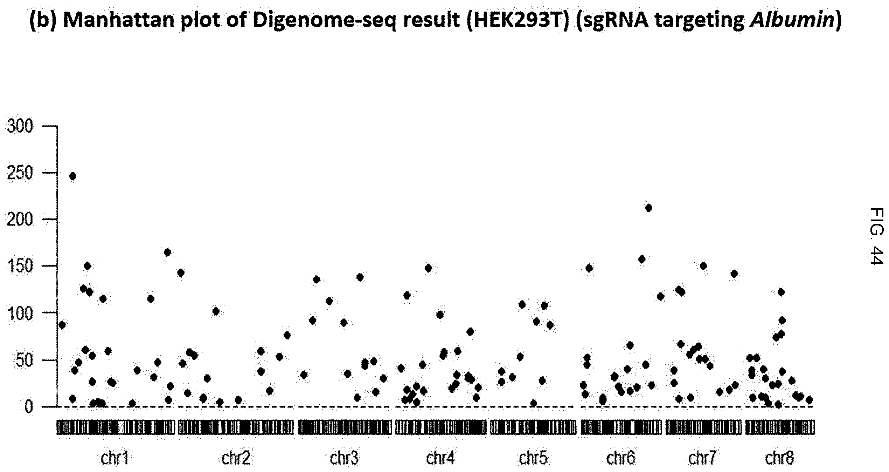

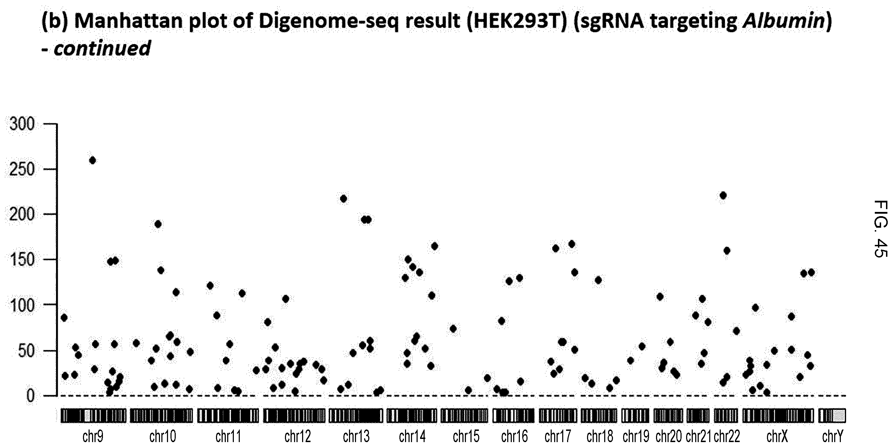

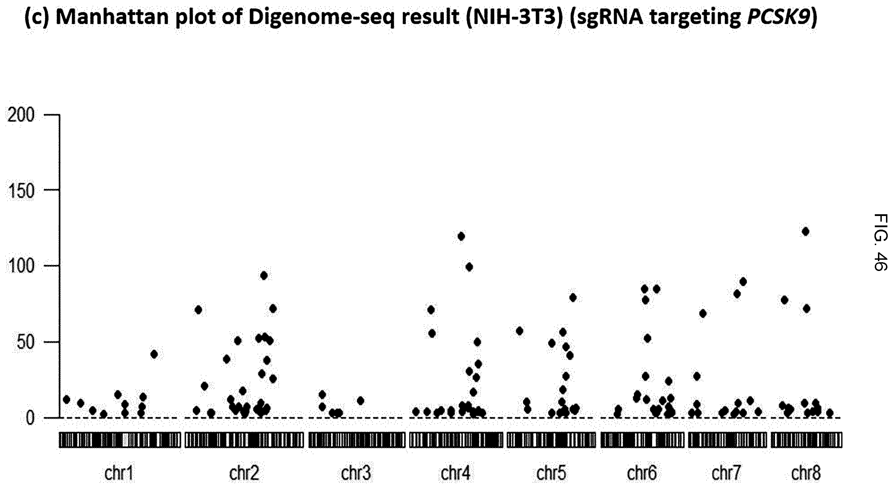

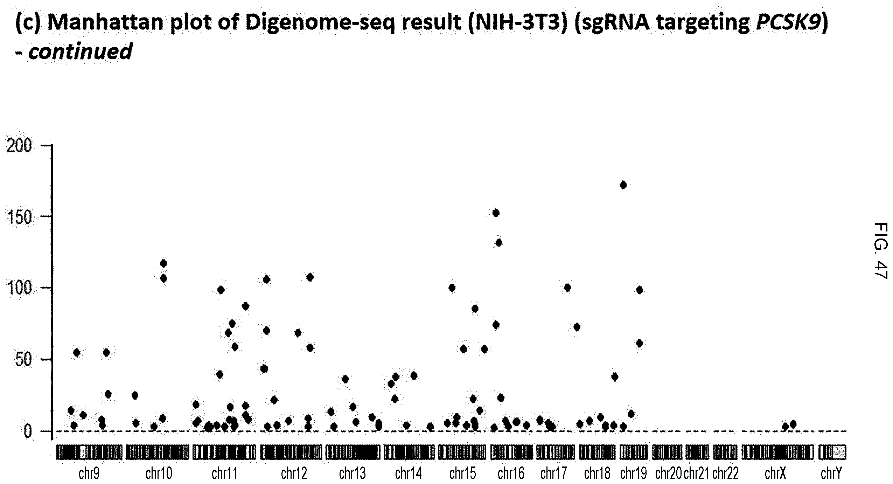

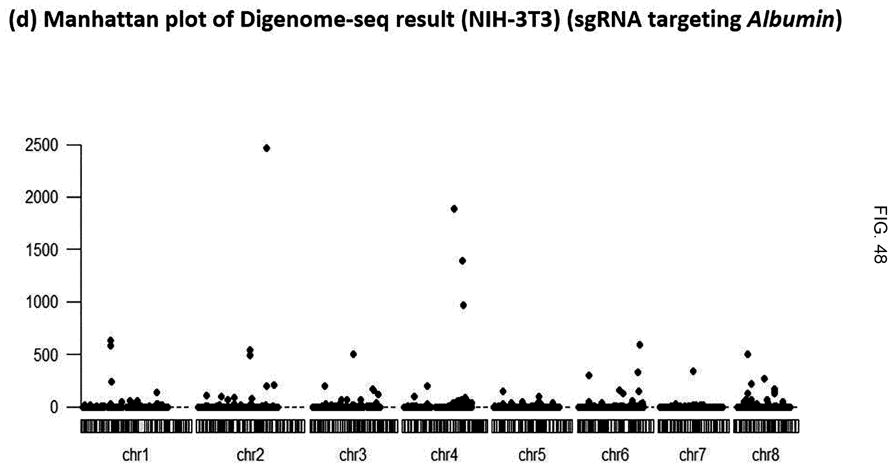

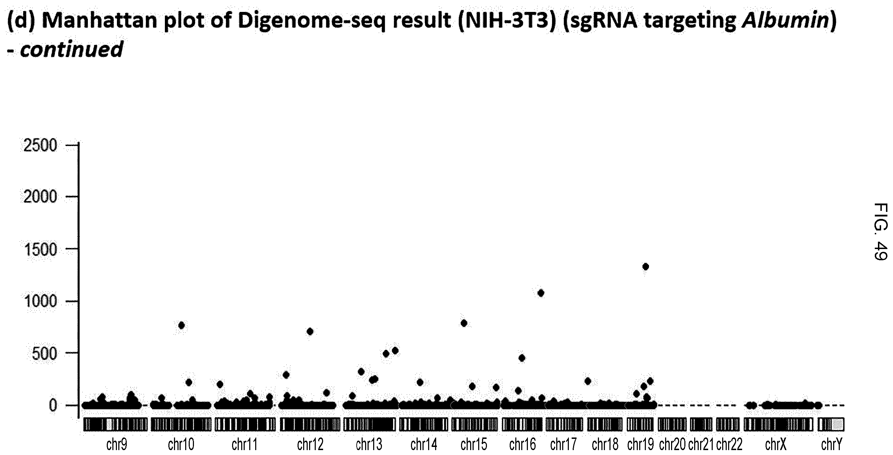

FIGS. 42 to 49 show the Manhattan plot results of off-target candidates predicted through Digenome-seq. The Y axis indicates a DNA cleavage score. Specifically, FIGS. 42 and 43 show the Manhattan plot results of Digenome-seq obtained from HEK293T using sgRNA targeting *PCSK9.* FIGS. 44 and 45 show the Manhattan plot results of Digenome-seq obtained from HEK293T using sgRNA targeting *Albumin.* FIGS. 46 and 47 show the Manhattan plot results of Digenome-seq obtained from NIH-3T3 using sgRNA targeting *PCSK9.* FIGS. 48 and 49 show the Manhattan plot results of Digenome-seq obtained from NIH-3T3 using sgRNA targeting *Albumin.*

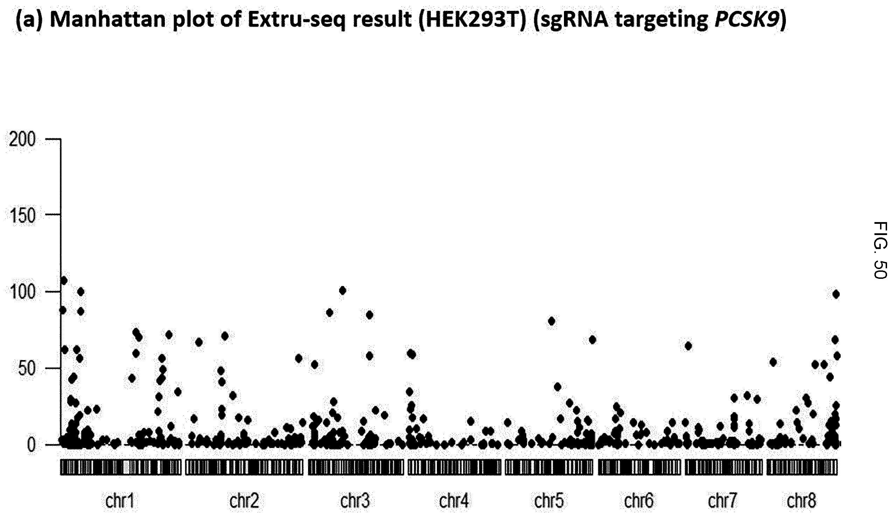

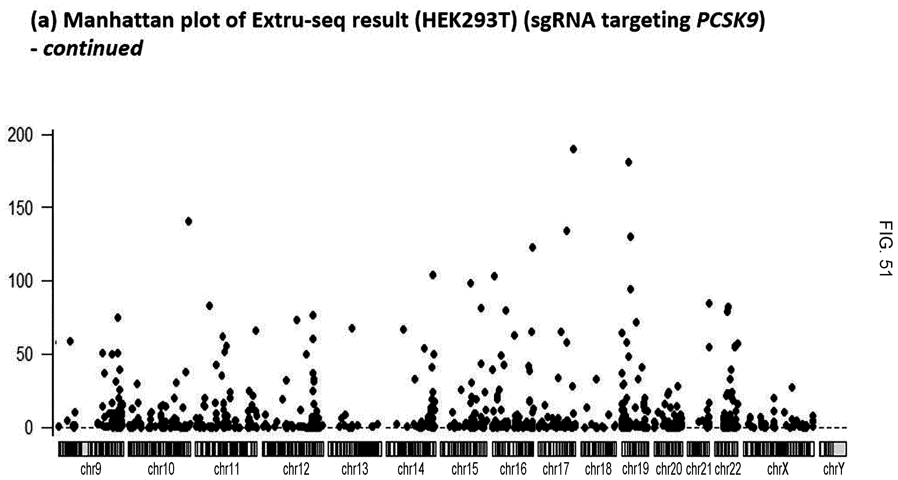

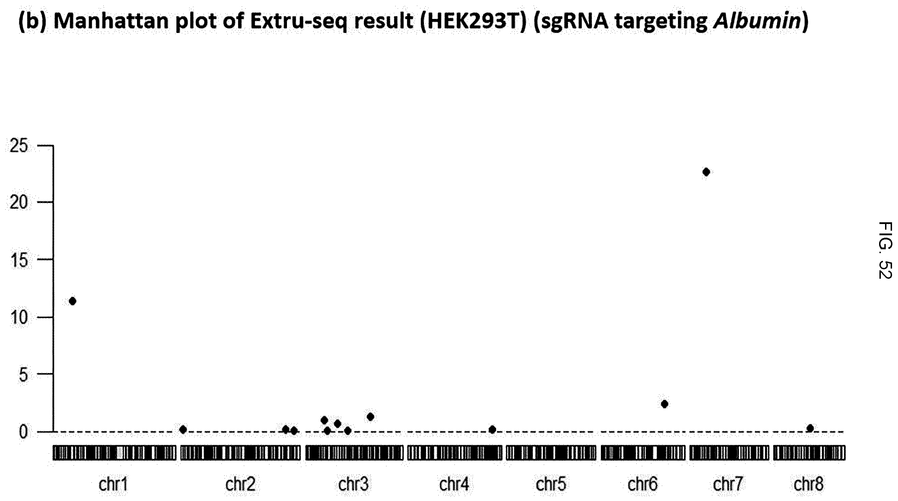

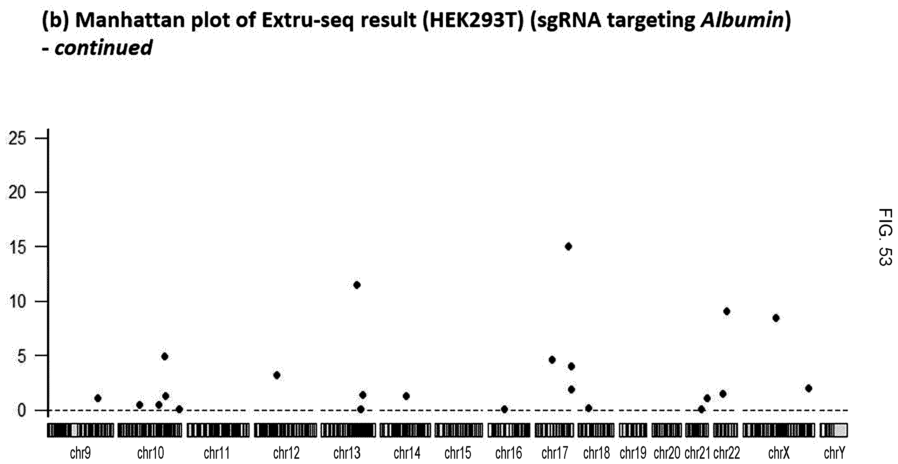

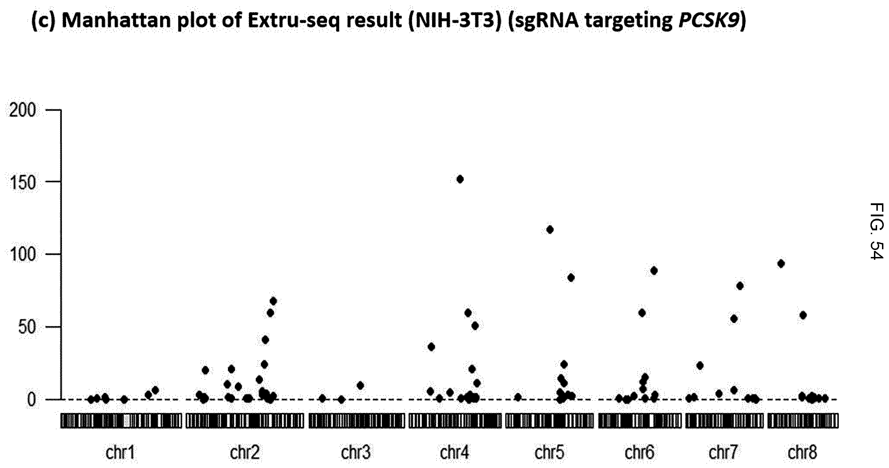

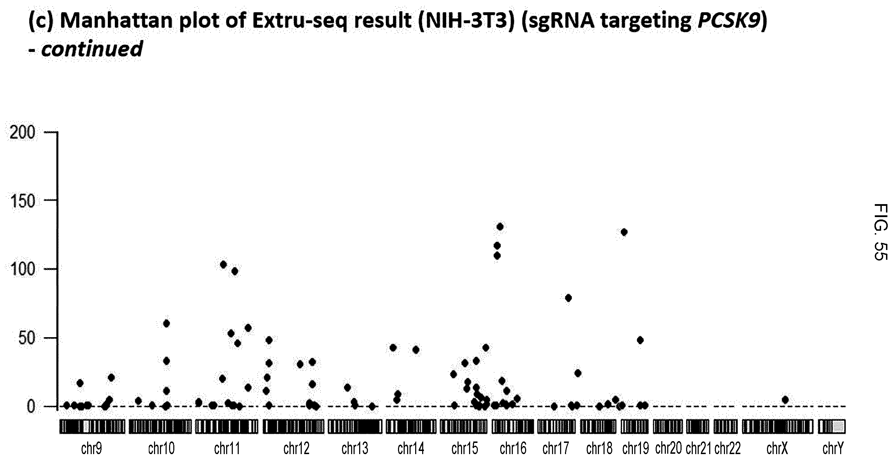

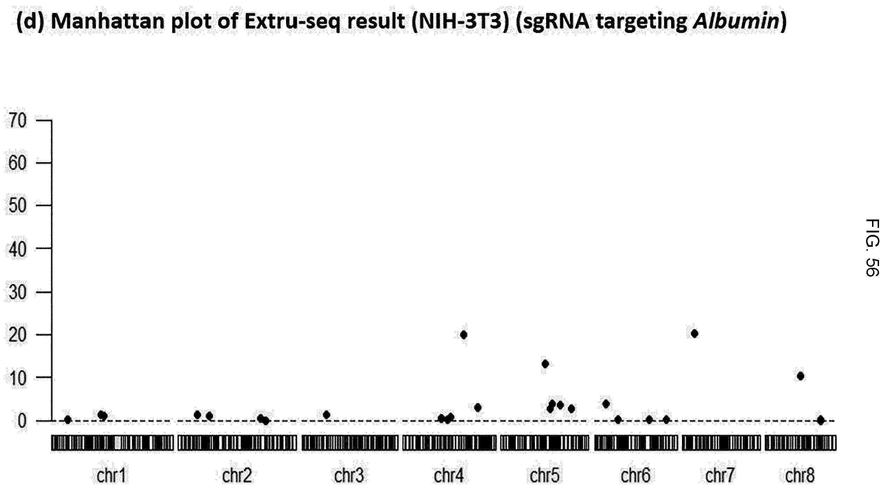

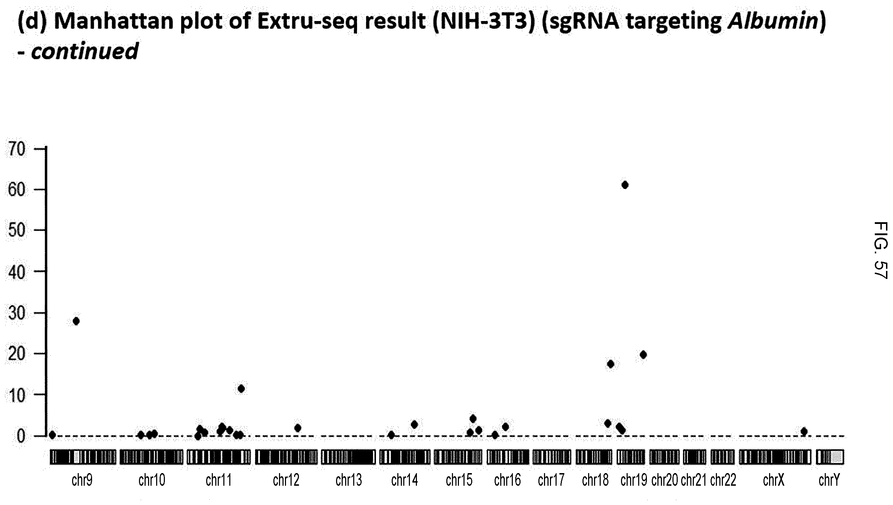

FIGS. 50 to 57 show the Manhattan plot results of off-target candidates predicted through Extru-seq. The Y axis indicates a DNA cleavage score. Specifically, FIGS. 50 and 51 show the Manhattan plot results of Extru-seq obtained from HEK293T using sgRNA targeting *PCSK9.* FIGS. 52 and 53 show the Manhattan plot results of Extru-seq obtained from HEK293T using sgRNA targeting *Albumin.* FIGS. 54 and 55 show the Manhattan plot results of Extru-seq obtained from NIH-3T3 using sgRNA targeting *PCSK9.* FIGS. 56 and 57 show the Manhattan plot results of Extru-seq obtained from NIH-3T3 using sgRNA targeting *Albumin.*
FIG. 58 shows the result related to a score (the score calculated from a sequence read count result) based on the number of mismatches between on-targets and off-targets, predicted using GUIDE-seq.
FIG. 59 shows the result related to a score (the cleavage score of Manhattan plot) based on the number of mismatches between on-targets and off-targets, predicted using Digenome-seq.
FIG. 60 shows the result related to a score (CROP score) based on the number of mismatches between on-targets and off-targets, predicted using an in silico system.
FIG. 61 shows the result related to a score (CFD score) based on the number of mismatches between on-targets and off-targets, predicted using an in silico system.
FIG. 62 shows the result related to a score (the cleavage score of Manhattan plot) based on the number of mismatches between on-targets and off-targets, predicted using Extru-seq.
FIGS. 63 and 64 show the results for the indel formation frequencies according to subretinal injection and systemic injection.
FIGS. 65 to 67 show the sequence read results for off-target candidates predicted through GUIDE-seq. Specifically, FIG. 65 shows the sequence read result for GUIDE-seq obtained from HeLa cells using sgRNA targeting *FANCF.* FIG. 66 shows the sequence read results for GUIDE-seq obtained from HeLa cells using sgRNA targeting *VEGFA.* FIG. 67 shows the sequence read results for GUIDE-seq obtained from HeLa cells using sgRNA targeting *HBB.*
FIGS. 68 to 73 show the Manhattan plot results for off-target candidates predicted through Extru-seq. The Y axis indicates a DNA cleavage score. Specifically, FIGS. 68 and 69 show the Manhattan plot results of Extru-seq obtained from HeLa cells using sgRNA targeting *FANCF.* FIGS. 70 and 71 are the Manhattan plot results of Extru-seq obtained from HeLa cells using sgRNA targeting *VEGFA.* FIGS. 72 and 73 show the Manhattan plot results of Extru-seq obtained from HeLa cells using sgRNA targeting *HBB.*
FIGS. 74 and 75 show the Venn diagram comparing the Extru-seq results obtained from MSCs and Extru-seq results obtained from HEK293T cells. FIG. 74 shows the results related to sgRNA targeting human *PCSK9.* FIG. 75 shows the results related to sgRNA targeting human *Albumin-targeting* sgRNA.
FIG. 76 shows the p-value obtained by a normalized rank sum test on each pair of off-target prediction methods for sgRNAs targeting *PCSK9* and Albumin in MSC and HEK293T cells.
FIGS. 77 to 116 show the results for off-target sites manually validated using IGV.
FIGS. 117 to 125 show the results for false-positive off-target candidates, manually excluded using IGV, from WGS data of Digenome-seq and Extru-seq.

### [Modes of the Invention]

### Definition of terms

### Nucleic acid

The term "nucleic acid" used herein is used as meaning a partial region in a molecule or a whole molecule, consisting of DNA (double-stranded or single-stranded), RNA (double-stranded or single-stranded), or a hybrid of DNA and RNA (double-stranded or single-stranded). The nucleic acid is used as meaning the assemble of nucleotides (a partial region in the molecule or the whole molecule), but is not limited. The term "nucleic acid" or "nucleic acid region" may be used to refer to a partial region in the molecule. The term "nucleic acid" or "nucleic acid area" may be used to refer to a whole molecule. The term "nucleic acid" should be interpreted appropriately according to the context, and the content of each context including the description of the term "nucleic acid" will help understand the meaning of the term "nucleic acid.". In addition, the above term comprise all meanings recognized by those of ordinary skill in the art, and can be appropriately interpreted depending on the context.

### "linked" or "linkage"

The term "linked" or "linkage" used herein refers that two or more elements present in one conceptualizable structure are linked directly or indirectly (e.g., via a different element such as a linker), and it is not intended that other additional elements cannot exist between the two or more elements. For example, the statement such as "element B linked to element A" is intended to include both of the case where one or more other elements are interposed between element A and element B (i.e., when element A is connected to element B via one or more third elements) and the case where there are no other elements interposed between element A and element B (i.e., when element A and element B are directly connected), and is not to be interpreted as limited.

### Sequence identity

The term "sequence identity" used herein is the term used in relation to the degree of similarity between two or more nucleotide sequences. For example, the term "sequence identity" is used along with terms referring to the reference sequence and ratios (e.g., percentage). For example, the term "sequence identity" may be used to explain a sequence that is similar to or substantially the same as the reference nucleotide. When described as "a sequence having 90% or more sequence identity with sequence A," the reference sequence used is sequence A. For example, the percentage of sequence identity can be calculated by aligning the reference sequence with the sequence subject to measurement of the percentage of sequence identity, and the percentage of sequence identity may be calculated by including all of a mismatch, a deletion, and an insertion in one or more nucleotides. The method of calculating and/or determining the percentage of sequence identity is not otherwise limited, and may be calculated and/or determined through a reasonable method or algorithm that can be used by those of ordinary skill in the art.

### Representation of amino acid sequence

Unless stated otherwise, using a one-letter notation or three-letter notation for amino acids, the amino acid sequence in this specification is written in the direction from the N-terminus to the C-terminus. For example, RNVP represents a peptide in which arginine, asparagine, valine, and proline are sequentially connected in the direction from the N-terminus to the C-terminus. In another example, Thr-Leu-Lys represents a peptide in which threonine, leucine, and lysine are sequentially connected in the direction from the N-terminus to the C-terminus. Amino acids that cannot be expressed with the one-letter notation are represented using different letters, and explained supplementarily.

The notation method for each amino acid is as follows: alanine (Ala, A); arginine (Arg, R); asparagine (Asn, N); aspartic acid (Asp, D); cysteine ( Cys, C); glutamic acid (Glu, E); glutamine (Gln, Q); glycine (Gly, G); histidine (His, H); isoleucine (Ile, I); leucine (Leu, L); lysine (Lys K); methionine (Met, M); phenylalanine (Phe, F); proline (Pro, P); serine (Ser, S); threonine (Thr, T); tryptophan (Trp, W); tyrosine (Tyr, Y); and valine (Val, V).

### Representation of nucleic acid sequence

The symbols A, T, C, G, and U used herein are interpreted as understood by those of ordinary skill in the art. Depending on the context and technology, it may be appropriately interpreted as a base, nucleoside, or nucleotide on DNA or RNA. For example, when referring to bases, each symbol may be interpreted as adenine (A), thymine (T), cytosine (C), guanine (G), or uracil (U), when referring to as nucleosides, each symbol may be interpreted as adenosine (A), thymidine (T), cytidine (C), guanosine (G), or uridine (U), and when referring to nucleotides in a sequence, each symbol should be interpreted as indicating a nucleotide comprising each nucleoside.

### Target sequence

The "target sequence" used herein refers to a specific sequence that is recognized by a guide RNA or gene editing tool (e.g., Cas/gRNA complex) to cleave a target gene or target nucleic acid. The target sequence may be appropriately selected according to its purpose. For example, the "target sequence" may refer to a sequence comprised in a target gene or target nucleic acid sequence, and a sequence complementary to the spacer sequence comprised in guide RNA. In another example, the "target sequence" may refer to a sequence comprised in a target gene or target nucleic acid sequence, and a sequence that is complementary to a sequence complementary to the spacer sequence comprised in guide RNA. As such, the target sequence is used to refer to a sequence complementary to the spacer sequence comprised in guide RNA and/or a sequence that is substantially the same as the spacer sequence of guide RNA, and should not be construed as limiting. In some embodiments, a target sequence may be disclosed as a sequence comprising a PAM sequence. In some embodiments, a target sequence may be disclosed as a sequence that does not comprise a PAM sequence. A target sequence should be interpreted appropriately according to the context. Generally, the spacer sequence is determined in consideration of the sequence of a target gene or target nucleic acid and the PAM sequence recognized by an editing protein of a CRISPR/Cas system. The target sequence may refer to only the sequence of a specific strand complementarily binding to the guide RNA of a CRISPR/Cas complex, only the sequence of a specific strand that does not complementarily bind to guide RNA, or a whole target double strand comprising the specific strand part, which is interpreted appropriately according to the context. The definition of the terms for the target sequence is provided to describe the strand on which the target sequence can exist, and is not intended to distinguish an on-target sequence and an off-target sequence through the term target sequence. That is, in some embodiments, an intended target sequence may be referred to as an on-target sequence, and an unintended target sequence may be referred to as an off-target sequence. With regard to an on-target and an off-target, the term "target sequence" may be interpreted appropriately according to the content of a related paragraph.

### Spacer-binding strand

The term "spacer-binding strand" used herein is used to refer to a strand comprising a sequence complementary to a partial or whole sequence of the spacer region of a guide nucleic acid in a gene editing system (e.g., CRISPR/Cas gene editing system) involved in the guide nucleic acid (e.g., guide RNA). A DNA molecule, such as a genome, generally has a double-stranded structure. In a double strand, the strand that has a sequence complementary to a partial or whole sequence of the spacer region of a guide nucleic acid and thus forms a complementary bond with a partial or whole sequence of the spacer region may be referred to as a spacer-binding strand.

### spacer-nonbinding strand

The term "spacer-nonbinding strand" used herein is used to refer to a strand, other than a 'spacer-binding strand' which is a strand comprising the sequence forming a complementary bond with a partial or whole sequence of the spacer region of a guide nucleic acid in a gene editing system (e.g., CRISPR/Cas gene editing system) involved in the guide nucleic acid (e.g., guide RNA). A DNA molecule, such as a genome, generally has a double-stranded structure, and the term "spacer-nonbinding strand" may be used to refer to a strand other than a spacer-binding strand in the double strand.

### Functional Equivalent

The term "functional equivalent" or "equivalent" refers to a second biomolecule that is functionally equivalent to a first biomolecule, but is not necessarily structurally equivalent. For example, a "Cas9 equivalent" refers to a protein that is the same as or substantially the same function as Cas9, but does not necessarily the same amino acid sequence. Throughout the present application, when referring to a specific protein, the specific protein mentioned above is intended to encompass all of its functional equivalents. For example, when described as a "X protein," the term "X protein" may be construed to encompass functional equivalents of the X protein. In this sense, the "functional equivalent" of the X protein encompasses any homologue, paralog, ortholog, fragment, naturally-occurring, engineered, mutated, or synthetic version of the protein X ensuring an equivalent function. When described as a Cas protein, the term "Cas protein" may be construed to encompass functional equivalents of the Cas protein.

### Nuclear localization signal of sequence (NLS)

The term "nuclear localization signal or sequence (NLS)" refers to an amino acid sequence that promotes the introduction of a protein into a cell nucleus. For example, the introduction of the protein may be promoted by nuclear transport. NLS is well known in the art, and will be apparent to those of ordinary skill in the art. For example, an exemplary sequence of NLS may be disclosed in PCT Application No. PCT/EP2000/011690 (Publication No. WO2021/038547), the contents of which for the exemplary NLS are incorporated as reference herein. In some embodiments, the NLS may comprise the amino acid sequence, such as PKKKRKV (SEQ ID NO: 01), KRPAATKKAGQAKKKK (SEQ ID NO: 02), PAAKRVKLD (SEQ ID NO: 03), RQRRNELKRSP (SEQ ID NO: 04), NQSSNFGPMKGGNFGGRSSGPYGGGGQYFAKPRNQGGY (SEQ ID NO: 05), RMRIZFKNKGKDTAELRRRRVEVSVELRKAKKDEQILKRRNV (SEQ ID NO: 06), VSRKRPRP (SEQ ID NO: 07), PPKKARED (SEQ ID NO: 08), POPKKKPL (SEQ ID NO: 09), SALIKKKKKMAP (SEQ ID NO: 10), DRLRR (SEQ ID NO: 11), PKQKKRK (SEQ ID NO: 12), RKLKKKIKKL (SEQ ID NO: 13), REKKKFLKRR (SEQ ID NO: 14), KRKGDEVDGVDEVAKKKSKK (SEQ ID NO: 15), RKCLQAGMNLEARKTKK (SEQ ID NO: 16), or MDSLLMNRRKFLYQFKNVRWAKGRRETYLC (SEQ ID NO: 17), but the present application is not limited thereto. The NLS may be selectively fused to a gene editing agent such as a Cas protein. The NLS fused to the protein may be used to promote the movement of the connected protein into a nucleus, which is a desired location.

### About

The term "about" used herein means a degree of approximation to a certain quantity, and refers to an amount, level, value, number, frequency, percentage, dimension, size, volume, weight, or length that is changed from reference amount, level, value, number, frequency, percentage, dimension, size, amount, weight, or length by approximately 30, 25, 20, 25, 10, 9, 8, 7, 6, 5, 4, 3, 2, or 1%.

### Directionality of disclosed sequence

The nucleotide sequence disclosed herein (e.g., a DNA sequence, an RNA sequence, or a DNA/RNA hybrid sequence) should be understood as disclosed in a 5' to 3' direction unless otherwise specified. The amino acid sequence disclosed herein should be understood as disclosed in the direction from the N-terminus to the C-terminus unless otherwise specified. For a sequence that is disclosed in a different direction from the above-mentioned direction, the directionality toward the other direction is separately specified in paragraphs related to the corresponding sequence.

### Overview of gene editing system

The present application relates to a method of predicting off-targets that can occur in a gene editing process using a gene editing system. Off-target prediction is used to encompass prediction of off-target sites. Before explaining the method of predicting off-targets provided by the present application, a gene editing system related to off-targets will be explained. The gene editing system (e.g., genome editing system) refers to a system used to achieve desired editing in a desired nucleic acid molecule (e.g., genomic DNA) through the use of a gene editing tool such as an editing protein and a guide nucleic acid. In many studies, a gene editing system is used for editing the genome of cells, and the term "gene editing system" may be used interchangeably with a genome editing system. However, the use of the gene editing system is not limited to genome editing. Further, the term "gene editing system" may be used to refer to a gene editing tool, and may be appropriately interpreted according to the related context. Examples of known gene editing systems are zinc-finger nucleases (ZFNs), transcription activator-like effector nucleases (TALENs), and CRISPR/Cas gene editing systems (refer to the document [Khan, Sikandar Hayat. "Genome-editing technologies: concept, pros, and cons of various genome-editing techniques and bioethical concerns for clinical application." Molecular Therapy-Nucleic Acids 16 (2019): 326-334.], the entire contents of which is incorporated herein by reference). Further, there are base editing and prime editing, which are developed based on a CRISPR/Cas gene editing system.

One of the characteristics of the off-target prediction method provided by the present application is rupturing the membrane structure of cells through a physical method (e.g., using an extruder) to bring a component of a gene editing system (e.g., an editing protein and/or a guide nucleic acid) into a contact with a genome. Accordingly, the off-target prediction method of the present application may be applied to all of the above-described gene editing systems.

As an example of gene editing system, a CRISPR/Cas gene editing system which is actively being studied to achieve the purpose of genome editing will be described in detail below.

### CRISPR/Cas gene editing system

### Overview of CRISPR/Cas gene editing system

A CRISPR/Cas gene editing system is used as an umbrella term referring to a gene editing system involving an editing protein containing a Cas protein and guide nucleic acid (e.g., guide RNA), which are used to induce desired editing of a gene (e.g., genomic DNA) at a desired location. A CRISPR/Cas gene system may be used as other terms that can be understood by those of ordinary skill in the art. For example, the CRISPR/Cas gene system may be referred to as CRISPR/Cas, a CRISPR/Cas system, a CRISRP system, or a Cas-based genome editing system, but the present application is not limited thereto. Further, the CRISPR/Cas gene editing system is used to encompass all development technologies such as base editing (refer to the document [Gaudelli, Nicole M., et al. "Programmable base editing of A· T to G· C in genomic DNA without DNA cleavage." Nature 551.7681 (2017): 464-471.]) and prime editing(refer to the document [Anzalone, Andrew V., et al. "Search-and-replace genome editing without double-strand breaks or donor DNA." Nature 576.7785 (2019): 149-157.]), which are developed based on a CRISPR/Cas gene. The results of gene editing (e.g., genome editing) may comprise cleavage, indel, insertion, deletion, substitution, base editing (e.g., can be achieved by base editing), and writing (e.g., can be achieved by prime editing), but the present application is not limited thereto. Hereinafter, a CRISPR/Cas gene editing system will be described in detail comprising the origin of the CRISPR/Cas gene editing system.

### CRISPR

The "CRISPR" section is a section for helping those of ordinary skill in the art understand, and the term used in this section is not intended to limit the terms disclosed herein.

CRISPR is a family of DNA sequences (i.e., CRISPR clusters) in bacteria and archaea, representing snippets of prior infections caused by viruses that have invaded the prokaryotes. The snippets of DNA are used by the prokaryotic cells to detect and disrupt DNA from subsequent attacks by similar viruses, and effectively constitute a prokaryotic immune defense system, along with an array of CRISPR-associated proteins (Cas proteins) and CRISPR-associated RNA. CRISPR clusters are transcribed and processed into CRISPR RNA (crRNA). Subsequently, Cas9/crRNA/tracrRNA endonucleolytically cleaves linear or cyclic dsDNA target complementary to RNA. Specifically, the target strand not complementary to crRNA is first cleaved endonucleolytically, and then trimmed 3'-5' exonucleolytically. DNA-binding and cleavage typically require a protein and two RNAs. However, single guide RNA (sgRNA, or simply gRNA) was developed, and single stranded RNA is engineered thing such that incorporating aspects of both the crRNA and tracrRNA into single RNA species. For example, the document [Jinek, Martin, et al. "A programmable dual-RNA-guided DNA endonuclease in adaptive bacterial immunity." science 337.6096 (2012): 816-821.] is referred, the entire contents of which is incorporated herein as reference. Cas9 recognizes a short motif (the PAM or protospacer adjacent motif) in the CRISPR repeat sequences to help distinguish self vs. non-self. CRISPR biology, as well as a Cas9 nuclease sequence and structure, are well known to those of ordinary skill in the art (refer to, e.g., the document [Ferretti, Joseph J., et al. "Complete genome sequence of an M1 strain of Streptococcus pyogenes." Proceedings of the National Academy of Sciences 98.8 (2001): 4658-4663.; Deltcheva, Elitza, et al. "CRISPR RNA maturation by trans-encoded small RNA and host factor RNase III." Nature 471.7340 (2011): 602-607.; and Jinek, Martin, et al. "A programmable dual-RNA-guided DNA endonuclease in adaptive bacterial immunity." science 337.6096 (2012): 816-821.], the entire contents of each of which are incorporated herein as reference). Cas9 orthologs have been described in various species, comprising Streptococcus pyogenes (S. pyogenes) and Streptococcus thermophilus (S. thermophilus), but the present application is not limited thereto. Additional suitable Cas9 nucleases and their sequences will be apparent to those of ordinary skill in the art based on the disclosure, and such Cas9 nucleases and their sequences comprise Cas9 sequences from the organisms and loci disclosed in the document [Chylinski, Krzysztof, Anais Le Rhun, and Emmanuelle Charpentier. "The tracrRNA and Cas9 families of type II CRISPR-Cas immunity systems." RNA biology 10.5 (2013): 726-737.], the entire contents of which are incorporated herein by reference.

### CRISPR/Cas gene editing system

A CRISPR/Cas gene editing system developed from the above-described CRISPR is a technology of editing a gene (e.g., the genome of cells) at a desired location using a Cas protein derived from the CRISPR system of cells, and a guide nucleic acid guiding the Cas protein to a target region. For example, a Cas protein forms a Cas/gRNA complex, along with guide RNA (gRNA). The Cas/gRNA complex guides the guide RNA comprised in the complex to a desired location. The Cas protein comprised in the Cas/gRNA complex induces a double strand break (DSB) or nick to a desired location. The CRISPR/Cas gene editing system can edit not only the cell's genome, but also a DNA molecule that is not located on the genome. Since the discovery of CRISPR, regarding the CRISPR/Cas genome editing system, as described above, single guide RNA to which tracrRNA and crRNA are connected was developed (refer to the document [Jinek, Martin, et al. "A programmable dual-RNA-guided DNA endonuclease in adaptive bacterial immunity." science 337.6096 (2012): 816-821.], the entire contents of which is incorporated herein by reference), and various kinds and/or types of Cas proteins (Cas equivalents) such as cas9, cas12a(cpf1), cas12b(c2c1), cas12e(casX), cas12k(c2c5), cas14, cas14a, cas13a(c2c2), cas13b(c2c6) cas9 nickase, dead cas were developed. Further, based on the CRISPR/Cas gene editing system, base editing that can achieve the purpose of base changing and prime editing that can achieve writing desired edits were developed. As described above, the CRISPR/Cas gene editing system may be used to encompass the traditional CRISPR/Cas gene editing system and technologies aiming at gene editing developed based on it. To understand the CRISPR/Cas gene editing system, the document WO2018/231018 (PCT No.) may be referred, the entire content of which is incorporated herein by reference. To help those of ordinary skill in the art understand, an editing protein (e.g., a Cas protein) that can be used in the CRISPR/Cas gene editing system will be further described below.

### CRISPR/Cas gene editing system 1- Editing protein (Editor protein)

### Overview of the editing protein and editing protein in CRISPR/Cas gene editing system

An editing protein (editor protein) may be used to refer to a protein that generates a DSB or nick in a desired region to achieve gene editing, or helps induce editing. Generally, a protein with nuclease activity, which cleaves a nucleic acid may be referred to as an editing protein. In the CRISPR/Cas gene editing system, the editing protein may be used interchangeably with a Cas protein. The representative example of Cas protein is Cas9. The term "Cas protein" used herein is used as a general term for gene editing proteins which can generate a DSB or nick in a target region or inert Cas proteins, used in the CRISPR/Cas gene editing system. Examples of Cas proteins comprise Cas9, Cas9 variant, Cas9 nickase (nCas9), dead Cas9, Cpf1 (type-V CRISPR-Cas system), C2c1 (type V CRISPR-Cas system), C2c2 (type VI CRISPR-Cas system), and C2c3 (type V CRISPR-Cas system), but the present application is not limited thereto. Additional examples of Cas proteins are disclosed in the document [Abudayyeh, Omar O., et al. "C2c2 is a single-component programmable RNA-guided RNA-targeting CRISPR effector." Science 353.6299 (2016): aaf5573.], the entire contents of which is incorporated herein by reference. In one embodiment, Cas proteins may be Cas9 or Cpf1 derived from various microorganisms such as Streptococcus pyogenes, Streptococcus thermophilus, Streptococcus sp., Staphylococcus aureus, Campylobacter jejuni, Nocardiopsis dassonvillei, Streptomyces pristinaespiralis, Streptomyces viridochromogenes, Streptomyces viridochromogenes, Streptosporangium roseum, Streptosporangium roseum, AlicyclobacHlus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, and Acaryochloris marina. Hereinafter, Cas9 proteins used in the CRISPR/Cas9 gene editing system will be illustrated.

### Cas9 protein

In the CRISPR/Cas9 gene editing system, proteins with nuclease activity cleaving a nucleic acid are referred to as Cas9 proteins. The Cas9 proteins correspond to Class 2 (Type II) in the classification of CRISPR/Cas systems, and comprise Cas9 proteins derived from Streptococcus pyogenes, Streptococcus thermophilus, Streptococcus sp., Streptomyces pristinaespiralis, Streptomyces viridochromogenes, Streptomyces viridochromogenes, Streptosporangium roseum, and Streptosporangium roseum. Additional Cas9 proteins and their sequences are disclosed in the document [Chylinski, Krzysztof, Anais Le Rhun, and Emmanuelle Charpentier. "The tracrRNA and Cas9 families of type II CRISPR-Cas immunity systems." RNA biology 10.5 (2013): 726-737.], the entire contents of which are incorporated herein by reference. For example, the DNA cleavage domain of Cas9 is known to comprise two subdomains, such as a NHN nuclease subdomain and a RucC1 subdomain. The NHN subdomain cleaves a strand complementary to gRNA, and the RuvC1 subdomain cleaves a non-complementary strand. The inactivation of these subdomains may silence the nuclease activity of Cas9. For example, both of the mutants D10A and H840A completely inactivate the nuclease activity of S. pyogenes Cas9 (refer to the document [Jinek, Martin, et al. "A programmable dual-RNA-guided DNA endonuclease in adaptive bacterial immunity." science 337.6096 (2012): 816-821.]). For example, the mutant H840A provides Cas9 nickase.

### CRISPR/Cas gene editing system 2 - guide nucleic acid

### Overview of guide nucleic acid

In the CRISPR/Cas gene editing system, a Cas protein is associated with a guide nucleic acid (e.g., guide RNA) to form a Cas/guide nucleic acid complex (e.g., Cas/gRNA complex). The Cas/gRNA complex may be referred to as a ribonucleoprotein (RNP). The Cas/gRNA complex generates a double-strand break (DSB) or nick in a target region comprising a sequence corresponding (e.g., complementary) to the spacer sequence of guide RNA (gRNA), and the DSB or nick is induced by a Cas protein. The location where the DSB or nick is generated may be near the PAM sequence on the genome. A protospacer-adjacent motif (PAM) on the genome and the spacer sequence of gRNA are involved in Cas/gRNA targeting. The Cas protein (e.g., Cas9) guided to a target region by PAM and the spacer sequence of the gRNA generates DSB in the target region.

In the CRISPR/Cas gene editing system, RNA having a function to guide the Cas protein to a target region in order to recognize a specific sequence comprised in the target DNA molecule is referred to as guide RNA.

When the composition of the guide RNA is divided functionally, it may be largely divided into 1) a scaffold sequence part, and 2) a guide domain comprising a guide sequence. The scaffold sequence part is a part that interacts with a Cas protein (e.g., Cas9 protein), and a part that can be bound with the Cas protein to form a complex. Generally, the scaffold sequence part comprises tracrRNA, and crRNA-repeat sequence part, and the scaffold sequence is determined depending on which the Cas9 protein is used. The guide sequence is a part that can bind complementary to a nucleotide sequence part of a certain length in a target nucleic acid (e.g., a target DNA molecule or a cell genome). The guide sequence may be artificially modified, and is determined by the target nucleotide sequence of interest related to desired gene editing.

In some embodiments, the guide RNA may be explained as comprising crRNA and tracrRNA. The crRNA may comprise a spacer and a repeat sequence. The repeat sequence part of crRNA may interact (e.g., binding complementary to) with the part of tracrRNA. As described above, single guide RNA (sgRNA) to which crRNA and tracrRNA are linked may be provided.

In one embodiment, the guide RNA may be provided as two strands. In one embodiment, the guide RNA may be provided as one strand. sgRNA in which tracrRNA and crRNA are linked (refer to the document [Jinek, Martin, et al. "A programmable dual-RNA-guided DNA endonuclease in adaptive bacterial immunity." science 337.6096 (2012): 816-821.], the entire contents of which are incorporated herein by reference) was developed. In a specific embodiment, the guide RNA may be sgRNA.

### Guide domain and guide sequence of guide RNA

A guide nucleic acid (e.g., guide RNA) may include a guide domain comprising a guide sequence. The guide sequence is used interchangeably with a spacer sequence. The guide sequence is determined by a target nucleotide sequence of interest, as a part that can be artificially designed. In some embodiments, the guide sequence may be designed to target a sequence adjacent to a PAM sequence located on a DNA molecule for editing. As described above, localization of the Cas/gRNA complex to a target location was induced. The structure of the guide nucleic acid may vary depending on a CRISPR type. For example, the guide RNA used in the CRISPR/Cas9 gene editing system may have the structure of 5'-[guide domain]-[scaffold]-3'.

In one embodiment, the guide sequence may have a length of 5 to 40 nt. In one embodiment, the guide sequence comprised in the guide domain of the guide RNA may have a length of 10 to 30 nt. In one embodiment, the guide sequence may have a length of 15 to 25 nt. In one embodiment, the guide sequence may have a length of 18 to 22 nt. In one embodiment, the guide sequence may have a length of 20 nt. In one embodiment, the target sequence (encompassing both a target sequence present in a spacer-binding strand and a target sequence present in a spacer non-binding strand), which is a sequence in the genome forming a complementary bond with the guide sequence, may have a length of 5 to 40 nt or 5 to 40 bp. In one embodiment, the target sequence, which is a sequence in the genome forming a complementary bond with the guide sequence, may have a length of 10 to 30 nt or 10 to 30 bp. In one embodiment, the target sequence may have a length of 15 to 25 nt or 15 to 25 bp. In one embodiment, the target sequence may have a length of 18 to 22 nt or 18 to 22 bp. In one embodiment, the target sequence may have a length of 20 nt or 20 bp.

### CRISPR/Cas gene editing system 3 - Protospacer adjacent motif (PAM)

Two conditions are needed to cleave a target gene or target nucleic acid by the CRISPR/Cas9 gene editing system.

First, there must be a base sequence (nucleotide sequence) of a certain length, which can be recognized by a Cas9 protein, within the target gene or target nucleic acid. Here, the base sequence (nucleotide sequence) of a certain length, recognized by the Cas9 protein, is called a protospacer-adjacent motif (PAM) sequence. The PAM sequence is a unique sequence determined by the Cas9 protein. Second, there must be a sequence that can bind complementary to a spacer sequence comprised in guide RNA around the PAM sequence of a certain length. Here, the PAM sequence may be used to encompass a sequence present on a spacer non-binding strand and a sequence present on a spacer-binding strand.

As described above, in the CRISPR/Cas gene editing system, a Cas/gRNA complex is guided to the target region by the PAM sequence on the target DNA molecule (e.g., genome of cell) and the guide sequence of gRNA . In the target DNA molecule, the PAM sequence may be located in the non-binding strand of the guide sequence, which is not a strand to which the guide sequence of the guide RNA binds. The PAM sequence may be independently determined according to the type of Cas protein used. In one embodiment, the PAM sequence may be any one selected from the following (shown in a 5' to 3' direction): NGG (SEQ ID NO: 18); NNNNRYAC (SEQ ID NO: 19); NNAGAAW (SEQ ID NO: 20); NNNNGATT (SEQ ID NO: 21); NNGRR(T) (SEQ ID NO: 22); TTN (SEQ ID NO: 23); and NNNVRYAC (SEQ ID NO: 24). Each N may be independently A, T, C, or G. Each R may be independently A or G. Each Y may be independently C or T. Each W may be independently A or T. For example, when spCas9 is used as the Cas protein, the PAM sequence may be NGG (SEQ ID NO: 18). For example, when Streptococcus thermophilus Cas9 (StCas9) is used as the Cas protein, the PAM sequence may be NNAGAAW (SEQ ID NO: 20). For example, when Neisseria meningitides Cas9 (NmCas9) is used, the PAM sequence may be NNNNGATT (SEQ ID NO: 21). For example, when Campylobacter jejuni Cas9 (CjCas9) is used, the PAM may be NNNVRYAC (SEQ ID NO: 24). In one embodiment, the PAM sequence may be connected to the 3'-end of the target sequence present on the spacer non-binding strand (here, the target sequence present on the spacer non-binding strand refers to a sequence not binding to the guide RNA). In one embodiment, the PAM sequence may be located at the 3'-end of the target sequence located on the spacer non-binding strand. The target sequence present on the spacer non-binding strand refers to a sequence that does not bind to the guide sequence of the guide RNA. The target sequence present on the spacer non-binding strand is complementary to the target sequence present on the spacer-binding strand.

The location where a DSB or nick is generated may be near the PAM sequence on the genome. In one embodiment, the location where a DSB or nick is generated may be -0 to -20 or +0 to +20 based on the 5' - or 3'-end of the PAM sequence present on the non-spacer-binding strand. In one embodiment, the location where a DSB or nick is generated may be -1 to -5 or +1 to +5 of the PAM sequence on the non-spacer-binding strand. For example, in the CRISPR/Cas gene editing system using spCas9, the spCas9 cleaves between the third nucleotide and the fourth nucleotide located upstream of the PAM sequence.

### Genome editing process using CRISPR/Cas gene editing system

To help those of ordinary skill in the art understand, the genome editing process using the CRISPR/Cas gene editing system is briefly described using the following examples.

For example, an environment where a DNA molecule for editing can come into contact with the Cas/gRNA complex may be provided. The DNA molecule may be a DNA molecule that is purpose for editing. For the purpose of genome editing in cells, a Cas protein or a nucleic acid encoding the same and a guide RNA or a nucleic acid encoding the same are introduced into cells, thereby creating an environment where the Cas protein and the guide RNA can come into a contact with the genomic DNA of the cells. In the environment where the Cas protein and the guide RNA can come into a contact with the genomic DNA of the cells, the Cas protein and the guide RNA may form a Cas/gRNA complex. Of course, the Cas protein and the gRNA can form the Cas/gRNA complex in an appropriate environment, even when the genomic DNA of the cells is not present. The guide sequence of the gRNA comprised in the Cas/gRNA complex and the PAM sequence on the genome are involved to allow the Cas/gRNA complex to guide to a target region where a predesigned target sequence is present. The Cas/gRNA complex induced to the target region generates DSBs (e.g., in the case of Cas9) in the target region. Afterward, as (cleaved) DNA where DSBs are generated is repaired in a DNA repairing process, gene editing is made at a target region or target location. There are two major pathways for repairing DSBs generated on DNA, such as homology-directed repair (HDR) and non-homologous end joining (NHEJ). Among these, HDR, which is a naturally-occurring DNA repair system, may be used to correct the genome in various organisms comprising a human. HDR-mediated repairing may be mainly used to insert a desired sequence into a target region or target site, or to induce a specific point mutation, but the present application is not limited thereto. HDR-mediated repairing may be performed by a DNA repair system HDR, and a HDR template (e.g., a donor template that can be provided from the outside of cells). NHEJ refers to a process of repairing DSBs on DNA, and in contrast to HDR, it connects cleaved ends without a HDR template. That is, the repair process does not require an HDR template. NHEJ may be a DNA repair mechanism that can mainly be selected to induce indels. Insertion/deletion may refer to mutations in which some nucleotides are deleted, any nucleotide is inserted, and/or the insertion and the deletion are mixed in the nucleotide sequence of a nucleic acid before gene editing. The occurrence of some indels in the target gene may inactivate the corresponding gene. The DNA repair mechanisms, HDR and NHEJ, are disclosed in detail in the document [Sander, Jeffry D., and J. Keith Joung. "CRISPR-Cas systems for editing, regulating and targeting genomes." Nature biotechnology 32.4 (2014): 347-355.], the entire contents of which are incorporated herein by reference.

To date, the gene editing system comprising the CRISPR/Cas gene editing system has been explained in detail. The present application relates to a method of predicting off-targets that may occur in a gene editing (e.g., genome editing) process using the gene editing system. Hereinafter, off-targets that may occur in the gene editing system will be described in detail.

### Off-target

In the field of gene editing (e.g., genome editing), an off-target refers to genetic modification that occurs at an unintended location. The genetic modification induced by an off-target may be non-specific. The developed genome editing tools comprise a CRISPR/Cas gene editing system, a transcription activator-like effector nuclease (TALEN), a meganuclease, and a zinc finger nuclease. These genome editing tools or gene editing systems are designed to perform editing in a target region through a special mechanism capable of binding to a predetermined sequence (e.g., a sequence in the target region). For example, in the CRISPR/Cas gene editing system, guide RNA (gRNA) induces a movement of the Cas/gRNA complex to an intended target location. A PAM sequence in the genome may also be involved in the movement to the target location. However, the Cas/gRNA complex still has possibility of binding to a sequence at an unintended location rather than a sequence in the target region. In this way, when the Cas/gRNA complex binds to a sequence at an unintended location, and DSBs are generated at the unintended location, unintended genetic modification occurs. The off-target effect leads to unintended genetic modifications, such as unintended point mutations, deletions, insertions, inversions, and translocations. It is known that the binding of a genome editing tool to an undesired region results from partially but sufficient matching to the target sequence of the sequence in the undesired region. Although not bound by the theory, the document [Lin, Yanni, et al. "CRISPR/Cas9 systems have off-target activity with insertions or deletions between target DNA and guide RNA sequences." Nucleic acids research 42.11 (2014): 7473-7485.], the entire contents of which are incorporated herein by reference, shows that the mechanisms of off-target binding can be grouped into base mismatch tolerance and bulge mismatch. For example, an off-target site may comprise 0, 1, 2, 3, 4, 5, 6, 7, 8, 9, or 10 or more mismatches with a guide RNA sequence, but the present application is not limited thereto.

The off-target problem implies the possibility of causing disruptions in important coding region leading to serious problems such as cancer. Further, in the research area, the off-target problem may also cause confusion in variables in biological research, and implies the possibility of causing unreproducible results (refer to the document [Eid, Ayman, and Magdy M. Mahfouz. "Genome editing: the road of CRISPR/Cas9 from bench to clinic." Experimental & Molecular Medicine 48.10 (2016): e265-e265.], the entire contents of which are incorporated herein by reference).

The problem of off-targets still remains not only in the CRISPR/Cas gene editing system, but also in base editing and prime editing, which had been developed based on the CRISPR/Cas gene editing system. In the specification, the off-targets may be used as a concept countered to on-target, and may be used to refer to genetic modification at an unintended location.

As described above, off-targets cause serious side effects (e.g., side effects hard to identify, and/or irreversible side effects) in various aspects. Accordingly, the identification of off-targets that can occur upon the use of a gene editing system (e.g., genome editing system) is very important in the development and research of treatments. A lot of costs and time are required to identify bona-fide off-targets occurring in a designed gene editing system (e.g., CRISPR/Cas9 gene editing system and specific guide RNA). For this reason, various methods that can identify off-target candidates, that is, predict off-targets, have been studied and developed. However, methods of predicting off-targets possible to occur in a gene editing process (e.g., a genome editing process using a gene editing system), which had been developed up to the filing date of the present application, still have various problems. Hereinafter, the off-target prediction systems that had been studied and developed conventionally and problems thereof will be disclosed.

### Known off target prediction system and limitations thereof

### Known off-target prediction system

As described above, to predict off-targets that can occur in genome editing using a gene editing system (e.g., CRISPR/Cas gene editing system), various methods have been developed. The conventional off-target prediction or off-target candidate identification methods may be classified into three categories such as a cell-based off-target prediction system, an in vitro off-target prediction system, and an in silico off-target prediction system according to a mechanism of action (MOA) of the method. Examples of prediction systems comprised in the categories are as follows:
- Cell-based system: GUIDE-seq (refer to the document [Tsai, S. Q., Zheng, Z., Nguyen, N. T., Liebers, M., Topkar, V. V., Thapar, V., ... & Joung, J. K. (2015). GUIDE-seq enables genome-wide profiling of off-target cleavage by CRISPR-Cas nucleases. Nature biotechnology, 33(2), 187-197.]), GUIDE-tag, DISCOVER-seq, BLISS, BLESS, integrase-defective lentiviral vector-mediated DNA break capture, HTGTS, ONE-seq, CReVIS-Seq, ITR-seq, and TAG-seq.
- In vitro system: Digenome-seq (refer to the document [Kim, Daesik, et al. "Digenome-seq: genome-wide profiling of CRISPR-Cas9 off-target effects in human cells." Nature methods 12.3 (2015): 237-243.]), DIG-seq (refer to the document [Kim, Daesik, and Jin-Soo Kim. "DIG-seq: a genome-wide CRISPR off-target profiling method using chromatin DNA." Genome research 28.12 (2018): 1894-1900.]), SITE-seq, CIRCLE-seq, and CHANGE-seq.
- In silico system: Cas-OFFinder (refer to the document [Bae, Sangsu, Jeongbin Park, and Jin-Soo Kim. "Cas-OFFinder: a fast and versatile algorithm that searches for potential an off-target effect of Cas9 RNA-guided endonucleases." Bioinformatics 30.10 (2014): 1473-1475.]), CHOPCHOP, and CRISPOR etc..

As described above, to predict off-targets that occur in the genome editing process using a CRISPR/Cas genome editing system (or tool), various methods were developed, but the prediction methods or tools that have been developed so fat still have limitations. Limitations for each category are shown below.

### Problems of cell-based classes

For example, a cell-based method shows the problem of sometimes missing a bona-fide off-target location, and the problem of reducing the efficiency of a prediction method in a type of clinically more highly relevant cells (e.g., clinically used cells) (refer to the document [Wienert, Beeke, et al. "Unbiased detection of CRISPR an off-target effect in vivo using DISCOVER-Seq." Science 364.6437 (2019): 286-289; and Shapiro, Jenny, et al. "Increasing CRISPR efficiency and measuring its specificity in HSPCs using a clinically relevant system." Molecular Therapy-Methods & Clinical Development 17 (2020): 1097-1107.]).

Further, the inventors of the present application additionally confirmed problems that can occur in a cell-based prediction method (e.g., GUIDE-seq) through their experiments. For example, the inventors of the present application confirmed a higher miss rate of the cell-based prediction method, and confirmed through the experiments that an off-target prediction results may vary depending on a cell type (refer to the experimental examples of the present application).

### Problems of in vitro and in silico classes

The in vitro off-target prediction method and in silico off-target prediction method have problems that provide too many false-positive data points, and problems that do not reflect an intracellular environment that may be cell-specific, such as a chromatin structure and an epigenetic modification (refer to the document [Kim, Daesik, and Jin-Soo Kim. "DIG-seq: a genome-wide CRISPR off-target profiling method using chromatin DNA." Genome research 28.12 (2018): 1894-1900.]).

Further, the inventors of the present application additionally identified problems that can arise by the in vitro-based prediction method (e.g., Digenome-seq, Dig-seq) and the in silico-based prediction method in their experiments. For example, the inventors of the present application confirmed a higher false-positive rate of the in vitro-based prediction method (refer to experimental examples of the present application).

### Offtarget prediction system used in IND process of gene therapy

To determine or confirm off-targets of a CRISPR/Cas genome editing system because each method has its own advantages and disadvantages, various prediction methods were used in combination. In a recent study conducted at Intellia, GUIDE-seq, SITE-seq, and Cas-OFFinder were used to identify potential off-target locations (NTLA-2001, refer to the literature [Gillmore, Julian D., et al. "CRISPR-Cas9 in vivo gene editing for transthyretin amyloidosis." New England Journal of Medicine 385.6 (2021): 493-502.]). EDITAS Medicine used the off-target prediction tools of GUIDE-seq, Digenome-seq, and Cas-OFFinder for the candidate therapeutic agent EDIT-101 (refer to the document [Maeder ML, Stefanidakis M, Wilson CJ, Baral R, Barrera LA, Bounoutas GS, Bumcrot D, Chao H, Ciulla DM, DaSilva JA et al: Development of a gene-editing approach to restore vision loss in Leber congenital amaurosis type 10. Nat Med 2019, 25(2):229-233.]).

However, the use of a combination of various prediction methods requires a lot of labors and costs, and is difficult to use in a general group. In addition, the use of various off-target prediction methods may not guarantee that more off-target candidates can be detected. For example, regarding NTLA-2001, seven valid off-target locations identified by SITE-seq used herein comprise all of valid off-target locations found by GUIDE-seq (three valid off-targets were found) and valid off-target locations found by Cas-OFFinder (three valid off-targets were found). In this case, the output of one in vitro method is the same as the output of the combination of three methods. For NTLA-2001, SITE-seq identified 475 off-target candidates, of which 468 candidates were identified as false-positive. In a clinical trial, it is difficult to verify all 475 off-target candidates for cells of each patient or organ.

Therefore, for various reasons comprising the above-described aspects, it is necessary to develop an intensive off-target prediction method. The inventors of the present application developed an effective off-target prediction method, which is more accurate and has a lower false-positive rate. The inventors of the present application confirmed excellent performance of a new off-target prediction method of the present application by comparing performance of a conventional method and the newly-developed off-target prediction method of the present application using various and multiple test methods in detail. Specifically, the inventors of the present application confirmed that the off-target prediction method of the present application has better performance than an in vitro off-target prediction method by comparing the in vitro off-target prediction method and the off-target prediction method of the present application. Further, the inventors of the present application confirmed that the off-target prediction method of the present application shows better performance than a cell-based off-target prediction method by comparing the cell-based off-target prediction method and the off-target prediction method of the present application. Furthermore, the inventors of the present application confirmed overall that the off-target prediction method of the present application shows better performance than other off-target prediction methods through various and multiple tests (refer to the experimental examples of the present application).

The off-target prediction method provided by the present application has all advantages of the cell-based prediction method and the in vitro prediction method. Regarding the advantages of the cell-based prediction method, the off-target prediction method provided by the present application may provide an environment where the Cas/gRNA complex can come into a contact with genomic DNA in which the chromatin structure and epigenetic modification are maintained. Regarding the advantages of the in vitro-based prediction method, the off-target prediction method provided by the present application may inhibit a DNA repair mechanism to accumulate a cut rate, thereby preventing missing a bona-fide off-target. Hereinafter, the off-target prediction system (method or tool) provided by the present application will be described in detail.

### Overview of off-target prediction system provided by the present application

### Overview of off-target prediction system provided by the present application

The present application provides a method of predicting off-targets that can occur in a gene editing process. The present application provides a method of predicting off-targets possible to occur in a genome editing process. In one embodiment, the gene editing process may be performed using a CRISPR/Cas gene editing system. In one embodiment, the genome editing process may be performed using a CRISPR/Cas gene editing system. In one embodiment of the present application, a method of predicting off-targets that can occur in a genome editing process using a CRISPR/Cas gene editing system is provided. Off-targets encompass the concept of an off-target site. For example, an off-target site or location may be described as an off-target. In the present application, the prediction of an off-target may mean identification of an off-target candidate. The prediction of an off-target may mean the identification of the location of an off-target candidate. The prediction of an off-target may mean the identification of an off-target candidate. The descriptions of "off-target," "off-target prediction," and "off-target candidate" used herein should not be construed as limiting.

The novel off-target prediction system provided by the present application physically rupture cells, thereby contacting genomic DNA of cells and a gene editing protein (e.g., a Cas protein such as a Cas9 protein), and gRNA, or a Cas/gRNA complex (e.g., Cas9/gRNA complex). By physically rupturing a cell, the cell membrane and/or nuclear membrane of the cell are ruptured, and an environment where the Cas/gRNA complex can contact with the genome may be created.

In one embodiment, physical disruption of cells may be performed using a filter with pores having an appropriate size so as to have a less effect on the genomic DNA of the cells. For example, to provide an environment where the genomic DNA of the cell can come into contact with the Cas/gRNA complex, an extruder comprising a filter having pores of an appropriate size may be used. Pressure may be applied to the region containing the cells, the cells may pass through the pores with a smaller diameter than the size of the cell due to the pressure, and the cells may be destroyed while passing through the pores. When the pore size is appropriately adjusted, in the cell disruption process, the genomic DNA in the cell or the structure of the genomic DNA (e.g., the structure according to epigenetic features such as a chromatin structure) may not be destroyed or modified.

According to the characteristics of the off-target system of the present application, an environment where the Cas protein and gRNA (or the Cas/gRNA complex) may approach or contact with the genomic DNA of the cell may be created, and at the same time, more intact genomic DNA may be maintained until cleaved by the Cas/gRNA complex.

The off-target prediction system (or method) provided by the present application may be referred to as Extru-seq.

The off-target prediction system provided by the present application may be largely divided into two processes, comprising: acquisition of a composition to be analyzed (subject composition), and analysis of the composition to be analyzed.

Here, the acquisition of a composition to be analyzed may be performed by a process that comprises providing an environment where genomic DNA can contact with a Cas/gRNA complex.

The analysis of the composition to be analyzed may be performed by a process that comprises analyzing DNA comprised in the composition to be analyzed (e.g., cleaved DNA or uncleaved DNA).

Hereinafter, a method of providing the environment where the genomic DNA can contact with the Cas/gRNA complex will be disclosed.

### Method of providing environment where genomic DNA can contact with Cas/gRNA complex

As described above, the method of predicting off-targets that can occur in a gene editing process, provided by the present application, may provide the environment where the genomic DNA can contact with the Cas/gRNA complex.

To provide the environment where the genomic DNA which was present in a cell (e.g., the nucleus) can contact with the Cas/gRNA complex, a process of disrupting cells by a physical method (e.g., using a physical force) may be performed. Through cell disruption, the membrane structure of a cell such as the cell membrane and/or the cell nuclear membrane may be disrupted, or a space such that the Cas protein and gRNA, or the Cas/gRNA complex can approach the genomic DNA may be created in the membrane structure. For example, the nuclear membrane of the cell may be disrupted, exposing the genomic DNA to the Cas protein and gRNA. In another example, the cell membrane may be disrupted, the environment where the Cas protein can contact with gRNA may be provided, and the Cas protein and gRNA (or the Cas/gRNA complex) may approach the genomic DNA through the nuclear membrane of the cell. In some embodiments, the Cas protein may be fused or linked with NLS (i.e., an NLS-linked Cas protein can be provided), and the NLS fused or liked to the Cas protein may help the Cas protein (or the Cas/gRNA complex) pass through the nuclear membrane of the cell. In one embodiment, cell disruption may result in the disruption of the membrane structure of the cell. In one embodiment, due to the cell disruption, the cell membrane of the cell may be destroyed. In one embodiment, due to the cell disruption, the nuclear membrane of the cell may be destroyed.

In one embodiment, the above-described cell disruption may be achieved through passing the cells through a porous structure with pores. The porous structure may be a filter or membrane with pores. For example, destroying cells may be performed by passing the cells through a filter with pores. For example, cell disruption may be performed by passing cells through pores with a smaller diameter than the size of the cells. For example, cell disruption may be performed by passing the cells through pores with a smaller diameter than the size of the cell nucleus. Here, a driving force that allows the cells to pass through the filter may be pressure. Specifically, pressure is applied to the portion where the cells are located, and the applied pressure forces the cells to pass through pores smaller than the cell size. Here, the cells may be disrupted while passing through smaller pores than the cell size. In one embodiment, disrupting the cell may be performed by an extrusion process.

In one embodiment, the cell disruption, and the contact of the genomic DNA and the Cas/gRNA complex may be achieved by the use of an extruder. The extruder and the use of the extruder is described in detail later in the present disclosure.

Although the above descriptions were exemplified through the Cas protein and the gRNA or Cas/gRNA complex, they may be fully applied to editing proteins used in gene editing systems, other than the CRISPR/Cas gene editing system.

When the genomic DNA contacts with the Cas/gRNA complex, cleavage occurs at on-target and off-target locations on the genomic DNA. Here, the cleavage may be achieved by DSBs or nicks induced by the Cas/gRNA complex (particularly, the Cas protein). Since the DNA repair mechanism of the cells may be disrupted in the cell disruption process, cleaved DNA may not be repaired. By analyzing the cleaved DNA or uncleaved DNA, the locations where off-targets are likely to occur may be analyzed. That is, off-targets (or off-target sites) may be predicted or off-target candidates (or candidate off-target sites) may be identified.

### Advantages of method of predicting off-targets disclosed in the present application

The inventors of the present application tested a method of predicting off-targets provided by the present application in detail. By comparing the method of predicting off-targets of the present application with another off-target prediction method, it was confirmed that the off-target prediction method of the present application shows better performance than another off-target prediction method (refer to the experimental examples of the present application). The off-target prediction method of the present application shows multiple advantages that another off-target prediction method does not have. The off-target prediction method of the present application may have both the advantages of a cell-based off-target prediction method and the advantages of an in vitro off-target prediction method.

The off-target prediction method of the present application may have a lower false-positive rate than an in vitro off-target prediction method. For example, since the in vitro off-target prediction method is difficult to reflect the epigenetic features such as a chromatin structure and epigenetic modification, the false-positive rate of the off-target prediction result is high. Detecting a site not a bona-fide off-target as an off-target candidate may be expressed by the false-positive result. The high false-positive rate may be associated with a low validation rate. Further, in the conventional in vitro off-target prediction method, the epigenetic features that may be cell-specific are difficult to be reflected in the in vitro off-target prediction results. However, in the off-target prediction method of the present application, since cells are physically disrupted without using a chemical additive to maintain the structure of genomic DNA, cell-specific environments may be partially maintained, resulting in a lower false-positive rate. The off-target prediction method of the present application may show a high validation rate. Further, the epigenetic features may be reflected in the off-target prediction results.

The off-target prediction method of the present application may exhibit a lower miss rate than a cell-based off-target prediction method. A miss rate may mean missing a bona-fide off-target. For example, a false-negative result such as the case in which a bona-fide off-target site is not detected as an off-target candidate, raises a miss rate. For example, in the processes of the cell-based prediction method, a DNA repair mechanism may be inevitably involved, and a repaired cleaved site repaired by the DNA repair mechanism disturbs the identification of a bona-fide off-target or off-target candidate. However, in the off-target prediction method of the present application, the DNA repair mechanism may not be involved because cells may be disrupted.

The off-target prediction method of the present application may be applied without limiting a cell type. For example, the cell-based prediction method may be difficult to perform on some cells, and may be difficult to apply on cells used in actual clinical practice. When off-target prediction is performed based on cells that are not related to cells used in actual practice, inaccurate results may be obtained. For example, because the epigenetic features vary by cell type, the use of a different cell type may lead to inaccurate results. However, the off-target prediction method has no or less limitations to a cell type.

Further, the off-target prediction method of the present application may be performed more conveniently and at a lower cost than the cell-based prediction method or the in vitro off-target prediction method.

The above-described advantages may arise because the off-target prediction method of the present application comprises physically disrupting cells. The inventors of the present application tested and validated the off-target prediction method of the present application through a large number and many kinds of experiments. The advantages of the off-target prediction method of the present application are confirmed through the experimental examples of the present application.

In one embodiment, the validation rate calculated based on top 10 off-target candidates of the off-target candidates identified by the off-target prediction method of the present application may be 20%, 25%, 30%, 35%, 40%, 45%, 50%, 55%, 60%, 65%, 70%, 75%, 80%, 85%, 90%, 95%, or 100% or more, but the present application is not limited thereto. In one embodiment, the validation rate calculated based on top 10 off-target candidates of the off-target candidates identified by the off-target prediction method of the present application may be in the range formed by two values selected from the aforesaid values, but the present application is not limited thereto. The validation rate may be affected by the type of gene editing tool used in the off-target prediction system, and the type of cells.

In one embodiment, the miss rate of the off-target prediction method of the present application may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40% or less, but the present invention is not limited thereto. In one embodiment, the miss rate of the off-target prediction method of the present application may be in the range formed by two values selected from the aforesaid values, but the present application is not limited thereto. The miss rate may be affected by the type of gene editing tool used in the off-target prediction system, and the type of cells.

In one embodiment, a receiver operating characteristic (ROC) curve for the off-target prediction method of the present application may be plotted. In one embodiment, an area under receiver operating characteristic curve (AUC) for the off-target prediction method of the present application may be calculated. The ROC curve and the AUC are potent tools that can exhibit the diagnostic ability of a binary classifier system. The ROC curve may generally be plotted by corresponding a true positive rate (TPR) and a false-positive rate (FPR), or by corresponding sensitivity and specificity. For example, the ROC curve may be plotted with TPR on the y-axis and FPR on the x-axis. For example, the ROC curve may be plotted with sensitivity on the y-axis and specificity on the x-axis. As AUC is close to 1 (i.e., as the area of AUC is larger), this means that the system is a high-performance model. In one embodiment, AUC for the off-target prediction method of the present application may be calculated, wherein AUC may be approximately 0.4, 0.42, 0.44, 0.46, 0.48, 0.5, 0.52, 0.54, 0.56, 0.58, 0.6, 0.62, 0.64, 0.66, 0.68, 0.7, 0.72, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99 or more, or 1, but the present application is not limited thereto. In one embodiment, the AUC calculated for the off-target prediction method of the present application may be in the range of two values selected from the aforesaid values, but the present application is not limited thereto. The AUC may be affected by the gene editing tool used in the off-target prediction system, and the type of cells.

As described above, the off-target prediction method of the present application comprises the acquisition of a composition to be analyzed and the analysis of the composition to be analyzed. It will be clear to those of ordinary skill in the art that additional processes may be further comprised, in addition to the above-described two processes. Hereinafter, the obtaining of a composition to be analyzed will be described in detail.

### Acquisition of composition to be analyzed

The off-target prediction method of the present application may comprise the process of obtaining the composition to be analyzed. Here, the composition to be analyzed may refer to a composition that comprises cleaved DNA and/or uncleaved DNA. Through the method that comprises acquiring a composition to be analyzed, and analyzing the composition to be analyzed (e.g., analyzing cleaved DNA comprised in the composition to be analyzed), the off-target prediction method of the present application may be achieved. To obtain the composition to be analyzed comprising the cleaved DNA, genomic DNA must be in contact with a Cas protein and gRNA (or a Cas/gRNA complex). The Cas/gRNA complex cleaves an on-target and/or off-target site by contacting with the genomic DNA. To contact the Cas/gRNA complex with the genomic DNA, cells may be disrupted. That is, the environment where the genomic DNA can be in contact with the Cas/gRNA complex may be provided by cell disruption (e.g., the disruption of the membrane structure of cells).

One of key processes for obtaining a composition to be analyzed is physically disrupting cells. In one embodiment, the cells may be disrupted by a physical method. In one embodiment, the cells may be physically disrupted. In one embodiment, the cells may be disrupted by a physical force. In one embodiment, the composition to be analyzed may be obtained through disrupting the cells from a starting composition. In a specific embodiment, the starting composition may comprise the cells. In a specific embodiment, the starting composition may comprise the cells and a gene editing tool (e.g., Cas protein and gRNA).

Hereinafter, one of the key features of the off-target prediction method of the present application, the physical disruption of cells, is disclosed in detail below.

### Physical disruption of cells

### Overview of physical disruption of cells

As described above, the off-target prediction method of one embodiment of the present application may comprise physically disrupting cells. Here, it is important to note that a chemical additive that can cause a damage to genomic DNA or a genomic DNA structure (e.g., a chromatin structure, etc.) is not used for the main purpose of disrupting cells. In one embodiment, the physical disruption of cells may be performed by forcing the cells to pass through a porous structure with pores smaller than the cell size. In one embodiment, the porous structure may be a filter with pores. Hereinafter, the process of physically disrupting cells performed by forcing the cells to pass through a porous structure with pores smaller than the cell size will be described in detail.

### Disruption of cells using filter with pores and pressure

In one embodiment, the physical disruption of cells may be performed by a method that comprises passing cells through a filter with pores smaller than the cell size. Here, the force to pass the cells through the filter may be pressure.

For example, pressure may be applied to a first container where a first composition comprising cells is located. Here, the applied pressure makes the cells disrupted while passing them through the filter having pores smaller than the cell size. That is, a driving force that makes the cells pass through the filter may be pressure. When the pressure is applied to the first container or the first composition, the mixed solution and the contained components (e.g., the cells) in the first container may be released from the first container through the pores of the filter. In this process, the cells may be disrupted by the pores smaller than the cell size.

In one embodiment, the cell membrane may be disrupted by the pores smaller than the cell size. In one embodiment, the cell membrane and the nuclear membrane may be disrupted by the pores smaller than the cell size.

When described for a plurality of cells, some or all of the cells may be disrupted in the process of passing through pores smaller than the cell size. Among the plurality of cells, some cells may not be disrupted by passing through pores larger than the cell size, or may not be disrupted even by passing through pores smaller than the cell size.

In one embodiment, the first composition located in the first container may further comprise a tool used in a gene editing system (e.g., gene editing tool). For example, the first composition located in the first container may further include a Cas protein and gRNA. When pressure is applied to the first container, as the cells are disrupted, components of the disrupted cells may move to a second container, and the Cas protein and gRNA may move to the second container through the pores. In the second container, which is located on the opposite side of the first container based on the filter, the Cas protein and gRNA (or the Cas/gRNA complex) and the genomic DNA of the cells may come into contact. Here, the contact between the Cas/gRNA and the genomic DNA may be performed in newly-produced vesicles (e.g., liposomes), and/or in an extravesicular environment not the inside the vesicles, but is not otherwise limited.

In another embodiment, the first composition located in the first container may comprise the cells, and the second container located on the opposite side of the first container based on the filter may comprise a tool used in the gene editing system. For example, when pressure is applied to the first container, as the cells are disrupted, components of the disrupted cells move to the second container where the gene editing tools are present. Accordingly, in the second container, the gene editing tools (e.g., the Cas protein and gRNA) may come into a contact with the DNA molecule derived from the cells.

In the second container, when the contact between the gene editing tools and the DNA molecule is achieved, an environment where the DNA molecule (e.g., genomic DNA) can be cleaved by the gene editing tools is created.

### Filter with pores

As described above, by allowing the cells to pass through a porous structure with pores, the disruption of the cells may be achieved.

In one embodiment, a porous structure may be a filter with pores. In one embodiment, the filter may be a filter formed of any one of polycarbonate, cellulose, a mixed cellulose ester membrane, glass, polyethersulfone, nylon, polytetrafluoroethylene (PTFE), and PVDF, or a combination thereof, but the present application is not limited thereto, and may be a filter that is conventionally used in bio and/or chemical field(s). In a specific embodiment, the filter may be a polycarbonate membrane filter, but is not otherwise limited.

In one embodiment, the filter may comprise pores with a smaller diameter than the cell size. In one embodiment, the filter may comprise pores with a smaller diameter than the average size of the cells. In one embodiment, the filter may comprise pores with a smaller diameter than the nuclear size of a cell. In one embodiment, the filter may comprise pores with a smaller size than the average size of the cell nucleus. The filter may be suitably designed depending on the type of cells. In one embodiment, the average diameter of the pores comprised in the filter may be smaller than the cell size (e.g., cell diameter). In one embodiment, the average diameter of the pores comprised in the filter may be smaller than the size of the cell nucleus (e.g., the diameter of the cell nucleus).

In one embodiment, the filter may comprise pore with any one diameter of approximately 0.1 µm, 0.2 µm, 0.3 µm, 0.4 µm, 0.5 µm, 0.6 µm, 0.7 µm, 0.8 µm, 0.9 µm, 1 µm, 1.5 µm, 2 µm, 2.5 µm, 3 µm, 3.5 µm, 4 µm, 4.54 µm, 5 µm, 5.5 µm, 6 µm, 6.5 µm, 7 µm, 7.5 µm, 8 µm, 8.5 µm, 9 µm, 9.5 µm, 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, 41 µm, 42 µm, 43 µm, 44 µm, 45 µm, 46 µm, 47 µm, 48 µm, 49 µm, 50 µm, 51 µm, 52 µm, 53 µm, 54 µm, 55 µm, 56 µm, 57 µm, 58 µm, 59 µm, 60 µm, 61 µm, 62 µm, 63 µm, 64 µm, 65 µm, 66 µm, 67 µm, 68 µm, 69 µm, 70 µm, 71 µm, 72 µm, 73 µm, 74 µm, 75 µm, 76 µm, 77 µm, 78 µm, 79 µm, 80 µm, 81 µm, 82 µm, 83 µm, 84 µm, 85 µm, 86 µm, 87 µm, 88 µm, 89 µm, 90 µm, 91 µm, 92 µm, 93 µm, 94 µm, 95 µm, 96 µm, 97 µm, 98 µm, 99 µm, and 100 µm. In one embodiment, the filter may comprise pore with a diameter of any one value or less among the aforesaid values.

In one embodiment, the average diameter of the pores comprised in the filter may be any one selected from approximately 0.1 µm, 0.2 µm, 0.3 µm, 0.4 µm, 0.5 µm, 0.6 µm, 0.7 µm, 0.8 µm, 0.9 µm, 1 µm, 1.5 µm, 2 µm, 2.5 µm, 3 µm, 3.5 µm, 4 µm, 4.54 µm, 5 µm, 5.5 µm, 6 µm, 6.5 µm, 7 µm, 7.5 µm, 8 µm, 8.5 µm, 9 µm, 9.5 µm, 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, 41 µm, 42 µm, 43 µm, 44 µm, 45 µm, 46 µm, 47 µm, 48 µm, 49 µm, 50 µm, 51 µm, 52 µm, 53 µm, 54 µm, 55 µm, 56 µm, 57 µm, 58 µm, 59 µm, 60 µm, 61 µm, 62 µm, 63 µm, 64 µm, 65 µm, 66 µm, 67 µm, 68 µm, 69 µm, 70 µm, 71 µm, 72 µm, 73 µm, 74 µm, 75 µm, 76 µm, 77 µm, 78 µm, 79 µm, 80 µm, 81 µm, 82 µm, 83 µm, 84 µm, 85 µm, 86 µm, 87 µm, 88 µm, 89 µm, 90 µm, 91 µm, 92 µm, 93 µm, 94 µm, 95 µm, 96 µm, 97 µm, 98 µm, 99 µm, and 100 µm, or any one value or less among the aforesaid values. In a specific embodiment, the average diameter of the pores comprised in the filter may be approximately 5 µm, 6 µm, 7 µm, 8 µm, 9 µm, 10 µm, 11 µm, 12 µm, 13 µm, 14 µm, or 15 µm. In a specific embodiment, the average diameter of the pores comprised in the filter may be 5 µm, 6 µm, 7 µm, 8 µm, 9 µm, 10 µm, 11 µm, 12 µm, 13 µm, 14 µm, or 15 µm or less.

In one embodiment, the average diameter of the pores in the filter may be in the range of two values selected from 0.1 µm, 0.2 µm, 0.3 µm, 0.4 µm, 0.5 µm, 0.6 µm, 0.7 µm, 0.8 µm, 0.9 µm, 1 µm, 1.5 µm, 2 µm, 2.5 µm, 3 µm, 3.5 µm, 4 µm, 4.54 µm, 5 µm, 5.5 µm, 6 µm, 6.5 µm, 7 µm, 7.5 µm, 8 µm, 8.5 µm, 9 µm, 9.5 µm, 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, 41 µm, 42 µm, 43 µm, 44 µm, 45 µm, 46 µm, 47 µm, 48 µm, 49 µm, 50 µm, 51 µm, 52 µm, 53 µm, 54 µm, 55 µm, 56 µm, 57 µm, 58 µm, 59 µm, 60 µm, 61 µm, 62 µm, 63 µm, 64 µm, 65 µm, 66 µm, 67 µm, 68 µm, 69 µm, 70 µm, 71 µm, 72 µm, 73 µm, 74 µm, 75 µm, 76 µm, 77 µm, 78 µm, 79 µm, 80 µm, 81 µm, 82 µm, 83 µm, 84 µm, 85 µm, 86 µm, 87 µm, 88 µm, 89 µm, 90 µm, 91 µm, 92 µm, 93 µm, 94 µm, 95 µm, 96 µm, 97 µm, 98 µm, 99 µm, and 100 µm.

In some embodiments, to achieve the physical disruption of the cells, one or more filters may be used. For example, one filter may be used, and for example, a first filter comprising pores with a first average diameter may be used. In another example, plurality of filters may be used. For example, a first filter comprising pores of the first average diameter may be firstly used, and a second filter comprising pores of a second average diameter (i.e., a filter having a different pore profile from the pore profile of the first filter) may be secondly used. The type and number of filters that can be used to achieve the physical disruption of cells are not otherwise limited.

### Pressure

As described above, in order for cells to pass through pores smaller than the cell size, a force must be applied to a region where the cells are located.

A force that makes cells pass through the pores (e.g., pass through the pores while disrupting) may be pressure. That is, when pressure is applied to the region where cells are located (e.g., the container containing the cells), the cells may pass through the pores with a smaller size than the cell size while disrupting. Here, the pressure may be applied by various methods, but is not otherwise limited.

In one embodiment, the applying the pressure may be performed by a person. For example, the pressure may be applied by pushing a piston designed to apply the pressure to a container containing cells. In one embodiment, applying the pressure may be achieved by a machine or device. For example, the pressure may be applied by pushing a piston designed to apply the pressure to a container containing cells through the machine. In another example, the application of the pressure may be achieved by centrifugation. In one embodiment, the pressure may be a centrifugal force or osmotic pressure. The size or intensity of the applied force (e.g., pressure) is not otherwise limited. For example, the minimum force or pressure to make cells pass through pores and/or a filter, or a higher force or pressure may be applied.

In one embodiment, cell disruption may be achieved using an extruder. Hereinafter, the method of disrupting cells using an extruder will be described in detail.

### Method of disrupting cells using extruder

### Overview of extruder

In the specification, an extruder may refer to a tool or machine that comprises a container and porous structure having pores and is designed to allow a composition loaded in the container to pass through the porous structure with pores through a force applied to the container. An example of extruder used in bio and chemical fields is Avanti^{®} Mini-Extruder. The Mini-Extruder has two containers comprised in two syringes, respectively, and comprises a porous filter (or membrane) located between the two containers. The inventors of the present application found that the structure of such an extruder is suitable for physically disrupting cells, and used the extruder to disrupt the cells. The above description of the Avanti^{®} Mini-Extruder is an example to aid the understanding of those of ordinary skill in the art, and the extruder disclosed herein is not limited to the above Mini-Extruder. The extruder disclosed herein may be recognized to encompass tool or machine that comprise at least one container and a porous structure (filter or membrane) and enable achieving disruption of cells.

In one embodiment, the term "extrusion" may be recognized to comprise a series of processes that allow components located in a container to pass through a porous structure (filter or membrane) by pressure. For example, the process of applying pressure to a first container where a composition containing cells is located and allowing the cells to pass through the filter while being disrupted by pressure is an example of extrusion. In another example, the process of passing a Cas protein and/or gRNA through a filter from the first container by pressure to move to a region (e.g., a second container) other than the first container is an example of extrusion.

In one embodiment, the extruder may be a one-way extruder that is designed to pass through a filter once, but the present application is not limited thereto. In one embodiment, the extruder may be a two-way extruder that is designed to pass through the filter multiple times, but the present invention is not limited thereto. The two-way extruder may comprise at least two containers and a filter located between the two containers. For example, the above-described Avanti^{®} Mini-Extruder may be a two-way extruder. In one embodiment, when the two-way extruder is used, a cell disruption rate may increase by passing cells through a filter multiple times, but is not otherwise limited.

Hereinafter, a method of creating an environment where a gene editing tool and a DNA molecule are able to contact, which comprises a cell disruption process, using the above-described extruder will be described in further detail.

### Cell disruption through extruder

The off-target prediction method of the present application may comprise the use of an extruder. For example, an extruder that comprises a first container, a second container, and a filter may be used. Here, the filter may be located between the first container and the second container. Hereinafter, an example of the use of the extruder comprising the first container, the second container, and the filter will be disclosed.

In one embodiment, the starting composition comprising a cell, a Cas protein, and a gRNA may be loaded in the first container. Pressure may be applied to the first container where the starting composition is located. For example, the application of pressure may be performed by pushing a piston connected to the first container, designed to apply pressure to the first container. That is, pressure may be applied to the first container by pushing the piston connected to the first container in the direction of the first container and the filter. In one embodiment, in order to move the components of the starting composition (comprising cells, a Cas proteins, and gRNAs) to the second container, pressure may be applied.

When pressure is applied to the first container, the components comprised in the starting composition may move to the second container through the filter with pores. Here, cells larger than the pore size may be disrupted while passing through the filter. As described above, cell disruption may be the disruption of a cell membrane, or the disruption of a cell membrane and a nuclear membrane. Eventually, a mixture comprising the components obtained from the disrupted cells, the Cas proteins, and the gRNAs may settle in the second container. In this process, the Cas/gRNA complex contacts with DNA (e.g., genomic DNA), which is one of the components obtained from the disrupted cells. Further, the mixture in the second container may or may not comprise undisrupted cells.

Afterward, optionally, pressure may be applied to the second container where the mixture is located, allowing the components of the mixture to move to the first container (through a filter). Therefore, the mixture may settle in the first container. Subsequently, optionally, pressure may be applied to the first container where the mixture is placed, allowing the components of the mixture to move to the second container (through a filter). Likewise, the extruder may be used so that the components of the composition loaded to the extruder or components derived from the composition pass through its filter several times. Passing through the filter several times may increase a cell disruption rate, and/or may increase a contact rate between the Cas/gRNA complex and the genomic DNA.

In one embodiment, an extrusion may be performed n times. In one embodiment, passing through a filter may be performed n times. Here, n may be an integer. Here, n may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100, but the present invention is not limited thereto. In one embodiment, n may be any one or less of the aforesaid values. In one embodiment, n may be any one or more of the aforesaid values. In one embodiment, n may be within a range determined by any two selected from the aforesaid values.

A composition to be analyzed may be obtained through a process comprising the use of an extruder. In one embodiment, after the extrusion process, any one or more of incubation, RNA removal, and DNA purification may be further performed.

In one embodiment, after the extrusion process, an incubation process for accumulating a cleavage rate may be performed. That is, after the cell disruption process, a process of incubating the composition comprising the components of the disrupted cells may be further performed. For example, incubation time may be approximately 1, 2, 3, 4, 5, 6, 7, 8, 9, 10, 11, 12, 13, 14, 15, 16, 17, 18, 19, 20, 21, 22, 23, 24, 26, 28, 30, 32, 36, 38, 40, 42, 44, 46, or 48 hours, or longer than the above-mentioned values, but is not otherwise limited. For example, after the extrusion process is completed, through incubation (e.g., incubation at 37 °C) and RNA removal, eventually, the composition to be analyzed may be obtained. Here, the composition to be analyzed is a composition that is to be used in a subsequent analysis process. Here, for example, DNA comprised in the composition to be analyzed may be suitable DNA for analysis (e.g., sequencing). For example, the composition to be analyzed may comprise cleaved DNA. The composition to be analyzed may comprise uncleaved DNA in addition to the cleaved DNA. The process of forming the cleaved DNA has been fully described above. For example, the Cas/gRNA complex may contact with DNA (e.g., genomic DNA), and the DNA may be cleaved via a DSB or nick induced by the Cas/gRNA complex.

### Analysis of composition to be analyzed

### Composition to be analyzed

As described above, the composition to be analyzed may be obtained by the method that comprises contacting a gene editing tool (e.g., a Cas/gRNA complex) and genomic DNA according to one embodiment of the present application. In one embodiment, the composition to be analyzed may comprise cleaved genomic DNA. In one embodiment, the composition to be analyzed may comprise one or more cleaved DNAs (e.g., double-stranded DNAs or single-stranded DNAs). In one embodiment, the cleaved genomic DNA may comprise one or more cleavages at one or more cleavage sites. For example, as described above, one or more cleavages may occur via a DSB or nick induced by the Cas/gRNA complex that comes into contact with the genomic DNA. In one embodiment, the cleavage site may be involved with an off-target site or on-target site. In one embodiment, the cleavage site may be an off-target site or on-target site. That is, cleavage may occur via a DSB or nick induced (or generated) at an off-target site or on-target site by the Cas/gRNA complex that contact with the genomic DNA.

In one embodiment, the composition to be analyzed may reflect the advantages of an in vitro off-target prediction system. For example, the cleaved genomic DNA comprised in the composition to be analyzed may not be repaired genomic DNA. This is because some or all of the DNA repair mechanisms are inactivated.

In one embodiment, the composition to be analyzed may reflect the advantages of a cell-based off-target prediction system. For example, the cleaved genomic DNA comprised in the composition to be analyzed may reflect cell-specific epigenetic features.

After the composition to be analyzed is obtained, it may be analyzed to obtain information on the cleavage of the genomic DNA. Accordingly, the information on off-target candidates that can be generated with the use of a gene editing system may be obtained. Information on off-target candidates may be applied to predict off-targets. That is, off-targets that are possibly generated when using a gene editing system (e.g., a CRISPR/Cas gene editing system) may be predicted.

Hereinafter, a method of obtaining the information on off-target candidates by analyzing the composition to be analyzed is disclosed.

### Overview of analysis of composition to be analyzed

As described above, the method of the present application comprises analyzing the composition to be analyzed comprising the cleaved genomic DNA obtained above. Information on the cleavage of the genomic DNA (e.g., information on one or more cleavage positions and/or cleavage scores at one or more cleavage positions) may be obtained by analyzing the composition to be analyzed. Based on the information on the cleavage of the genomic DNA, information on off-target candidates (e.g., information on one or more off-targets and/or scores for one or more off-targets ) may be obtained.

### Analysis of composition to be analyzed and obtaining of information on cleavage of genomic DNA

In one embodiment, information on the cleavage of the genomic DNA may be obtained by analyzing DNA (e.g., cleaved and/or uncleaved genomic DNA) comprised in the composition to be analyzed. In one embodiment, information on the cleavage of the genomic DNA may be obtained by analyzing the cleaved DNA comprised in the composition to be analyzed. In one embodiment, information on the cleavage of the genomic DNA may be obtained by analyzing one or more cleavage sites. Here, an analysis method that can identify the cleavage site of the cleaved DNA is not particularly limited. For example, any analysis method that can identify the cleavage site of the cleaved DNA can be used enough in the off-target prediction method of the present application.

In one embodiment, DNA analysis may be performed through a DNA analysis method well known to those of ordinary skill in the art. In one embodiment, DNA analysis may be performed by any one or more selected from a PCR-based assay (refer to the document [Cameron, Peter, et al. "Mapping the genomic landscape of CRISPR-Cas9 cleavage." Nature methods 14.6 (2017): 600-606.]) and sequencing (refer to the documents [Metzker, Michael L. "Sequencing technologies-the next generation." Nature reviews genetics 11.1 (2010): 31-46.; and Kumar, Kishore R., Mark J. Cowley, and Ryan L. Davis. "Next-generation sequencing and emerging technologies." Seminars in thrombosis and hemostasis. Vol. 45. No. 07. Thieme Medical Publishers, 2019.]) (e.g., DNA sequencing).

For example, sequencing may be any one or more sequencing methods called whole-genome sequencing (WGS), deep sequencing, high-throughput sequencing (HTS), de-novo sequencing, second-generation sequencing, next-generation sequencing, third generation sequencing, large-scale sequencing, shotgun sequencing, long-read sequencing, and short-read sequencing, but is not otherwise limited.

In one embodiment, a sequencing depth of the sequencing method used in the analysis of the composition to be analyzed may be approximately 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 42, 44, 46, 48, 50, 55, 60, 65, 70, 75, 80, 85, 90, 95, 100, 200, 300, 400, 500, 600, 700, 800, 900, or 1000x. In one embodiment, the sequencing depth may be in a range of two values selected from the aforesaid values. In one embodiment, the sequencing depth may be lower or higher than the aforesaid value. In a specific embodiment, the sequencing depth of the sequencing used in analysis may be approximately 10 to 40x. The sequencing depth is not otherwise limited, and any sequencing depth that can confirm the cleavage site on the DNA is sufficient.

### Information on DNA cleavage

In one embodiment, information on DNA cleavage (e.g., information on a cleavage site of the genomic DNA) may be obtained by analyzing the composition to be analyzed.

In one embodiment, the information on DNA cleavage may comprise information on one or more cleavage sites. Here, the cleavage sites may be generated by a gene editing tool.

In one embodiment, the information on DNA cleavage may comprise information on the location on genomic DNA for one or more cleavage sites. For example, the information on DNA cleavage may comprise location information for each cleavage site on the genomic DNA among all cleavage sites on the cleaved DNA comprised in the composition to be analyzed. For example, the information on DNA cleavage may comprise location information for each cleavage site on the genomic DNA among one or more cleavage sites on the cleaved DNA comprised in the composition to be analyzed. That is, through the analysis, the location information on all cleavage sites or the location information on some cleavage sites may be obtained. The location information obtained thereby may be associated with off-target candidates and/or on-target sites. For example, by comparing the location information on the identified cleavage site and the predetermined on-target site, it may be determined whether the cleavage site is associated with an off-target candidate or an on-target.

In one embodiment, the information on DNA cleavage may comprise cleavage scores for one or more cleavage sites. For example, the information on DNA cleavage may comprise a cleavage score of each cleavage site among all cleavage sites on the cleaved DNA comprised in the composition to be analyzed. For example, the information on DNA cleavage may comprise the cleavage score of each cleavage site among one or more cleavage sites on the cleaved DNA of the composition to be analyzed. That is, through analysis, cleavage scores for all cleavage sites or scores for some cleavage sites may be obtained. In one embodiment, the cleavage scores may be calculated from sequence reads. In one embodiment, the cleavage scores may be calculated from the result of a Manhattan plot. The calculation mechanism for the cleavage scores is not limited otherwise, and may be suitably selected depending on what analysis method is used. In one embodiment, based on the cleavage scores, a cleavage rank may be calculated. For example, cleavage sites showing high cleavage scores may be ranked higher. For example, the cleavage site showing the highest cleavage score may be ranked first. In one embodiment, the cleavage score may be related to the cleavage rate of the corresponding cleavage site. Information on the cleavage score obtained thereby may be associated with scores of the off-target candidates and/or on-target candidates.

In one embodiment, the information on DNA cleavage may comprise information on the number of generated cleavage sites. For example, the total number of cleavage sites may be calculated. For example, in one calculation of the number of cleavage sites, overlapping sites may be counted as one. In another example, in another calculation of the number of cleavage sites, overlapping sites may be counted as multiple. For example, when 5 DNAs showing a cleavage at the cleavage site x, the result may be counted as 1 or 5 if needed. From the information on the number of cleavage sites, the total number of off-target candidates that may be generated by using a gene editing system may be confirmed.

In one embodiment, the information on DNA cleavage obtained by analyzing the composition to be analyzed may comprise, but is not otherwise limited to, any one or more of the following:
a location of one or more cleavage sites on the genomic DNA;
a cleavage score for one or more cleavage sites; and
the number of the occurring cleavages.

In one embodiment, the process of obtaining information on DNA cleavage by analyzing the composition to be analyzed may further comprise an additional process to obtain the information on DNA cleavage. For example, a process(s) such as information (or data) processing, and/or the normalization of the obtained information (or data) may be further comprised. For example, a process of comparing the obtained cleavage information with the information on the previously determined on-target may be further comprised. The process of obtaining cleavage information may further comprise an additional process as described above, but is not otherwise limited.

In one embodiment, the information on DNA cleavage may further comprise other information that can be obtained through the analysis of the composition to be analyzed (e.g., DNA sequencing), but is not otherwise limited.

### Obtaining of information on off-targets

In one embodiment, from the obtained cleavage information, information on off-target candidates may be obtained. Those of ordinary skill in the art related to the present application may obtain information on off-targets without difficulty based on the cleavage information, and therefore, the present disclosure does not limit the process of the off-target prediction system of the present application. Those of skill in the art related to the present application may obtain information on off-targets using the cleavage information (e.g., information on DNA cleavage) obtained by analyzing the composition to be analyzed through an appropriate process or without an additional process.

In one embodiment, the off-target prediction method of the present application may comprise a process of identifying information on off-target candidates from the obtained information on cleavage.

In one embodiment, the information on off-target candidates may comprise information on the location of one or more off-target candidates on genomic DNA (e.g., information on candidate off-target sites). For example, the information on the location of off-target candidates may comprise information on each location (location on genomic DNA) of all off-target candidates. For example, the information on the location of off-target candidates may comprise information on each location of one or more off-target candidates. That is, the location information for all candidate off-target sites may be obtained, or the location information for one or more candidate off-target sites but not for all candidate off-target sites may be obtained. Among the off-target candidates, a bona-fide off-target (e.g., a genuine off-target generated from the use of a gene editing system) may be present. The information on the location of off-target candidates may be obtained based on the above-mentioned cleavage information (e.g., location information for one or more cleavage sites).

In one embodiment, the information on off-target candidates may comprise off-target scores (e.g., off-target prediction scores) for one or more off-target candidates. For example, the information on off-target candidates may comprise the off-target score for each off-target candidate with respect to all off-target candidates. For example, the information on off-target candidates may comprise the off-target score for each off-target candidate with respect to one or more off-target candidates. That is, the off-target scores for all candidate off-target sites may be obtained, or the off-target scores for one or more candidate off-target sites but not for all candidate off-target sites may be obtained. The information on the off-target scores for off-target candidates may be obtained based on the above-mentioned cleavage information (e.g., scores for one or more cleavage sites). In one embodiment, the ranking of the off-target candidates may be calculated based on the obtained off-target scores. For example, an off-target candidate (e.g., a candidate off-target site) with a higher off-target score may be ranked high. For example, the off-target candidate with the highest off-target score may be ranked first. For example, the high off-target scores of off-target candidates may be associated with bona-fide off-targets, but is not otherwise limited.

In one embodiment, the information on off-target candidates may comprise information on the number of off-target candidates. For example, the total number of off-target candidates may be calculated. For example, in the calculation of the number of off-target candidates, overlapping sites may be counted as one. In another example, in the calculation of the number of off-target candidates, overlapping sites may be counted as multiple. For example, when five candidate off-target sites x are found, they may be counted as 1 or 5. From the information on the number of off-target candidates, the total number of off-target candidates that can be generated by using a gene editing system may be confirmed. That is, the total number of predicted off-targets may be confirmed.

In one embodiment, the information on off-target candidates may comprise any one or more of the following, but is not otherwise limited:
the location on genomic DNA for each of one or more off-target candidates;
the off-target score for each of the one or more off-target candidates; and
the number of predicted off-target candidates

In one embodiment, the process of obtaining information on off-target candidates may further comprise an additional process for obtaining the information on off-target candidates. For example, a process(s) such as information (or data) processing, and/or the normalization of the obtained information (or data) may be further comprised. For example, a process of comparing the obtained information on off-target candidates with the information on the previously determined on-target may be further comprised. As described above, the process of obtaining the information on off-target candidates may further include an additional process, but is not otherwise limited.

In one embodiment, the information on off-target candidates may further comprise additional information that helps predict off-targets that are possibly generated by using a gene editing system, but is not otherwise limited.

### Relationship with gene editing system subject to prediction

The off-target prediction system of the present application may be associated with a gene editing system subject to prediction. Here, the gene editing system subject to prediction may refer to a gene editing system that is determined to be used for research or treatment, but is not otherwise limited. That is, the gene editing system subject to prediction may refer to a gene editing system (or gene editing process) that has to predict off-targets.

For example, when a specific cell is used in the gene editing system subject to prediction, the specific cell may also be used in the method of predicting off-targets of the present application. For example, when guide RNA having a specific guide sequence is used in the gene editing system subject to prediction, guide RNA having the same guide sequence may also be used in the method of predicting off-targets of the present application.

In this aspect, the method of predicting off-targets according to one embodiment of the present application may further comprise a process of confirming the gene editing system subject to prediction. The gene editing system subject to prediction may be referred to as the predetermined gene editing system. The predetermined gene editing (e.g., genome editing) system may comprise cells subject to gene editing (genome editing), and one or more of the predetermined gene editing tools. The predetermined gene editing tools may comprise, for example, types of guide RNA, guide sequence, and gene editing protein (e.g., Cas protein).

In one embodiment, the method of predicting off-targets of the present application may further comprise confirming or designing the predetermined gene editing system. The predetermined gene editing system may be confirmed, and thereby components that will be suitably used in an off-target prediction system may be designed. Here, the process of confirming the predetermined gene editing system may be performed before the composition to be analyzed is obtained. Hereinafter, an example of the confirmation of the predetermined gene editing system (that is to be predicted) is illustrated based on a CRISPR/Cas gene editing system. The exemplary description based on the CRISPR/Cas gene editing system does not limit the aspect of the off-target prediction system of the present application, and it implies that the exemplary description is fully applicable to different gene editing systems in a similar or identical context to the following description.

In one embodiment, the method of predicting off-targets of the present application may comprise the predetermined CRISPR/Cas gene editing system. Here, confirming the predetermined CRISPR/Cas gene editing system may comprise confirming any one or more of a predetermined cell (i.e., cells for editing to be used in editing of a CRISPR/Cas-based gene subject to prediction), a predetermined type of Cas protein (i.e., the type of Cas protein to be used in editing of a CRISPR/Cas-based gene subject to prediction), and information on a predetermined guide RNA (a sequence of guide RNA or guide sequence).

In a specific embodiment, confirming the predetermined CRISPR/Cas gene editing system may comprise confirming a predetermined cell. In a specific embodiment, in the off-target prediction system of the present application, a cell identical to the predetermined cell may be used. As a result, cell-specific characteristics may be reflected in the result of the off-target prediction system. The cell subject to genome editing are not otherwise limited. In one embodiment, the predetermined cell may be an animal cell or a plant cell. In one embodiment, the predetermined cell may be a human cell or a cell derived from a non-human animal (e.g., a mouse, a rat, a dog, a cat, a cow, a pig, a horse, and sheep), but are not otherwise limited. In a specific embodiment, the predetermined cell may be a human cell.

In a specific embodiment, confirming the predetermined CRISPR/Cas gene editing system may comprise confirming the predetermined Cas protein. In a specific embodiment, in the off-target prediction system of the present application, a Cas protein identical to the predetermined Cas protein may be used. As a result, characteristics that can be affected by the Cas protein may be reflected in the result of the off-target prediction system. In one embodiment, it may be a gene editing system using SpCas9.

In a specific embodiment, confirming the predetermined CRISPR/Cas gene editing system may comprise confirming the predetermined guide RNA. In a specific embodiment, in the off-target prediction system of the present application, guide RNA identical to the predetermined guide RNA may be used. As a result, characteristics that can be affected by the guide RNA may be reflected in the result of the off-target prediction system.

In a specific embodiment, confirming the predetermined CRISPR/Cas gene editing system may comprise confirming the predetermined guide sequence. In a specific embodiment, in the off-target prediction system of the present application, a guide RNA having the same guide sequence as the predetermined guide sequence may be used. As a result, characteristics that are affected by the guide sequence may be reflected in the result of the off-target prediction system.

In a specific embodiment, in the off-target prediction system of the present application, any one or more selected from a cell identical to the predetermined cell, a Cas protein identical to the predetermined Cas protein, and a guide RNA having the same guide sequence as the predetermined guide sequence may be used.

The above description does not limit that components identical to the components used in the predetermined gene editing system have to be necessarily used in the off-target prediction system, and the off-target prediction system of the present application may be suitably selected depending on the purpose of the off-target prediction system used by one of skill in the art. For example, a type of Cas protein different from the predetermined Cas protein (e.g., a Cas protein known to have similar characteristics) may be used in the off-target prediction system. In another example, a type of cells different from the predetermined cells (e.g., cells known to have similar characteristics) may be used in the off-target prediction system. In still another example, a type of guide RNA different from the predetermined guide RNA (e.g., guide RNA modified to be more effectively applied to the off-target prediction system) may be used in the off-target prediction system.

### Can be used along with a different off-target prediction system

In one embodiment, the off-target prediction system of the present application may be used together with a different off-target prediction system. For example, the off-target prediction system of the present application may be used along with any one or more selected from an in silico-based off-target prediction system, an in vitro-based off-target prediction system, and a cell-based off-target prediction system. For example, the off-target prediction system of the present application may be used along with any one or more selected from Cas-OFFinder, CHOPCHOP, CRISPOR, Digenome-seq, DIG-seq, SITE-seq, CIRCLE-seq, CHANGE-seq, GUIDE-seq, GUIDE-tag, DISCOVER-seq, BLISS, BLESS, integrase-defective lentiviral vector-mediated DNA break capture, HTGTS, ONE-seq, CReVIS-Seq, ITR-seq, and TAG-seq. To more effectively find a bona-fide off-target site, an off-target prediction system different from the off-target prediction system of the present application may be used, and the different off-target prediction system may be an off-target prediction system that has been developed prior to the filing date of the present application, or an off-target prediction system that is developed after the filing date of the present application, but is not otherwise limited.

### Starting composition and components that can be comprised in starting composition

### Overview of starting composition

As described above, according to one embodiment of the present application, a composition to be analyzed may be obtained through disrupting the cells. In addition, information on cleavage of genomic DNA may be obtained by analyzing the composition to be analyzed.

In one embodiment, the composition to be analyzed may be obtained, through disrupting the cells, from a starting composition comprising cells. In one embodiment, the starting composition may further comprise gene editing tools (e.g., a Cas protein and guide RNA) in addition to the cells. Hereinafter, conditions for components that can be comprised in the starting composition of the off-target prediction method of one embodiment of the present application will be disclosed.

### Cells and cell concentration

In one embodiment, the starting composition may comprise cells. In one embodiment, the concentration of the cells comprised in the starting composition may be approximately 1×10⁵ cells/mL, 2×10⁵ cells/mL, 3×10⁵ cells/mL, 4×10⁵ cells/mL, 5×10⁵ cells/mL, 6×10⁵ cells/mL, 7×10⁵ cells/mL, 8×10⁵ cells/mL, 9×10⁵ cells/mL, 1×10⁶ cells/mL, 2×10⁶ cells/mL, 3×10⁶ cells/mL, 4×10⁶ cells/mL, 5×10⁶ cells/mL, 6×10⁶ cells/mL, 7×10⁶ cells/mL, 8×10⁶ cells/mL, 9×10⁶ cells/mL, 1×10⁷ cells/mL, 2×10⁷ cells/mL, 3×10⁷ cells/mL, 4×10⁷ cells/mL, 5×10⁷ cells/mL, 6×10⁷ cells/mL, 7×10⁷ cells/mL, 8×10⁷ cells/mL, 9×10⁷ cells/mL, 1×10⁸ cells/mL, 2×10⁸ cells/mL, 3×10⁸ cells/mL, 4×10⁸ cells/mL, 5×10⁸ cells/mL, 6×10⁸ cells/mL, 7×10⁸ cells/mL, 8×10⁸ cells/mL, 9×10⁸ cells/mL, 1×10⁹ cells/mL, 2×10⁹ cells/mL, 3×10⁹ cells/mL, 4×10⁹ cells/mL, 5×10⁹ cells/mL, 6×10⁹ cells/mL, 7×10⁹ cells/mL, 8×10⁹ cells/mL, 9×10⁹ cells/mL, 1×10¹⁰ cells/mL, 2×10¹⁰ cells/mL, 3×10¹⁰ cells/mL, 4×10¹⁰ cells/mL, 5×10¹⁰ cells/mL, 6×10¹⁰ cells/mL, 7×10¹⁰ cells/mL, 8×10¹⁰ cells/mL, or 9×10¹⁰ cells/mL, but is not otherwise limited. In one embodiment, the concentration of the cells comprised in the starting composition may be in the range between two values selected from the above-mentioned values. In one embodiment, the concentration of the cells comprised in the starting composition may be any one value or more or less selected from the aforesaid values. In a specific embodiment, the concentration of the cells comprised in the starting composition may be approximately 1×10⁶ cells/mL, 2×10⁶ cells/mL, 3×10⁶ cells/mL, 4×10⁶ cells/mL, 5×10⁶ cells/mL, 6×10⁶ cells/mL, 7×10⁶ cells/mL, 8×10⁶ cells/mL, 9×10⁶ cells/mL, 1×10⁷ cells/mL, 2×10⁷ cells/mL, 3×10⁷ cells/mL, 4×10⁷ cells/mL, 5×10⁷ cells/mL, 6×10⁷ cells/mL, 7×10⁷ cells/mL, 8×10⁷ cells/mL, 9×10⁷ cells/mL, or 1×10⁸ cells/mL.

The cells that can be used in the off-target prediction system of the present application are not otherwise limited. In one embodiment, the cells may be animal cells or plant cells. In one embodiment, the cells may be human cells or cells derived from a non-human animal (e.g., a mouse, a rat, a dog, a cat, a cow, a pig, a horse, and sheep), but are not otherwise limited. In a specific embodiment, the cells may be human cells.

### Gene editing tools and concentration of editing tools

In one embodiment, the starting composition may comprise gene editing tools. In one embodiment, the starting composition may comprise a Cas protein and a gRNA.

In one embodiment, the concentration of the Cas protein comprised in the starting composition may be approximately 10 nM, 20 nM, 30 nM, 40 nM, 50 nM, 60 nM, 70 nM, 80 nM, 90 nM, 100 nM, 200 nM, 300 nM, 400 nM, 500 nM, 600 nM, 700 nM, 800 nM, 900 nM, 1000 nM (1µM), 2000 nM, 3000 nM, 4000 nM, 5000 nM, 6000 nM, 7000 nM, 8000 nM, 9000 nM, 10000 nM (10 µM), 20000 nM, 30000 nM, 40000 nM, 50000 nM, 60000 nM, 70000 nM, 80000 nM, 90000 nM, or 100000 nM (100 µM), but is not otherwise limited. In one embodiment, the concentration of the Cas protein comprised in the starting composition may be in the range between two values selected from the aforesaid values. In one embodiment, the concentration of the Cas protein comprised in the starting composition may be any one value or more or less selected from the aforesaid values. In a specific embodiment, the concentration of the Cas protein comprised in the starting composition may be approximately 1000 nM (1 µM), 2000 nM, 3000 nM, 4000 nM, 5000 nM, 6000 nM, 7000 nM, 8000 nM, 9000 nM, or 10000 nM (10 µM).

In one embodiment, the concentration of the guide RNA comprised in the starting composition may be approximately 10 nM, 20 nM, 30 nM, 40 nM, 50 nM, 60 nM, 70 nM, 80 nM, 90 nM, 100 nM, 200 nM, 300 nM, 400 nM, 500 nM, 600 nM, 700 nM, 800 nM, 900 nM, 1000 nM (1 µM), 2000 nM, 3000 nM, 4000 nM, 5000 nM, 6000 nM, 7000 nM, 8000 nM, 9000 nM, 10000 nM (10 µM), 20000 nM, 30000 nM, 40000 nM, 50000 nM, 60000 nM, 70000 nM, 80000 nM, 90000 nM, or 100000 nM (100 µM), but is not otherwise limited. In one embodiment, the concentration of the guide RNA comprised in the starting composition may be in the range between two values selected from the aforesaid values. In one embodiment, the concentration of the guide RNA comprised in the starting composition may be any one value or more or less selected from the aforesaid values. In a specific embodiment, the concentration of the guide RNA comprised in the starting composition may be approximately 1000 nM (1 µM), 2000 nM, 3000 nM, 4000 nM, 5000 nM, 6000 nM, 7000 nM, 8000 nM, 9000 nM, or 10000 nM (10 µM).

In one embodiment, the starting composition may comprise a ribonucleoprotein (RNP) (e.g., a Cas/gRNA complex). In this case, in order for the Cas protein and the gRNA to be present in the form of RNP in the starting composition, the off-target prediction method of the present application may further comprise mixing the guide RNA and the Cas protein and pre-incubating the mixture. That is, before providing the starting composition, the process of incubating the mixture comprising the guide RNA and the Cas protein may be further comprised. For example, RNP (Cas/gRNA complex) may be obtained from the incubated mixture comprising the guide RNA and the Cas protein, and the obtained RNP may be mixed with the cells to obtain the starting composition. In one embodiment, the concentration of the RNP (e.g., Cas/gRNA complex) comprised in the starting composition may be approximately 10 nM, 20 nM, 30 nM, 40 nM, 50 nM, 60 nM, 70 nM, 80 nM, 90 nM, 100 nM, 200 nM, 300 nM, 400 nM, 500 nM, 600 nM, 700 nM, 800 nM, 900 nM, 1000 nM (1µM), 2000 nM, 3000 nM, 4000 nM, 5000 nM, 6000 nM, 7000 nM, 8000 nM, 9000 nM, 10000 nM (10µM), 20000 nM, 30000 nM, 40000 nM, 50000 nM, 60000 nM, 70000 nM, 80000 nM, 90000 nM, or 100000 nM (100 µM), but is not otherwise limited. In one embodiment, the concentration of the RNP comprised in the starting composition may be in the range between two values selected from the aforesaid values. In one embodiment, the concentration of the RNP comprised in the starting composition may be any one value or more or less selected from the aforesaid values. In a specific embodiment, the concentration of the RNP comprised in the starting composition may be approximately 1000 nM (1 µM), 2000 nM, 3000 nM, 4000 nM, 5000 nM, 6000 nM, 7000 nM, 8000 nM, 9000 nM, or 10000 nM (10 µM).

### Exemplary embodiments of off-target prediction system of the present application (1)

Hereinafter, exemplary embodiments (non-limiting embodiments) of the off-target prediction system of the present application will be disclosed. The following embodiments may be characterized by a mechanism of the off-target prediction system of the present application. Some or all of the following embodiments may comprise one or all of embodiments disclosed in the embodiments characterized that the use of an extruder, which will be described below.

One embodiment of the present application provides a method of predicting off-targets that can be occurred in a gene editing (e.g., genome editing) process. One embodiment of the present application provides a method of confirming off-target candidate that can be occurred in genome editing process. One embodiment of the present application provides a method of predicting off-targets of a CRISPR/Cas gene editing system. One embodiment of the present application provides a method of confirming off-target candidates that can be generated in a gene editing process using a CRISPR/Cas gene editing system. Descriptions of a method of predicting off-targets that can be generated in a genome editing process or a method of confirming information on off-targets may be used without limitation.

One embodiment of the present application provides a method of predicting off-targets that can be occurred in a gene editing process, which comprises:
(i) obtaining a composition to be analyzed, which comprises cleaved genomic DNA; and
(ii) obtaining information on a cleavage site by analyzing the composition to be analyzed.

In a specific embodiment, the cleaved genomic DNA comprised in the composition to be analyzed may be cleaved genomic DNA formed by cleaving the genomic DNA of physically disrupted cells by a gene editing system.

In a specific embodiment, the cleaved genomic DNA may have cell-specific epigenetic features.

In a specific embodiment, the cleaved genomic DNA may not be repaired genomic DNA.

One embodiment of the present application provides a method of predicting off-targets that can be occurred in a gene editing (e.g., genome editing) process, which comprises:
(i) preparing a starting composition comprising a gene editing tool and a first cell;
(ii) obtaining a composition to be analyzed by physically disrupting the first cell, wherein the physical disruption of the first cell leads to the creation of an environment where the genomic DNAs in the cells can come into contact with the gene editing tool, whereby the genomic DNA and the gene editing tools are contact, whereby the genomic DNA are cleaved at one or more cleavage sites; and
(iii) obtaining information on the cleavage sites by analyzing the composition to be analyzed.

In a specific embodiment, the method of predicting off-targets may further comprises:
(iv) confirming information on off-target candidates from the information on the cleavage sites obtained in (iii).

One embodiment of the present application provides a method of predicting off-targets that can be occurred in a CRISPR/Cas gene editing system, which comprises:
(i) preparing a starting composition comprising a first Cas protein, first guide RNA, and a first cell, wherein the Cas protein and the guide RNA are capable of forming a Cas/gRNA complex;
(ii) obtaining a composition to be analyzed by physically disrupting the first cell, wherein, through the physically disrupting the first cell, an environment where a genomic DNA can contact with a Cas/gRNA complex is created, whereby the genomic DNA and the Cas/gRNA complex contact, wherein the genomic DNA is cleaved at one or more cleavage sites; and
(iii) obtaining information on the cleavage sites by analyzing the composition to be analyzed.

In a specific embodiment, the method of predicting off-targets may further comprise: (iv) confirming information on off-target candidates from the information on the cleavage sites obtained in (iii).

In a specific embodiment, the information on the cleavage sites may comprise one or more of the following: the location of one or more cleavage sites on the genomic DNA, cleavage scores for one or more cleavage sites, and the number of cleavage sites.

In a specific embodiment, the location of one or more cleavage sites on the genomic DNA may be the location of each of the one or more cleavage sites on the genomic DNA.

In a specific embodiment, the cleavage scores for one or more cleavage sites may be the cleavage score for each of the one or more cleavage sites.

In a specific embodiment, the number of cleavage sites may be the total number of the cleavage sites.

In a specific embodiment, the information on off-target candidates may comprise one or more of the following: the location of one or more off-target candidates on genomic DNA; off-target prediction scores for one or more off-target candidates; and the number of the predicted off-target candidates.

In a specific embodiment, the location of one or more off-target candidates on genomic DNA may be the location on the genomic DNA of each of the one or more off-target candidates.

In a specific embodiment, the off-target prediction scores for one or more off-target candidates may be off-target prediction scores for each of the one or more off-target candidates.

In a specific embodiment, the number of the off-target candidates may be the total number of predicted off-target candidates.

In a specific embodiment, in (ii), a membrane structure comprising a cell membrane of the first cell may be disrupted by physically disrupting the first cell, thereby providing an environment where the Cas/gRNA complex can come into contact with the genomic DNA.

In a specific embodiment, in (ii), a membrane structure comprising a nuclear membrane of the first cell may be disrupted by physically disrupting the first cell, thereby an environment where the Cas/gRNA complex can come into contact with the genomic DNA may be prepared.

In a specific embodiment, the physical disruption of the first cell may comprise passing the first cell through a filter with pores.

In a specific embodiment, a force to make the first cell pass through pores smaller than a size of first cell may be pressure.

In a specific embodiment, an average pore diameter of the filter may be smaller than the size of the first cell.

In a specific embodiment, the filter may comprise pores with a smaller diameter than the size of the first cell.

In a specific embodiment, the average pore diameter of the filter may be 5 to 15 µm.

In a specific embodiment, the average pore diameter of the filter may be approximately 8 µm.

In a specific embodiment, the filter may comprise pores with a diameter of 5 to 15 µm.

In a specific embodiment, the physical disruption of the first cell may be performed by the use of an extruder.

In a specific embodiment, the extruder may comprise a filter with pores, wherein the filter may comprise pores with a smaller diameter than the first cell size.

In a specific embodiment, the extruder may comprise a filter with pores, and the average pore diameter of the filter may be 5 to 15 µm.

In a specific embodiment, the exposed genomic DNA by the physical disruption of the first cell may maintain cell-specific epigenetic features (e.g., the features of a chromatin structure) of the first cell.

In a specific embodiment, the information on off-target candidates may be information reflecting the first cell-specific epigenetic features.

In a specific embodiment, the environment where the genomic DNA and the Cas/gRNA complex can contact may be an environment where a DNA repair mechanism is inactivated.

In a specific embodiment, DNA repair mechanism of the cell is inactivated according to the cell disruption, and therefore the cleaved DNA may not be repaired.

A specific embodiment may provide a method of predicting off-targets, which further comprises: confirming a predetermined CRISPR/Cas gene editing system subject to off-target prediction. Here, the predetermined CRISPR/Cas gene editing system may comprise any one or more of the use of a predetermined cell, the use of a predetermined Cas protein, and the use of a predetermined guide RNA. Here, the confirmation of the predetermined CRISPR/Cas gene editing system may be performed prior to (i).

In a specific embodiment, the guide sequence of the first guide RNA may have the same sequence as the guide sequence of the predetermined guide RNA.

In a specific embodiment, the predetermined CRISPR/Cas gene editing system may comprise the use of the predetermined cell, and the first cell and the predetermined cell may be the same.

In a specific embodiment, in (iii), the analysis of the composition to be analyzed may comprise: analyzing DNA comprised in the composition to be analyzed through sequencing.

In a specific embodiment, in (iii), the analysis of the composition to be analyzed may comprise: analyzing cleaved genomic DNA comprised in the composition to be analyzed through sequencing.

In a specific embodiment, in (iii), the analysis of the composition to be analyzed may comprise: analyzing DNA comprised in the composition to be analyzed through a PCR-based analysis method.

In a specific embodiment, in (iii), the analysis of the composition to be analyzed may comprise: analyzing cleaved genomic DNA comprised in the composition to be analyzed through a PCR-based analysis method.

In a specific embodiment, the concentration of the Cas protein comprised in the starting composition may be approximately 5000 nM.

In a specific embodiment, the concentration of the first cell comprised in the starting composition may be approximately 1×10⁷ cells/mL.

In a specific embodiment, the obtaining of the composition to be analyzed may further comprise: incubating the composition obtained through cell disruption.

In a specific embodiment, the obtaining of the composition to be analyzed may further comprise: incubating the composition comprising the disrupted cell components, the Cas protein, and the guide RNA.

In a specific embodiment, the obtaining of the composition to be analyzed may further comprise: removing RNA from the composition obtained by cell disruption.

In a specific embodiment, the obtaining of the composition to be analyzed may further comprise: removing an RNA component of the composition comprising the disrupted cell components, the Cas protein, and the guide RNA.

In a specific embodiment, the obtaining of the composition to be analyzed may further comprise: purifying DNA from the composition obtained by cell disruption.

In a specific embodiment, the obtaining of the composition to be analyzed may further comprise: purifying DNA from the composition comprising the disrupted cell components, the Cas protein, and the guide RNA.

In a specific embodiment, the method of predicting off-targets of the present application may be used by combining one or more other off-target prediction methods. Here, other off-target prediction methods may be any one or more selected from Cas-OFFinder, CHOPCHOP, CRISPOR, Digenome-seq, DIG-seq, SITE-seq, CIRCLE-seq, CHANGE-seq, GUIDE-seq, GUIDE-tag, DISCOVER-seq, BLISS, BLESS, integrase-defective lentiviral vector-mediated DNA break capture, HTGTS, ONE-seq, CReVIS-Seq, ITR-seq, and TAG-seq.

Hereinafter, an exemplary embodiment of the off-target prediction system of the present application characterized that the use of an extruder will be described.

In one embodiment, a method of predicting off-targets that can be occurred in a CRISPR/Cas gene editing system may be provided, the method comprising:
(i) loading a starting composition comprising a first editing protein, first guide RNA, and a first cell to a first container of an extruder;
(ii) performing an extrusion process comprising the following process using the extruder to obtain a composition to be analyzed:
   (a) applying pressure to the first container to move the components of the starting composition from the first container of the extruder to a second container of the extruder,

   wherein the components of the starting composition move from the first container to the second container through a filter with pores, located between the first container of the extruder and the second container of the extruder, by the applied pressure, and thus a mixture settles in the second container;
   wherein the first cell, which are larger than the diameter of pores of the filter, are disrupted by the applied pressure and at the same time, pass through the pores of the filter, and
   wherein, through the physical disruption of the first cell, an environment where the genomic DNA in cell can come into contact with a Cas/gRNA complex,
   thereby contacting the genomic DNA with the Cas/gRNA complex,
   resulting in the cleavage of the genomic DNA at one or more cleavage sites; and
(iii) analyzing the composition to be analyzed to obtain information on the cleavage sites.

In a specific embodiment, the off-target prediction method may further comprise the following:
(iv) confirming information on off-target candidates from the information on the cleavage sites obtained from (iii) to predict the off-targets occurring in the CRISPR/Cas gene editing system.

In a specific embodiment, the extrusion process in (ii) may comprise the following:
(a) applying pressure to the first container to move the components of the starting composition from the first container of the extruder to the second container of the extruder, wherein the components of the starting composition move from the first container to the second container through a filter with pores, located between the first container of the extruder and the second container of the extruder, by the applied pressure, and thus a mixture settles in the second container,
(b) applying pressure to the second container to move components of the mixture comprised in the second container from the second container to the first container,
   wherein the components of the mixture comprised in the second container move from the second container to the first container through the filter with pores, located between the first container and the second container, by the applied pressure, and thus a mixture moving through the filter by pressure from the second container to the first container settles in the first container, and
(c) repeatedly performing the processes of (a) and (b) the predetermined number of times,

wherein the predetermined number of times is counted in 0.5 increments, and 0.5 indicates the performance of a single process of (a) or (b), and
wherein the first cell which is a component larger than the pore diameter of the filter, is disrupted by the applied pressured, and at the same time, pass through the pores of the filter, and
wherein, through the physically disruption of the first cell, an environment where the genomic DNA in the cell can come into contact with a Cas/gRNA complex is created,
thereby contacting the genomic DNA with the Cas/gRNA complex,
resulting in the cleavage of the genomic DNA at one or more cleavage sites.

In a specific embodiment, the pressure applied to the first container may be produced through a process of pushing a piston designed to apply pressure to the first container in the direction of the first container and the filter.

In a specific embodiment, the pressure applied to the first container may be produced through a process of pushing a piston designed to apply pressure to the first container in the direction of the first container and the filter, and the pressure applied to the second container may be produced through a process of pushing a piston designed to apply pressure to the second container in the direction of the second container and the filter.

In a specific embodiment, the information on the cleavage sites may comprise one or more of the following: the location on the genomic DNA for one or more cleavage sites, cleavage scores for the one or more cleavage sites, and the number of the cleavage sites.

In a specific embodiment, the information on off-target candidates may comprise one or more of the following: the location on the genomic DNA for one or more off-target candidates, off-target prediction scores for the one or more off-target candidates, and the number of the predicted off-target candidates.

In a specific embodiment, a membrane structure comprising the cell membrane of the first cell may be disrupted by physically disrupting the first cell, and thereby an environment where the Cas/gRNA complex can come into contact with the genomic DNA of the first cell may be prepared.

In a specific embodiment, in (ii), the physical disruption of the first cell leads to the disruption of a membrane structure comprising the nuclear membrane of the first cell, and thereby an environment where the Cas/gRNA complex can come into contact with the genomic DNA of the first cell may be prepared.

In a specific embodiment, the filter may comprise pores whose diameter is smaller than the first cell size.

In a specific embodiment, the average diameter of pores of the filter may be 5 to 15 µm.

In a specific embodiment, the average diameter of pores of the filter may be 8 µm.

In a specific embodiment, the predetermined number of times may be 4 to 7.

In a specific embodiment, the predetermined number of times may be 5.5.

In a specific embodiment, in (ii), the exposed genomic DNA through by the physical disruption of the first cell may maintain first cell-specific epigenetic features.

In a specific embodiment, the information on the cleavage sites obtained in (iii) may be information reflecting the first cell-specific epigenetic features.

In a specific embodiment, the information on off-target candidates obtained in (iv) may be information reflecting first cell-specific epigenetic features.

In a specific embodiment, the DNA repair mechanism of the cell may be broken due to the disruption of the cell, and thus the cleaved DNA may not be repaired.

In a specific embodiment, the off-target prediction method may further comprise the following:
confirming the CRISPR/Cas gene editing system subject to off-target prediction, wherein the CRISPR/Cas gene editing system subject to prediction comprises the use of a Cas protein subject to prediction and the use of guide RNA subject to prediction.

In a specific embodiment, the off-target prediction method may further comprise the following:
confirming the CRISPR/Cas gene editing system subject to off-target prediction, wherein the CRISPR/Cas gene editing system subject to prediction comprises the use of a Cas protein subject to prediction and the use of guide RNA subject to prediction, and the confirmation of the CRISPR/Cas gene editing system subject to prediction is performed before (i).

In a specific embodiment, a guide sequence of the first guide RNA may have the same sequence as the guide sequence of the guide RNA subject to prediction.

In a specific embodiment, the CRISPR/Cas gene editing system subject to prediction may comprise the use of a cell subject to prediction, and here, the first cell and the cell subject to prediction may be the same.

In a specific embodiment, the analysis of the composition to be analyzed in (iii) may comprise analyzing the cleaved genomic DNA comprised in the composition to be analyzed through sequencing.

In a specific embodiment, the analysis of the composition to be analyzed in (iii) may comprise analyzing the cleaved genomic DNA comprised in the composition to be analyzed through sequencing.

In a specific embodiment, the analysis of the composition to be analyzed in (iii) may comprise analyzing DNA comprised in the composition to be analyzed through a PCR-based analysis method.

In a specific embodiment, the analysis of the composition to be analyzed in (iii) may comprise analyzing the cleaved genomic DNA comprised in the composition to be analyzed through a PCR-based analysis method.

In a specific embodiment, the concentration of the Cas protein comprised in the starting composition may be 5000 nM.

In a specific embodiment, the concentration of the first cell comprised in the starting composition may be 1×10⁷ cells/mL.

In a specific embodiment, to obtain the composition to be analyzed, the following process may be further performed: incubating the composition comprising the disrupted cell components, the Cas protein, and the guide RNA.

In a specific embodiment, to obtain the composition to be analyzed, the following process may be further performed: incubating the composition obtained through cell disruption.

In a specific embodiment, to obtain the composition to be analyzed, the following process may be further performed: removing RNA components of the composition comprising the disrupted cell components, the Cas protein, and the guide RNA.

In a specific embodiment, to obtain the composition to be analyzed, the following process may be further performed: removing RNA from the composition obtained by cell disruption.

In a specific embodiment, to obtain the composition to be analyzed, the following process may be further performed: purifying DNA of the composition comprising the disrupted cell components, the Cas protein, and the guide RNA.

In a specific embodiment, to obtain the composition to be analyzed, the following process may be further performed: purifying DNA from the composition obtained by cell disruption.

In a specific embodiment, the method of predicting off-targets of the present application may be used in combination with one or more different off-target prediction methods. Here, the different off-target prediction methods may be any one or more selected from Cas-OFFinder, CHOPCHOP, CRISPOR, Digenome-seq, DIG-seq, SITE-seq, CIRCLE-seq, CHANGE-seq, GUIDE-seq, GUIDE-tag, DISCOVER-seq, BLISS, BLESS, integrase-defective lentiviral vector-mediated DNA break capture, HTGTS, ONE-seq, CReVIS-Seq, ITR-seq, and TAG-seq.

### Exemplary embodiments of off-target prediction system of the present application (2)

Hereinafter, exemplary embodiments (non-limiting embodiments) will be disclosed through a description mode different from the above-described "Exemplary embodiments of off-target prediction system of the present application (1)."

### Exemplary embodiments characterized by mechanism

A01. A method of predicting off-targets that can be occurred in a genome editing process using a CRISPR/Cas gene editing system, comprising:
(i) preparing a starting composition comprising a first Cas protein, a first guide RNA, and a first cell, wherein the Cas protein and the first guide RNA are capable of forming a Cas/gRNA complex;
(ii) obtaining a composition to be analyzed through physically disrupting the first cell, wherein, through physical disruption of the first cell, an environment where genomic DNA can contact with the Cas/gRNA complex is prepared, whereby the genomic DNA and the Cas/gRNA complex contact, whereby the genomic DNA is cleaved at one or more cleavage sites;
(iii) obtaining information on the cleavage sites by analyzing the composition to be analyzed; and
(iv) identifying information on off-target candidates from the information on the cleavage sites obtained from (iii) to predict the off-targets occurring in the CRISPR/Cas gene editing system.

A02. The method of A01, wherein
the information on the cleavage sites comprises one or more of the following:
locations on the genomic DNA for one or more cleavage sites,
cleavage scores for the one or more cleavage sites, and
the number of the cleavage sites.

A03. The method of any one of A01 and A02, wherein
the information on off-target candidates comprises one or more of the following:
locations on the genomic DNA for one or more off-target candidates,
off-target prediction scores for the one or more off-target candidates, and
the number of the predicted off-target candidates.

A04. The method of any one of A01 to A03, wherein,
in (ii), a membrane structure comprising a cell membrane of the first cell is disrupted by physically disrupting the first cell, and thereby an environment where the Cas/gRNA complex can come into contact with the genomic DNA of the first cell is prepared.

A05. The method of any one of A01 to A04, wherein,
in (ii), a membrane structure comprising a nuclear membrane of the first cell is disrupted by physically disrupting the first cell, and thereby an environment where the Cas/gRNA complex can come into contact with the genomic DNA of the first cell is prepared.

A06. The method of any one of A01 to A05, wherein
physically disrupting the first cell comprises passing the first cell through a filter having pores with a smaller size than the size of the first cell, wherein the first cell is disrupted while passing through pores with a smaller size than the first cell size.

A07. The method of any one of A01 to A06, wherein
physically disrupting the first cell comprises passing a composition comprising the first cell or a composition comprising cell components derived from the disrupted cell through a filter having pores with a smaller size than the size of the first cell twice or more, wherein the first cell is disrupted while passing through pores having a smaller size than the first cell size.

A08. The method of any one of A06 and A07, wherein
the filter has pores with a smaller diameter than the size of the first cell.

A09. The method of any one of A06 to A08, wherein
the average pore diameter of the filter is smaller than the first cell size.

A10. The method of any one of A06 to A09, wherein
the average pore diameter of the filter is 5 to 15 µm.

A11. The method of any one of A06 to A10, wherein
the average pore diameter of the filter is approximately 8 µm.

A12. The method of any one of A01 to A05, wherein
physically disrupting the first cell is achieved by the use of an extruder.

A13. The method of any one of A01 to A05, and A12, wherein
physically disrupting the first cell is achieved by the use of an extruder, and a filter comprised in the extruder has pores whose diameter is smaller than the first cell size.

A14. The method of any one of A01 to A05, A12 and A13, wherein
physically disrupting the first cell is achieved by the use of an extruder, and the average pore diameter of the filter comprised in the extruder is 5 to 15 µm.

A15. The method of any one of A01 to A14, wherein
in (ii), the genomic DNA exposed by the physically disrupting the first cell maintains first cell-specific epigenetic features.

A16. The method of any one of A01 to A15, wherein
the information on the cleavage sites obtained in (iii) is information reflecting the first cell-specific epigenetic features.

A17. The method of any one of A01 to A16, wherein
in (iv), the information on off-target candidates is information reflecting the first cell-specific epigenetic features.

A18. The method of any one of A01 to A17, wherein
the DNA repair mechanism of the cell is broken according to cell disruption, and therefore the cleaved DNA is not repaired.

A19. The method of any one of A01 to A18, further comprising:
confirming a CRISPR/Cas gene editing system subject to off-target prediction, wherein the CRISPR/Cas gene editing system subject to prediction comprises the use of a Cas protein subject to prediction and the use of a guide RNA subject to prediction.

A20. The method of any one of A01 to A18, further comprising:
confirming a CRISPR/Cas gene editing system subject to off-target prediction, wherein the CRISPR/Cas gene editing system subject to prediction comprises the use of a Cas protein subject to prediction and the use of a guide RNA subject to prediction, and the confirmation of the CRISPR/Cas gene editing system subject to prediction is performed before (i).

A21. The method of any one of A19 and A20, wherein
a guide sequence of the first guide RNA has the same sequence as the guide sequence of the guide RNA subject to prediction.

A22. The method of any one of A19 to A21, wherein
the CRISPR/Cas gene editing system subject to prediction comprises the use of a cell subject to prediction, and wherein the first cell and the cell subject to prediction are the same.

A23. The method of any one of A01 to A22, wherein
analyzing the composition to be analyzed in (iii) comprise analyzing the DNA comprised in the composition to be analyzed through sequencing.

A24. The method of any one of A01 to A23, wherein
analyzing the composition to be analyzed in (iii) comprises analyzing the cleaved genomic DNA comprised in the composition to be analyzed through sequencing.

A25. The method of any one of A01 to A22, wherein
analyzing the composition to be analyzed in (iii) comprises analyzing the DNA comprised in the composition to be analyzed through a PCR-based analysis method.

A26. The method of any one of A01 to A22 and A25, wherein
analyzing the composition to be analyzed in (iii) comprises analyzing the genomic DNA comprised in the composition to be analyzed through a PCR-based analysis method.

A27. The method of any one of A01 to A26, wherein
the concentration of the Cas protein comprised in the starting composition is 5000 nM.

A28. The method of any one of A01 to A27, wherein
the concentration of the first cell comprised in the starting composition is 1×10⁷ cells/mL.

A29. The method of any one of A01 to A28, wherein
the obtaining of the composition to be analyzed further comprises:
incubating the composition comprising the disrupted cell components, the Cas protein, and the guide RNA.

A30. The method of any one of A01 to A29, wherein
the obtaining of the composition to be analyzed further comprises:
removing an RNA component of the composition comprising the disrupted cell components, the Cas protein, and the guide RNA.

A31. The method of any one of A01 to A30, wherein
the obtaining of the composition to be analyzed further comprises:
purifying DNA of the composition comprising the disrupted cell components, the Cas protein, and the guide RNA.

### Exemplary embodiments characterized by use of extruder

B01. A method of predicting off-targets that can be occurred in a genome editing process using a CRISPR/Cas gene editing system comprising the following:
(i) loading a starting composition comprising a first Cas protein, a first guide RNA, and a first cell to the first container;
(ii) performing extrusion comprising the following processes using an extruder to obtain a composition to be analyzed:
   (a) applying pressure to the first container to move the components of the starting composition from the first container of the extruder to the second container of the extruder,

   wherein the components of the starting composition pass through a filter with pores, located between the first container of the extruder and the second container of the extruder, by the applied pressure to move from the first container to the second container, and thus the mixture settles in the second container;
   wherein, the first cell which is a component larger than the diameter of pores of the filter is disrupted and pass through the pores of the filter, by the applied pressure, and
   wherein through the physically disrupting the first cell, an environment in which a genomic DNA is able to contact the Cas protein and the guide RNA is created,
   whereby the genomic DNA contacts with a Cas/gRNA complex,
   whereby the genomic DNA is cleaved at one or more cleavage sites; and
(iii) analyzing the composition to be analyzed to obtain information on the cleavage sites; and
(iv) identifying information on off-target candidates from the information on the cleavage sites obtained in (iii) to predict the off-targets occurring in the CRISPR/Cas gene editing system.

B02. The method of B01, wherein the extrusion process in (ii) comprises the following processes:
(a) applying pressure to the first container to move the components of the starting composition from the first container of the extruder to the second container of the extruder, wherein the components of the starting composition pass through a filter with pores, located between the first container of the extruder and the second container of the extruder, by the applied pressure to move from the first container to the second container, and thus the mixture settles in the second container,
(b) applying pressure to the second container to move components of the mixture comprised in the second container from the second container to the first container,
   wherein the components of the mixture comprised in the second container pass through the filter with pores, located between the first container and the second container, by the applied pressure to move from the second container to the first container, and thus a mixture moved from the second container to the first container by passing through the filter by pressure settles in the first container, and
(c) repeatedly performing the processes of (a) and (b) the predetermined number of times,

wherein the predetermined number of times is counted in 0.5 increments, and 0.5 indicates the performance of a single process of (a) or (b), and
wherein the first cell which is larger than the diameter of pores of the filter, pass through the pores of the filter while disrupting by the applied pressure, and
wherein, through the physically disruption of the first cell, an environment where the genomic DNA in the cell can come into contact with the Cas/gRNA complex is created,
whereby the genomic DNA with the Cas/gRNA complex contact,
whereby the genomic DNA is cleaved at one or more cleavage sites.

B03. The method of any one of B01 and B02, wherein
the pressure applied to the first container may be produced through a process of pushing a piston designed to apply pressure to the first container in the direction of the first container and the filter.

B04. The method of B02, wherein
the pressure applied to the first container may be produced through a process of pushing a piston designed to apply pressure to the first container in the direction of the first container and the filter, and
the pressure applied to the second container may be produced through a process of pushing a piston designed to apply pressure to the second container in the direction of the second container and the filter.

B05. The method of any one of B01 to B04, wherein
the information on the cleavage sites comprises one or more of the following:
the location on the genomic DNA for one or more cleavage sites,
cleavage scores for the one or more cleavage sites, and
the number of the cleavage sites.

B06. The method of any one of B01 to B05, wherein
the information on off-target candidates comprises one or more of the following:
the location on the genomic DNA for one or more off-target candidates,
off-target prediction scores for the one or more off-target candidates, and
the number of the predicted off-target candidates.

B07. The method of any one of B01 to B06, wherein
in (ii), a membrane structure comprising a cell membrane is disrupted through the physical disruption of the first cell, and whereby an environment where the Cas/gRNA complex can come into contact with the genomic DNA of the first cell is prepared.

B08. The method of any one of B01 to B07, wherein
in (ii), a membrane structure comprising the nuclear membrane of the first cell is disrupted through the physical disruption of the first cell, and whereby an environment where the Cas/gRNA complex can come into contact with the genomic DNA of the first cell is prepared.

B09. The method of any one of B01 to B08, wherein
the filter comprises pores whose diameter is smaller than the first cell size.

B10. The method of any one of B01 to B09, wherein
the average pore diameter of the filter is 5 to 15 µm.

B11. The method of any one of B01 to B10, wherein
the average pore diameter of the filter is 8 µm.

B12. The method of any one of B02 to B10, wherein
the predetermined number of times is 4 to 7.

B13. The method of any one of B02 to B12, wherein
the predetermined number of times is 5.5.

B14. The method of any one of B01 to B13, wherein
in (ii), the genomic DNA exposed by the physical disruption of the first cell maintains first cell-specific epigenetic features.

B15. The method of any one of B01 to B14, wherein
the information on the cleavage sites obtained in (iii) is information reflecting the first cell-specific epigenetic features.

B16. The method of any one of B01 to B15, wherein
the information on off-target candidates obtained in (iv) is information reflecting the first cell-specific epigenetic features.

B17. The method of any one of B01 to B16, wherein
A DNA repair mechanism of the cell is broken due to the cell disruption, whereby the cleaved DNA is not repaired.

B18. The method of any one of B01 to B17, wherein the method further comprises:
confirming a CRISPR/Cas gene editing system subject to off-target prediction, wherein the CRISPR/Cas gene editing system subject to prediction comprises a use of a Cas protein subject to prediction and a use of a guide RNA subject to prediction.

B19. The method of any one of B01 to B17, wherein the method further comprises:
confirming a CRISPR/Cas gene editing system subject to off-target prediction, wherein the CRISPR/Cas gene editing system subject to prediction comprises a use of a Cas protein subject to prediction and a use of guide RNA subject to prediction, and wherein the confirmation of the CRISPR/Cas gene editing system subject to prediction is performed before (i).

B20. The method of any one of B18 and B19, wherein
a guide sequence of the first guide RNA has the same sequence as the guide sequence of the guide RNA subject to prediction.

B21. The method of any one of B18 to B20, wherein
the CRISPR/Cas gene editing system subject to prediction comprises a use of cell subject to prediction, and wherein the first cell and the cell subject to prediction are the same.

B22. The method of any one of B01 to B21, wherein
(iii) analyzing of the composition to be analyzed comprise analyzing the cleaved genomic DNA comprised in the composition to be analyzed through sequencing.

B23. The method of any one of B01 to B22, wherein
(iii) analyzing the composition to be analyzed comprises analyzing the cleaved genomic DNA comprised in the composition to be analyzed through sequencing.

B24. The method of any one of B01 to B21, wherein
(iii) analyzing the composition to be analyzed comprises analyzing the DNA comprised in the composition to be analyzed through a PCR-based analysis method.

B25. The method of any one of B01 to B21, wherein
(iii) analyzing the composition to be analyzed in (iii) comprises analyzing the cleaved genomic DNA comprised in the composition to be analyzed through a PCR-based analysis method.

B26. The method of any one of B01 to B25, wherein
the concentration of the Cas protein comprised in the starting composition is 5000 nM.

B27. The method of any one of B01 to B26, wherein
the concentration of the first cell comprised in the starting composition is 1×10⁷ cells/mL.

B28. The method of any one of B01 to B27, wherein the method further comprises the following process to obtain the composition to be analyzed:
incubating the composition comprising the disrupted cell components, the Cas protein, and the guide RNA.

B29. The method of any one of B01 to B28, wherein the method further comprises the following process to obtain the composition to be analyzed:
removing RNA component of the composition comprising the disrupted cell components, the Cas protein, and the guide RNA.

B30. The method of any one of B01 to B29, wherein the method further comprises the following process to obtain the composition to be analyzed:
purifying DNA of the composition comprising the disrupted cell components, the Cas protein, and the guide RNA.

### Expected uses of off-target prediction system of the present application

Hereinafter, examples of the expected use of the off-target prediction system of the present application (expectation of the scene in which a person of skill in the art uses an off-target prediction system of the present application) will be described without limitation. The off-target prediction system of the present application (e.g., Extru-seq) is an off-target prediction system characterized by the physical disruption of cells, and a more efficient and more accurate off-target prediction system which has the advantages of a conventional in vitro-based off-target prediction system and the advantages of an in vivo-based off-target prediction system. Accordingly, all of methods of confirming off-target candidates or method of predicting off-targets, which uses the above-described characteristics of the off-target prediction system and performs to achieve the purpose of confirming off-targets that can be possibly occurred in a gene editing process, are comprised as one embodiment of the use or application of the off-target prediction method of the present application, and the following examples do not limit the scope of the present application.

For example, the off-target prediction method (or system) of the present application may be used by a technician or researcher that uses a CRISPR/Cas gene editing system for editing cell genome.

For example, a researcher chooses a gene editing system to be used in cell genome editing. For example, a researcher chooses a CRISPR/Cas gene editing system as a gene editing system to be used in cell genome editing. Further, a researcher may choose cells that are main purpose of genome editing. In the process of selecting a gene editing system to be used for cell genome editing, an in silico-based off-target prediction method may be used to design an appropriate guide sequence. Here, a researcher is to develop a treatment that includes the use of a gene editing system. In the development of a treatment, information on off-targets of the selected gene editing system (particularly, guide RNA) must be confirmed. Based on the selected gene editing system, details of the off-target prediction method of the present application are designed to fit the purpose. By performing the off-target prediction method of the present application, information on off-target candidates that can be possibly occurred in the use of the selected gene editing system is confirmed. Afterward, using the confirmed information on the off-target candidates, information on off-targets that are problematic in the use of the selected gene editing system is confirmed. Specifically, a bona-fide off-target is finally confirmed by verifying candidate off-target sites identified by the off-target prediction system of the present application. In this process, the known off-target prediction methods (in silico, in vitro, and cell-based off-target prediction methods) may be used in combination to find a bona-fide off-target site.

In another example, the off-target prediction system of the present application may be used in the selection process of a gene editing system (particularly, a guide sequence of guide RNA). A researcher produces a guide RNA library comprising various types of guide RNAs. The off-target prediction method is performed on a gene editing system comprising one or more guide RNAs comprised in a guide RNA library. Afterward, based on the result of the off-target prediction method of the present application, a gene editing system that will be used in development or research of treatments is selected. In this process, the known off-target prediction methods (in silico, in vitro, and cell-based off-target prediction methods) may be used in combination to find a bona-fide off-target site.

As described above, the off-target prediction system of the present application may be used in various scenes, and the aspect of uses of the off-target prediction system are not limited to the above-mentioned examples.

### [Examples]

Hereinafter, the invention provided by the present application will be described in further detail with reference to experimental examples or examples. Theses experimental examples merely provided for illustrating the contents disclosed by the present application, and it will be obvious to those of ordinary skill in the art that the scope of the invention disclosed by the specification would not be construed as being limited to the following experimental examples.

### Experimental Examples

### Experimental methods

### Experimental Method 1. Design of promiscuous sgRNAs

Candidate target sequences comprising protospacer-adj acent motif (NGG PAM) located in mouse genome (mm 10) *PCSK9* and *Albumin* genes were extracted by Cas-Designer (refer to the document [Park, Jeongbin, Sangsu Bae, and Jin-Soo Kim. "Cas-Designer: a web-based tool for choice of CRISPR-Cas9 target sites." Bioinformatics 31.24 (2015): 4014-4016.]). The extracted sequences were aligned to the human genome (hg19). Among the extracted sequences, when the extracted sequences were aligned to a human genome, sequences in which the number of targets with 0 mismatch was 1 or more were selected. The selected candidates were analyzed by Cas-OFFinder (refer to the document [Bae, Sangsu, Jeongbin Park, and Jin-Soo Kim. "Cas-OFFinder: a fast and versatile algorithm that searches for potential off-target sites of Cas9 RNA-guided endonucleases." Bioinformatics 30.10 (2014): 1473-1475.]). Candidates with various sets of related sequences comprising various numbers of mismatches (0 to 5 mismatches per site) widely distributed all over human and mouse genomes, were selected as targets. Information on the target and guide sequences of promiscuous sgRNA used in subsequent experiments is as follows:
Target sequence of single-stranded guide RNA (sgRNA) targeting mouse *PCSK9* (excluding NGG PAM):
   AGGTGGGAAACTGAGGCTT (SEQ ID NO: 25)
Target sequence of sgRNA targeting mouse *Albumin* (excluding NGG PAM):
   ACATGCATATGTATGTGTG (SEQ ID NO: 26)

As described below in the experimental results, these sgRNAs perfectly matched a target sequence present on a human genome (however, on the human genome, *PCSK9* and *Albumin* loci are not targets). Although the target and guide sequences targeted loci other than *PCSK9* or *Albumin* on a human genome, for convenience, they were indicated as human *PCSK9*-targeting sgRNA (sgRNA targeting human *PCSK9*) and human *Albumin-targeting* sgRNA (sgRNA targeting human *Albumin*)*.*

That is, a target sequence of the promiscuous sgRNA referred as a human *PCSK9-*targeting sgRNA is the same as a target sequence of mouse *PCSK9*-targeting sgRNA (sgRNA targeting mouse *PCSK9*)*.* The target sequence of the human *PCSK9*-targeting sgRNA is as follows (excluding NGG PAM): AGGTGGGAAACTGAGGCTT (SEQ ID NO: 25).

A target sequence of the promiscuous sgRNA referred as human *Albumin-targeting* sgRNA is the same as a target sequence of mouse *Albumin-targeting* sgRNA (sgRNA targeting mouse *Albumin*)*.* The target sequence of the human *Albumin-targeting* sgRNA is as follows (excluding NGG PAM): ACATGCATATGTATGTGTG (SEQ ID NO: 26).

### Experimental Method 2. Construction of plasmid for expressing sgRNA and Cas9

Streptococcus pyogenes Cas9 sequence (refer to the document [Cho, Seung Woo, et al. "Targeted genome engineering in human cells with the Cas9 RNA-guided endonuclease." Nature biotechnology 31.3 (2013): 230-232.]) and the designed promiscuous sgRNA sequences (*Albumin-*targeting sgRNA and *PCSK9*-targeting sgRNA sequences) were cloned to the backbone of AAV plasmids used in the previous research (refer to the document [Kim, Eunji, et al. "In vivo genome editing with a small Cas9 orthologue derived from Campylobacter jejuni." Nature communications 8.1 (2017): 1-12.]) to produce Cas9 (pAAV-Cas9) and sgRNA (pAAV-Albumin and pAAV-PCSK9) expression vectors. The Cas9 expression was performed under the control of a CMV promoter, and the sgRNA expression was performed under the control of a U6 promoter. Guide sequences targeting *FANCF, VEGFA,* and *HBB* genes were cloned in a pRG2 vector (Addgene #104174).

### Experimental Method 3. GUIDE-seq

Human HEK293T cells (ATCC, Cat# CRL-3216) and mouse NIH-3T3 cells (ATCC, Cat# CRL-1658) were maintained in Dulbecco's modified eagle medium (DMEM) supplemented with 10% fatal bovine serum (FBS) and 1% penicillin-streptomycin under conditions of 5% CO2 and 37 °C. The HEK293T and NIH3T3 cells were subcultured every 72 hours to maintain 80% confluency. For GUIDE-seq, 2×10⁵ HEK293T cells were transfected with a sgRNA-expressing plasmid (500 ng, pAAV-Albumin or pAAV-PCSK9), a Cas9-expressing plasmid (500 ng, p3s-Cas9HC; Addgene plasmid #43945), and 5 pmol dsODN using lipofectamine 2000. 2×10⁵ NIH-3T3 cells were transfected with a sgRNA-expressing plasmid (250 ng, pAAV-Albumin or pAAV-PCSK)), a Cas9-expressing plasmid (500 ng, p3s-Cas9HC; Addgene plasmid #43945), and 100 pmol dsODN using an Amaxa P3 electroporation kit (V4XP-3032; program EN-158). The transfected cells were transferred to 24-well plates containing the pre-cultured DMEM (1mL/well) at 37 °C. After 72 hours, genomic DNA was isolated using a QIAamp DNA mini kit (Qiagen).

Human HeLa cells (ATCC, Cat# CCL-2) were maintained in a DMEM supplemented with 10% FBS and 1% penicillin-streptomycin under conditions of 5% CO2 and 37 °C. The HeLa cells were subcultured every 72 hours to maintain 80% confluency. For GUIDE-seq, 2×10⁵ HeLa cells were transfected with a sgRNA-expressing plasmid (500 ng, pRG2-FANCF, pRG2-VEGFA or pRG2-HBB), a Cas9-expressing plasmid (500 ng, p3s-Cas9HC; Addgene plasmid #43945), and 25 pmol dsODN using Amaxa 4D-nucleofector (V4XC-1024; program CN-114). The transfected cells were transferred to a 24-well plate containing pre-cultured DMEM (1 mL/well) at 37 °C. After 72 hours, genomic DNA was isolated using a QIAamp DNA mini kit (Qiagen).

1000 nm of the purified DNA was fragmented using a Covaris system (duty factor: 10%, PIP: 50, cycles per burst: 200, time: 50s, temperature: 20 °C), and purified using Ampure XP beads (A63881). A sequencing library was produced from DNA using a NEBNext^{®} Ultra^{™} II DNA Library Prep Kit for Illumina (E7546L) according to a manufacturer's protocol. Subsequently, the region of the library containing a dsODN sequence was amplified using a dsODN-specific primer and sequenced using Miseq (Illumina, TruSeq HT kit). The other procedures were the same as described in previous studies (refer to the document [Tsai, Shengdar Q., et al. "GUIDE-seq enables genome-wide profiling of off-target cleavage by CRISPR-Cas nucleases." Nature biotechnology 33.2 (2015): 187-197.]). For data analysis, GUIDE-seq (1.0.2; https://pypi.org/project/guide-seq/) compatible with Python 3 was used.

### Experimental Method 4. Construction of plasmids for sgRNA transcription and in vitro transcription reactions

To improve the yield and accuracy of sgRNA transcription, the inventors of the present application modified the previously described method (refer to the document [Kim, Daesik, Beum-Chang Kang, and Jin-Soo Kim. "Identifying genome-wide off-target sites of CRISPR RNA-guided nucleases and deaminases with Digenome-seq." Nature Protocols 16.2 (2021): 1170-1192.]). Briefly, the sgRNA template was produced by annealing two complementary oligonucleotides, followed by PCR amplification. BamHI, BsaI, and KpnI restriction sites were attached at an end of the sgRNA template through second PCR. The tailed sgRNA template was inserted into a pUC19 plasmid digested with BamHI and Kpnl. The sgRNA-encoding plasmid was linearized by Bsal, resulting in producing an appropriate sgRNA terminal sequence. The linearized plasmid was incubated in a reaction butter (NEB, B9012S) containing 14 mM MgCl₂ (NEB, B0510A), 10 mM DTT (Sigma, 43816), 0.02 U/µl yeast inorganic pyrophosphatase (NEB, M2403L), 1 U/µl murine RNase inhibitor (NEB, M0314L), 4 mM ATP (NEB, N0451AA), 4 mM GTP (NEB, N0452AA), 4 mM UTP (NEB, N0453AA), and 4 mM CTP (NEB, N0454AA) together with 7.5U/µl T7 RNA polymerase (NEB, M0251L) for 8 hours at 37 °C. The yeast inorganic phosphatase was comprised to enhance sgRNA synthesis. After the reaction, the mixture was mixed and incubated with DNase I to remove the DNA template; and then the transcribed sgRNA was purified using a PCR purification kit (Favorgen, #FAGCK001-1).

### Experimental Method 5. Digenome-seq

Genomic DNAs were purified from HEK293T cells (ATCC, Cat# CRL-3216) and NIH-3T3 cells (ATCC, Cat# CRL-1658) using a DNeasy blood & tissue kit (Qiagen). The two types of genomic DNAs (10 µg) were incubated in 1 mL of a reaction solution containing an NEB3 buffer [100 mM NaCl, 50 mM Tris-HCl, 10 mM MgCl2, 100 µg/mL bovine serum albumin (BSA), at pH 7.9] together with a Cas9 protein (10 µg) and *Albumin* or *PCSK9-*targeting sgRNA (10 µg each) (8 hrs, 37 °C). The digested genomic DNA was treated with RNase A(50 µg/mL, Qiagen) for 10 minutes to degrade sgRNA, and purified using a DNeasy blood & tissue kit (Qiagen).

Genomic DNA (1 µg) was fragmented to 300 bp range using a Covaris system (Life Technologies) and blunt-ended using an End Repair Mix (Thermo Fischer). The fragmented DNA was ligated to an adaptor to produce a library, and then applied to WGS using a HiSeq X Ten Sequencer (Illumina) in Macrogen. The WGS was performed at a sequencing depth of 30 to 40x. A DNA cleavage site was identified using Digenome 1.0 program (refer to the document [Park, Jeongbin, et al. "Digenome-seq web tool for profiling CRISPR specificity." Nature methods 14.6 (2017): 548-549.]).

### Experimental Method 6. In silico prediction of off-target sites

hg19 genome-wide candidate off-target sites with fewer than 7 mismatches with the selected sgRNAs were obtained using Cas-OFFinder. CROP scores (heuristic scores that indicate if the candidate off-target sites would be edited) were computed using a CROP prediction model and optimized parameters (https://github.com/vaprilyanto/crop) based on the previous paper ( [Liu, Qiaoyue, et al. "Deep learning improves the ability of sgRNA off-target propensity prediction." BMC bioinformatics 21.1 (2020): 1-15.]). CFD scores (percent activity values provided in a matrix of penalties based on mismatches of each possible type at each position within the guide RNA sequence) were calculated using a "crisprScore" R package (refer to the document [Doench, John G., et al. "Optimized sgRNA design to maximize activity and minimize off-target effects of CRISPR-Cas9." Nature biotechnology 34.2 (2016): 184-191.]). For two calculations, a GX19 (GACATGCATATGTATGTGTG (SEQ ID NO: 27) for *Albumin* and GAGGTGGGAAACTGAGGCTT (SEQ ID NO: 28) for *PCSK9)* sgRNA sequence and a X20 target sequence were used.

### Experimental Method 7. Extru-seq

To prepare Extru-seq, the transcribed sgRNAs were refolded in a 1X NEBuffer 3.1 reaction buffer (100 mM NaCl, 50 mM Tris-HCl, 10 mM MgCl2, 100 µg/mL BSA, at pH 7.9). After heating the sgRNA for 2 minutes at 98 °C, its temperature was lowered at a speed of 0. 1°C/s until reaching 20 °C. To reduce reaction inhibition by a high concentration glycerol, a Cas9 buffer (10 mM Tris-HCl, 0.15 M NaCl, 50% glycerol, at pH 7.4) was exchanged with an elution buffer (100 mM NaCl, 50 mM Tris-HCl, 10 mM MgCl2, at pH 8.0). Buffer exchange was performed using a 10K Amicon^{®} Ultra-15 centrifugal filter (Millipore).

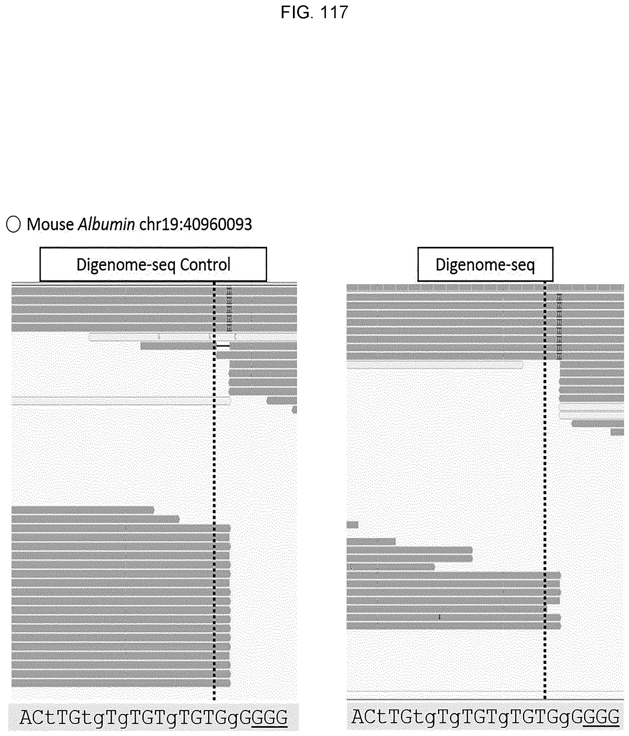

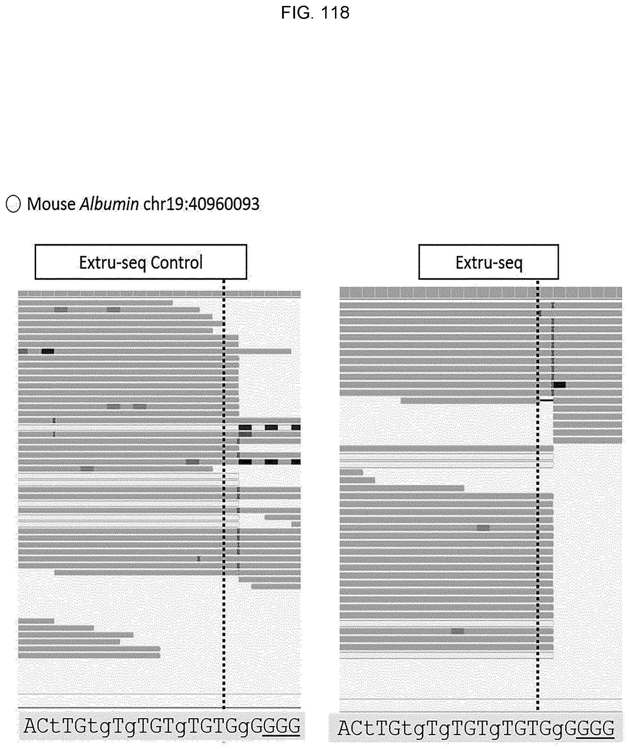

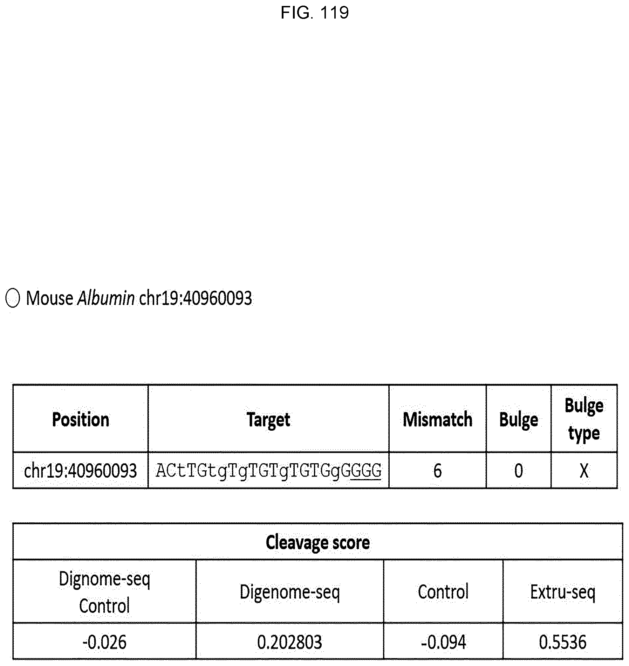

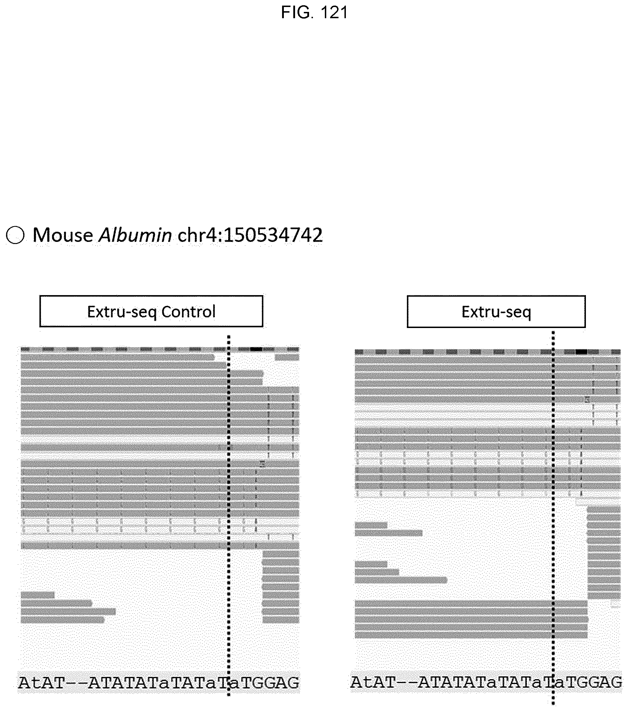

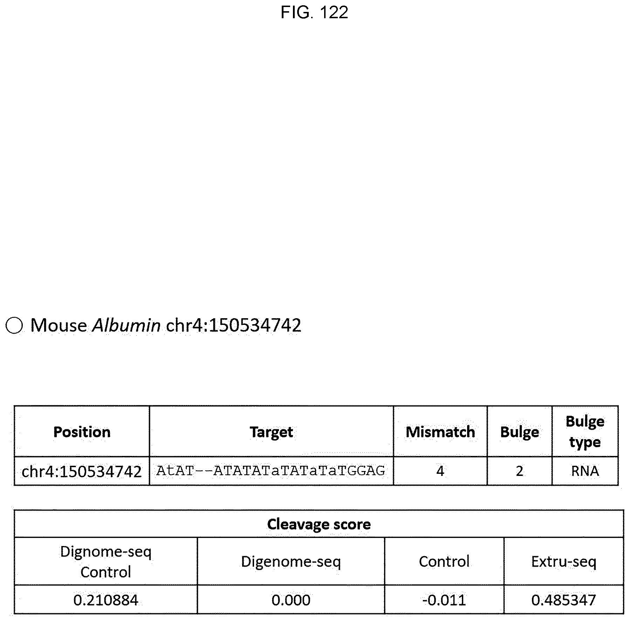

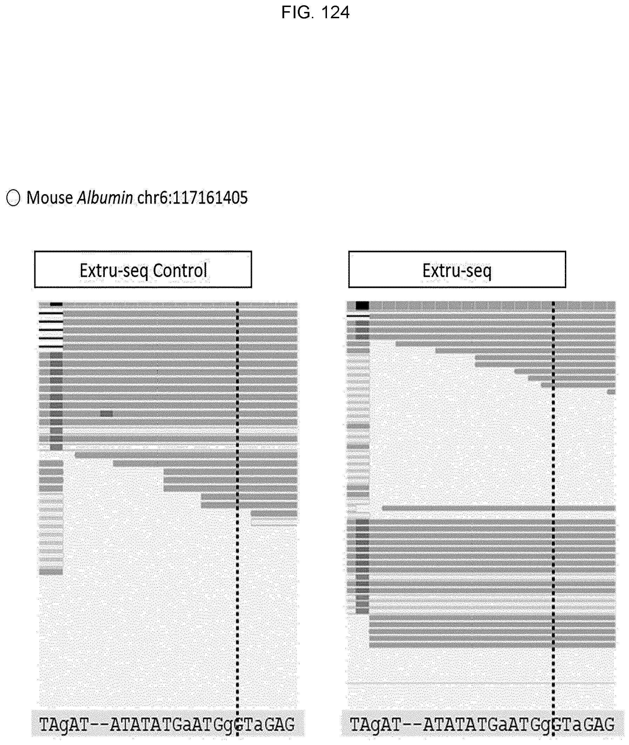

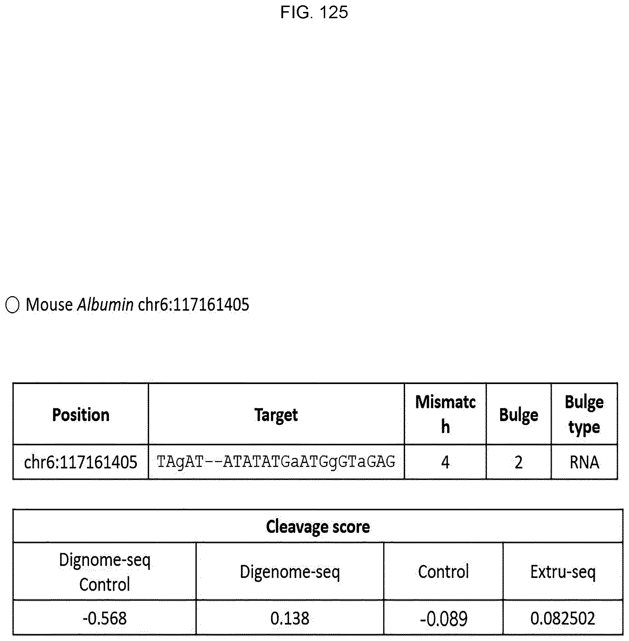

Each type of HEK293T (ATCC, Cat# CRL-3216), NIH-3T3 (ATCC, Cat# CRL-1658), and HeLa (ATCC, Cat# CCL-2) cells were harvested with 0.25% trypsin-EDTA, and human bone marrow mesenchymal stem cell (BM-MSCs; Lonza, Cat# PT-2501) were harvested with 0.05% trypsin-EDTA. The harvested cells were resuspended in Dulbecco's phosphate-buffered saline (PBS). The buffer-exchanged Cas9 (800 mg) and the refolded sgRNA (530 µg) were pre-cultured at room temperature for 10 minutes to form an RNP complex (For multiplex Extru-seq, the buffer-exchanged Cas9 (800 mg) and five different refolded sgRNAs (106 µg each) were used). 1×10⁷ cells were mixed with 5000 nM RNP complexes in 1 mL 1X NEBuffer 3.1 reaction buffer (100 mM NaCl, 50 mM Tris-HCl, 10 mM MgCl2, 100 µg/mL BSA, at pH 7.9). To perform Extru-seq in the presence of SCR7, SCR7 pyrazine (Sigma, SML1546) (1 µM) was added. After gently pipetting, suspended cells were extruded 11 times through an 8-µm pore-sized polycarbonate membrane filter (Whatman) using a mini extruder (Avanti Polar Lipids). The extruded sample was incubated at 37 °C for 16 hours. To remove sgRNA and RNA, RNase A (2 mg/mL) was added, and then genomic DNA was purified from the extruded sample using a FavorPrep Blood Genomic DNA Extraction mini kit (Favorgen, #FAGCK001-2). Whole genome sequencing (WGS) was performed at a sequencing depth of 30 to 40x. WGS was performed in Macrogen using Nova-seq equipment according to a manufacturer's standard protocol. DNA cleavage sites were identified using the Digenome-seq standalone program (http://www.rgenome.net/digenome-js/standalone). Analysis filtering options were as follows: a minimum depth of 10, a minimum score of 0.05, and a minimum ratio of 0.01; and the other options were the default. Further, the inventors of the present application, as developers of a new tool, checked all the sites identified by Extru-seq using an integrative genomic viewer (IGV). Some loci looked like false positive candidates (i.e., non-cleavage site according to IGV (refer to FIGS. 117 to 125)). The false positives (non-cleavage sites) identified using IGV were treated as negative and excluded from analysis. FIGS. 117 to 125 show false positive off-target sites that are manually excluded from Digenome-seq and Extru-seq WGS data. They were visualized using IGV. These false positives were observed in Digenome-seq. Related bam files are available at NCBI Bioproject (https://www.ncbi.nlm.nih.gov/bioproject/) under accession number PRJNA796642.

Referring to FIGS. 117 to 119, the sequence ACtTGtgTgTGTgTGTGgGGGG (SEQ ID NO: 49) is disclosed. In these drawings, mismatches are indicated by small letters, and bulge (if present) is indicated by a dash. In these drawings, the PAM sequence is underlined.

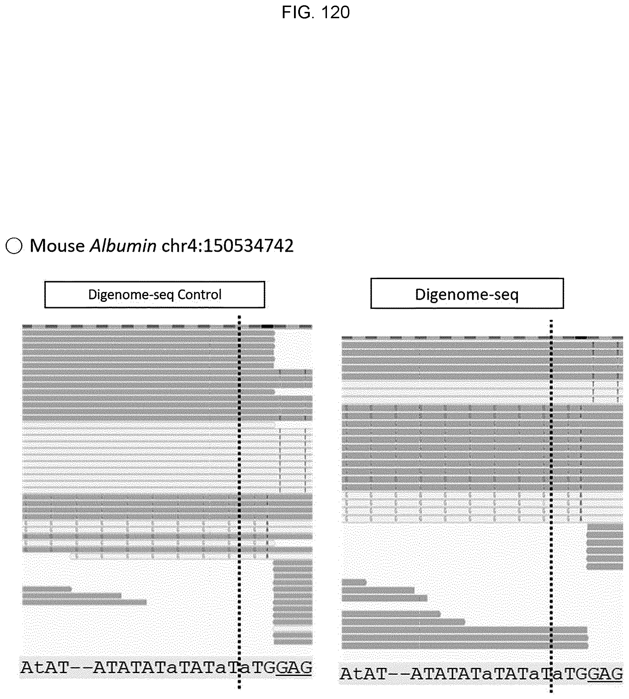

Referring to FIGS. 120 to 122, the sequence AtATATATATaTATaTaTGGAG (SEQ ID NO: 50) (bulge-related marks are omitted) is disclosed. In these drawings, mismatches are indicated by small letters, and bulge (if present) is indicated by a dash. In these drawings, the PAM sequence is underlined.

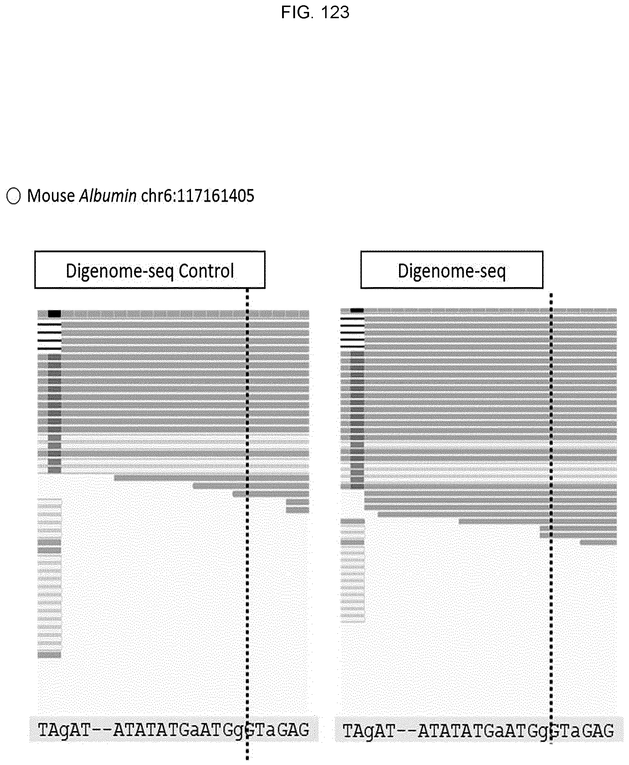

Referring to FIGS. 123 to 125, the sequence TAgATATATATGaATGgGTaGAG (SEQ ID NO: 51) (bulge-related marks are omitted) is disclosed. In these drawings, mismatches are indicated by small letters, and bulge (if present) is indicated by a dash. In these drawings, the PAM sequence is underlined.

### Experimental Method 8. Assignment of non-target results of Digenome-seq and Extru-seq to CAS-OFFinder results

Unlike GUIDE-seq and CAS-OFFinder, the standalone Digenome-seq program does not have an sgRNA:off-target alignment function that provides information on the number of mismatches and bulge type (DNA or RNA) between guide and off-target sites. A web version of the Digenome-seq analysis tool (http://www.rgenome.net/digenome-js/#!) has a optional alignment function with an alignment score which does not provide any information on the number of mismatches or type of bulge. Instead, the inventors of the present application used CAS-OFFinder to identify off-target sites with up to 7 mismatches and 2 bulges related to the target sequence. The positions of off-target candidates identified by Digenome-seq and Extru-seq were compared with those identified by CAS-OFFinder. The information on mismatches and bulge type obtained from CAS-OFFinder was able to be assigned to the loci identified by Digenome-seq and Extru-seq.

### Experimental Method 9. Validation of candidate offtarget sites using human cell lines

Each of human HEK293T and HeLa cells were maintained in DMEM supplemented with 10% FBS (ATCC, CRL-3216) and 1% penicillin-streptomycin at 37 °C in the presence of 5% CO2. To determine indel frequencies at candidate off-target sites, each of 2×10⁵ HEK293T cells and 8×10⁴ HeLa cells were transfected with sgRNA-expressing plasmids (500 ng, pAAV-Albumin, pAAV-PCSK9, pRG2-HBB, pRG2-FANCF or pRG2-VEGFA) and Cas9-expressing plasmids (500 ng, pAAV-Cas9 or p3s-Cas9HC; Addgene plasmid #43945) using lipofectamine 2000 (vendor, amount). After incubating the cells at 37 °C for 3 days, genomic DNA was prepared using a FavorPrep Blood Genomic DNA Extraction Mini Kit (Favorgen, #FAGCK001-2). Deep sequencing was then used to analyze target sites and potential off-target sites. Deep sequencing libraries were produced by PCR. TruSeq HT dual index primers were used to label each sample. Paired-end sequencing was performed on pooled libraries using MiSeq (Illumina). Specifically, several targets were combined by PCR performed using primers with different indices, and then subjected to deep sequencing analysis.

Deep sequencing data are available at NCBI Bioproject (https://www.ncbi.nlm.nih.gov/bioproject/) under accession number PRJNA796642. To determine whether the target was validated or false, the inventors of the present application used the following criteria, which were used in EDITAS Medicine (refer to the document [Maeder, Morgan L., et al. "Development of a gene-editing approach to restore vision loss in Leber congenital amaurosis type 10." Nature medicine 25.2 (2019): 229-233.]). First, to be validated, the indel of the sample must be higher than 0.1%. Second, a treated/control ratio must be higher than 2. The validation results for the off-target candidates through deep sequencing are shown in detail in Table 1. Mismatches of off-target candidates, which are subject to validation, with the targets were indicated in small letters. Regarding human *PCSK9* of Table 1, SEQ ID NOs: 74 to 132 were assigned to the off-target sequences 1 to 59 disclosed in human *PCSK9,* in the order of their disclosure. Regarding human *Albumin* of Table 1, SEQ ID NOs: 133 to 174 were assigned to the off-target sequences 1 to 42 disclosed in human *Albumin,* in the order of their disclosure. Regarding mouse *PCSK9* in Table 1, SEQ ID NOs: 175 to 211 were assigned to the off-target sequences 1 to 37 disclosed in Mouse *PCSK9,* in the order of their disclosure. Regarding mouse *Albumin* in Table 1, SEQ ID NOs: 212 to 249 were assigned to the off-target sequences 1 to 38 disclosed in mouse *Albumin,* in the order of their disclosure. Regarding human *HBB* in Table 1, SEQ ID NOs: 250 to 293 were assigned to the off-target sequences 1 to 44 disclosed in human *HBB,* in the order of their disclosure. Regarding human *VEGFA* in Table 1, SEQ ID NOs: 294 to 343 were assigned to the off-target sequences 1 to 50 disclosed in human *VEGFA,* in the order of their disclosure. Regarding human *FANCF* in Table 1, SEQ ID NOs: 344 to 383 were assigned to the off-target sequences 1 to 40 disclosed in human *FANCF,* in the order of their disclosure. Table 1 is shown below.

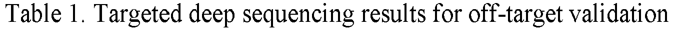

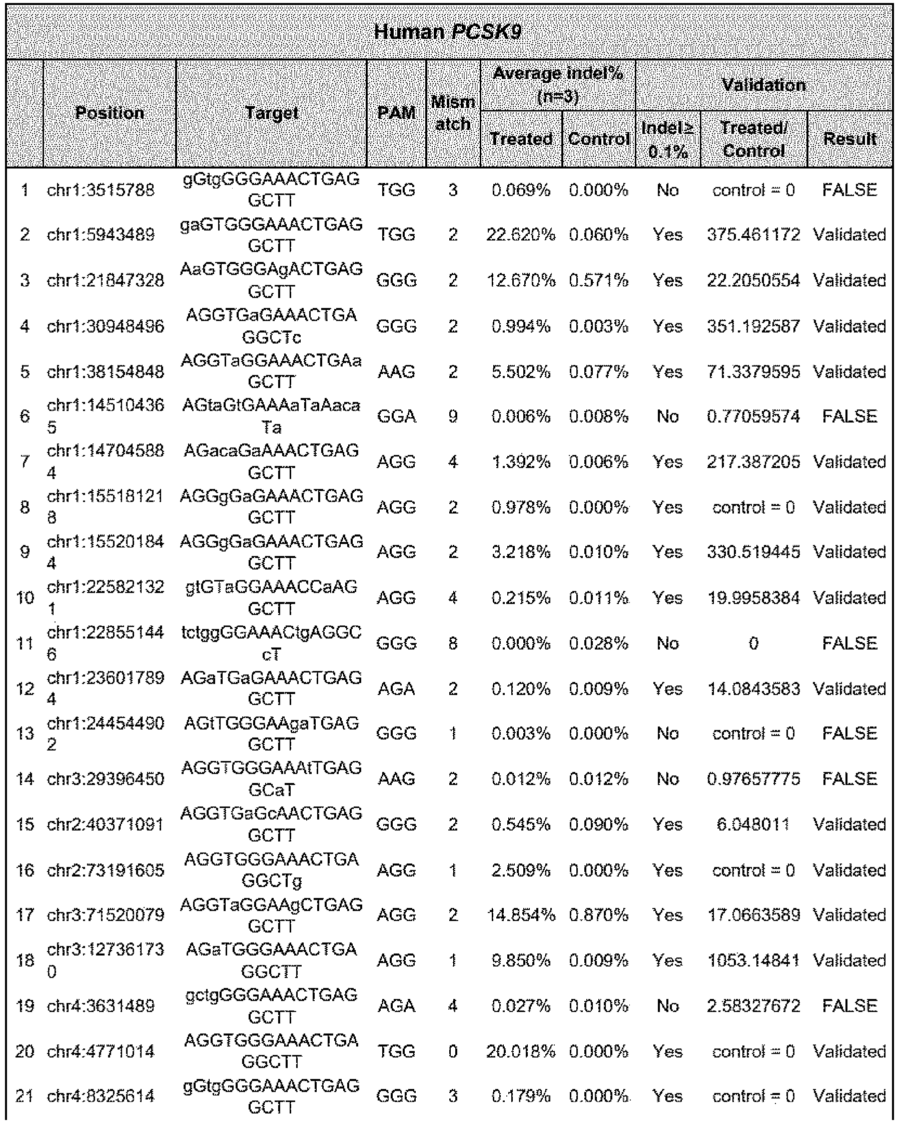

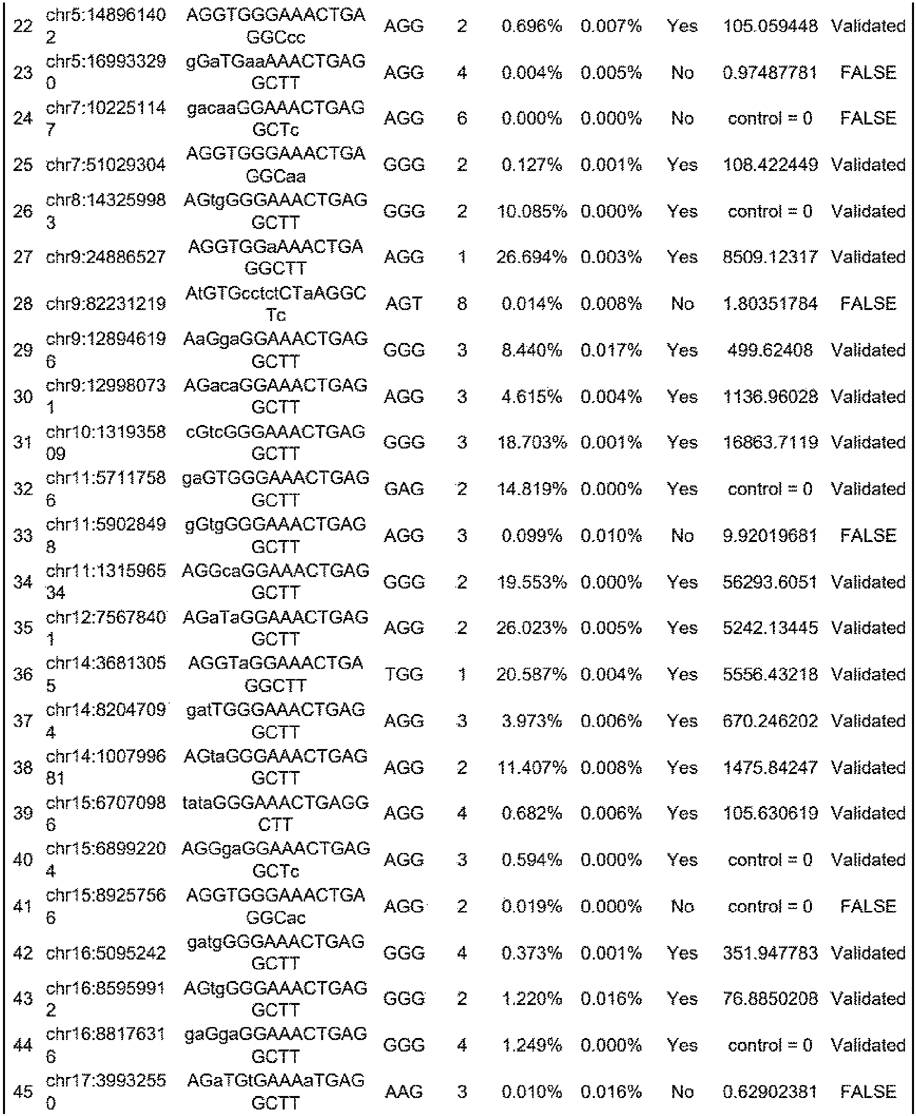

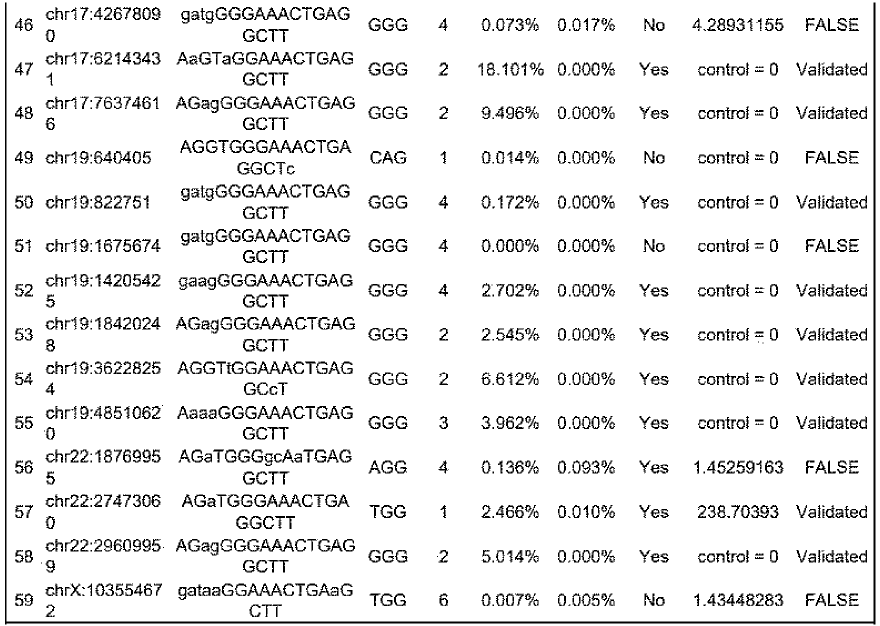

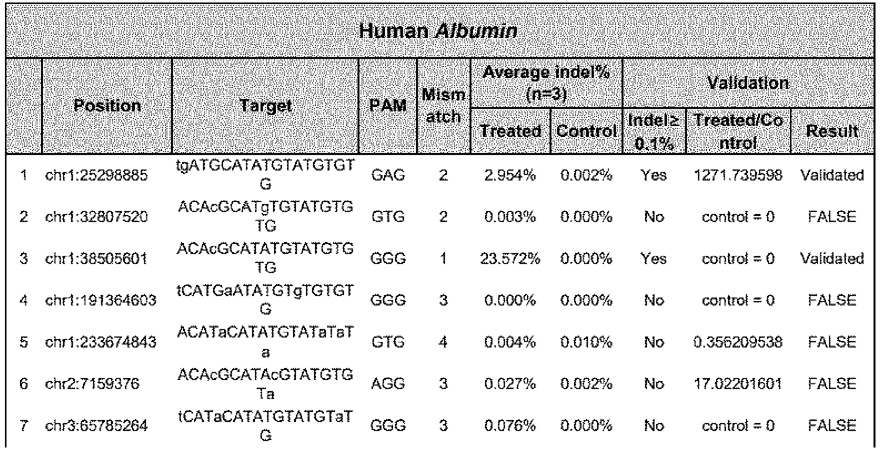

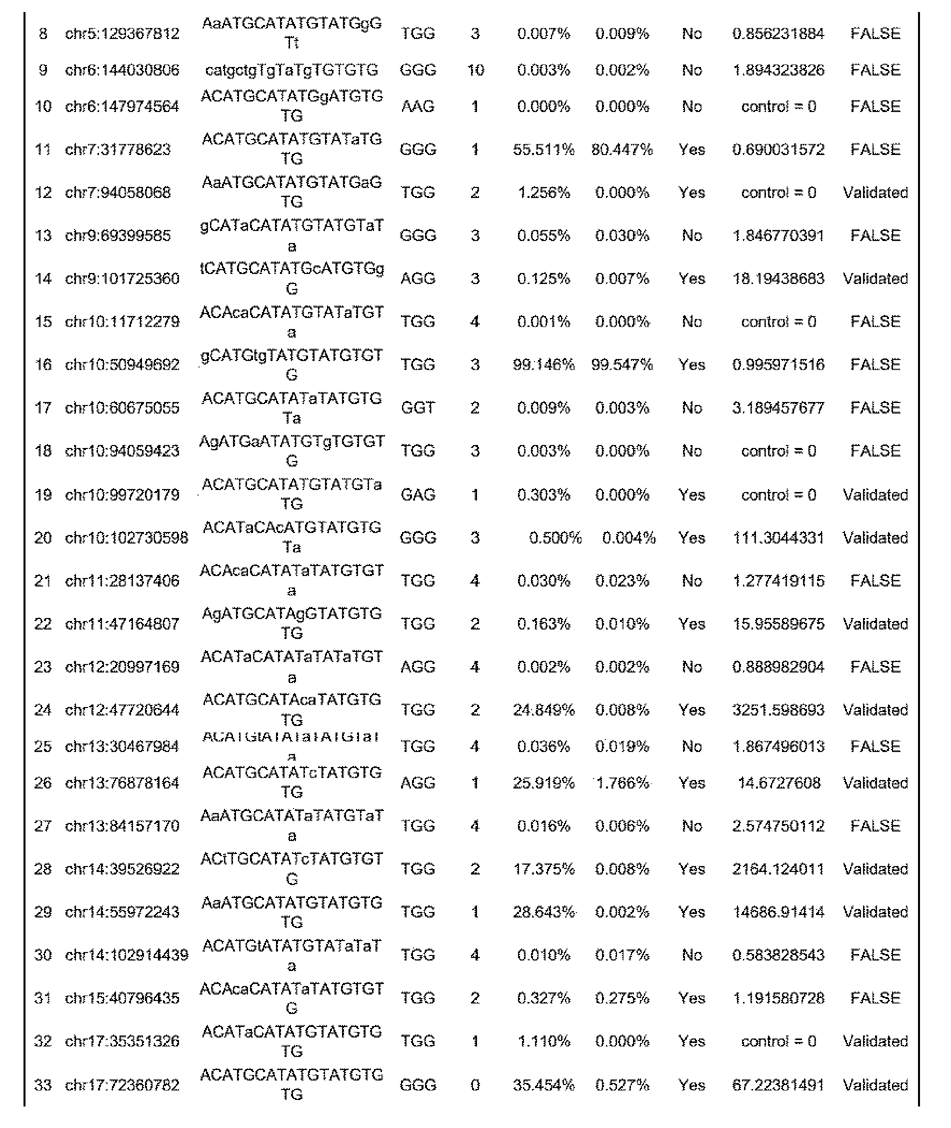

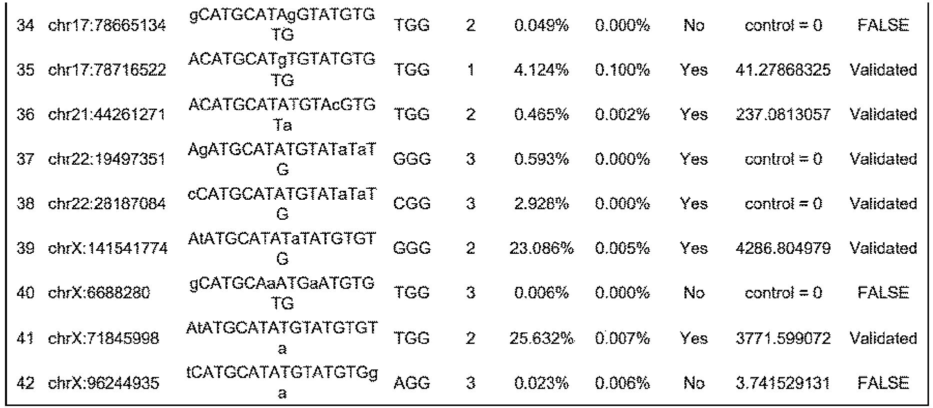

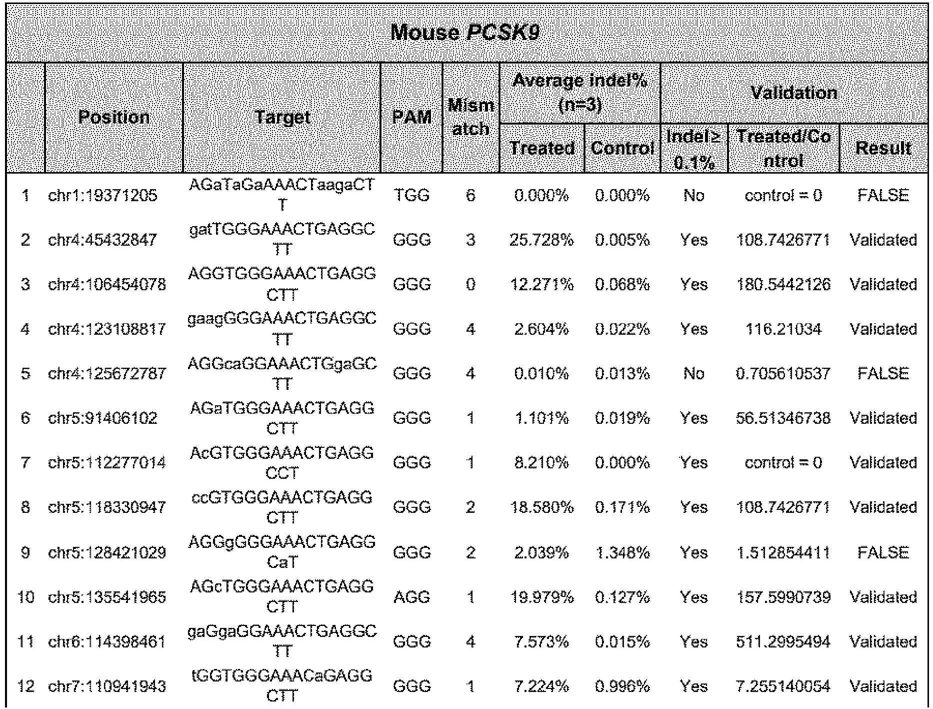

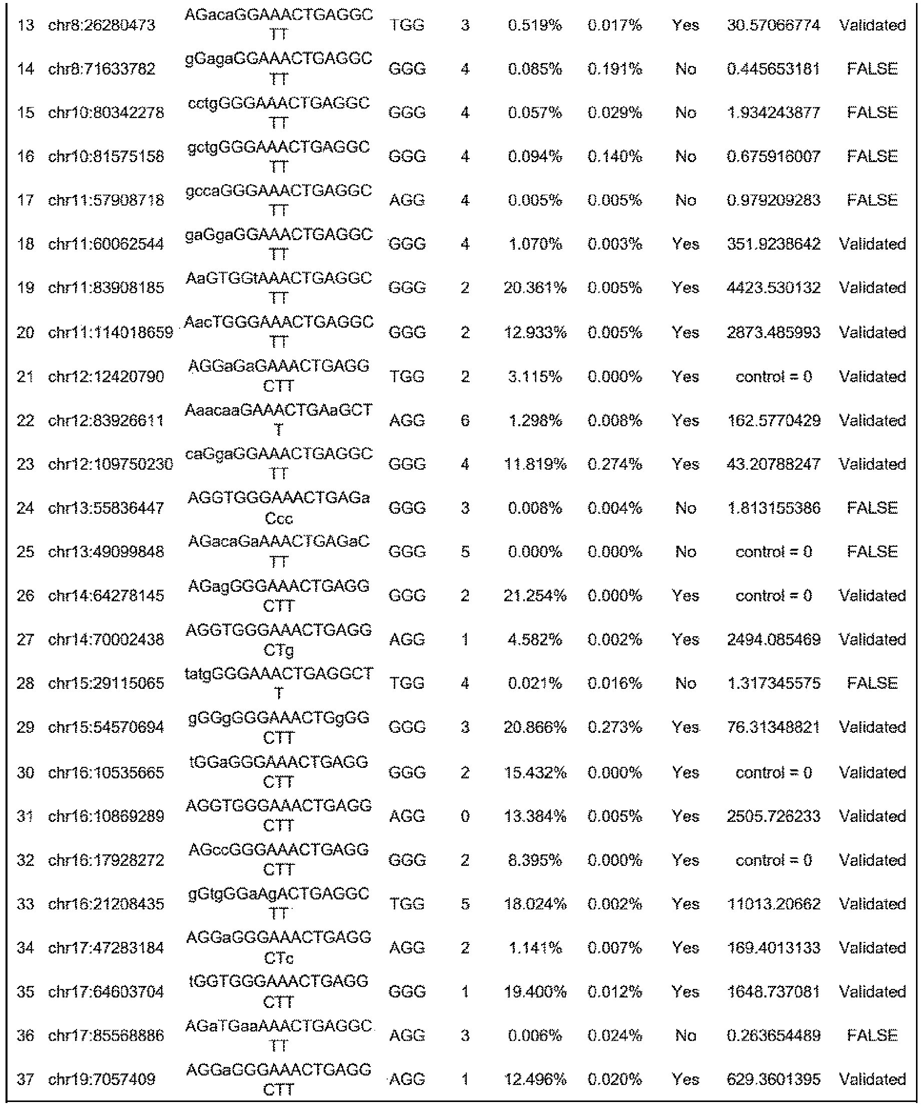

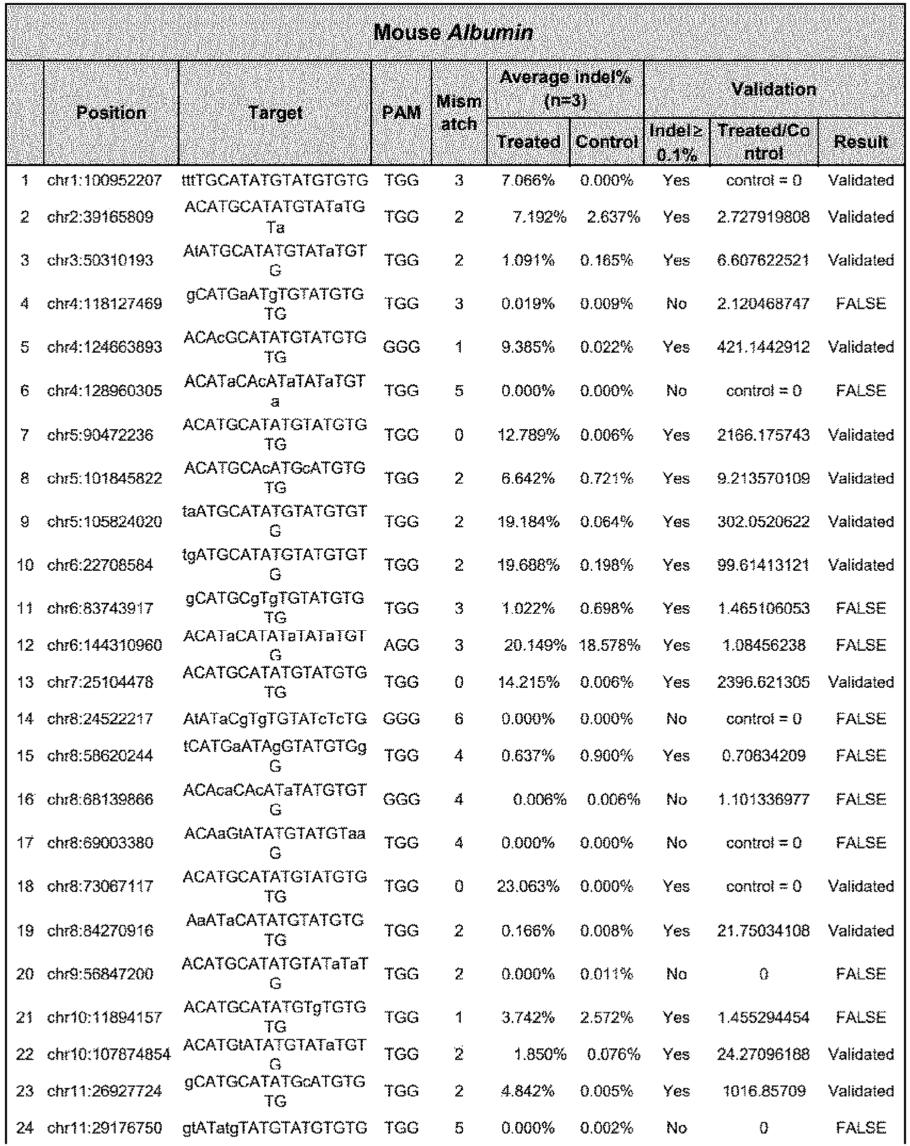

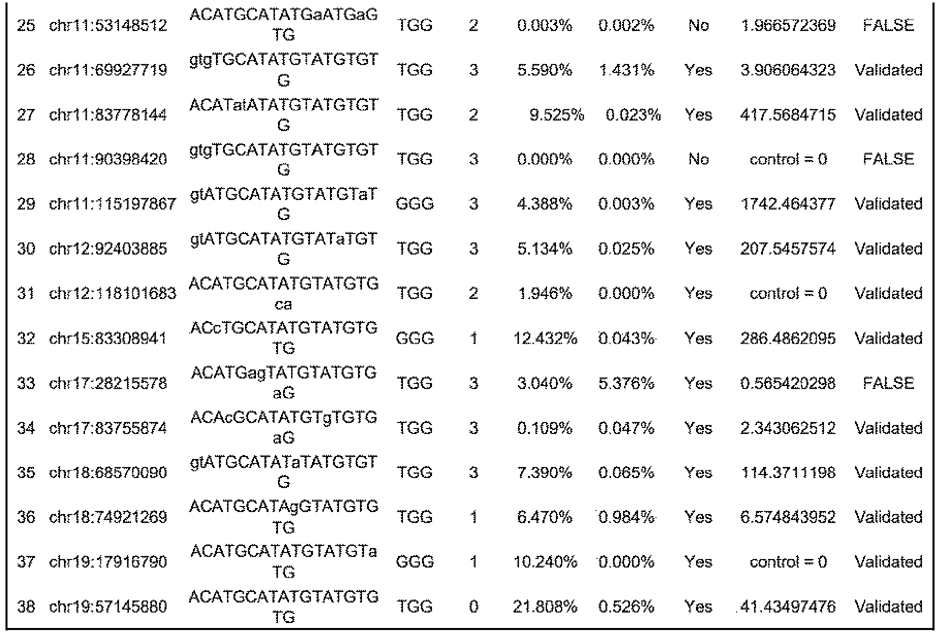

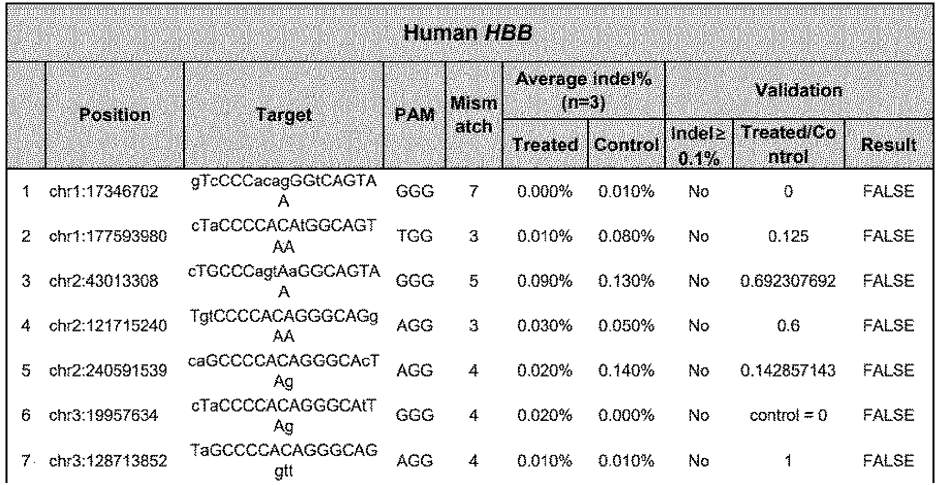

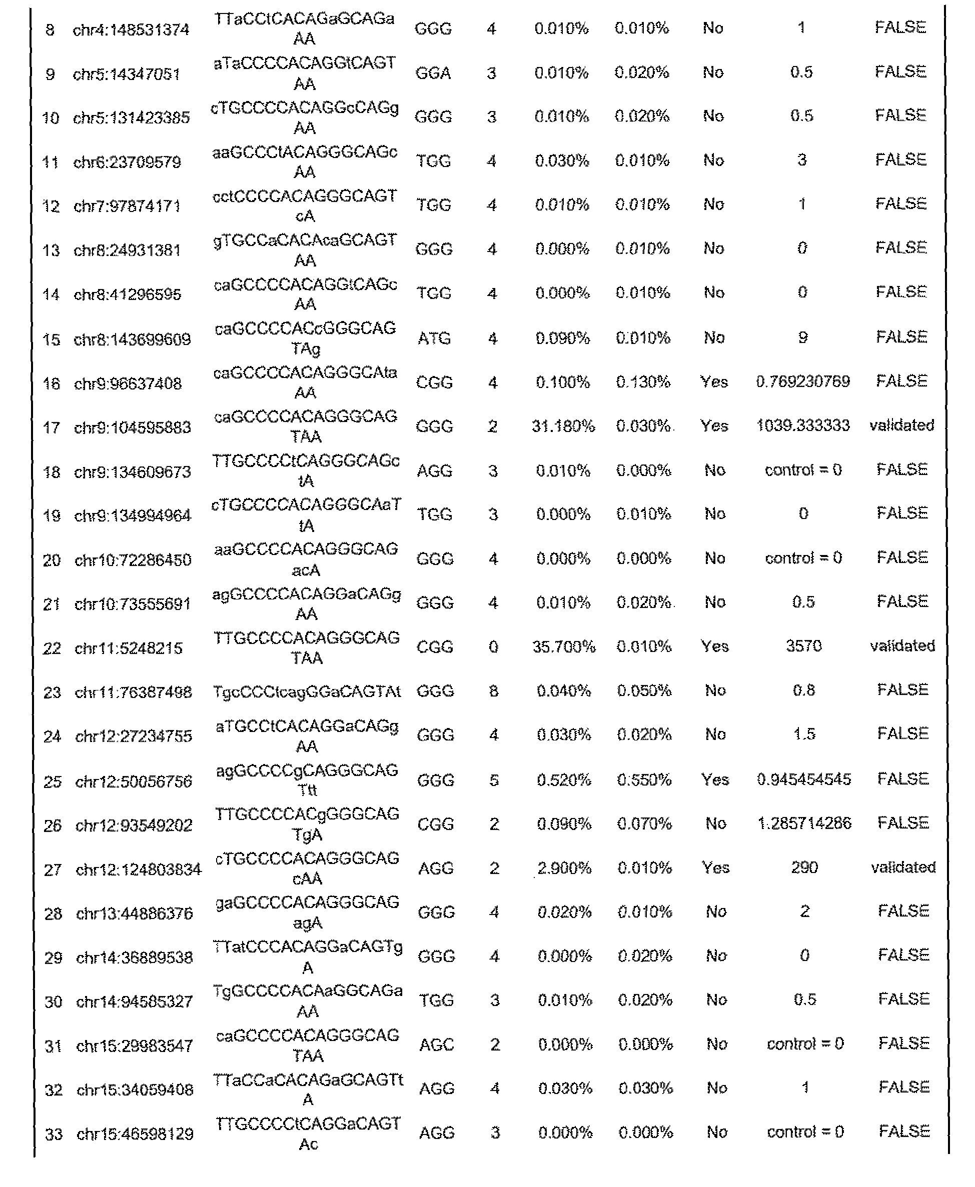

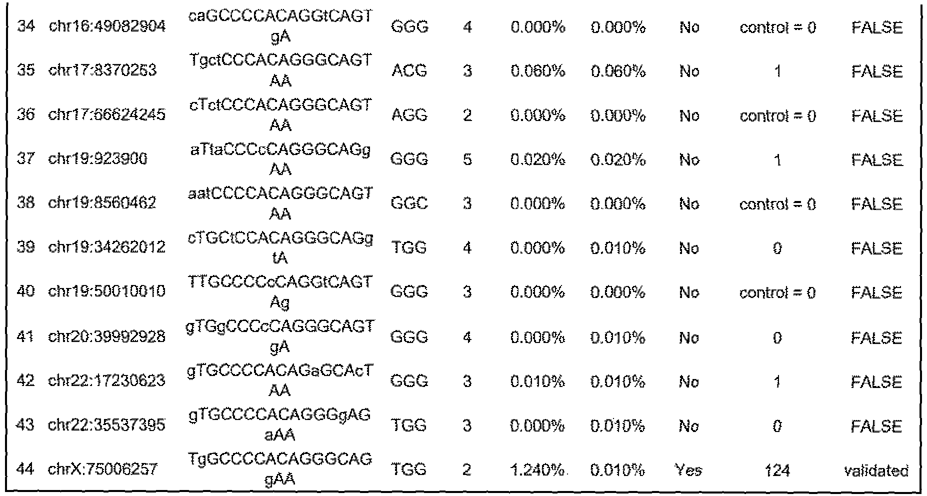

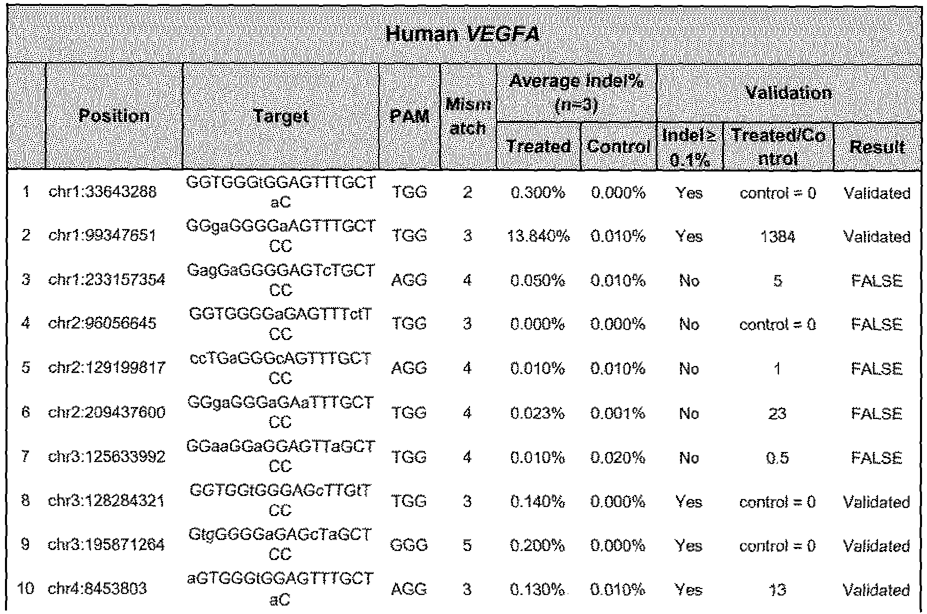

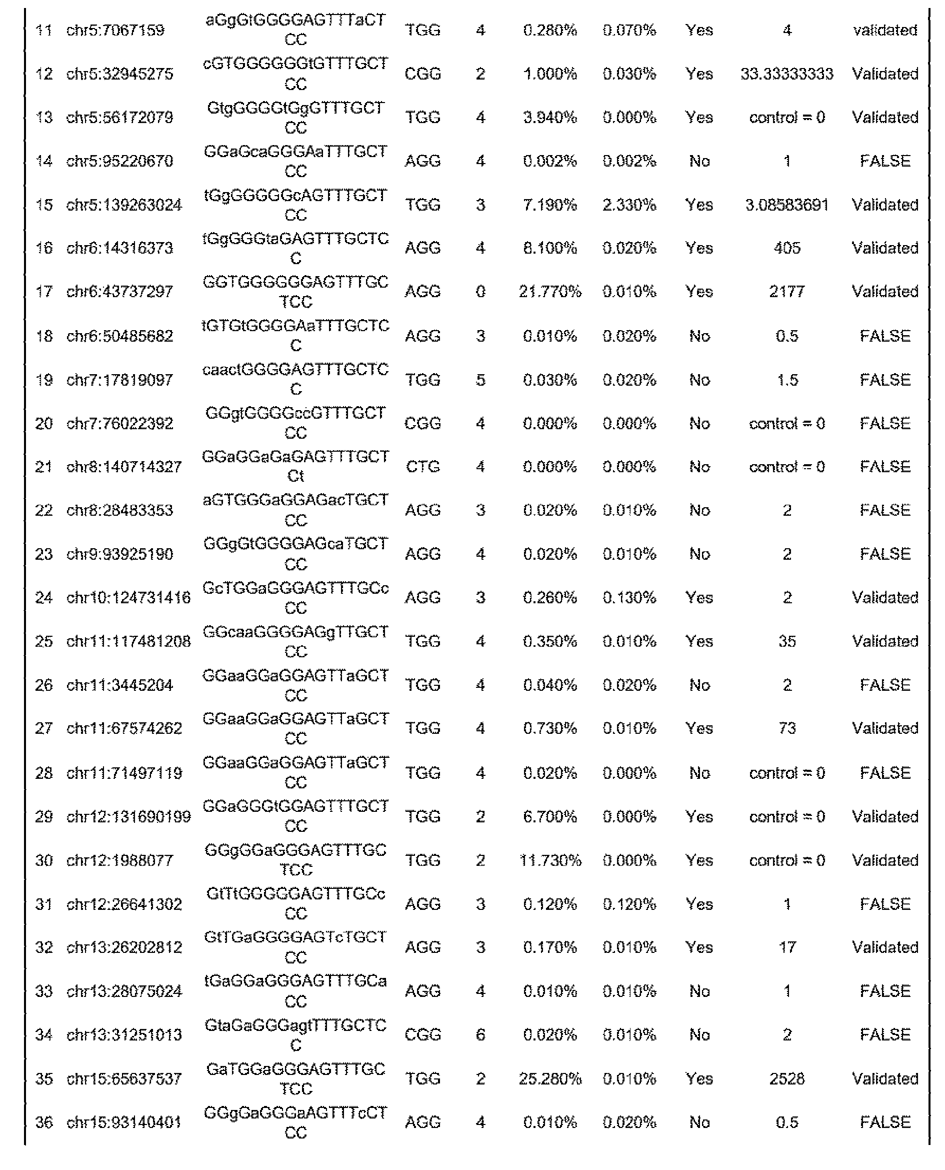

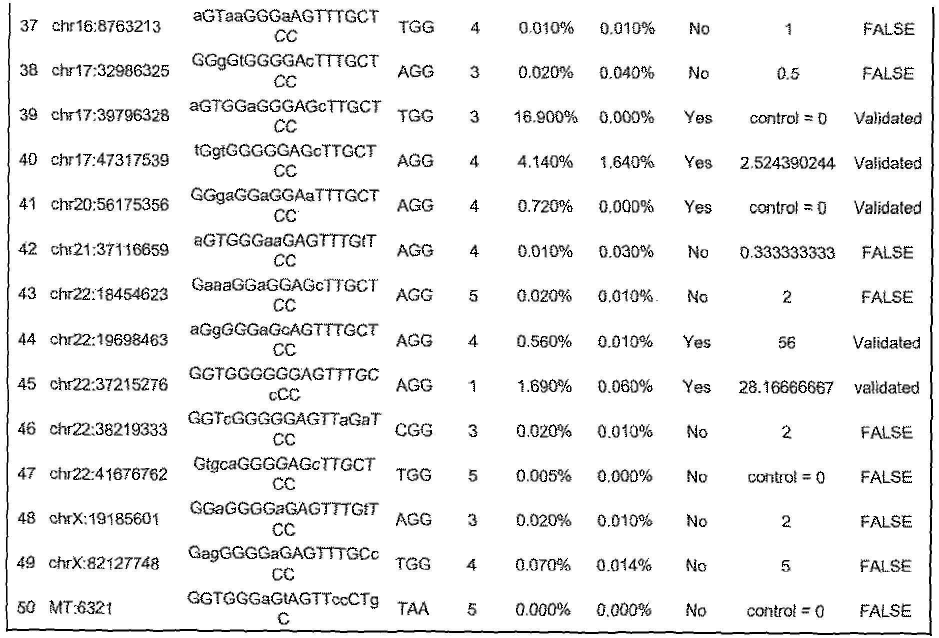

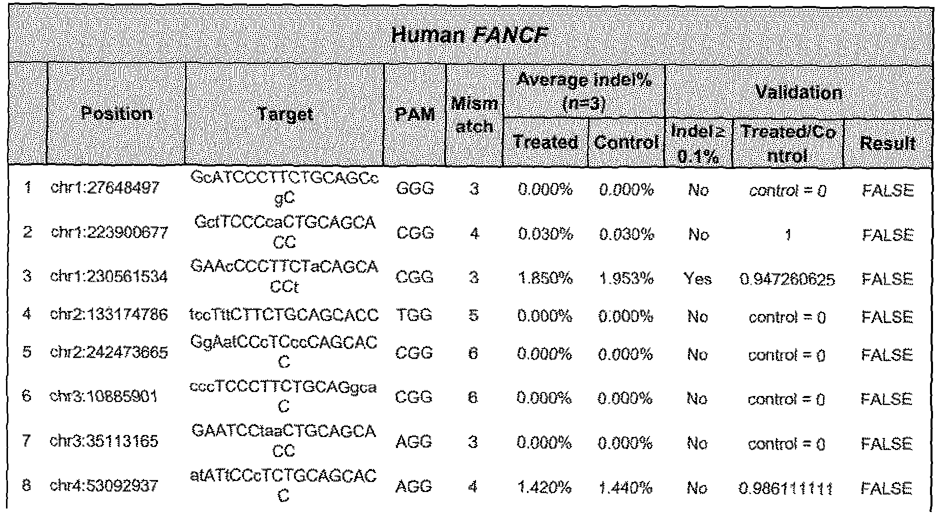

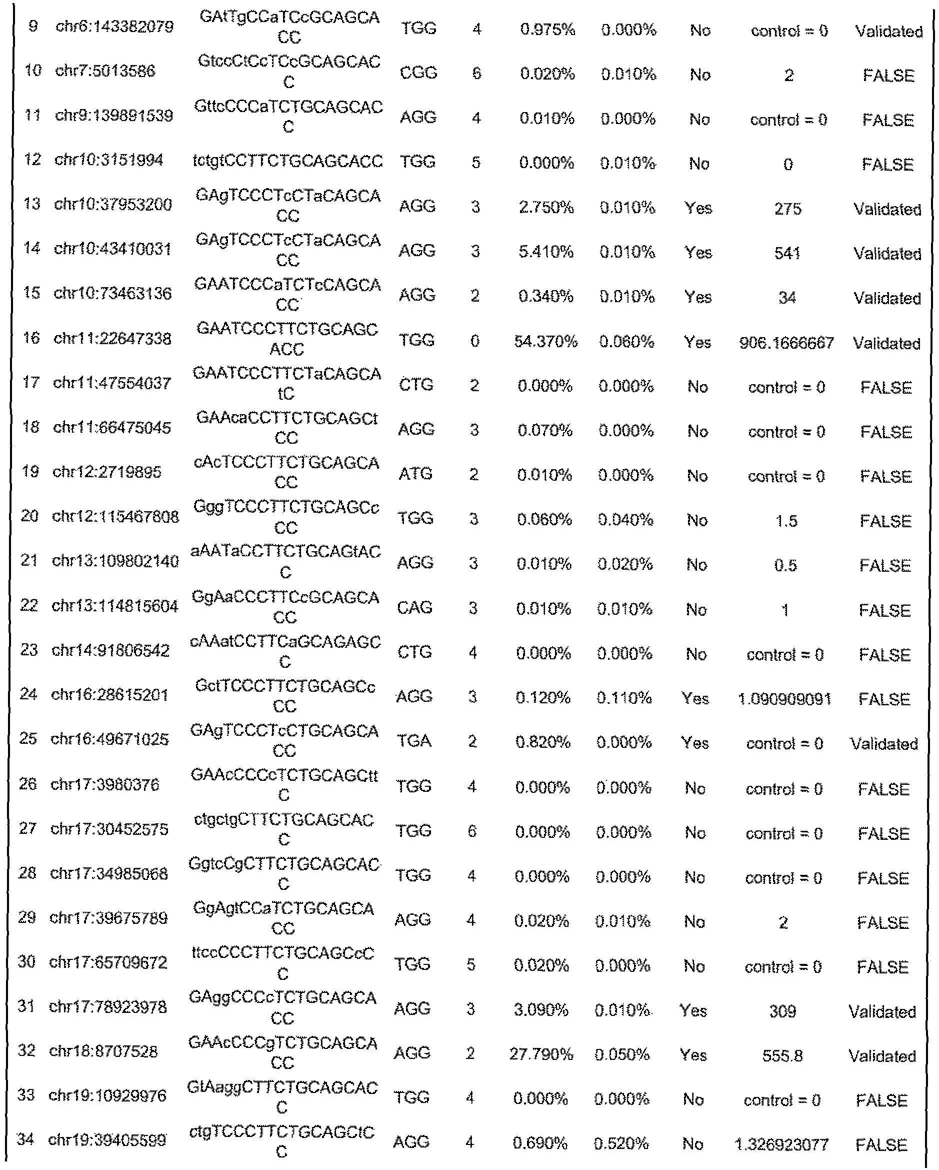

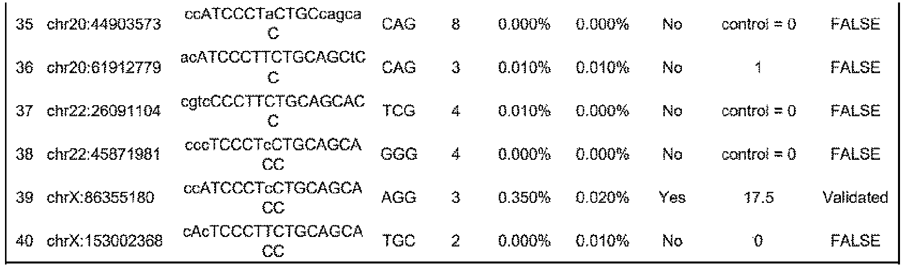

### Experimental Method 10. AAV production

AAV8 carrying a desired cloned sequence (pAAV-PCSK9, pAAV-Albumin, and pAAV-Cas9) was produced by VigeneBioscience in a large scale (10¹³ genome copies (GC)/mL). The produced AAV was dispensed and stored at-70 °C until use.

### Experimental Method 11. Animal research

All animal experiments were approved by the Institutional Animal Care and Use Committee (IACUC) in the medical school of Yonsei University (IACUC number 2019-0215). C57BL/6 mice were maintained on a 12:12h light/dark cycle.

### Experimental Method 12. AAV injection

Each of two types of AAV8 that carry pAAV-Cas9 and one of two pAAV-sgRNAs (pAAV-PCSK9 or pAAV-Albumin) was delivered into a C57BL/6 mouse through systemic (intravenous) and subretinal injections. Both injections were performed in a ratio of 1:1 GC (pAAV-Cas9:pAAV-sgRNA). A dose for intravenous injection was 2.5 × 10¹¹ GC/animal, and a dose for subretinal injection was 1.5 × 10¹⁰ GC/eye.

For systemic injection, 200 µL of AVV8 diluted in PBS was injected into a 7- to 9-week-old male mouse through tail vein injection. A dose was 2.5 × 10¹¹ GC AAV8.

For subretinal injection, 7- to 9-week-old male mice were selected. Under systemic anesthesia, one pupil per mouse was dilated with eye drops containing tropicamide and phenylephrine. During the experiment, the body temperature of the mouse was maintained at 37 °C with a heating pad. A small incision was made with a 1/2 30G needle 1 mm apart from the limbus of the cornea. A Hamilton syringe with a 33G blunt needle loaded with a 2 µL solution containing AAV8 mixture was inserted through an incision to the point of feeling resistance (subretinal area). To avoid unnecessary tissue damage, the volume was injected carefully and gently, and waited for 20 to 30 seconds to spread evenly, and then the syringe was slowly removed. Subsequently, antibiotic ointment was applied to the eye surface. Four mice were used for different injection methods and different sgRNAs.

### Experimental Method 13. DNA preparation from harvested organs and tissue

Organs and tissue were removed 2 weeks and 3 months after injection. At the end of the experiment, animals were euthanized by cardiac puncture under isoflurane anesthesia. The organs comprising eyes, liver, spleen, lungs, kidneys, muscles, brain, and testes were dissected, flash-frozen in liquid nitrogen, and stored at -70 °C until additional analysis.

For subretinal injection, the neural retina and the retinal pigment epithelium (RPE) were separated and prepared. The cornea, iris, lens, and vitreous body were removed from the enucleated eye. The remaining eye tissue was incubated in a hyaluronidase solution for 45 minutes (37 °C, 5% CO₂). Afterward, it was incubated in cold PBS for 30 minutes to inactivate hyaluronidase activity. Next, the eye tissue was transferred to fresh PBS and the neural retina was gently separated from a retina/RPE/choroid/sclera complex. The remaining retina/RPE/choroid/sclera complex was incubated in a trypsin solution at 37 °C in 5% CO₂ for 45 minutes, and gently shaken until the RPE sheet was completely detached. All isolated RPE sheets and RPE cells were collected. Genomic DNA was extracted using a DNeasy Blood & Tissue Kit (Qiagen, Cat No. 69506) according to the manufacturer's instructions.

### Experimental Method 14. Targeted deep sequencing

The genomic DNAs of mouse retinal pigment epithelial (RPE) cells were amplified using a REPLI-g single cell kit (Qiagen) according to the manufacturer's protocol.

Target sites and potential off-target sites were analyzed through targeted deep sequencing. Deep sequencing libraries were produced by PCR. TruSeq HT Dual Index primers were used for labeling of each sample. Paired-end sequencing was performed on polled libraries using MiSeq (Illumina). Specifically, several targets were combined by PCR performed with primers having different indexes, and then targeted deep sequencing analysis was performed.

### Experimental Method 15. Statistical analysis

Score/sequence read counts were min-max normalized. In each group, the maximum value was normalized to 1, and the minimum value was normalized to 0. A Wilcoxon Rank-Sum Test was performed on samples in each intersection of the Venn diagram to test whether the score medians of two different groups are the same or not. The results of a two-sided unpaired Mann-Whitney test calculated by Prism (version 9.4.1) are shown.

### Nucleic acid sequences of sgRNAs and amino acid sequence of spCas9 used in experiments

Hereinafter, the sequences of sgRNAs used herein and related sequences thereof, and the spCas9 sequence are shown. As described above, human *PCSK9*-targeting sgRNA actually targets a different locus other than *PCSK9* on a human genome, but for convenience, it is referred to as human *PCSK9*-targeting sgRNA. Human *Albumin-*targeting sgRNA actually targets a different locus other than *Albumin* on a human genome, but for convenience, it is referred to as human *Albumin*-targeting sgRNA.

### Total sequence of mouse PCSK9-targeting sgRNA

GAGGUGGGAAACUGAGGCUUGUUUUAGAGCUAGAAAUAGCAAGUUAAA
AUAAGGCUAGUCCGUUAUCAACUUGAAAAAGUGGCACCGAGUCGGUGCUUUU
U (SEQ ID NO: 29)

### Guide sequence of mouse PCSK9-targeting sgRNA

GAGGUGGGAAACUGAGGCUU (SEQ ID NO: 30)

### Target sequence of mouse PCSK9-targeting sgRNA (target sequence on space non-binding strand, excluding PAM)

AGGTGGGAAACTGAGGCTT (SEQ ID NO: 25)

### Whole sequence of mouse Albumin-targeting sgRNA

GACAUGCAUAUGUAUGUGUGGUUUUAGAGCUAGAAAUAGCAAGUUAAA
AUAAGGCUAGUCCGUUAUCAACUUGAAAAAGUGGCACCGAGUCGGUGCUUUUU
U (SEQ ID NO: 31)

### Guide sequence of mouse Albumin-targeting sgRNA

GACAUGCAUAUGUAUGUGUG (SEQ ID NO: 32)

### Target sequence of mouse Albumin-targeting sgRNA (target sequence on space non-binding strand, excluding PAM)

ACATGCATATGTATGTGTG (SEQ ID NO: 26)

### Whole sequence of human PCSK9-targeting sgRNA

GAGGUGGGAAACUGAGGCUUGUUUUAGAGCUAGAAAUAGCAAGUUAAA
AUAAGGCUAGUCCGUUAUCAACUUGAAAAAGUGGCACCGAGUCGGUGCUUUU
U (SEQ ID NO: 29)

### Guide sequence of human PCSK9-targeting sgRNA

GAGGUGGGAAACUGAGGCUU (SEQ ID NO: 30)

### Target sequence of human PCSK9-targeting sgRNA (target sequence on space non-binding strand, excluding PAM)

AGGTGGGAAACTGAGGCTT (SEQ ID NO: 25)

### Whole sequence of human Albumin-targeting sgRNA

GACAUGCAUAUGUAUGUGUGGUUUUAGAGCUAGAAAUAGCAAGUUAAA
AUAAGGCUAGUCCGUUAUCAACUUGAAAAAGUGGCACCGAGUCGGUGCUUUUU
U (SEQ ID NO: 31)

### Guide sequence of human Albumin-targeting sgRNA

GACAUGCAUAUGUAUGUGUG (SEQ ID NO: 32)

### Target sequence of human Albumin-targeting sgRNA (target sequence on space non-binding strand, excluding PAM)

ACATGCATATGTATGTGTG (SEQ ID NO: 26)

### Whole sequence of FANCF-targeting sgRNA

GGAAUCCCUUCUGCAGCACCGUUUUAGAGCUAGAAAUAGCAAGUUAAA
AUAAGGCUAGUCCGUUAUCAACUUGAAAAAGUGGCACCGAGUCGGUGCUUUUU
U (SEQ ID NO: 33)

### Guide sequence of FANCF-targeting sgRNA

GGAAUCCCUUCUGCAGCACC (SEQ ID NO: 34)

### Target sequence of FANCF-targeting sgRNA (target sequence on space non-binding strand, excluding PAM)

GAATCCCTTCTGCAGCACC (SEQ ID NO: 35)

### Whole sequence of VEGFA-targeting sgRNA

GGGUGGGGGGAGUUUGCUCCGUUUUAGAGCUAGAAAUAGCAAGUUAAA
AUAAGGCUAGUCCGUUAUCAACUUGAAAAAGUGGCACCGAGUCGGUGCUUUUU
U (SEQ ID NO: 36)

### Guide sequence of VEGFA-targeting sgRNA

GGGUGGGGGGAGUUUGCUCC (SEQ ID NO: 37)

### Target sequence of VEGFA-targeting sgRNA (target sequence on space non-binding strand, excluding PAM)

GGTGGGGGGAGTTTGCTCC (SEQ ID NO: 38)

### Whole sequence of HBB-targeting sgRNA

GUUGCCCCACAGGGCAGUAAGUUUUAGAGCUAGAAAUAGCAAGUUAAA
AUAAGGCUAGUCCGUUAUCAACUUGAAAAAGUGGCACCGAGUCGGUGCUUUUU
U (SEQ ID NO: 39)

### Guide sequence of HBB-targeting sgRNA

GUUGCCCCACAGGGCAGUAA (SEQ ID NO: 40)

### Target sequence of HBB-targeting sgRNA (target sequence on space non-binding strand, excluding PAM)

TTGCCCCACAGGGCAGTAA (SEQ ID NO: 41)

### Amino acid sequence of spCas9

IAKSEQEIGKATAKYFFYSNIMNFFKTEITLANGEIRKRPLIETNGETGEIVWDK
GRDFATVRKVLSMPQVNIVKKTEVQTGGFSKESILPKRNSDKLIARKKDWDPKKYGG
FDSPTVAYSVLVVAKVEKGKSKKLKSVKELLGITIMERSSFEKNPIDFLEAKGYKEVK
KDLIIKLPKYSLFELENGRKRMLASAGELQKGNELALPSKYVNFLYLASHYEKLKGSP
EDNEQKQLFVEQHKHYLDEIIEQISEFSKRVILADANLDKVLSAYNKHRDKPIREQAE
NIIHLFTLTNLGAPAAFKYFDTTIDRKRYTSTKEVLDATLIHQSITGLYETRIDLSQLGG
D (SEQ ID NO: 42)

### spCas9-encoding DNA sequence

TGAGCCAGCTGGGCGGCGAC (SEQ ID NO: 43)

### Results

### Result 7. Selection of offtarget prediction method to be used for comparison

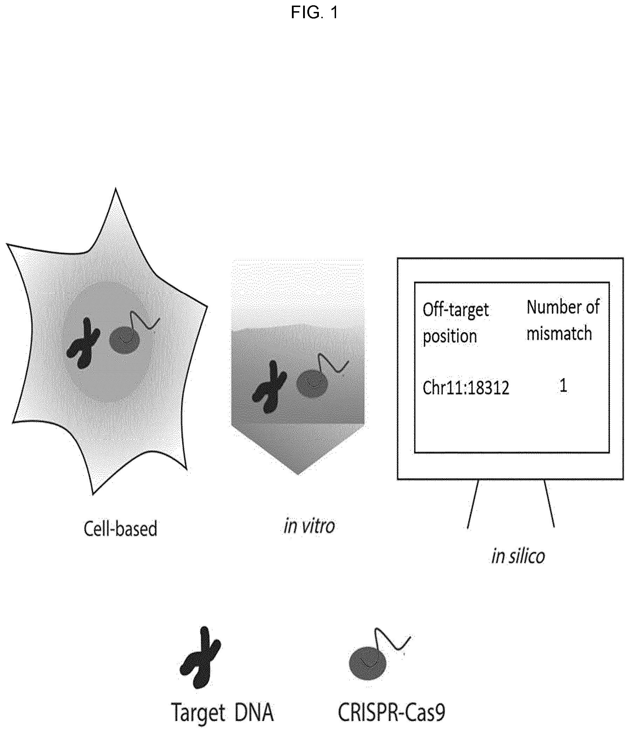

Genome-wide off-target prediction methods can be categorized into three groups, such as cell-based, in vitro, and in silico methods according to their approaches. Examples of the approaches for the three groups are shown in FIG. 1.

Different combinations of off-target prediction methods have been used in IND studies for genome editing treatments. Information on the off-target prediction methods used in IND studies for genome editing treatments is shown in Table 2.

**Table 2. Off-target prediction method used in gene editing drugs and IND studies (TALEN: Transcription activator-like effector; NA: Not available; LCA10: Leber congenital amaurosis 10; MPS: Mucopolysaccharidosis.)**

| **Name** | **Genome editor** | **Target gene** | **Company** | **Clinical phase** | **Disease** | **Off-target prediction methods used** | **Number of methods used** | **Reference** |
|---|---|---|---|---|---|---|---|---|
| Universal CAR T-Cells | TALEN | *TRAC*/*CD52* | Cellectis | Phase 2 | Cancer | *in silica* (TAL Effector-Nucleotide Targeter (TALE-NT) 2.0) | 1 | [24] |
| PBCAR019 1 | I-Crel | *TRAC* | Precision Bioscience | Phase 1/2 | Cancer | *in silico* (COSMID) | 1 | [25] |
| 5B-728 | Zinc Finger | *CCRS* | Sangamo | Phase 1/2 | HIV | Cell-based (integration deficient lentivirus end-capture-based integration site analysis), *in vitro* (SELEX-based oligo capture) | 2 | [42] |
| CTX110, CTX120 | CRISPR-Cas9 | *TRAC* | CRISPR Therapeutics | Phase 1/2 | Cancer | NA | NA | [43] |
| NY-ESO-1 directed Cell | CRISPR-Cas9 | *TRAC, PCDC1, TRBC* | UPenn | Phase 1 | Cancer | Cell-based (GUIDE-seq) | 1 | [26] |
| LBP-EC01 | Cas3 | Bacterial genomic DNA-specific sequence | Locus Bioscience | Phase 1b | Urinary tract infection | NA | NA | [44] |
| EDIT 101 | CRISPR-Cas9 | *CEP290* | EDITAS Medicine | Phase 1/2 | LCA10 | Cell-based (GUIDE-seq), *in vitro* (Digenome-seq), *in silico* (Cas-OFFinder) | 3 | [23] |
| SB Therapeutic s | Zinc Finger | *Albumin* | Sangamo | Phase 1/2 | MPS I, MPS II | Cell-based (Unbiased off-target assessment via AAV integration site analysis), *in vitro* (SELEX-based oligo capture) | 2 | [45] |
| CTX001 | CRISPR-Cas9 | *BCL11a* | CRISPR Therapeutics | Phase 1/2 | Sickle cell disease | Cell-based (GUIDE-seq), *in silico* (NA) | 2 | [35] |
| NTLA-2001 | CRISPR-Cas9 | *TTR* | Intellia | Phase 1 | Hereditary transthyretin amyloidosis with polyneuropathy | Cell-based (GUIDE-seq), *in vitro* (SITE-seq), *in silica* (Cas-OFFinder) | 3 | [22] |

To compare the performance of off-target prediction methods, the inventors of the present application selected one method for each of the above-mentioned categories. For a cell-based off-target prediction method, GUIDE-seq was selected. For an in silico off-target prediction method, CAS-OFFinder was selected. GUIDE-seq and CAS-OFFinder were most frequently used to predict off-targets of Cas9 therapeutic agents comprising EDIT101 and NTLA-2001. For an in vitro off-target prediction method, Digenome-seq was selected. Because Digenome-seq was used in studies of EDIT101 and it is one of the most popular protocols in the previous study for comparison.

### Result 2. Overview of Extru-seq and optimization of Extru-seq conditions

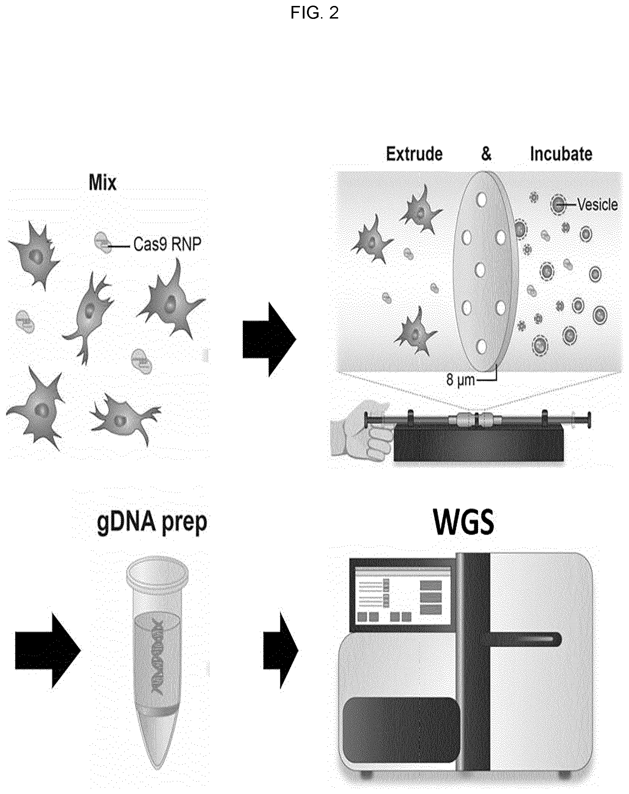

The inventors of the present application aimed to design a new method that combines the positive features of a cell-based method and an in vitro method. To this end, an off-target prediction method, Extru-seq, characterized by lysing cells using a physical force and mixing the genomic DNA with Cas9 and sgRNA was developed. The schematic diagram of the novel off-target prediction method, Extru-seq, is shown in FIG. 2.

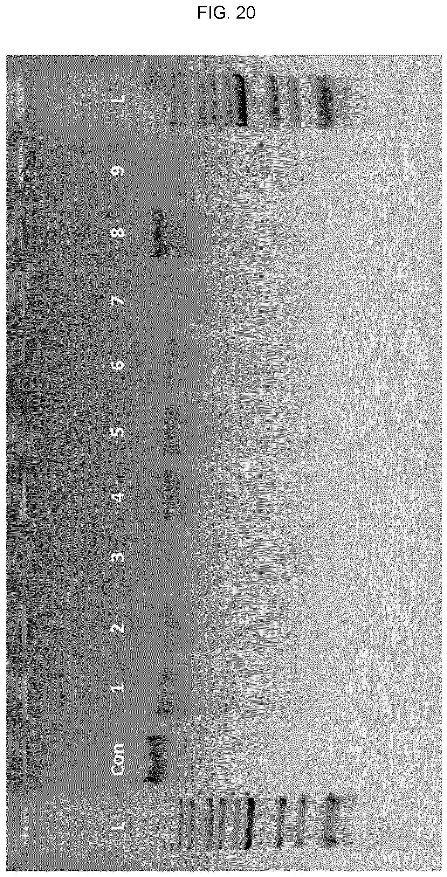

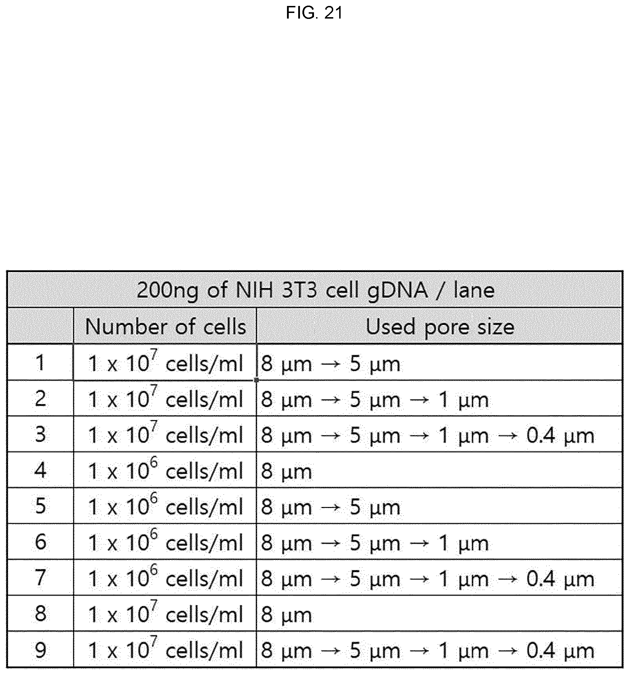

For example, live HEK294T or NIH-3T3 cells is mixed with the pre-incubated Cas9-sgRNA RNP complex. Under the use of an extruder (refer to the document [Goh, Wei Jiang, et al. "Bioinspired cell-derived nanovesicles versus exosomes as drug delivery systems: a cost-effective alternative." Scientific reports 7.1 (2017): 1-10.]), the mixture pass through a filter (e.g., a filter paper) with a pore size smaller than the cell diameter. As it passed through a filter with a pore size smaller than the cell diameter, the cells (e.g., cell membrane) is destroyed, allowing the Cas9 RNP to access the genomic DNA. FIGS. 20 and 21 show the results of experiments performed to find the optimization conditions for the average pore size of a filter, the Cas9 RNP concentration of the mixture, and the number of cells in Extru-seq.

Specifically, FIG. 20 shows the quality of the genomic DNA cultured overnight with Cas9 RNP, analyzed through gel electrophoresis. Various numbers of NIH-3T3 cells and various pore sizes were tested. Here, 'Con' represents the genomic DNA of a control with quality sufficient for WGS analysis. 'L' represents ladder DNA. FIG. 21 shows information on each of samples 1 to 9. For example, the electrophoresis result for the genomic DNA of an extruded sample under the conditions of sample 8 (1×10⁷ cells/mL; 8 µm pore size) is shown in line 8 of FIG. 20.

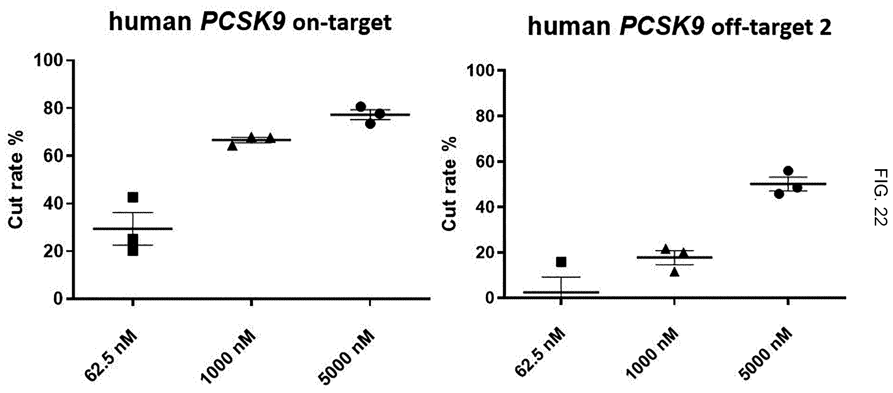

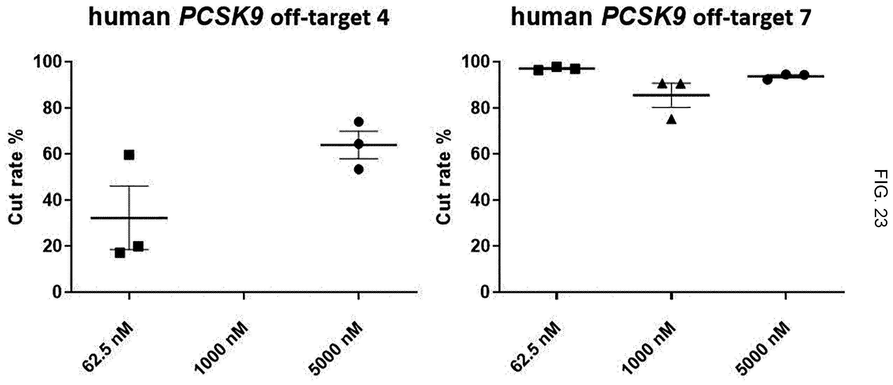

FIGS. 22 and 23 show cleavage rates for on- and off-target sites recognized by sgRNA targeting a human *PCSK9* site, measured by quantitative PCR (qPCR). FIG. 22 shows the result of cleavage rates for on-target and off-target 2 sites for a sample using human *PCSK9-*targeting sgRNA. FIG. 23 shows the result of cleavage rates for off-target 4 and off-target 7 sites of a sample using human *PCSK9*-targeting sgRNA.

Through this experiment, the inventors of the present application determined the optimized conditions for Extru-seq for subsequent experiments as follows: a pore size of 8 µM, a Cas9 RNP concentration of 5000 nM, and 10⁷ cells. Under the optimized condition, it was confirmed that the quality of genomic DNA cultured overnight with Cas9 RNP at 37 °C was sufficiently high for constructing a whole genome sequencing (WGS) library.

### Result 3. Measurement of NHEJ level after extrusion step

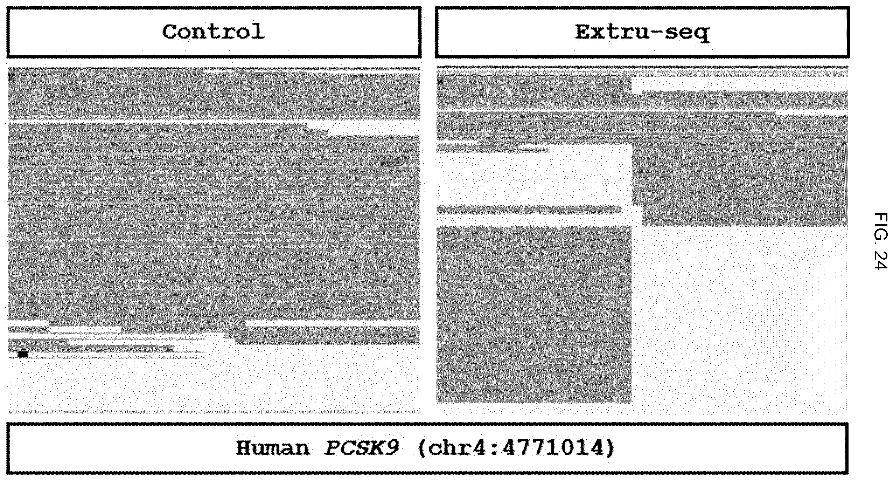

The inventors of the present application formulated a hypothesis in that a DNA repair mechanism for re-ligating genomic DNA cleaved by Cas9 would not exist in the Extru-seq process. In fact, when DNA was cleaved through the Extru-seq process, and the cleavage rate of a target site was measured using quantitative PCR, an average rate of 70% was observed. The results for the cleavage rates of target sites are shown in FIGS. 24 to 31.

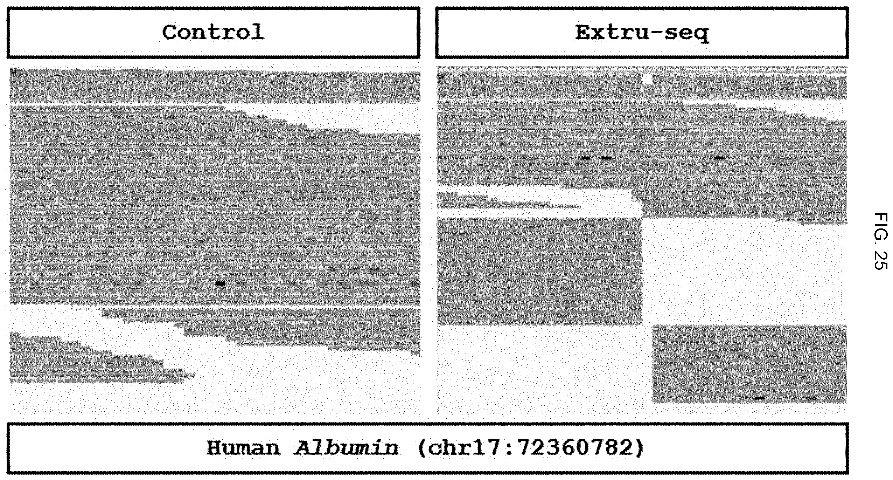

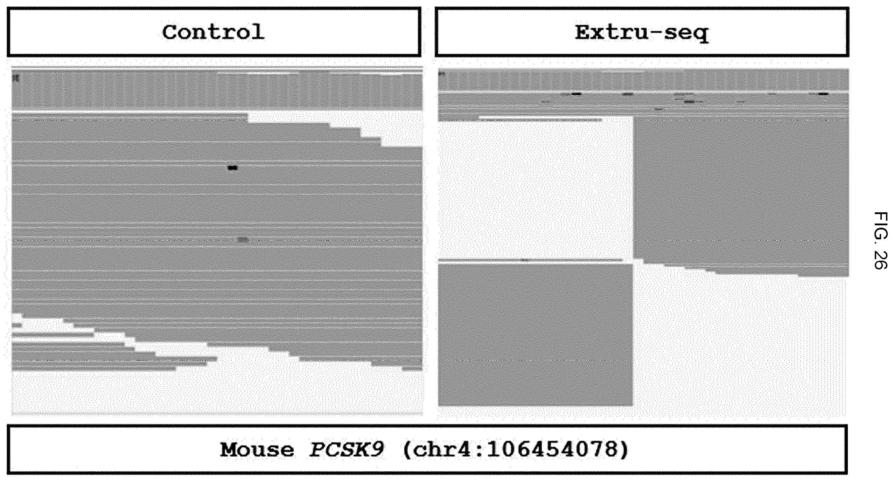

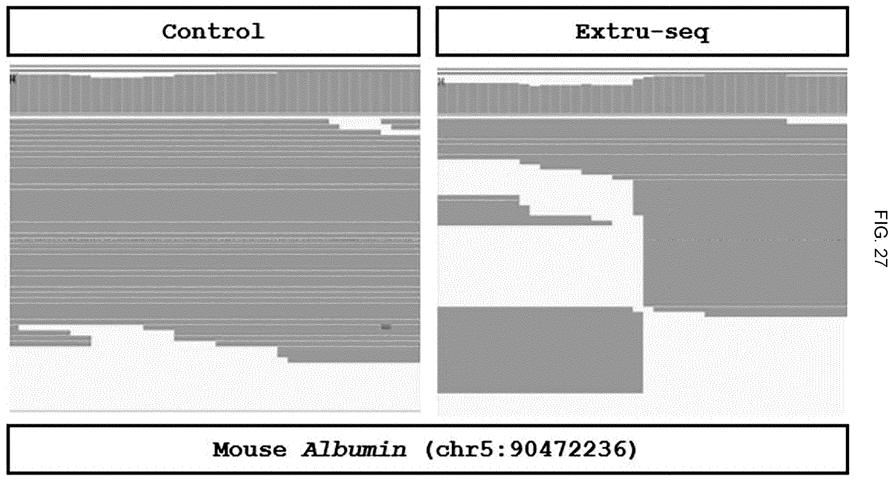

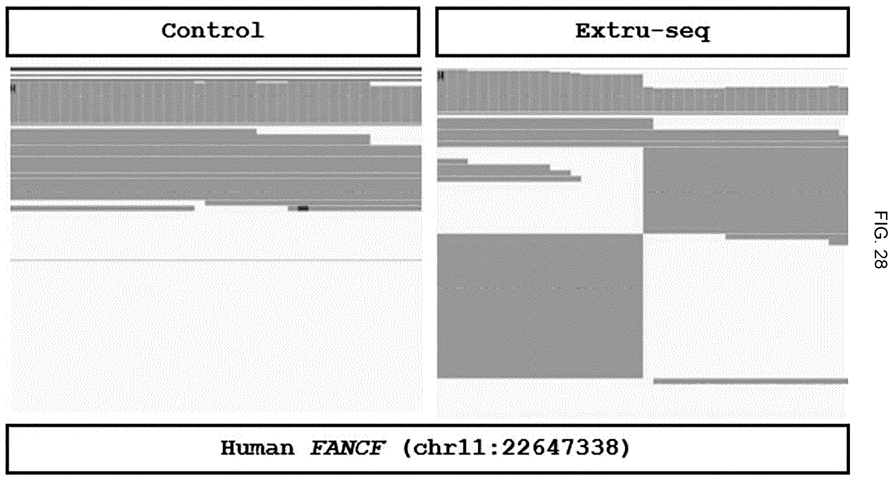

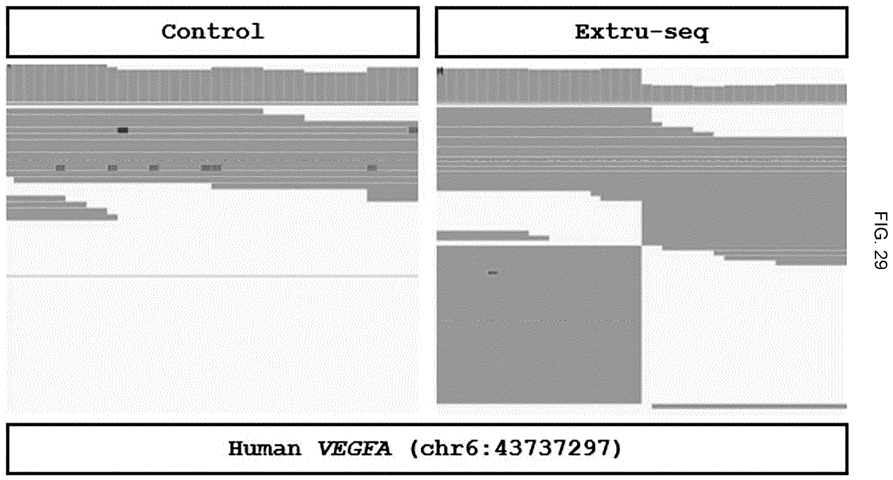

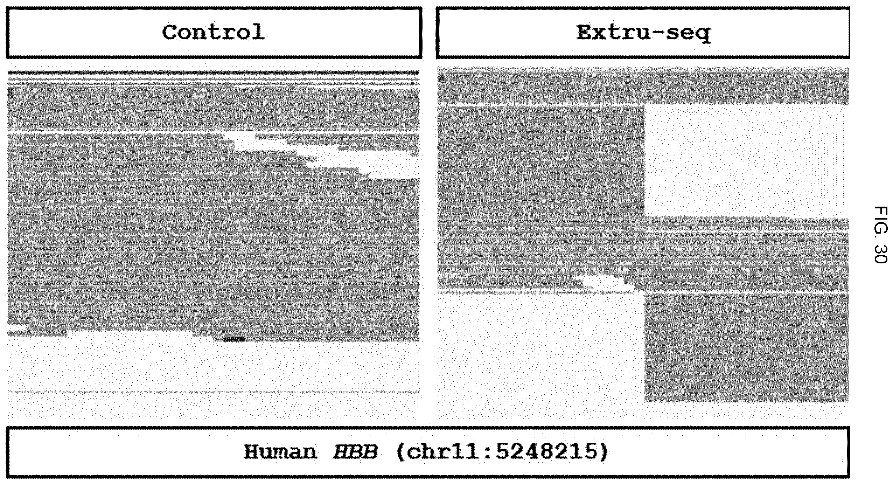

FIGS. 24 to 30 show the WGS data of Extru-seq analyzed using IGV to identify a cleavage pattern. FIG. 24 shows the result obtained using human *PCSK9-*targeting sgRNA. FIG. 25 shows the result obtained using human *Albumin*-targeting sgRNA. FIG. 26 shows the result obtained using mouse *PCSK9*-targeting sgRNA. FIG. 27 shows the result obtained using mouse *Albumin-targeting* sgRNA. FIG. 28 shows the result obtained using human *FANCF*-targeting sgRNA. FIG. 29 shows the result obtained using human *VEGFA*-targeting sgRNA. FIG. 30 shows the result obtained using human *HBB*-targeting sgRNA.

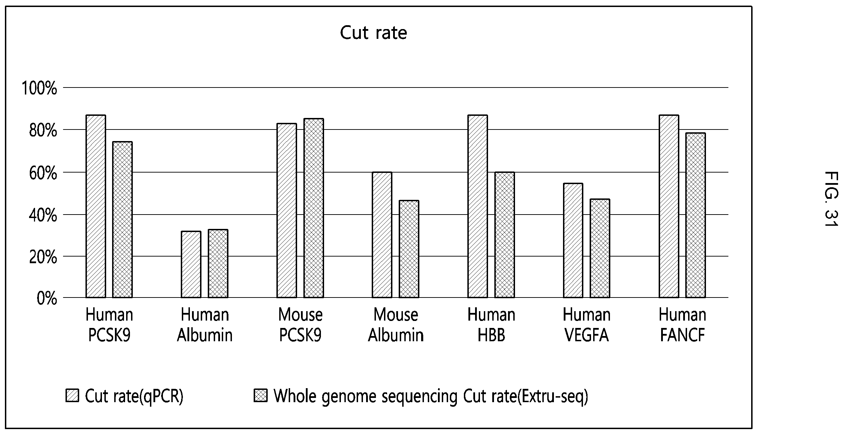

FIG. 31 shows the cleavage rates of seven on-target sites of each target, obtained through manual calculation based on qPCR and IGV analysis of WGS data. In FIG. 31, the y-axis represents a cleavage rate.

The results shown in FIGS. 24 to 31 demonstrate that there is no DNA repair mechanism such as NHEJ, indicating that Extru-seq is able to reflect positive features in vitro.

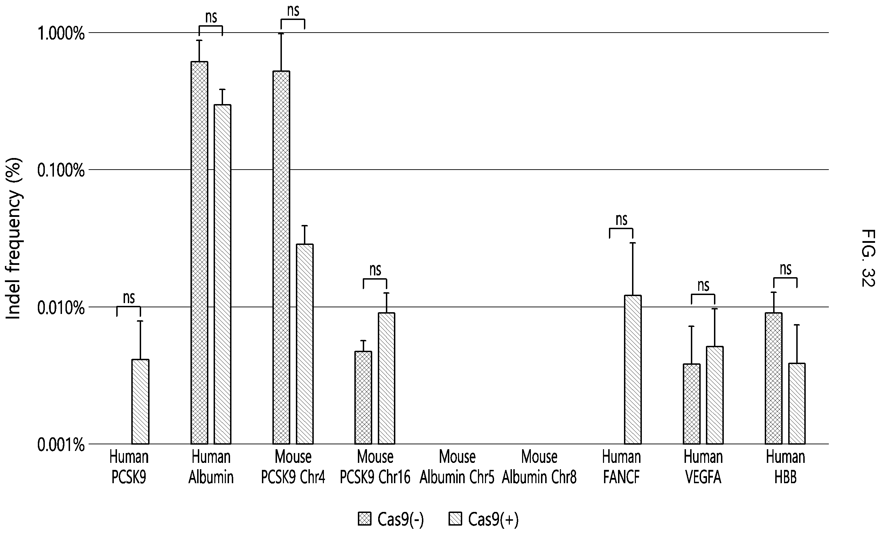

Further, the inventors of the present application analyzed cut and uncut populations of on-target sites to investigate which NHEJ occurs or the degree to the NHEJ occurs after the extrusion process. First, considering that indel mutations would accumulate in the uncut group if the NHEJ process was intact during the incubation period after the extrusion process, the uncut population in the Extru-seq sample was analyzed through deep sequencing. The deep sequencing result for Extru-seq sample treated with a Cas9 RNP complex was compared with the result for a control sample that was not treated with the Cas9 RNP complex. As a comparison result, there was no significant difference between the two samples. The deep sequencing result of the uncut population is shown in FIG. 32. Particularly, FIG. 32 shows indel frequencies, measured by targeted deep sequencing, for the uncut population of the Extru-seq sample related to FIGS. 24 to 30. Indel frequencies were measured for untreated samples and Cas9-treated samples. In FIG. 32, the samples not treated with Cas9 are indicated as Cas9(-), and the Cas9-treated samples are indicated as Cas9(+). For a t-test, an unpaired student t-test was used. Error bars indicate standard deviation (n=3).

These results demonstrate that the NHEJ level after the extrusion process is not significant.

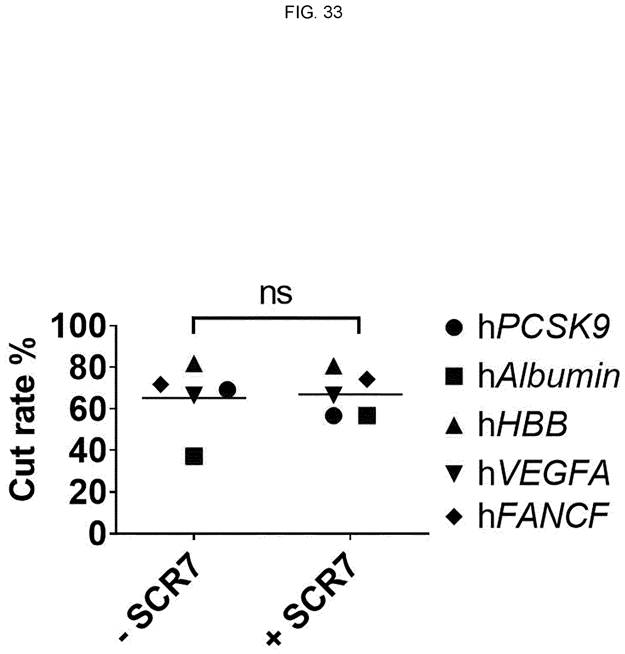

Second, using the protocol from multiplex Digenome-seq (refer to the document [Kim, Daesik, et al. "Genome-wide target specificities of CRISPR-Cas9 nucleases revealed by multiplex Digenome-seq." Genome research 26.3 (2016): 406-415.]), the inventors of the present application performed multiplex Extru-seq to measure a change in cleavage rates at five different on-target sites in the presence or absence of SCR7 (chemical DNA ligase IV or NHEJ inhibitor; refer to the document [Chu, Van Trung, et al. "Increasing the efficiency of homology-directed repair for CRISPR-Cas9-induced precise gene editing in mammalian cells." Nature biotechnology 33.5 (2015): 543-548.]). If NHEJ occurs, a cleavage rate increases due to the presence of SCR7, and this effect would also accumulate during the incubation step. However, the difference in average cleavage rates at five on-target sites in the presence or absence of SCR7 was not significant. The results related to the presence or absence of SCR7 are shown in FIG. 33. Specifically, FIG. 33 shows the cleavage rates (%) measured using qPCR at five target positions. This result was obtained by multiplex Extru-seq in the presence (in FIG. 33, +SCR7) of 1 µM SCR7 or absence (in FIG. 33, -SCR7). The horizontal lines on the graph represent the average for the experiments (n=5). For a t-test, an unpaired student t-test was used.

These results further reveal that NHEJ does not have a significant effect on the on-target cleavage rates.

The inventors of the present application hypothesized that as cell components other than genomic DNA would not be damaged such that a cleavage pattern would be similar to that of a cell-based off-target prediction method. The hypothesis was tested by comparing the Extru-seq result with those of the cell-based and in vitro-based methods (to be described below). It was confirmed that Extru-seq is an off-target prediction method that can reflect the positive features of both a cell-based off-target prediction method (intact cell components other than genomic DNA) and an in vitro off-target prediction method (the absence of a DNA repair mechanism).

### Result 4. Design and use of promiscuous guide sequences

The second goal of this study was to conduct a standard test that can effectively measure performance metrics for each method. Previous studies used guide sequences predicted to recognize only a small number of off-target sites in the genome to compare other methods. As a result, only a few guide sequence-validated off-target loci were found, so it was difficult to effectively compare different prediction methods using the statistically significant number of loci. In more recent papers (refer to the document [Wienert, Beeke, et al. "Unbiased detection of CRISPR off-targets in vivo using DISCOVER-Seq." Science 364.6437 (2019): 286-289.; and Akcakaya, Pinar, et al. "In vivo CRISPR editing with no detectable genome-wide off-target mutations." Nature 561.7723 (2018): 416-419.]), promiscuous guide sequences predicted to recognize a large number of off-target loci were used. The use of promiscuous guide sequences provided a powerful test bed for a genome-wide off-target prediction method. However, these promiscuous guide sequences were not used in this study. One of them involved a mouse guide sequence (indicated as a previous study) targeting *PCSK9,* which is not complementary to sequences of human cells. Another one targeting *VEGFA* lacked off-target loci predicted as a single mismatch (refer to Table 3).

To overcome such limitation, the research of the present application searched for two types of promiscuous guide sequences targeting *PCSK9* and *Albumin* of the mouse genome. The guide sequences also perfectly matched target sequences present in the human genome (However, in the human genome, the *PCSK9* and *Albumin* loci is not targets). Even though the guide sequences targeted loci other than *PCSK9* or *Albumin* in the human genome, they are referred to as human *PCSK9* and human *Albumin* for convenience. In each of the human and mouse genomes, the number of off-target sequences for these promiscuous guide sequences was calculated using Cas-OFFinder. It was confirmed that the selected guide sequences were associated with numerous off-target sequences in both genomes. Information on target sequences of the guide sequences used in the previous research and the promiscuous guide sequences used in this research and the investigation results for off-target sites are shown in Table 3 below. Specifically, Table 3 shows the investigation results for genome-wide off-target loci comprising 0 to 6 mismatches. In the genome hg19 (Table 3 (a)) and the genome mm10 (Table 3 (b)), off-target sites were predicted through CAS-OFFinder. Referring to Table 3, the sequence GACCCCCTCCACCCCGCCTC (SEQ ID NO: 72) (*VEGFA* target sequence), the sequence AGCAGCAGCGGCGGCAACAG (SEQ ID NO: 73) (*PCSK9* target sequence, previous study), the sequence ACATGCATATGTATGTGTG (SEQ ID NO: 26) (*Albumin-*target sequence), and the sequence AGGTGGGAAACTGAGGCTT (SEQ ID NO: *25) (PCSK9* target sequence) are shown.

**Table 3. Investigation results for whole genome off-target sites according to target sequences or guide sequences**

| (a) | | | | | | | | |
|---|---|---|---|---|---|---|---|---|
| **Target gene** | **Target** | **M0** | **M1** | **M2** | **M3** | **M4** | **M5** | **M6** |
| *VEGFA* | GACCCCCTCCACCCCGCCTC | 1 | 0 | 2 | 35 | 446 | 3898 | 17450 |
| *PCSK9* (Previou s study) | AGCAGCAGCGGCGGCAACAG | 0 | 1 | 27 | 188 | 821 | 3471 | 16235 |
| *Albumin* (This study) | ACATGCATATGTATGTGTG | 1 | 6 | 42 | 281 | 1740 | 8940 | 43234 |
| *PCSK9* (This study) | AGGTGGGAAACTGAGGCTT | 1 | 5 | 48 | 259 | 1610 | 8985 | 46803 |

| (b) | | | | | | | | |
|---|---|---|---|---|---|---|---|---|
| **Target gene** | **Target** | **M0** | **M1** | **M2** | **M3** | **M4** | **M5** | **M6** |
| VEGFA | GACCCCCTCCACCCCGCCTC | 1 | 0 | 1 | 23 | 268 | 2048 | 11579 |
| *PCSK9* ( Previous study) | AGCAGCAGCGGCGGCAACAG | 1 | 5 | 41 | 354 | 1072 | 3343 | 21891 |
| *Albumin* (This study) | ACATGCATATGTATGTGTG | 4 | 13 | 94 | 684 | 3486 | 16144 | 65390 |
| *PCSK9* ( This study) | AGGTGGGAAACTGAGGCTT | 2 | 5 | 22 | 136 | 986 | 6504 | 41972 |

### Result 5. Prediction of genome-wide off-target sites using GUIDE-seq, Digenome-seq, in silico method, and Extru-seq

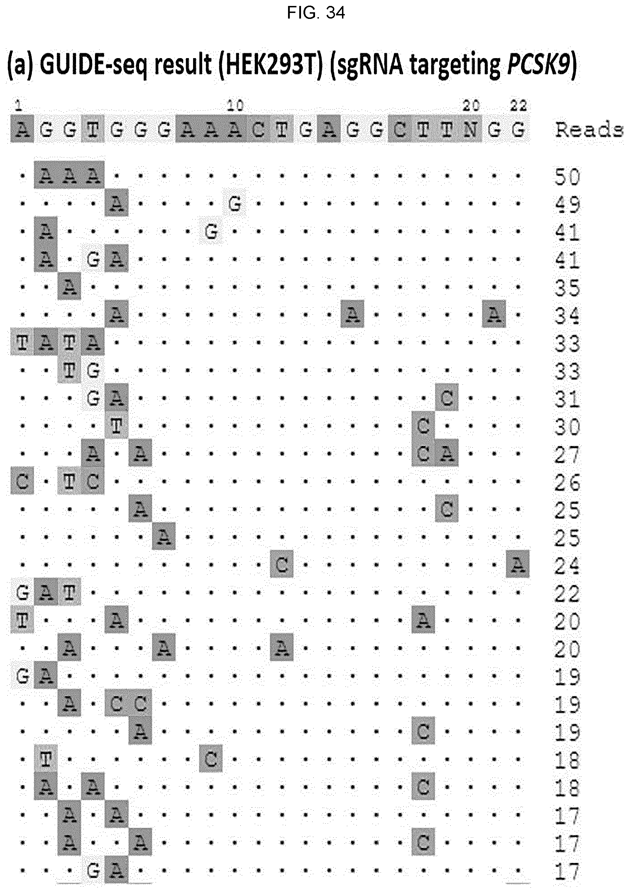

Using the promiscuous sgRNA sequences targeting *PCSK9* and *Albumin,* respectively, GUIDE-seq, Digenome-seq, Extru-seq, and in silico prediction based on CAS-OFFinder were performed. The off-target prediction system for each sgRNA sequence was performed on a human cell line (HEK293T) and a mouse cell line (NIH-3T3). The results are shown in FIGS. 34 to 62, and FIGS. 3 and 4.

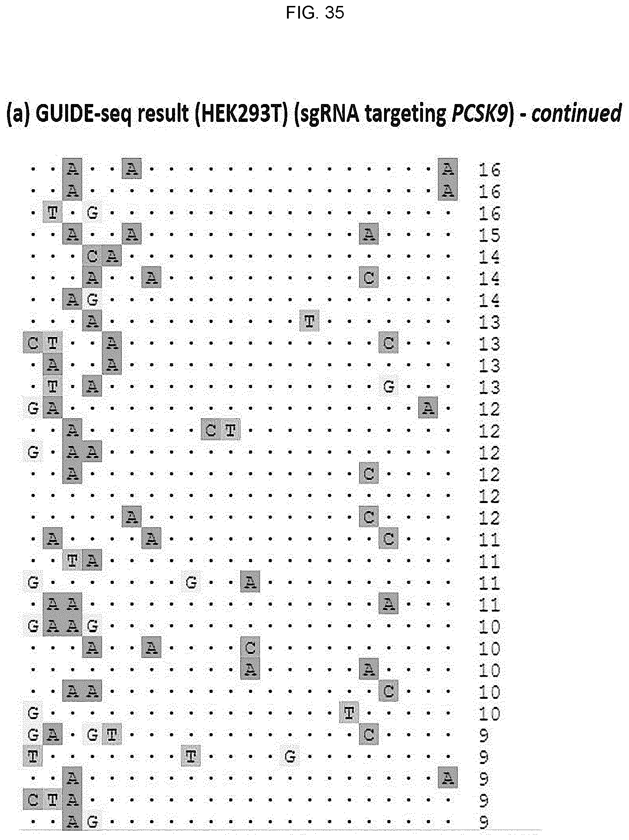

Specifically, FIGS. 34 and 35 show GUIDE-seq results obtained from HEK293T cells using *PCSK9*-targeting sgRNA. In FIGS. 34 and 35, the target sequence (comprising a PAM sequence) AGGTGGGAAACTGAGGCTTNGG (SEQ ID NO: 44) is shown.

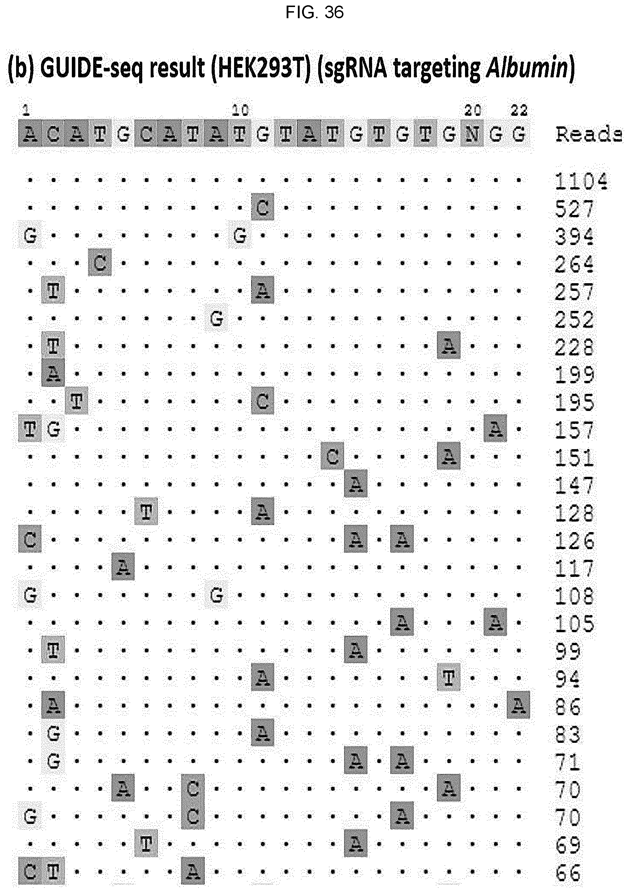

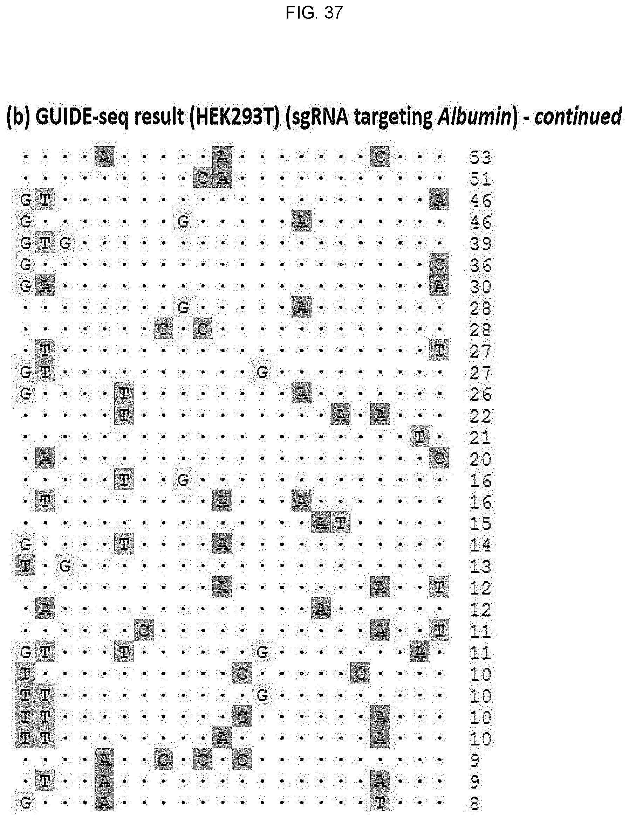

FIGS. 36 and 37 show GUIDE-seq results obtained from HEK293T cells using *Albumin-targeting* sgRNA. In FIGS. 36 and 37, the target sequence (comprising a PAM sequence) ACATGCATATGTATGTGTGNGG (SEQ ID NO: 45) is shown.

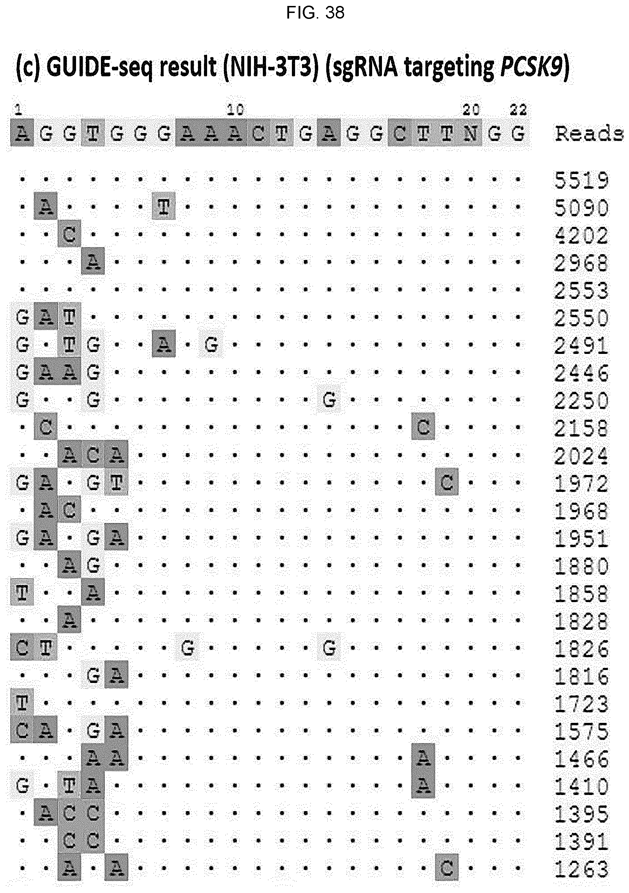

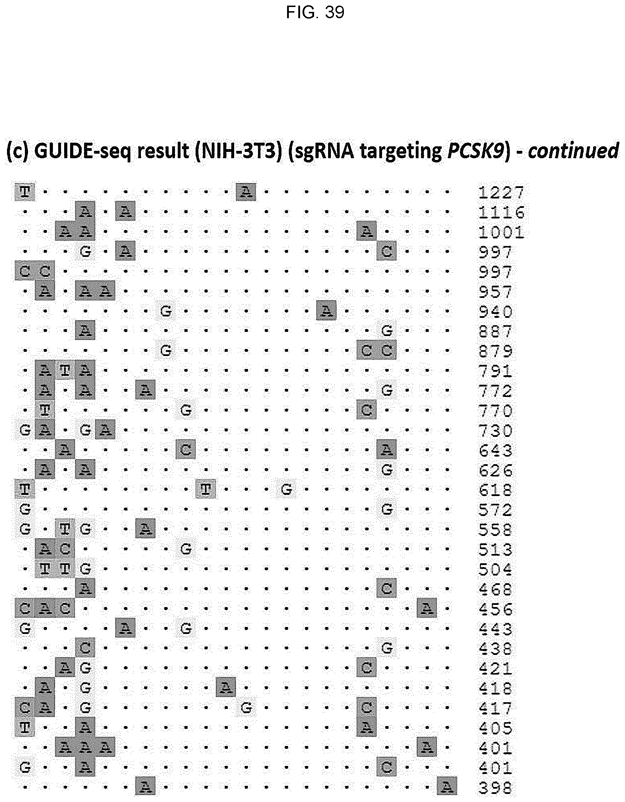

FIGS. 38 and 39 show GUIDE-seq results obtained from NIH-3T3 cells using *PCSK9-*targeting sgRNA. In FIGS. 38 and 39, the target sequence (comprising a PAM sequence)AGGTGGGAAACTGAGGCTTNGG (SEQ ID NO: 44) is shown.

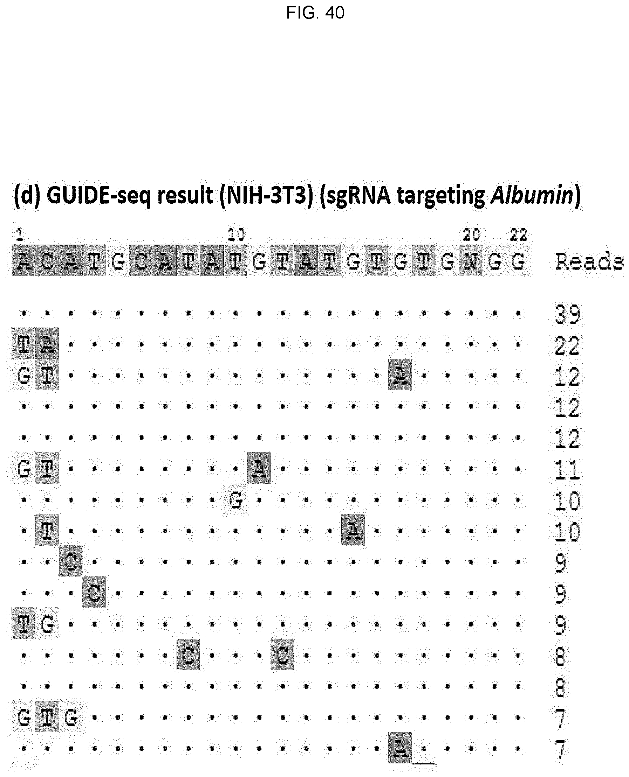

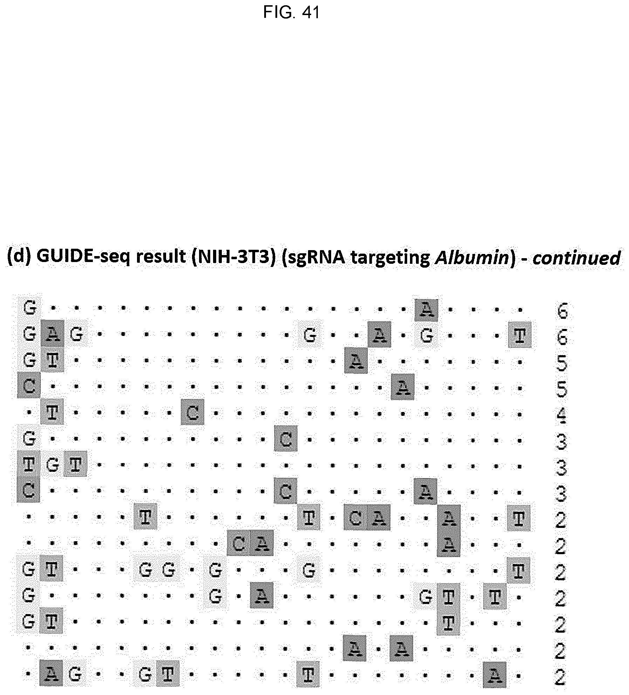

FIGS. 40 and 41 show GUIDE-seq results obtained from NIH-3 T3 cells using *Albumin-*targeting sgRNA. In FIGS. 40 and 41, the target sequence (comprising a PAM sequence) ACATGCATATGTATGTGTGNGG (SEQ ID NO: 45) is shown.

Relatively low-ranking off-target loci were omitted from the corresponding drawing by the GUIDE-seq analysis program. The omitted loci were comprised in subsequent analysis.

FIGS. 42 and 43 show the Manhattan plot results of Digenome-seq obtained from HEK293T cells using *PCSK9*-targeting sgRNA. Here, the y-axis represents a DNA cleavage score.

FIGS. 44 and 45 show the Manhattan plot results of Digenome-seq obtained from HEK293T cells using *Albumin*-targeting sgRNA. Here, the y-axis represents a DNA cleavage score.

FIGS. 46 and 47 show the Manhattan plot results of Digenome-seq obtained from NIH-3T3 cells using *PCSK9*-targeting sgRNA. Here, the y-axis represents a DNA cleavage score.

FIGS. 48 and 49 show the Manhattan plot results of Digenome-seq obtained from NIH-3T3 cells using *Albumin*-targeting sgRNA. Here, the y-axis represents a DNA cleavage score.

FIGS. 50 and 51 show the Manhattan plot results of Extru-seq obtained from HEK293T cells using *PCSK9*-targeting sgRNA. Here, the y-axis represents a DNA cleavage score.

FIGS. 52 and 53 show the Manhattan plot results of Extru-seq obtained from HEK293T cells using *Albumin*-targeting sgRNA. Here, the y-axis represents a DNA cleavage score.

FIGS. 54 and 55 show the Manhattan plot results of Extru-seq obtained from NIH-3T3 cells using *PCSK9*-targeting sgRNA. Here, the y-axis represents a DNA cleavage score.

FIGS. 56 and 57 show the Manhattan plot results of Extru-seq obtained from NIH-3T3 cells using *Albumin*-targeting sgRNA. Here, the y-axis represents a DNA cleavage score.

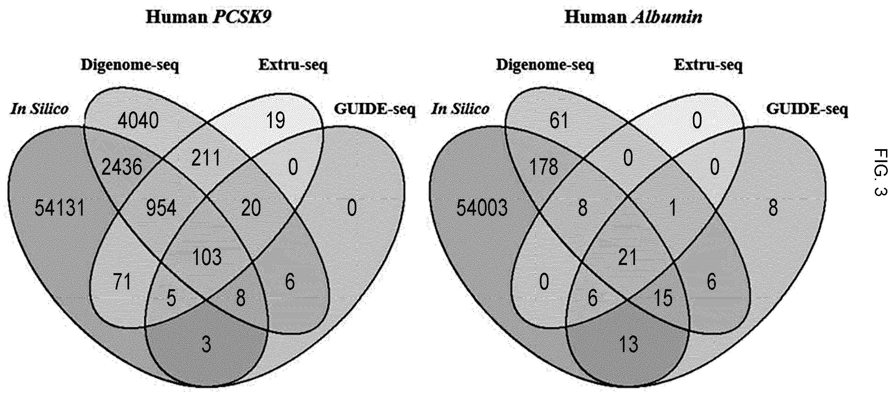

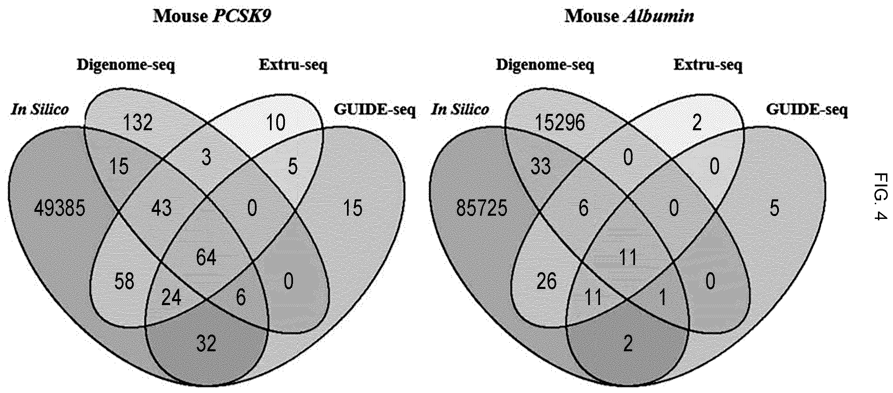

The inventors of the present application predicted off-target sties (candidate off-target sites) for sgRNAs (sgRNA targeting human *PCSK9,* sgRNA targeting human *Albumin,* sgRNA targeting mouse *PCSK9,* and sgRNA targeting mouse *Albumin)* by GUIDE-seq, Digenome-seq, Extru-seq, and in silico methods, and the results were compared. The comparison results are shown in FIGS. 3 and 4 using Venn diagrams. Specifically, FIG. 3 shows the comparison result for sgRNA targeting human *PCSK9,* and the comparison result for sgRNA targeting human *Albumin.* FIG. 4 shows the comparison result for sgRNA targeting mouse *PCSK9,* and the comparison result for sgRNA targeting mouse *Albumin.* Regarding the results of FIGS. 3 and 4, a human cell line (HEK293T) and a mouse cell line (NIH-3T3) were used.

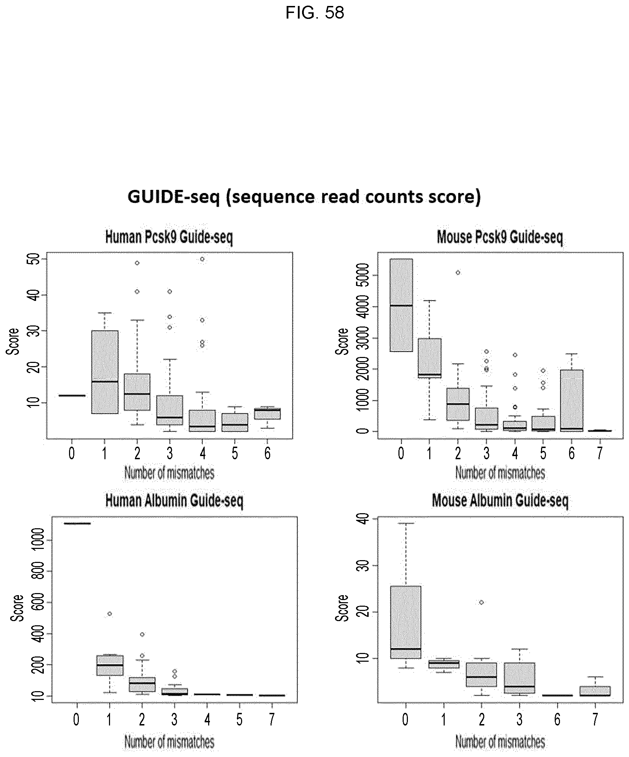

Candidate off-target loci were able to be ranked using the sequence read counts of GUIDE-seq and the DNA cleavage scores from Digenome-seq and Extru-seq. For in silico prediction based on CAS-OFFinder, there are no scores that can be used for these rankings. Accordingly, the inventors of the present application calculated prediction scores for candidate off-target sites for ranking using two different scripts of the machine learning studies (refer to the documents [Liu, Qiaoyue, et al. "Deep learning improves the ability of sgRNA off-target propensity prediction." BMC bioinformatics 21.1 (2020): 1-15.; and Doench, John G., et al. "Optimized sgRNA design to maximize activity and minimize off-target effects of CRISPR-Cas9." Nature biotechnology 34.2 (2016): 184-191.]). The prediction scores are as follows: CRISPR off-target predictor (CROP) scores (heuristic scores indicating if candidate off-target sites would be edited) and cutting frequency determination (CFD) scores (percent activity values provided to a penalty matrix based on mismatches of each possible type at each position in a guide RNA sequence). The distribution of sequence read counts, DNA cleavage, and in silico prediction scores for each candidate off-target locus were tabulated against the number of mismatches with guide sequences. The tables showing the distribution of sequence read counts, DNA cleavage scores, and in silico prediction scores according to the number of mismatches with guide sequences are shown in FIGS. 58 to 62.

Specifically, FIG. 58 shows the results related to the scores (scores calculated from the sequence read count results) of on-target and off-targets according to the number of mismatches, predicted using GUIDE-seq. The x-axis is indicated by the number of mismatches, and the score for each number of mismatches is shown. That is, the number of mismatches for on-target and off-target sites is displayed on the x-axis. Scores converted from sequence read counts are displayed on the y-axis.

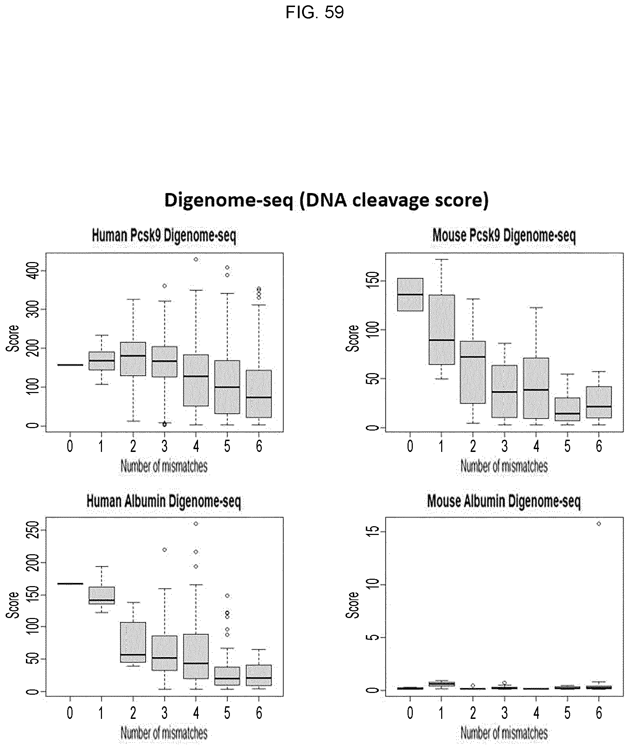

FIG. 59 shows the results related to the scores (cleavage scores of the Manhattan plot) of on-target and off-targets according to the number of mismatches, predicted using Digenome-seq. The number of mismatches is shown on the x-axis, and the score for each number of mismatches is shown. That is, the number of mismatches for on-target and off-target sites is shown on the x-axis. The cleavage score is shown on the y-axis.

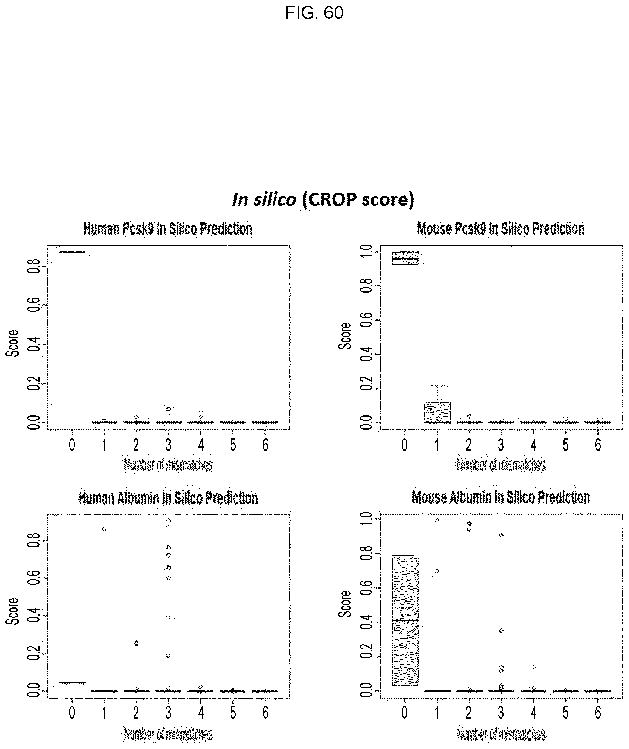

FIG. 60 shows the results related to scores (CROP scores) of on-target and off-targets according to the number of mismatches, predicted using an in silico system. The x-axis is indicated by the number of mismatches, showing scores for each number of mismatches. That is, the number of mismatches for on-target and off-targets is shown on the x-axis. The CROP score is shown on the y-axis.

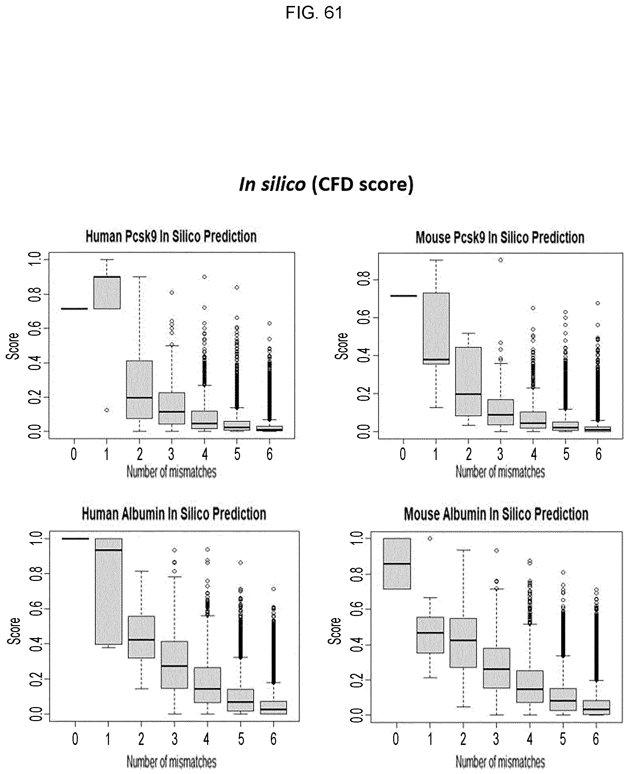

FIG. 61 shows the results related to scores (CFD scores) of on-target and off-targets according to the number of mismatches, predicted using an in silico system. The x-axis is indicated by the number of mismatches, showing scores for each number of mismatches. That is, the number of mismatches for on-target and off-targets is shown on the x-axis. The CFD score is shown on the y-axis.

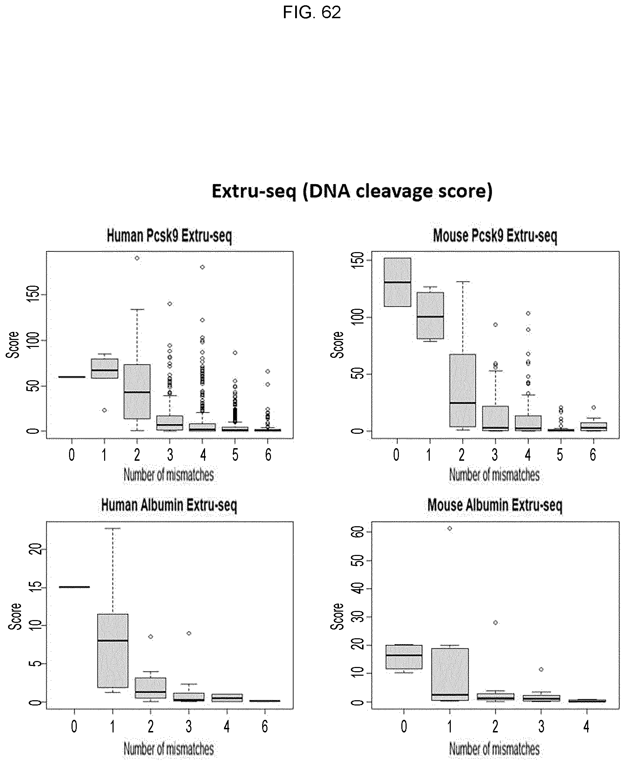

FIG. 62 shows the results related to scores (cleavage scores of the Manhattan plot) of on-target and off-targets according to the number of mismatches, predicted using Extru-seq. The x-axis is indicated by the number of mismatches, showing scores for each number of mismatches. That is, the number of mismatches for on-target and off-targets is shown on the x-axis. The cleavage score is shown on the y-axis.

It was expected that as the number of mismatches increases, the corresponding prediction scores decrease. While GUIDE-seq and in silico predictions followed such trend, outliers with high DNA cleavage scores were observed even when there were 4, 5, or 6 mismatches in the Digenome-seq results. When DNA cleavage scores for off-target site candidates were calculated using the Extru-seq approach, unlike the Digenome-seq results, high DNA cleavage scores for off-target candidates of sgRNAs with 4 or more mismatches were not observed. This result indicates that Extru-seq identified fewer false positives than Digenome-seq. The inventors of the present application confirmed such an idea by validation of the off-target candidates with the top scores.

### Result 6. Validation rates of GUIDE-seq and Extru-seq

GUIDE-seq and Extru-seq showed high validation rates. The validation of predicted off-target loci was performed in a human cell line and a mouse model. In the human cell line experiment, plasmids encoding Cas9 protein and sgRNA were transfected into HEK293T cells. In a mouse experiment, sequences encoding Cas9 protein and sgRNA were packaged in adeno-associated virus (AAV) serotype 8 (i.e., AAV8). Afterward, these AAVs were delivered to a C57BL/6 mouse through systemic or subretinal injection. Since only the subretinal injection showed a high frequency of on-target indel formation, retinal pigment epithelial cells of the model were used in the validation experiment.

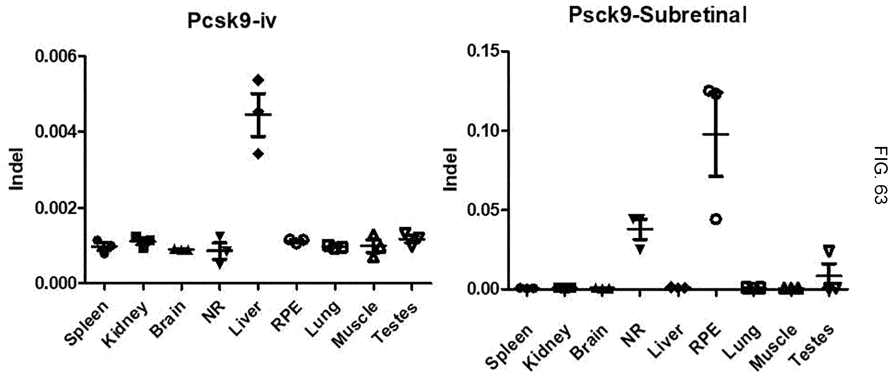

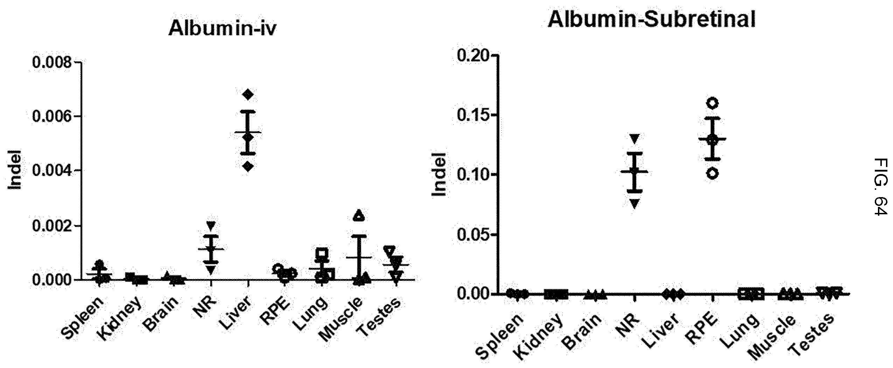

In the subretinal injection and systemic injection, the results for indel formation frequencies are shown in FIGS. 63 and 64. Specifically, the indel rates were calculated through the analysis of genomic DNA obtained from organs of the C57BL/6 mouse into which two AAV8 vectors expressing Cas9 and sgRNA targeting *PCSK9* or *Albumin,* respectively, were injected. In FIGS. 63 and 64, a result marked as iv represents a result for systemic injection, and a result marked as subretinal represents a result for subretinal injection. In FIGS. 63 and 64, error bars indicate s.e.m (n=3). NR indicates neural retina, and RPE indicates retinal pigment epithelial cells.

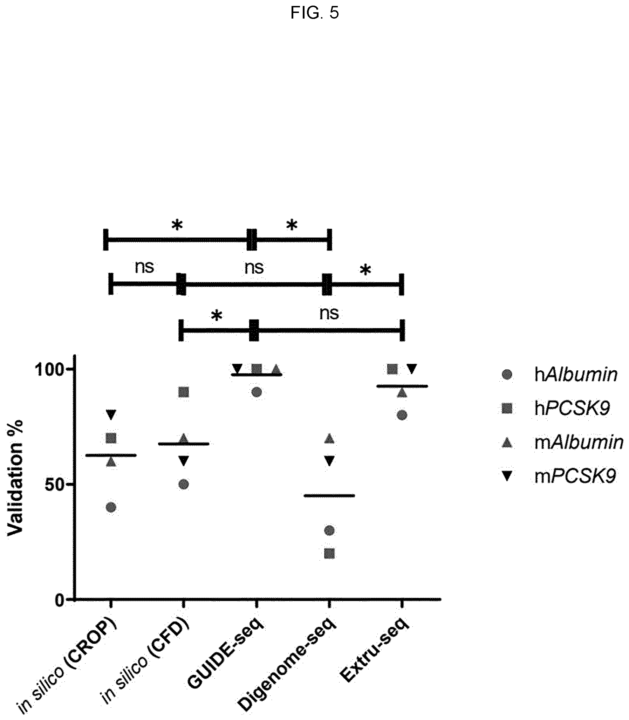

Top 10 candidates of each prediction method were examined using targeted deep sequencing. As a result of the examination, Extru-seq showed a validation rate of 92.5%, and GUIDE-seq showed a validation rate of 97.5%. However, Digenome-seq showed a validation rate of 45%, and the in silico method showed a validation rate of 62.5% in CROP, and a validation rate of 67.5% in CFD. Extru-seq and GUIDE-seq showed much higher validation rates than Digenome-seq and the in silico methods. The results of the validation rates according to each prediction method are shown in FIG. 5 and Table 4.

Specifically, FIG. 5 shows the validation rates of the top off-target sites, predicted by the in silico method, GUIDE-seq, Digenome-seq, and Extru-seq. This shows the experimental results in human cells and mouse cells for promiscuous sgRNA targeting *PCSK9* and *Albumin* (*P<0.05, ns, no significance in two-sided unpaired Mann-Whitney test).

Table 4 below shows the validation results obtained through targeted deep sequencing of the top 10 off-target sites (using sgRNA targeting human *PCSK9,* sgRNA targeting human *Albumin,* sgRNA targeting mouse *PCSK9,* and sgRNA targeting mouse *Albumin*) predicted by each method. For validation, the indel frequency at an off-target site must be higher than 0.1%, and the equation '(indel frequency at the off-target locus)/(indel frequency in the control with no Cas9 treatment) > 2' must be satisfied (refer to the document [Frangoul, Haydar, et al. "CRISPR-Cas9 gene editing for sickle cell disease and β-thalassemia." New England Journal of Medicine 384.3 (2021): 252-260.]). * indicates that a target is manually confirmed. Here, the manually confirmed (off) target site refers to a site determined as positive when IGV software was used among sites determined as negative when using Digenome software. As the confirmation result using the IGV software, one site was additionally identified in the human *PCSK9* sample. Five sites were additionally identified in the human *Albumin* sample. Three sites were additionally identified in the mouse *PCSK9* sample. Three sites were additionally identified in the mouse *Albumin* sample.

**Table 4. Validation results for top 10 candidates off-target site predicted by each off-target prediction method**

| (a) | | | | | | |
|---|---|---|---|---|---|---|
| Targeted gene | **human *PCSK9*** | In-silico (CROP) | In-silico (CFD) | GUIDE-seq | Digenome-seq | Extru-seq |
| Number of candidate off-target sites found | | **57711** | **57711** | **145** | **7778** | **1383+1*** |
| Validation results for the 10 sites with the highest scores | Validated | 7 | 9 | 10 | 2 | 10 |
| | FALSE | 3 | 1 | 0 | 8 | 0 |
| | Total | 10 | 10 | 10 | 10 | 10 |
| | Validation ratio | 70.00% | 90.00% | 100.00% | 20.00% | 100.00% |

| (b) | | | | | | |
|---|---|---|---|---|---|---|
| Targeted gene | **human *Albumin*** | In-silico (CROP) | In-silico (CFD) | GUIDE-seq | Digenome-seq | Extru-seq |
| Number of candidate off-target sites found | | **54244** | **54244** | **70** | **290** | **36+5*** |
| Validation results for the 10 sites with the highest scores | Validated | 4 | 5 | 9 | 3 | 8 |
| | FALSE | 6 | 5 | 1 | 7 | 2 |
| | Total | 10 | 10 | 10 | 10 | 10 |
| | Validation ratio | 40.00% | 50.00% | 90.00% | 30.00% | 80.00% |

| (c) | | | | | | |
|---|---|---|---|---|---|---|
| Targeted gene | **Mouse *PCSK9*** | In-silico (CROP) | In-silico (CFD) | GUIDE-seq | Digenome-seq | Extru-seq |
| Number of candidate off-target sites found | | **49627** | **49627** | **146** | **263** | **207+3*** |
| Validation results for the 10 sites with the highest scores | Validated | 8 | 6 | 10 | 6 | 10 |
| | FALSE | 2 | 4 | 0 | 4 | 0 |
| | Total | 10 | 10 | 10 | 10 | 10 |
| | Validation ratio | 80.00% | 60.00% | 100.00% | 60.00% | 100.00 % |

| (d) | | | | | | |
|---|---|---|---|---|---|---|
| Targeted gene | **Mouse *Albumin*** | In-silico (CROP) | In-silico (CFD) | GUIDE-seq | Digenome-seq | Extru-seq |
| Number of candidate off-target sites found | | **85815** | **85815** | **30** | **15347** | **56+3*** |
| Validation results for the 10 sites with the highest scores | Validated | 6 | 7 | 10 | 7 | 9 |
| | FALSE | 4 | 3 | 0 | 3 | 1 |
| | Total | 10 | 10 | 10 | 10 | 10 |
| | Validation ratio | 60.00% | 70.00% | 100.00% | 70.00% | 90.00% |

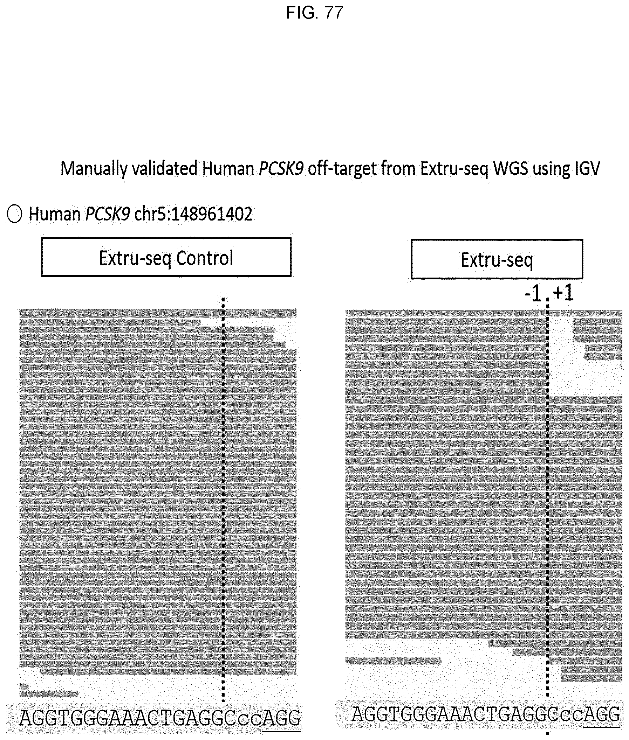

Information on manually identified targets is shown in FIGS. 77 to 116. Specifically, WGS data visualized using IGV for off-target sites manually validated from Extru-seq are shown in FIGS. 77 to 116. Regarding FIGS. 77 to 116, the sequences of the off-target sites are shown, and the sequences mismatching guide sequences are indicated in small letters. The PAM sequence was underlined.

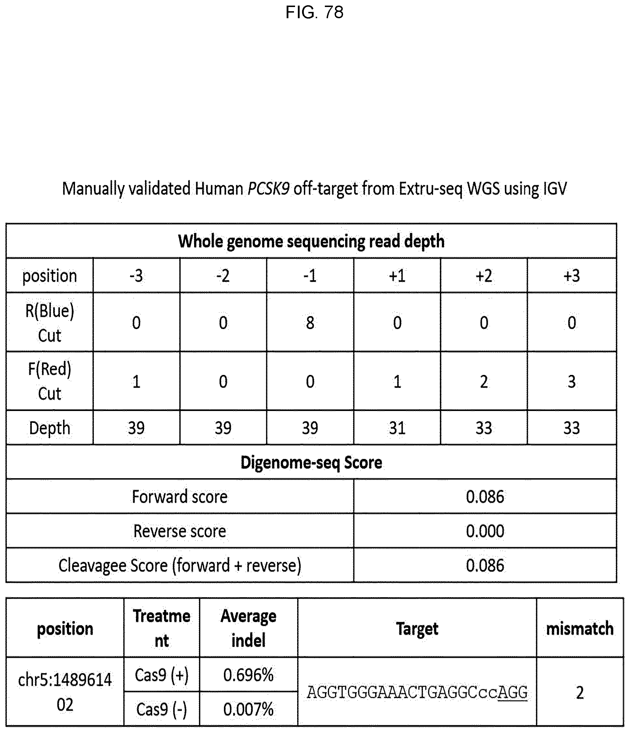

Regarding FIGS. 77 and 78, the sequence AGGTGGGAAACTGAGGCccAGG (SEQ ID NO: 52) of an off-target site is disclosed.

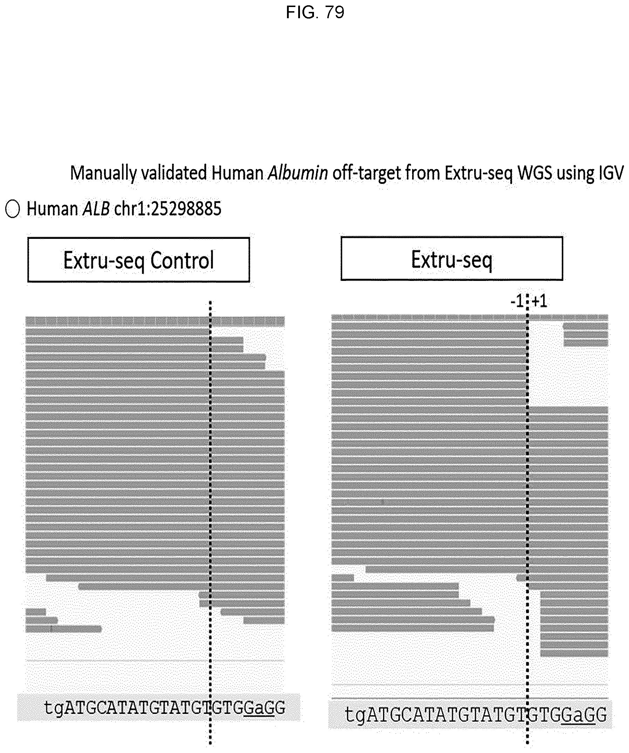

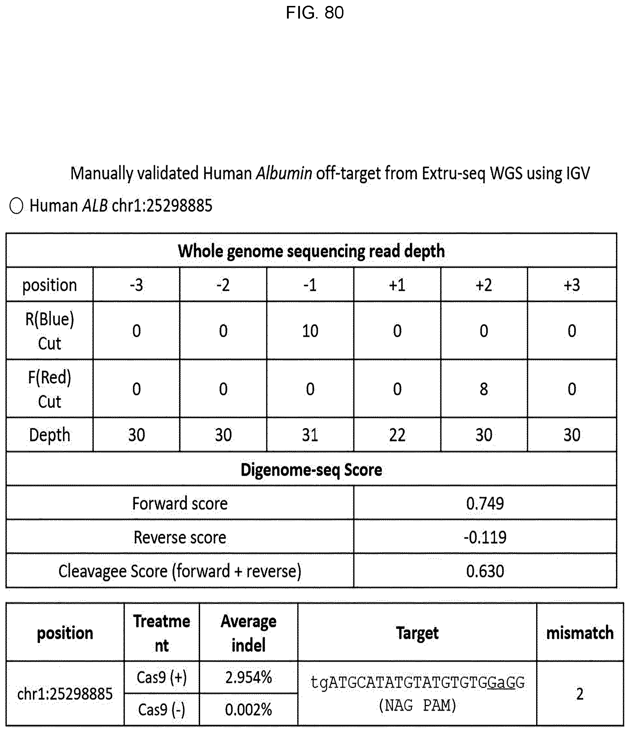

Regarding FIGS. 79 and 80, the sequence tgATGCATATGTATGTGTGGaGG (SEQ ID NO: 53) of an off-target site is disclosed.

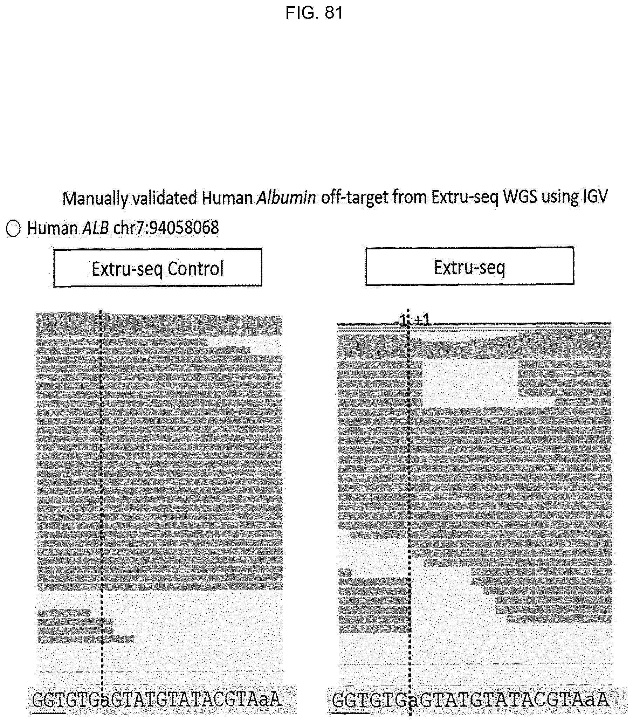

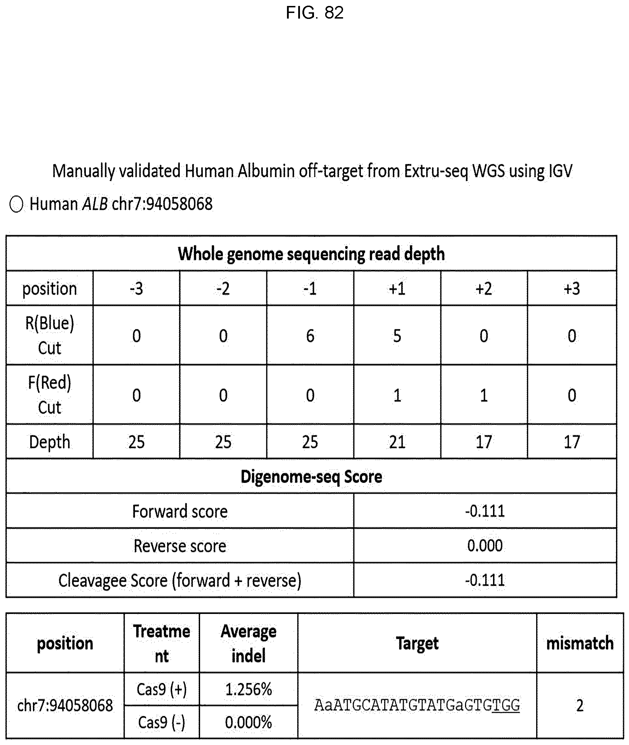

Regarding FIGS. 81 and 82, the sequence AaATGCATATGTATGaGTGTGG (SEQ ID NO: 54) of an off-target site is disclosed.

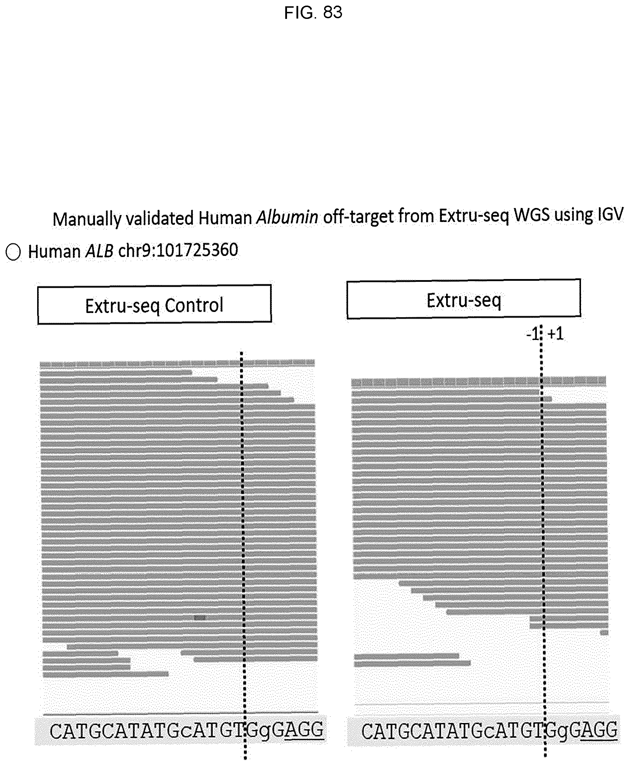

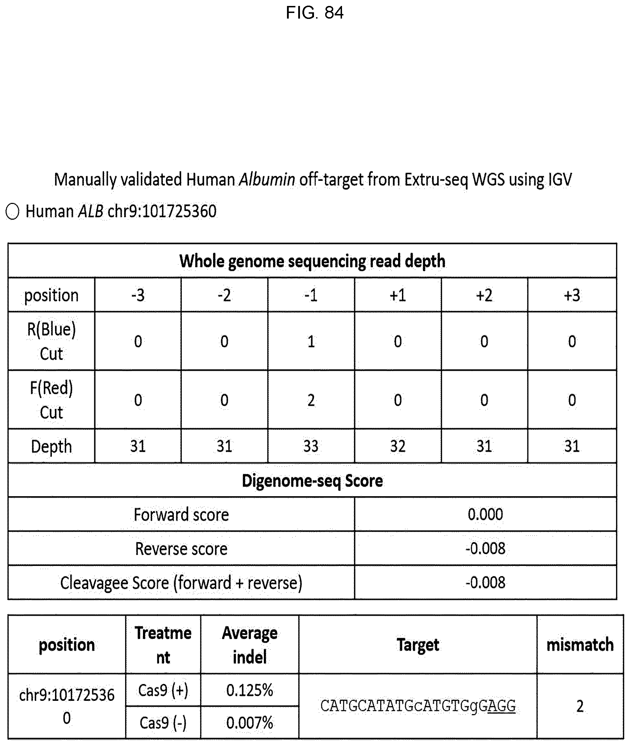

Regarding FIGS. 83 and 84, the sequence CATGCATATGcATGTGgGAGG (SEQ ID NO: 55) of an off-target site is disclosed.

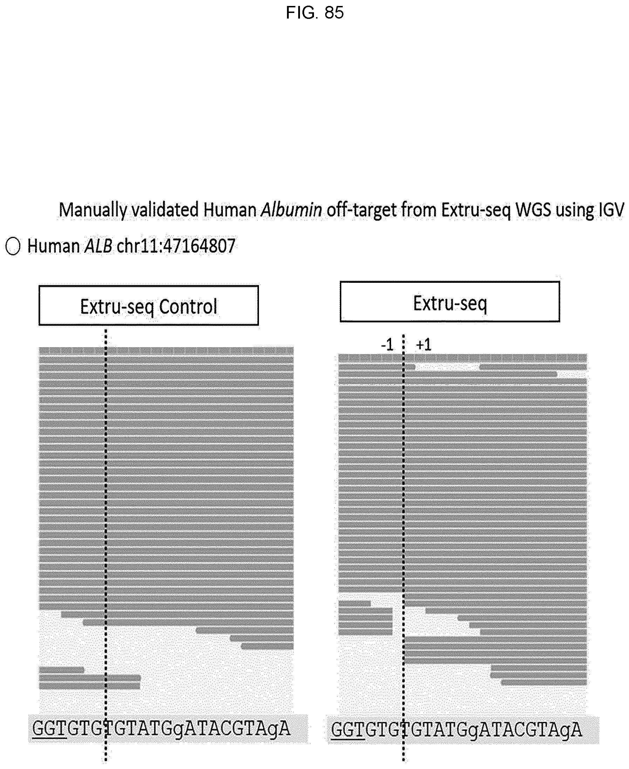

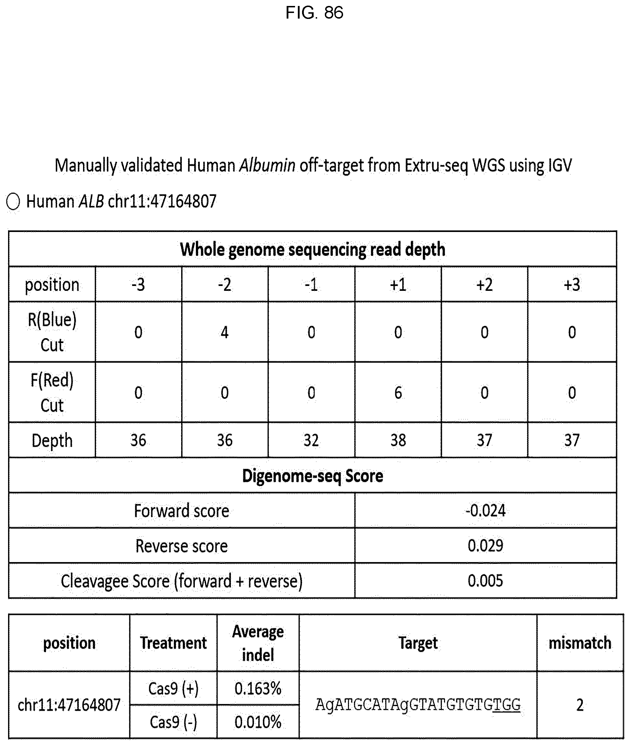

Regarding FIGS. 85 and 86, the sequence AgATGCATAgGTATGTGTGTGG (SEQ ID NO: 56) of an off-target site is disclosed.

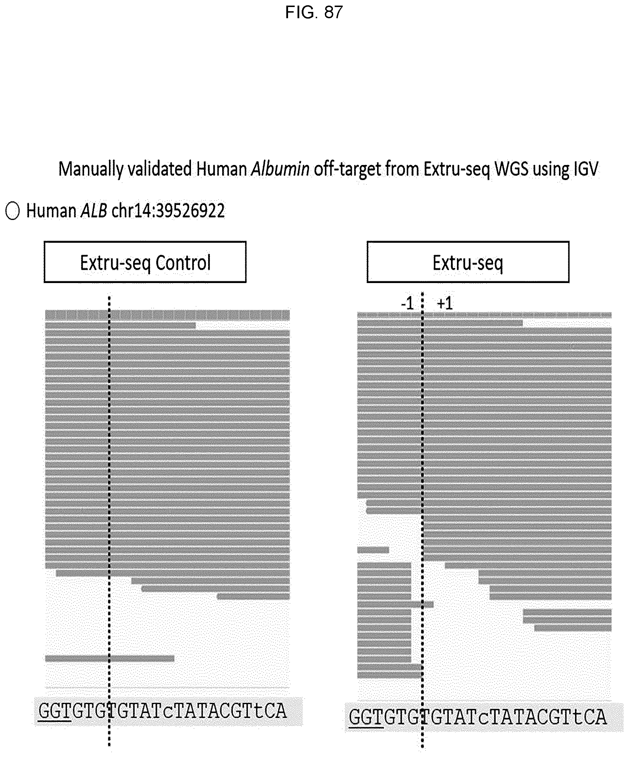

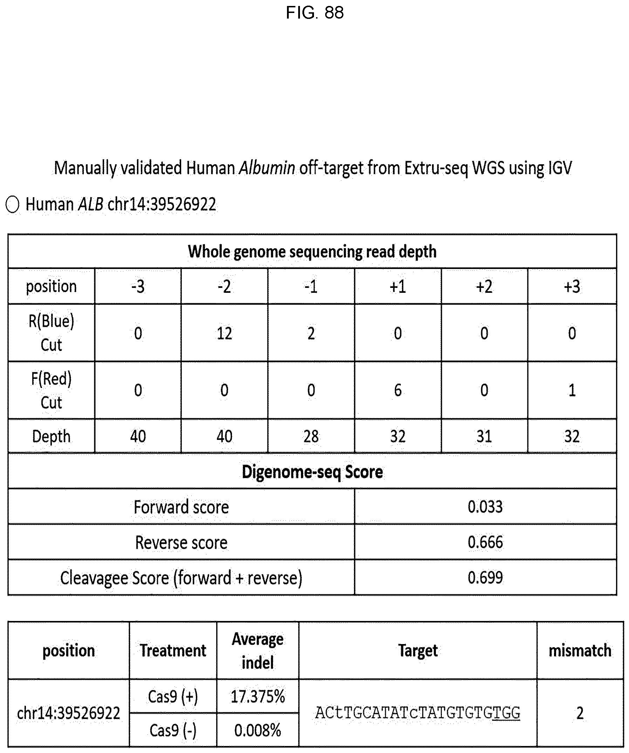

Regarding FIGS. 87 and 88, the sequence ACtTGCATATcTATGTGTGTGG (SEQ ID NO: 57) of an off-target site is disclosed.

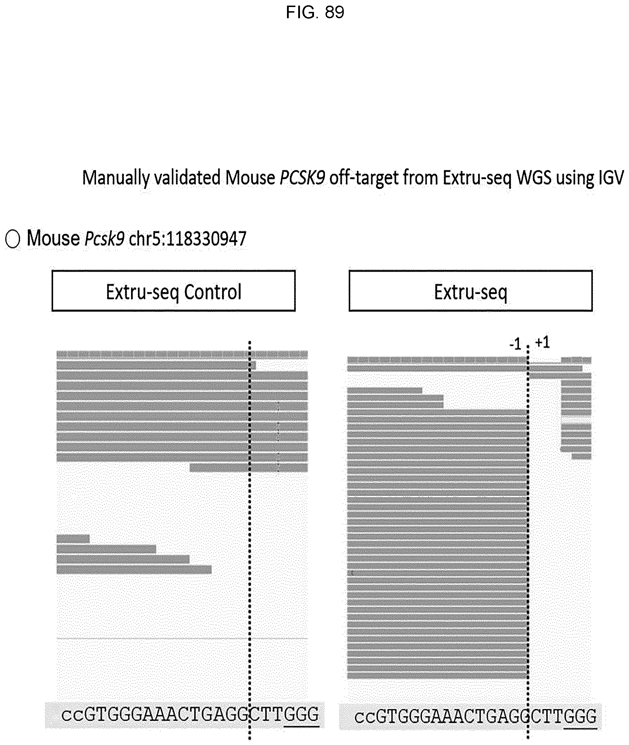

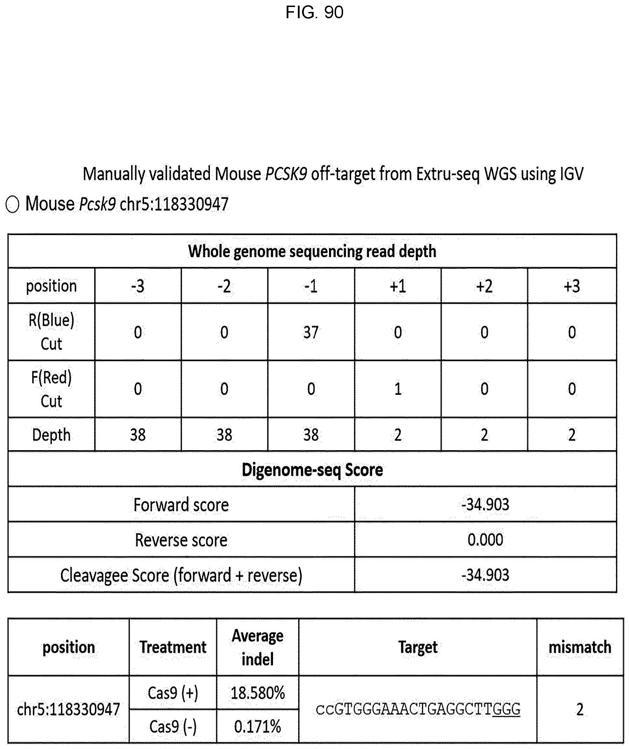

Regarding FIGS. 89 and 90, the sequence ccGTGGGAAACTGAGGCTTGGG (SEQ ID NO: 58) of an off-target site is disclosed.

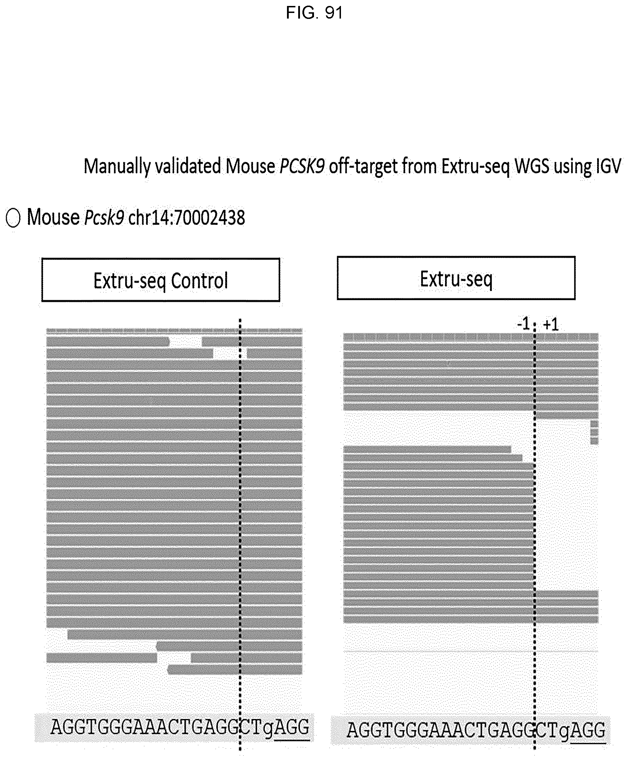

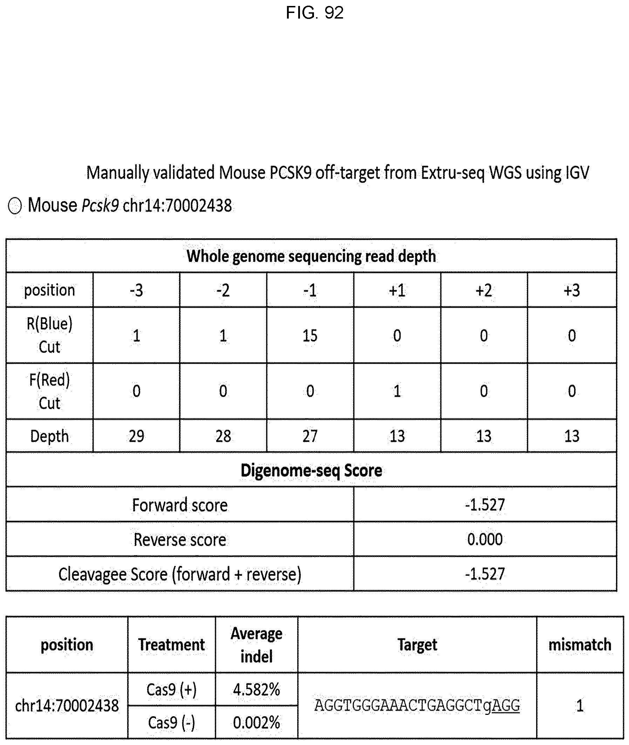

Regarding FIGS. 91 and 92, the sequence AGGTGGGAAACTGAGGCTgAGG (SEQ ID NO: 59) of an off-target site is disclosed.

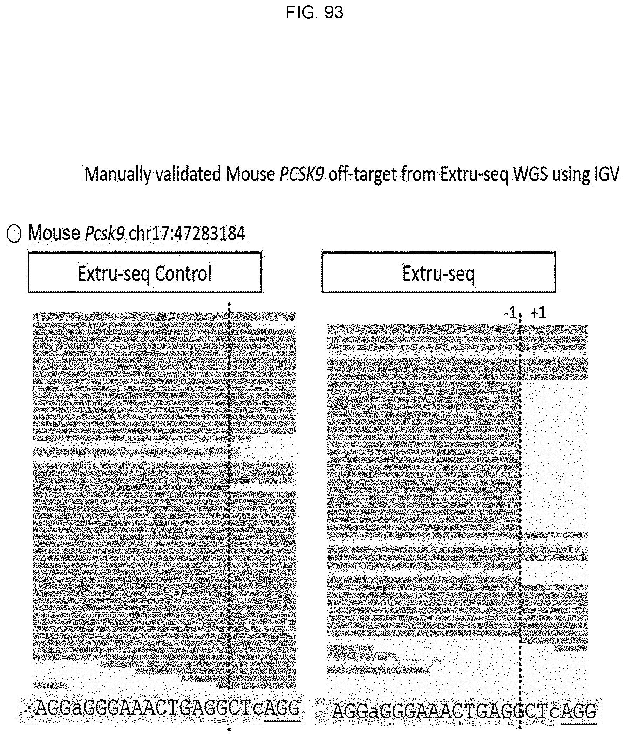

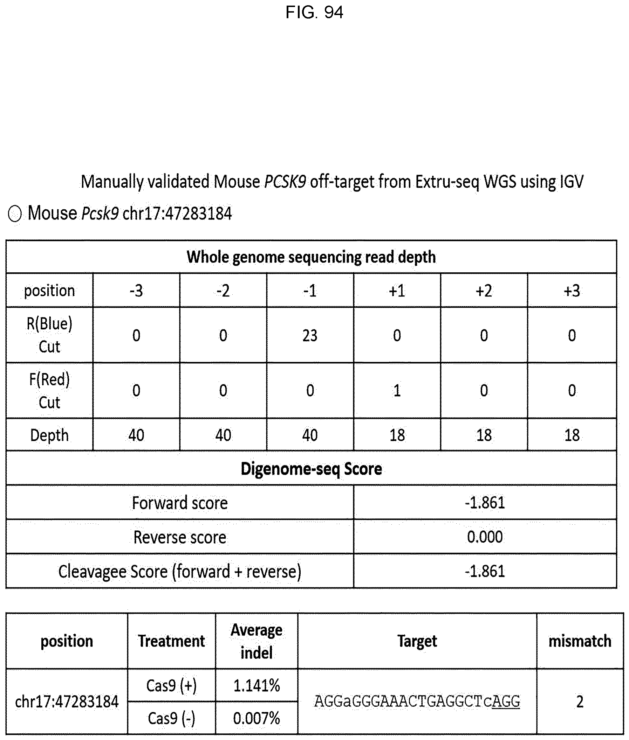

Regarding FIGS. 93 and 94, the sequence AGGaGGGAAACTGAGGCTcAGG (SEQ ID NO: 60) of an off-target site is disclosed.

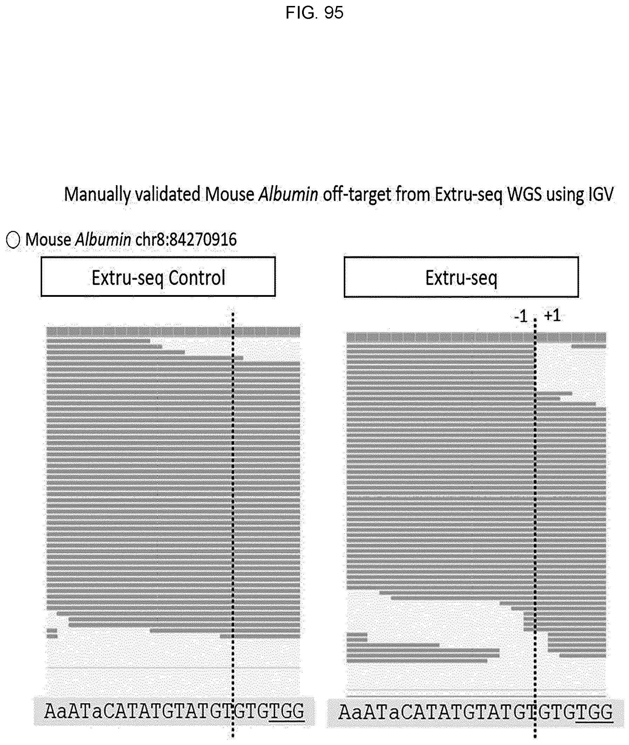

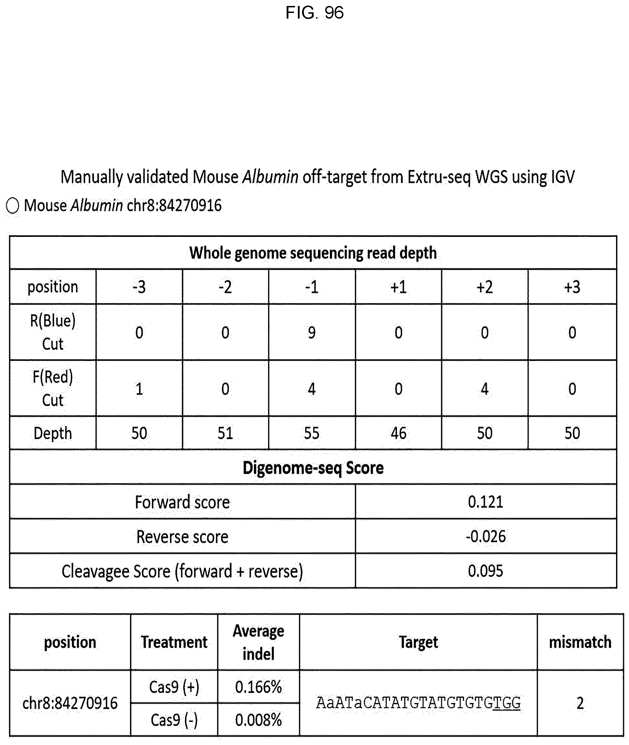

Regarding FIGS. 95 and 96, the sequence AaATaCATATGTATGTGTGTGG (SEQ ID NO: 61) of an off-target site is disclosed.

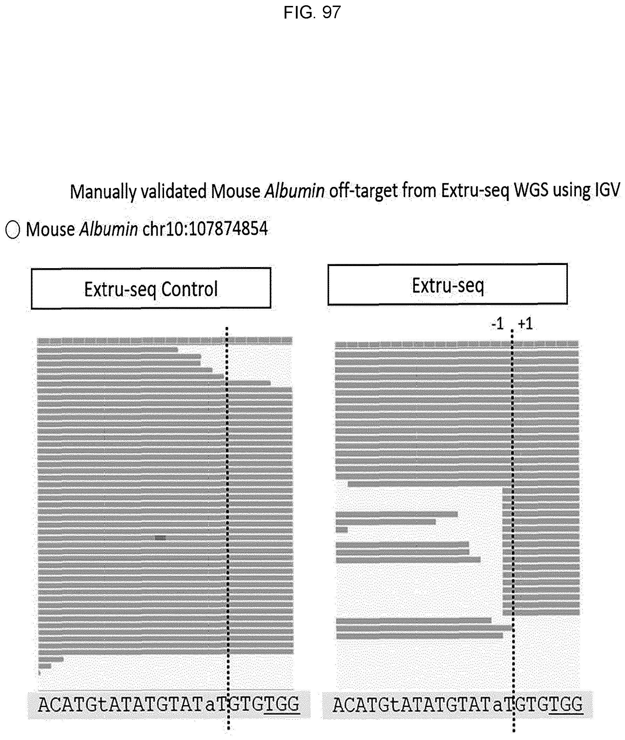

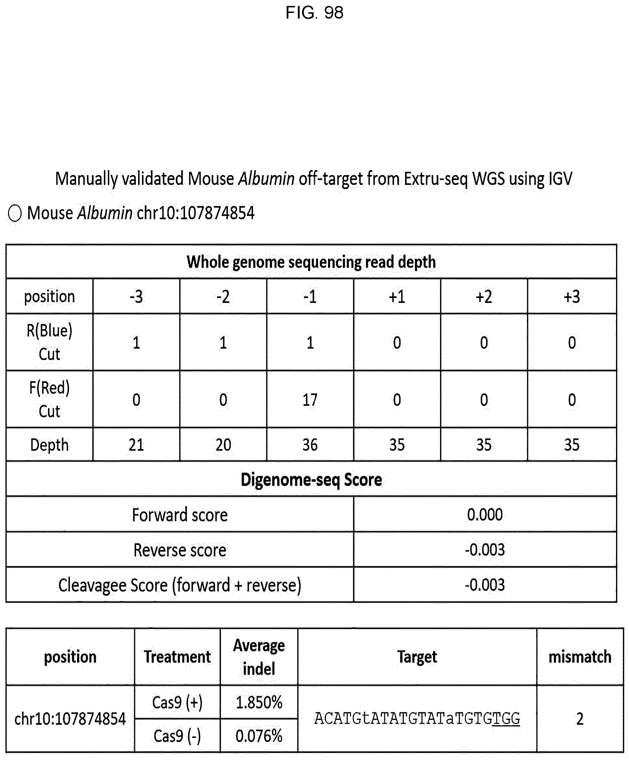

Regarding FIGS. 97 and 98, the sequence ACATGtATATGTATaTGTGTGG (SEQ ID NO: 62) of an off-target site is disclosed.

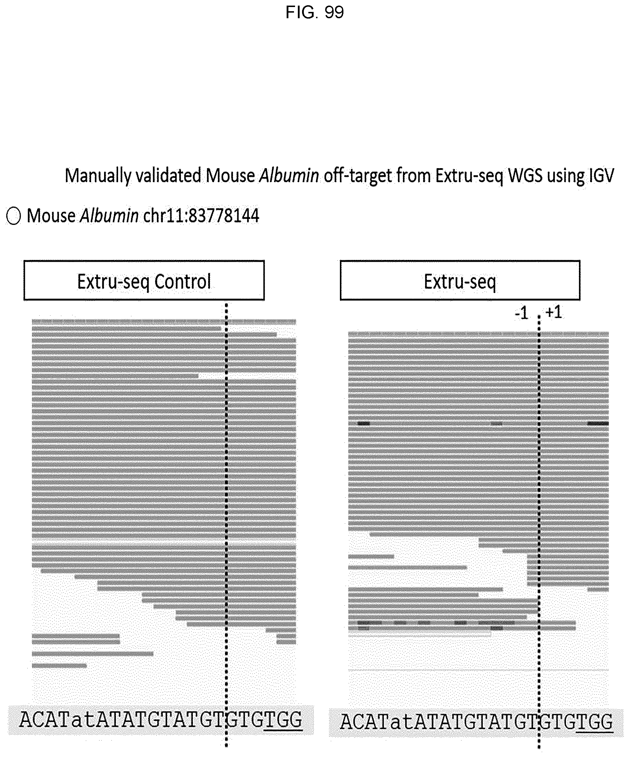

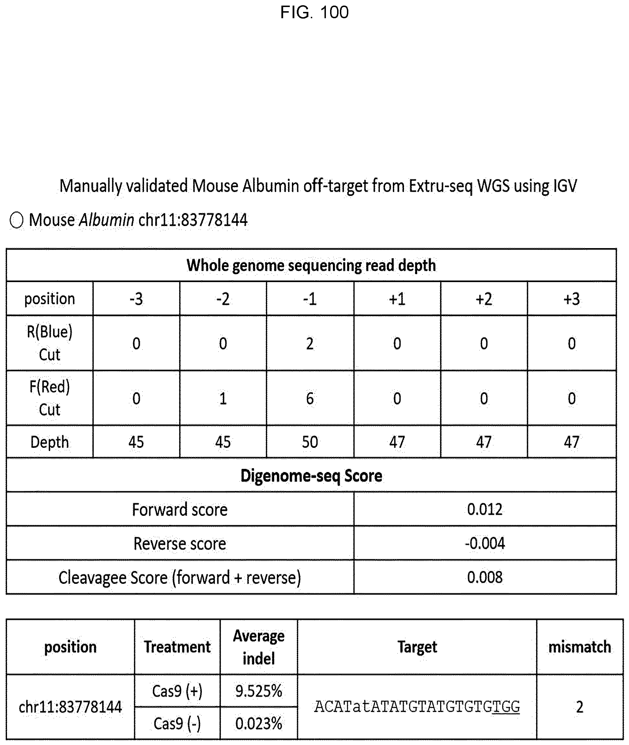

Regarding FIGS. 99 and 100, the sequence ACATatATATGTATGTGTGTGG (SEQ ID NO: 63) of an off-target site is disclosed.

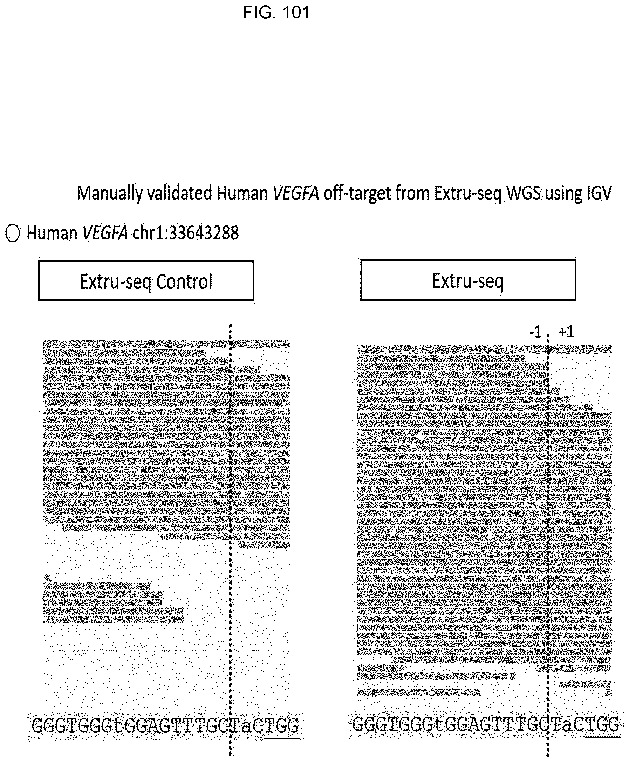

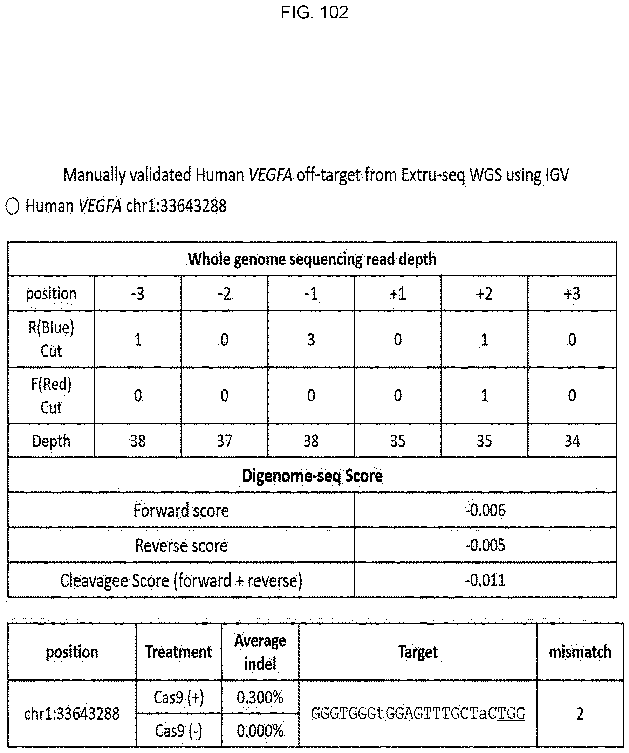

Regarding FIGS. 101 and 102, the sequence GGGTGGGtGGAGTTTGCTaCTGG (SEQ ID NO: 64) of an off-target site is disclosed.

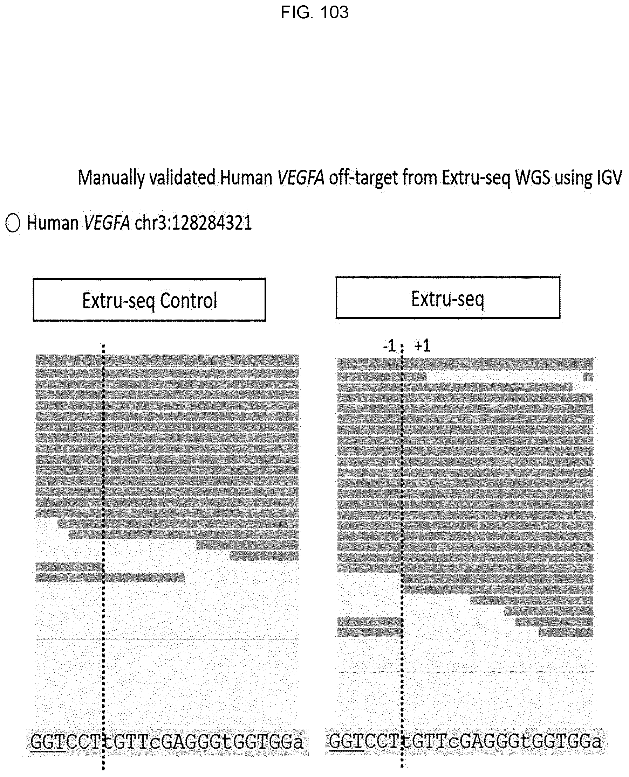

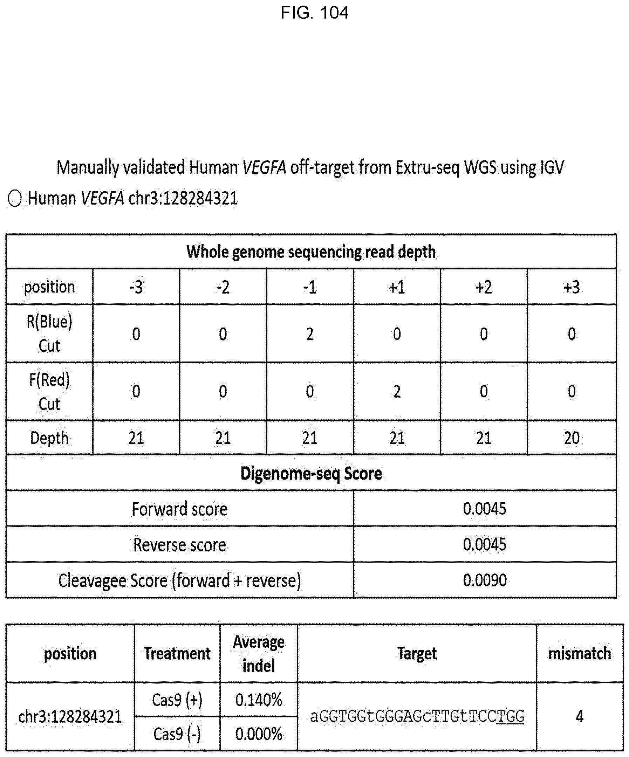

Regarding FIGS. 103 and 104, the sequence aGGTGGtGGGAGcTTGtTCCTGG (SEQ ID NO: 65) of an off-target site is disclosed.

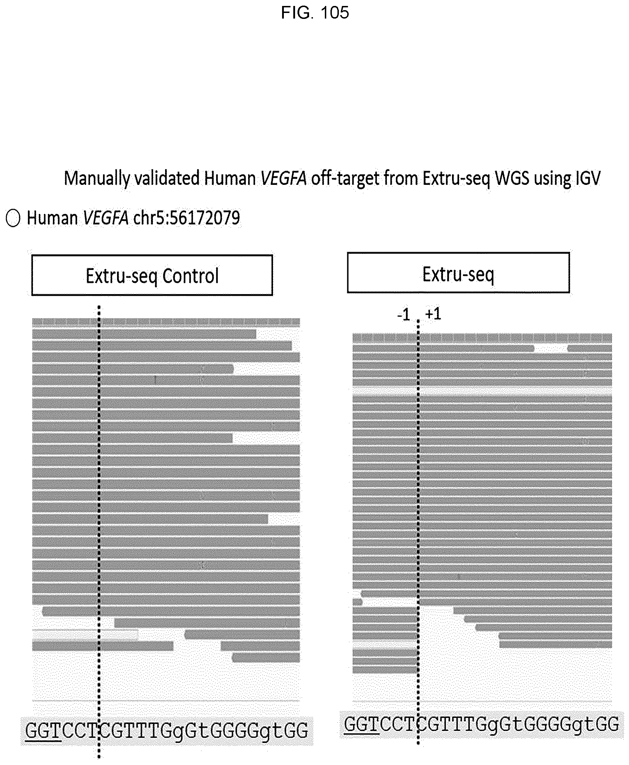

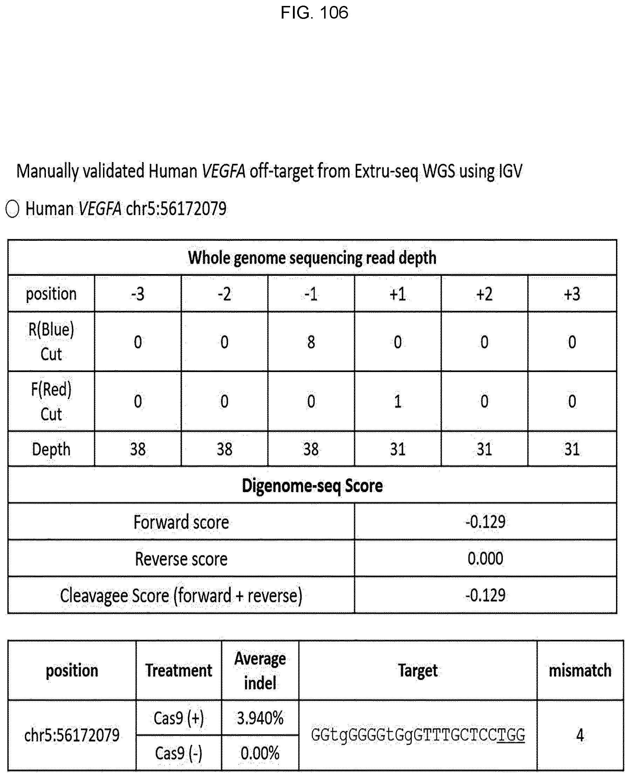

Regarding FIGS. 105 and 106, the sequence GGtgGGGGtGgGTTTGCTCCTGG (SEQ ID NO: 66) of an off-target site is disclosed.

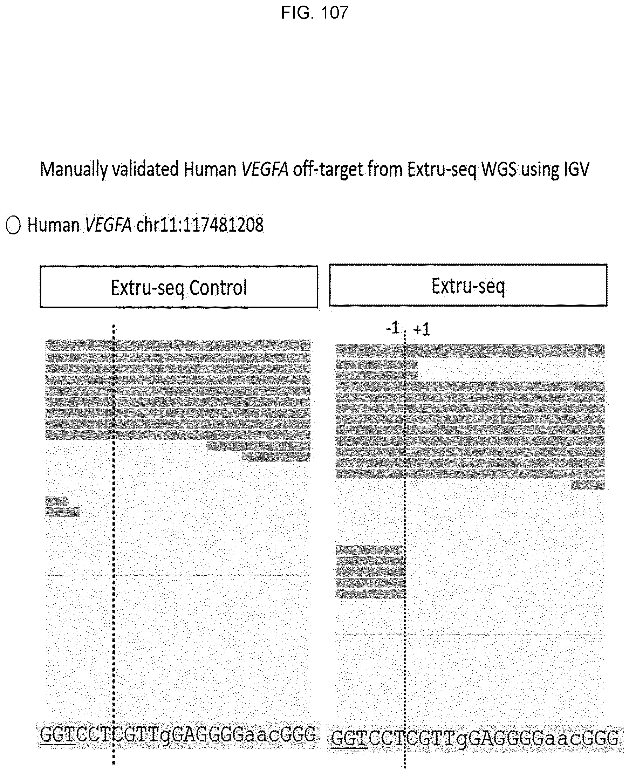

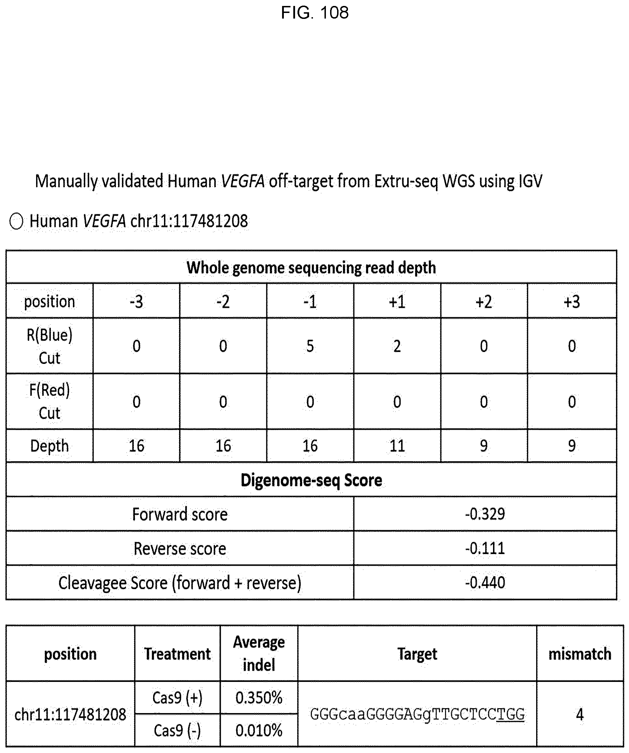

Regarding FIGS. 107 and 108, the sequence GGGcaaGGGGAGgTTGCTCCTGG (SEQ ID NO: 67) of an off-target site is disclosed.

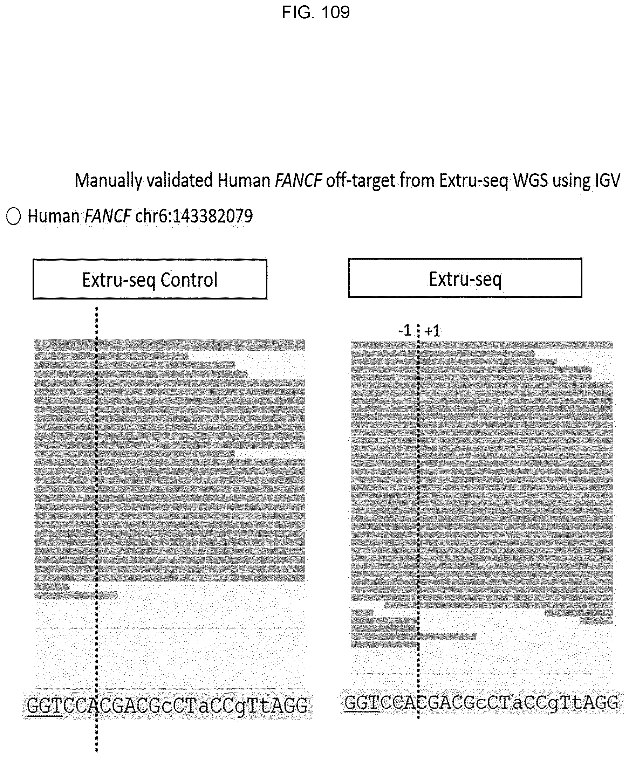

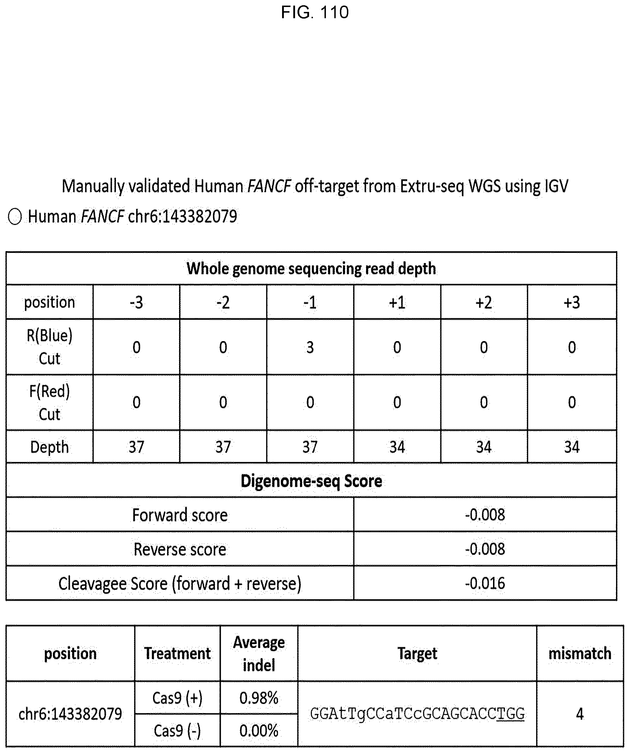

Regarding FIGS. 109 and 110, the sequence GGAtTgCCaTCcGCAGCACCTGG (SEQ ID NO: 68) of an off-target site is disclosed.

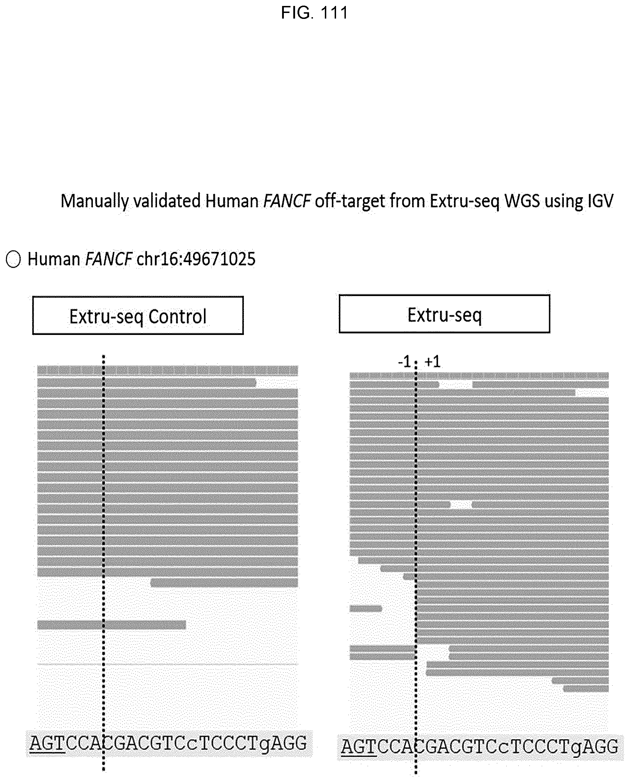

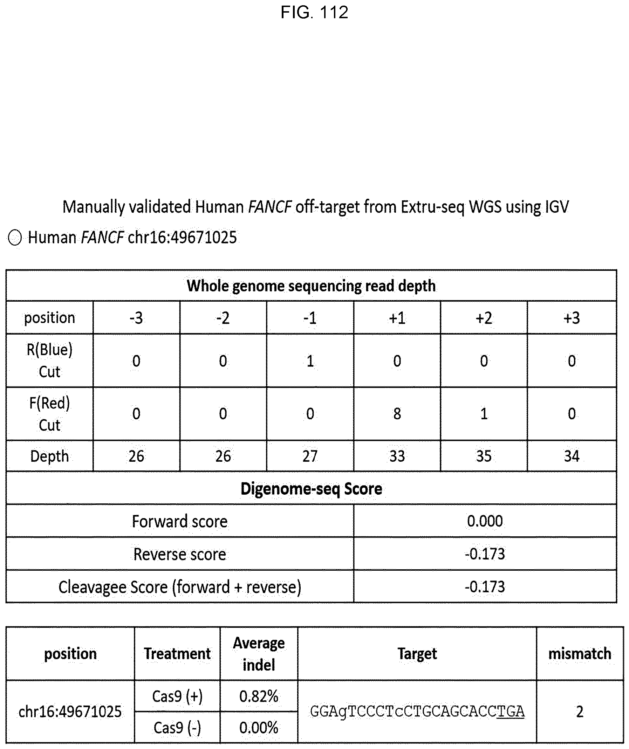

Regarding FIGS. 111 and 112, the sequence GGAgTCCCTcCTGCAGCACCTGA (SEQ ID NO: 69) of an off-target site is disclosed.

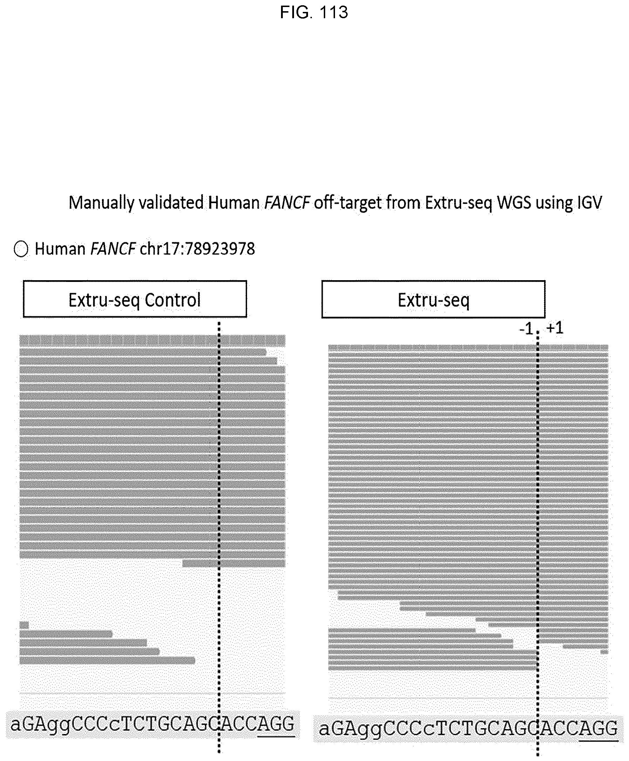

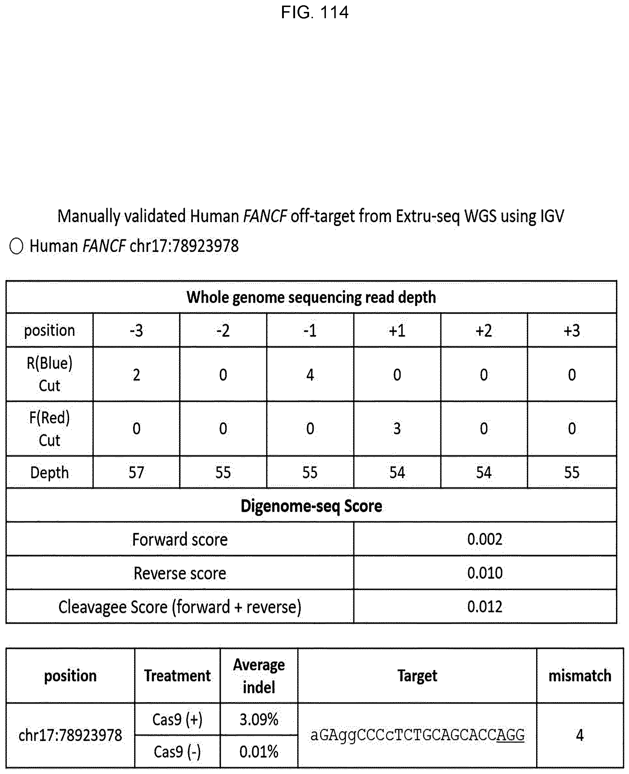

Regarding FIGS. 113 and 114, the sequence aGAggCCCcTCTGCAGCACCAGG (SEQ ID NO: 70) of an off-target site is disclosed.

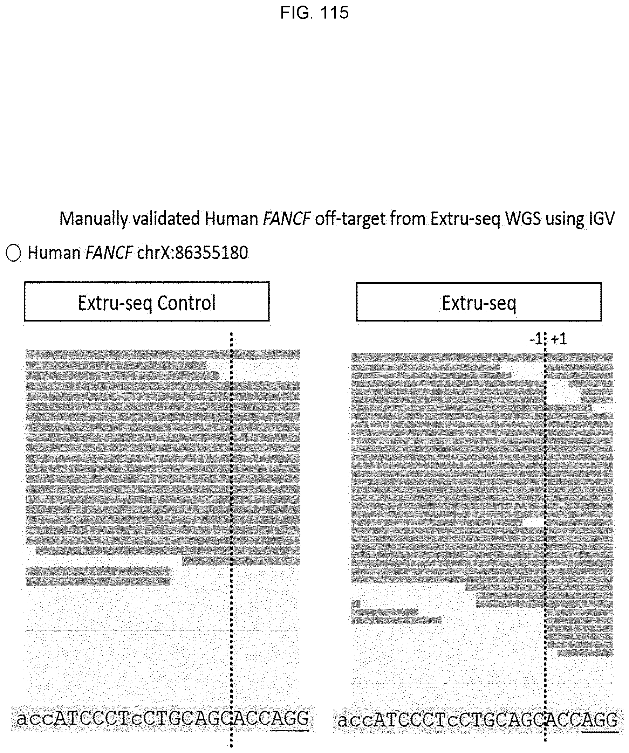

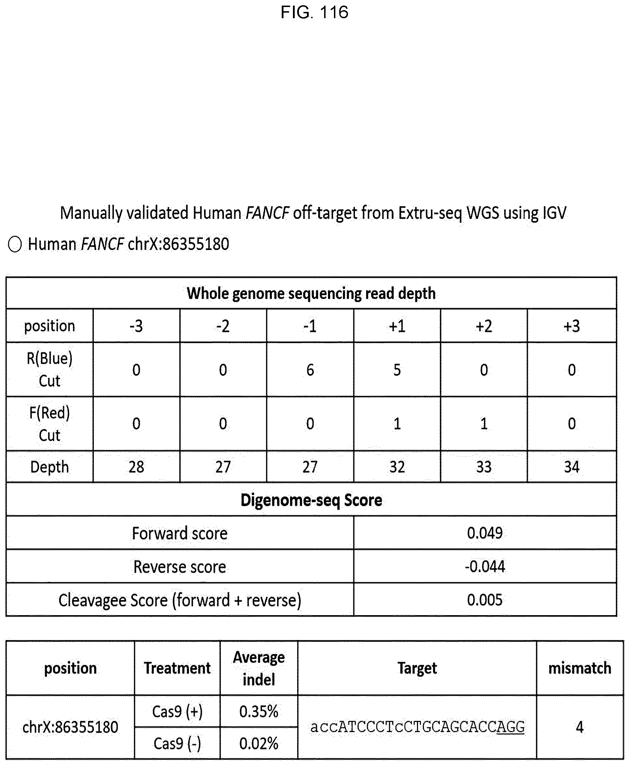

Regarding FIGS. 115 and 116, the sequence accATCCCTcCTGCAGCACCAGG (SEQ ID NO: 71) of an off-target site is disclosed.

### Result 7. Further comparison of Extru-seq, GUIDE-seq, Digenome-seq, and DIG-seq

Digenome-seq uses purified genomic DNA from which components like chromatin proteins are lost. To overcome this problem, previous studies developed an improved version of Digenome-seq, named DIG-seq. DIG-seq, which uses cell-free chromatin DNA rather than histone-free DNA, predicted fewer false positive than Digenome-seq. A mild detergent used to lyse cells in the DIG-seq approach can affect the chromatin state of cellular DNA, it may affect a Cas9 cleavage mechanism, so the inventors of the present application expected that Extru-seq using a physical force for cell lysis would reflect more features of a cell-based method compared to DIG-seq.

To compare Extru-seq with another in vitro method, the inventors of the present application performed GUIDE-seq and Extru-seq using guide sequences targeting *FANCF, VEGFA,* and *HBB* in HeLa cells. Each of the guide sequences targeting *FANCF, VEGFA,* and *HBB* is used to compare DIG-seq and Digenome-seq in previous studies (refer to the document [Kim, Daesik, and Jin-Soo Kim. "DIG-seq: a genome-wide CRISPR off-target profiling method using chromatin DNA." Genome research 28.12 (2018): 1894-1900.]).

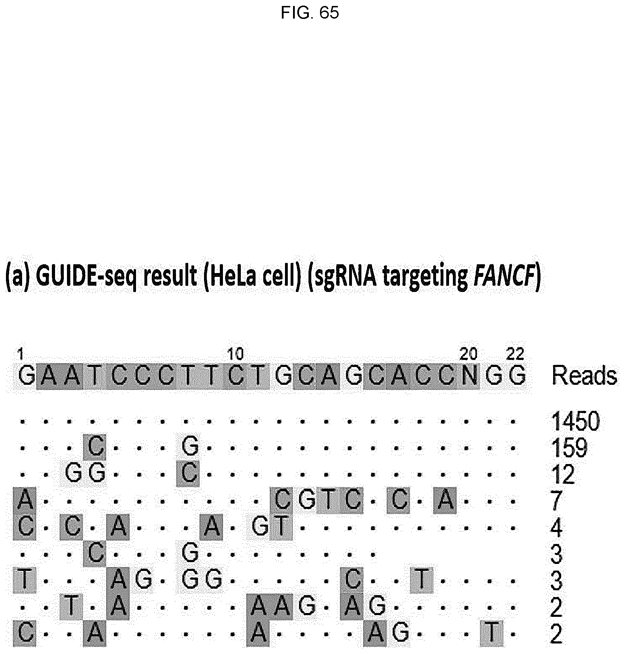

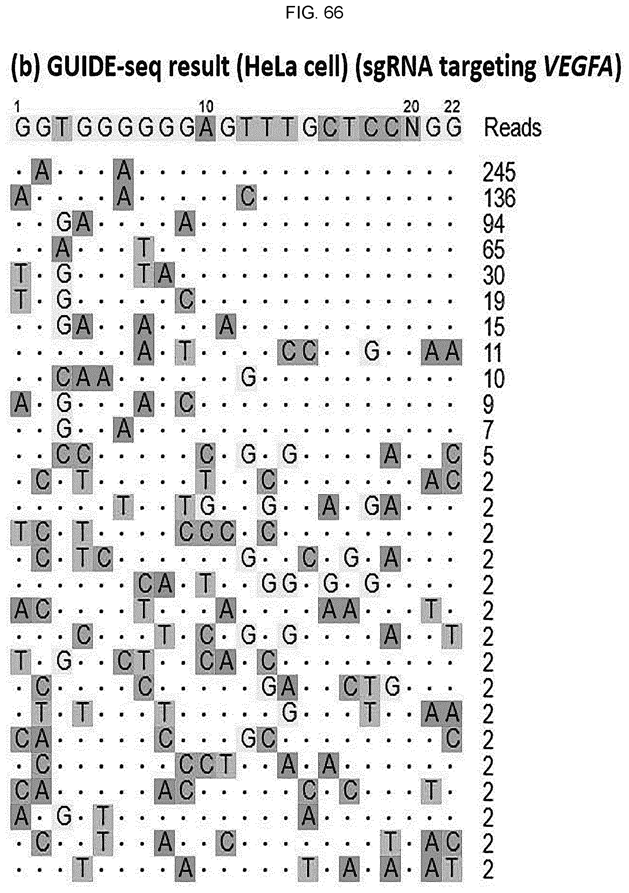

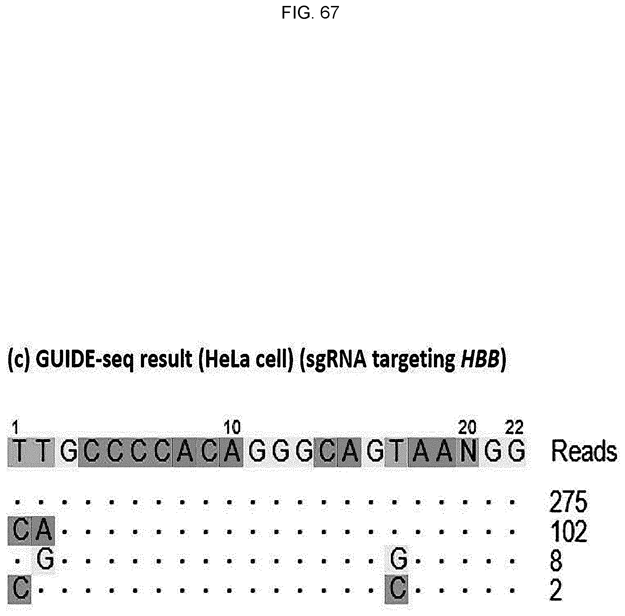

The GUIDE-seq results in HeLa cells are shown in FIGS. 65 to 67 (using sgRNAs targeting *FANCF, VEGFA,* and *HBB*)*.* Regarding FIG. 65, the target sequence (comprising PAM) GAATCCCTTCTGCAGCACCNGG (SEQ ID NO: 46) is shown. Regarding FIG. 66, the target sequence (comprising PAM) GGTGGGGGGAGTTTGCTCCNGG (SEQ ID NO: 47) is shown. Regarding FIG. 67, the target sequence (comprising PAM) TTGCCCCACAGGGCAGTAANGG (SEQ ID NO: 48) is shown. Specifically, FIG. 65 shows the result (sequence read result) of predicting off-targets through GUIDE-seq, using *FANCF-*targeting sgRNA. FIG. 66 shows the result (sequence read result) of predicting off-targets through GUIDE-seq, using *VEGFA*-targeting sgRNA. FIG. 67 shows the result (sequence read result) of predicting off-targets through GUIDE-seq, using *HBB*-targeting sgRNA.

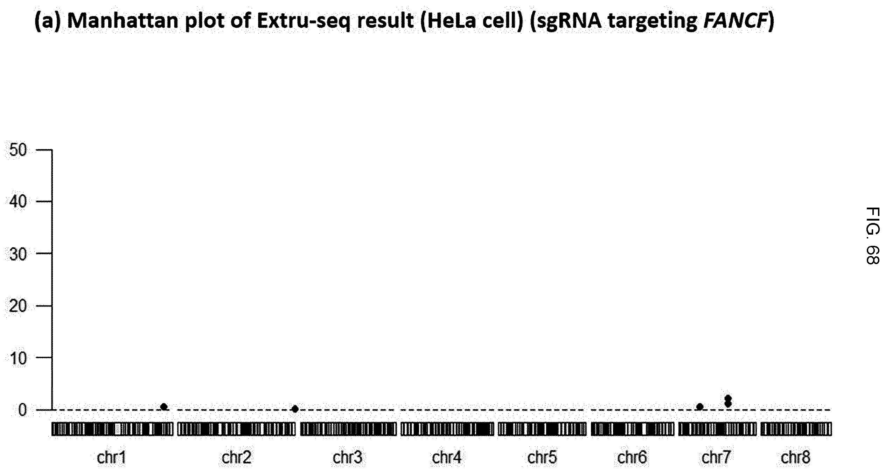

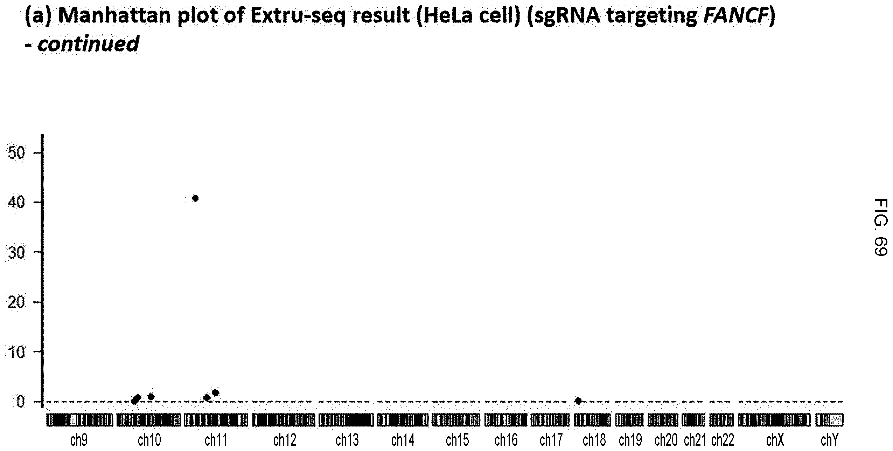

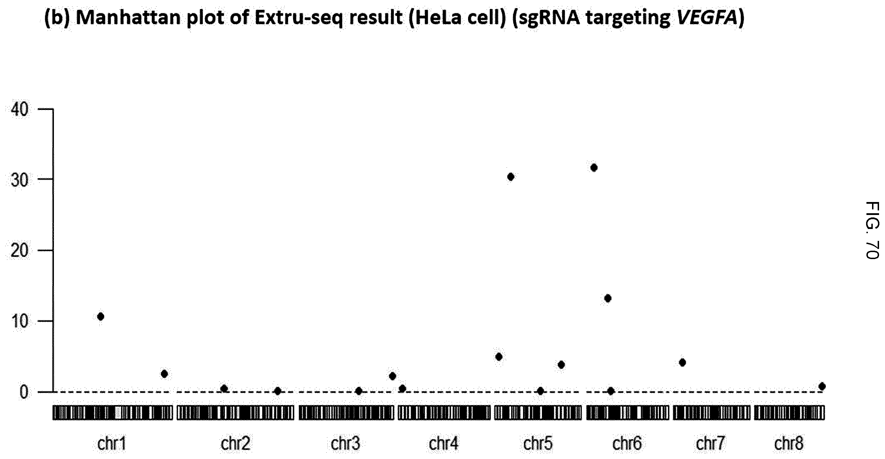

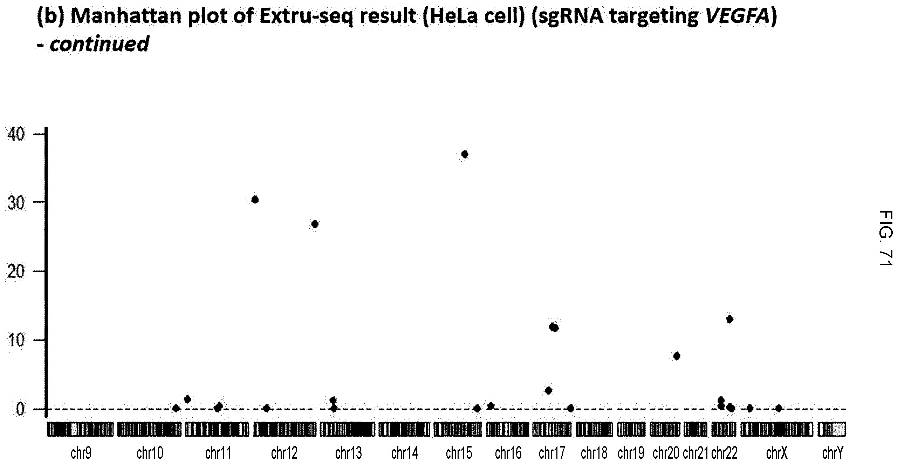

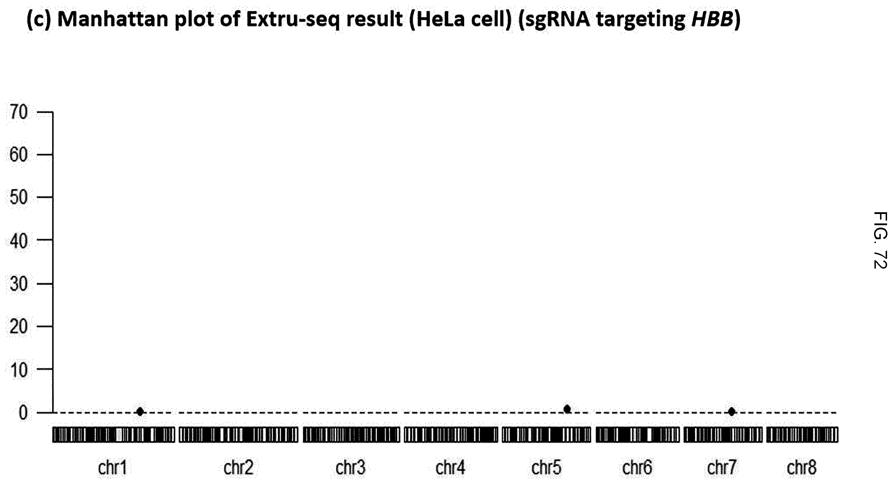

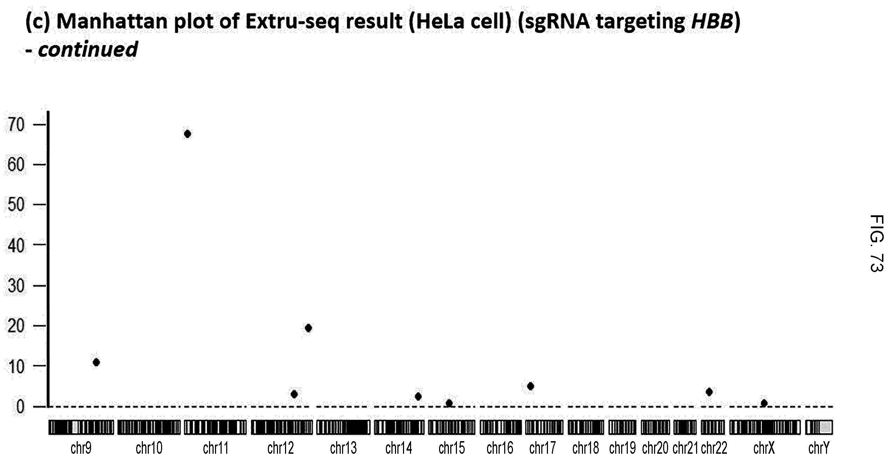

The Extru-seq result in HeLa cells is shown in FIGS. 68 to 73 (using sgRNA targeting *FANCF, VEGFA,* and *HBB*)*.* Specifically, FIGS. 68 and 69 show the results (Manhattan plot results) of predicting off-targets through Extru-seq, using *FANCF*-targeting sgRNA. FIGS. 70 and 71 show the results (Manhattan plot results) of predicting off-targets through Extru-seq, using VEGFA-targeting sgRNA. FIGS. 72 and 73 show the results (Manhattan plot results) of predicting off-targets through Extru-seq, using *HBB*-targeting sgRNA. The y-axis indicates DNA cleavage scores.

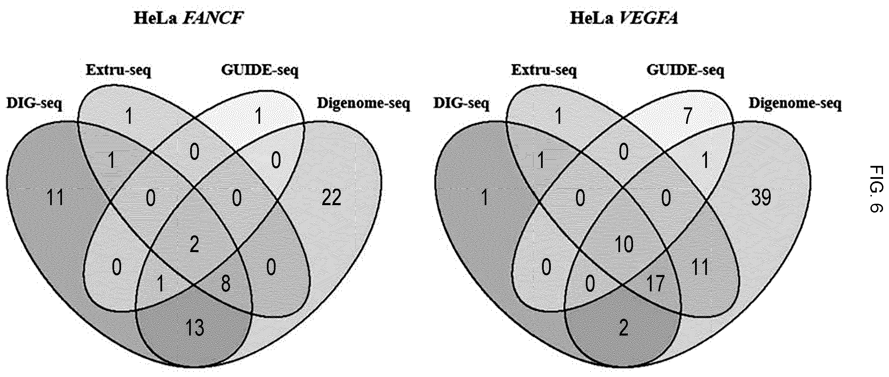

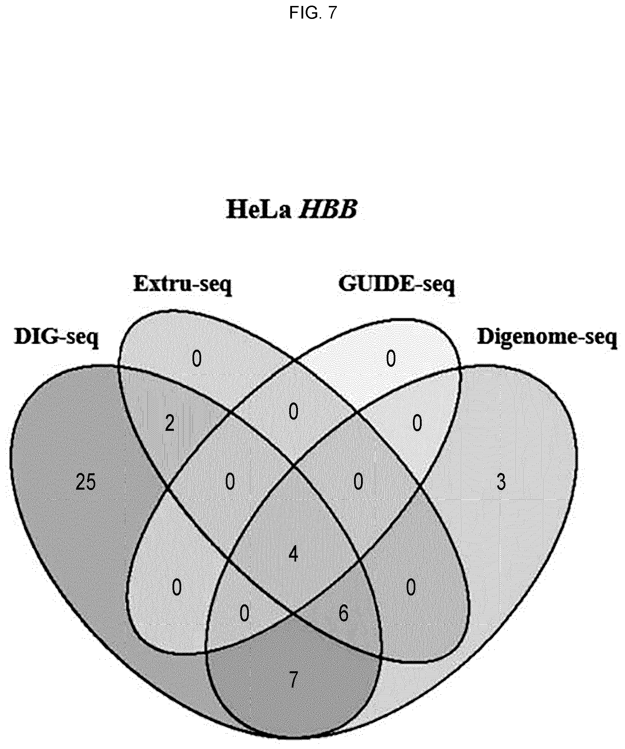

FIGS. 6 and 7 show the comparison of the results for off-target candidates according to each method (Extru-seq, GUIDE-seq, DIG-seq, Digenome-seq) through Venn diagram (using HeLa cells, human *FANCF* targeting sgRNA, human *VEGFA* targeting sgRNA, and human *HBB* targeting sgRNA). Analysis results through Venn diagram indicate that Digenome-seq and DIG-seq predicted a number of different off-target loci. On the other hand, it shows that most off-target loci predicted by Extru-seq were identified by at least one of different off-target prediction methods.

When tested to confirm if candidate loci could be validated, Extru-seq exhibited higher validation rates than DIG-seq and Digenome-seq. The validation results are shown in Table 5 below.

Specifically, Table 5 (a) shows the validation results of the top 10 predicted off-target sites for sgRNA targeting human *FANCF.* Table 5 (b) shows the validation result of the top 10 predicted off-target sites for sgRNA targeting human *VEGFA.* Table 5 (c) shows the validation result of the top predicted 10 off-target sites for human *HBB*-targeting sgRNA. The off-target sites were validated through target deep sequencing. For validation, the indel frequencies of the off-target sites are higher than 0.1%, and the equation '(Indel frequency at the off-target locus)/(Indel frequency in the control) > 2' must be satisfied. In Table 5, * indicates that a target is manually confirmed. The results for manually confirmed targets are shown in FIGS. 101 to 116.

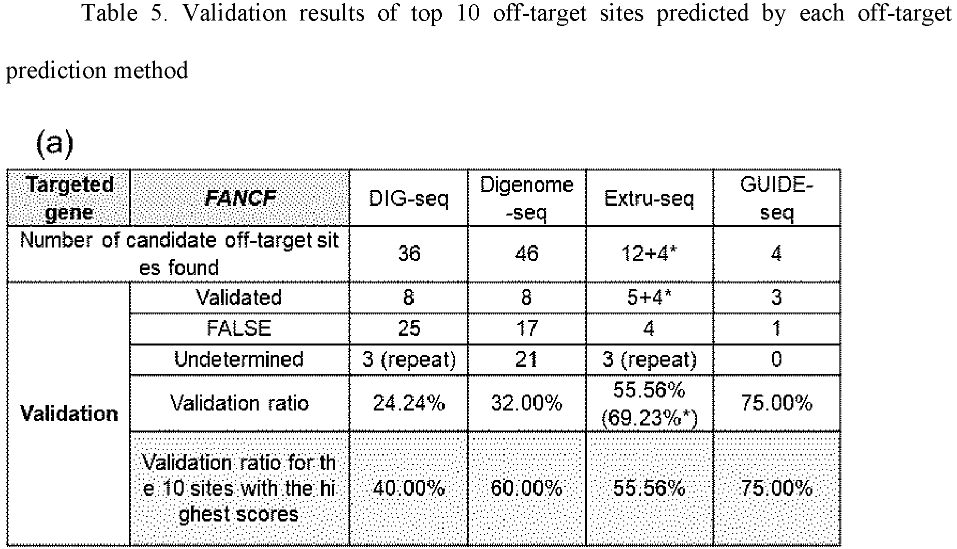

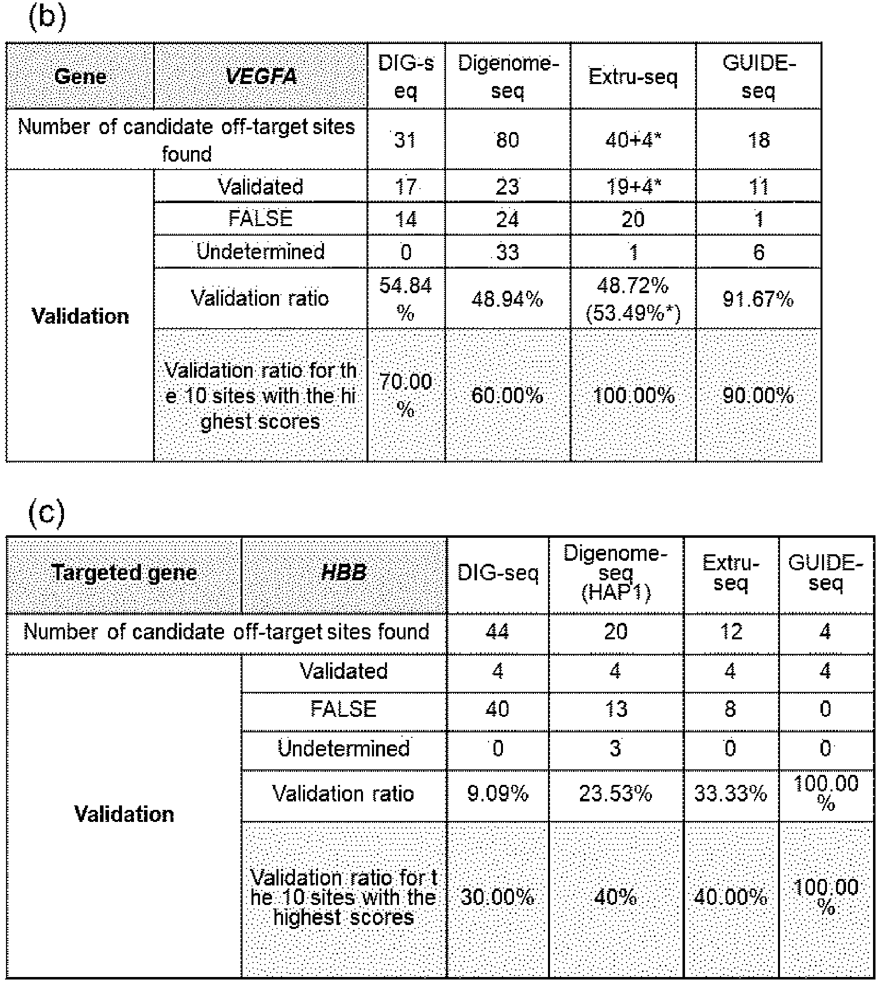

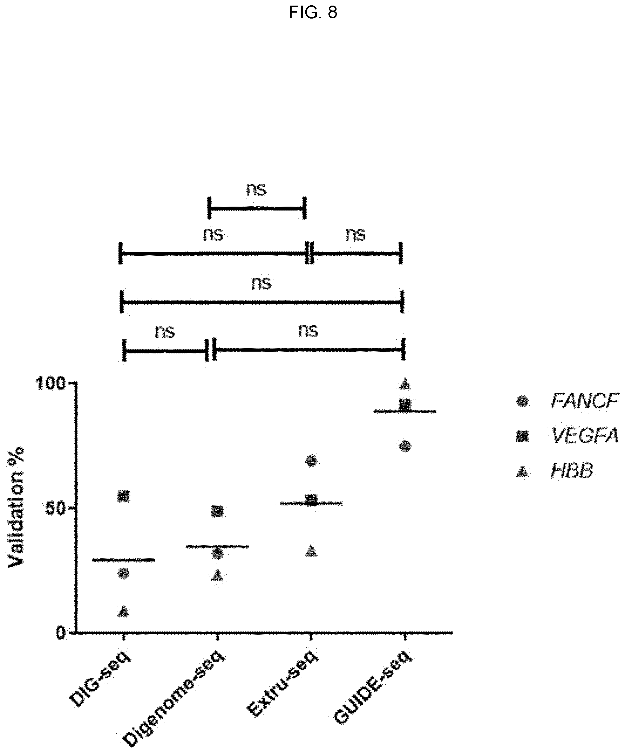

FIG. 8 shows the validation results for off-target sites predicted by each method (DIG-seq, Digenome-seq, Extru-seq, and GUIDE-seq). Specifically, the validation rates for the results related to FIGS. 6 and 7, and Table 6 are depicted in graphs (ns, no significance in two-sided unpaired Mann-Whitney test).

### Result 8. Comparison of rank distributions of off-target sites predicted by GUIDE-seq and Extru-seq

The inventors of the present application compared the degree of agreement between prediction results obtained by Extru-seq, cell-based, in vitro, and in silico methods. The top 10 candidate off-target loci for each prediction method were tabulated and the rankings of these loci for each different method were tabulated. Afterward, the rankings of the off-target loci in each method were compared. The results are shown in Tables 6 to 9. The loci predicted to be in the top 10 by the pair of method were counted, and the sharing ratio was calculated. To this end, the sharing rate (%) of the top 10 (rank) was calculated. That is, the top 10 off-target candidates were extracted in method A, and the ranks occupied by off-target candidates corresponding to the top 10 off-target candidates of A in another method (e.g., method B) were compared. If the corresponding off-target candidates are in the top 10 in another method, it was determined that they contribute to a sharing rate. As a result of calculation, low similarity (the overall average sharing rate in the top 10 = 22%) was observed in most cases. The highest similarity was consistently found between GUIDE-seq and Extru-seq pair-wise comparisons (the average sharing percentage in the top 10 for the GUIDE-seq and Extru-seq pair = 43%).

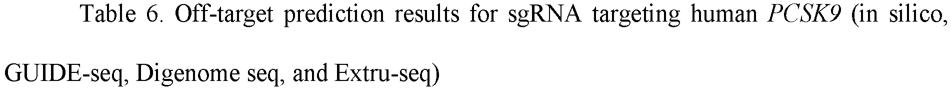

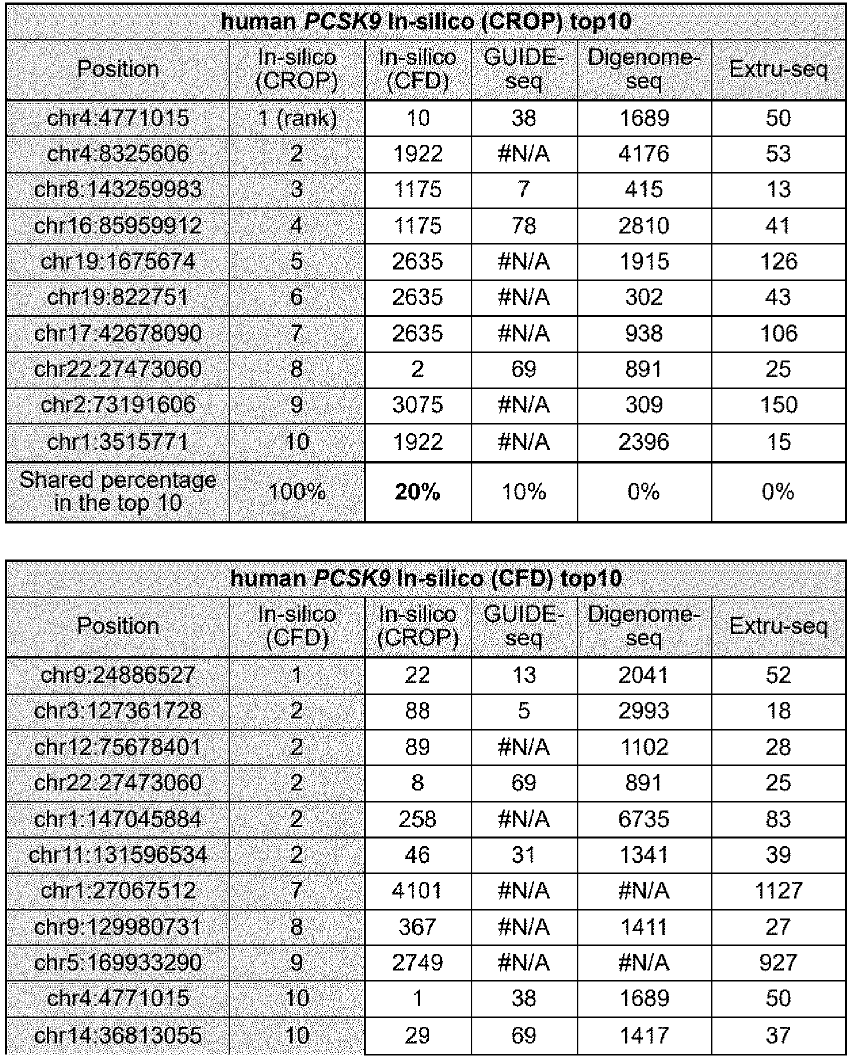

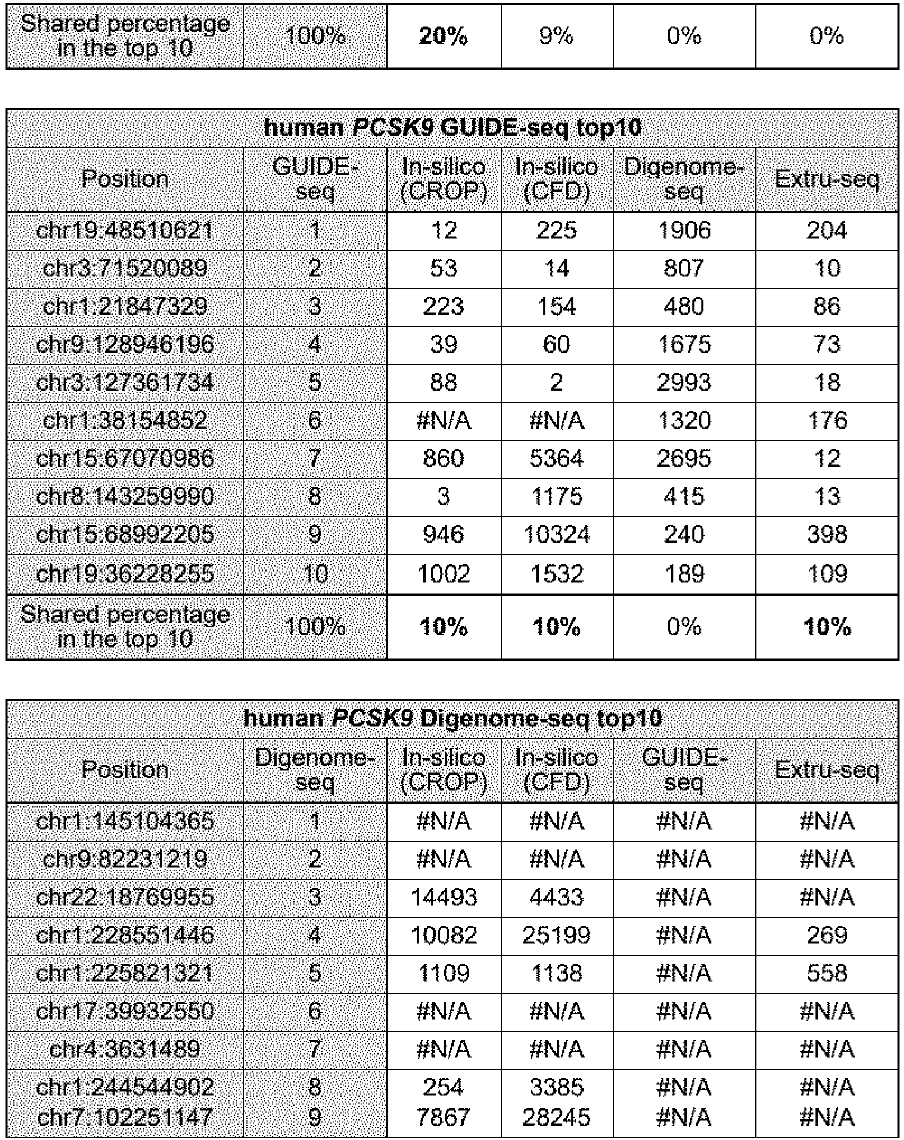

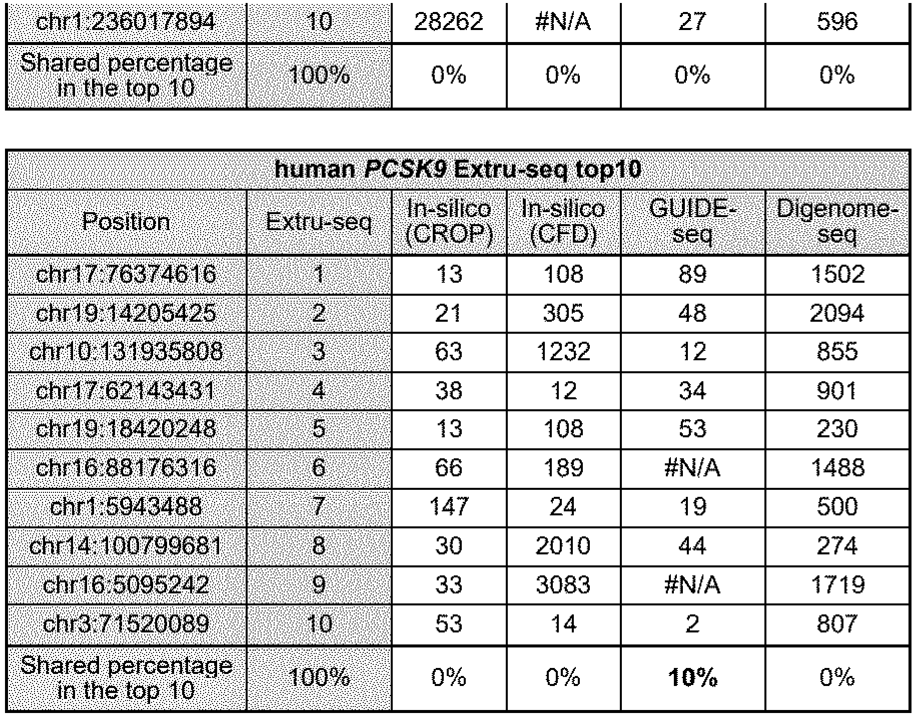

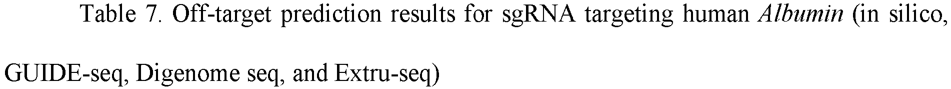

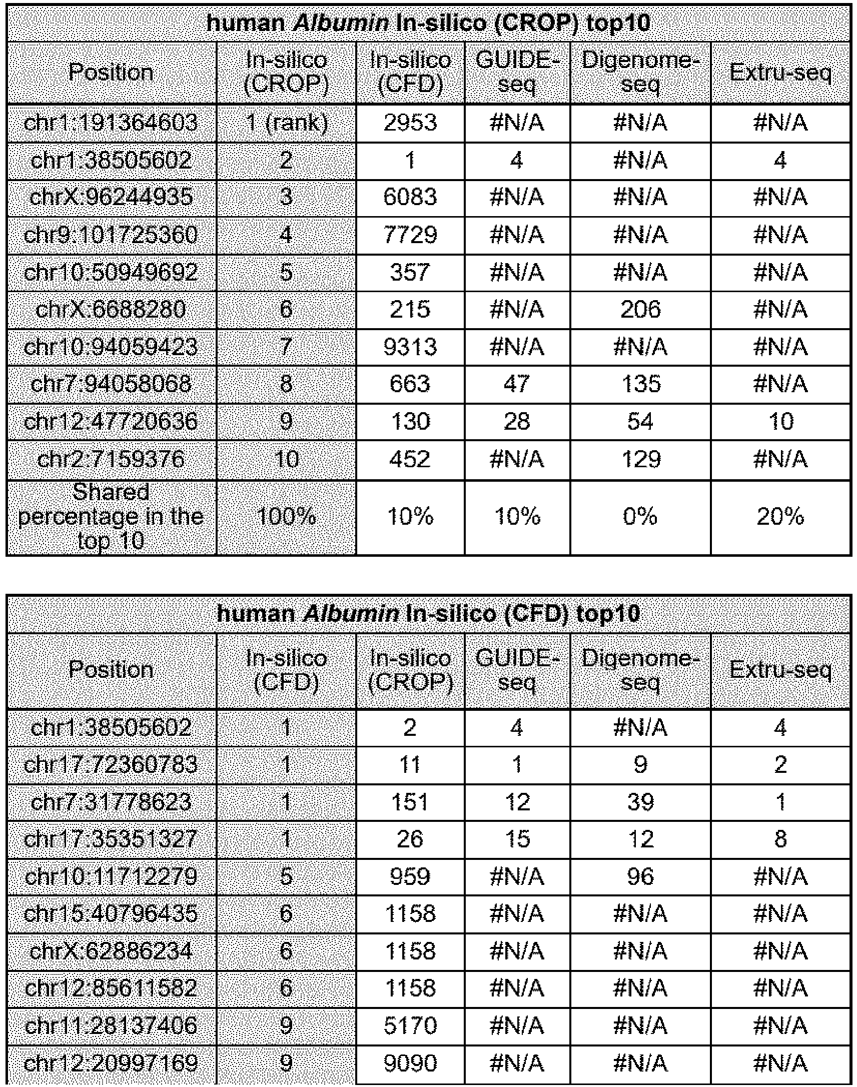

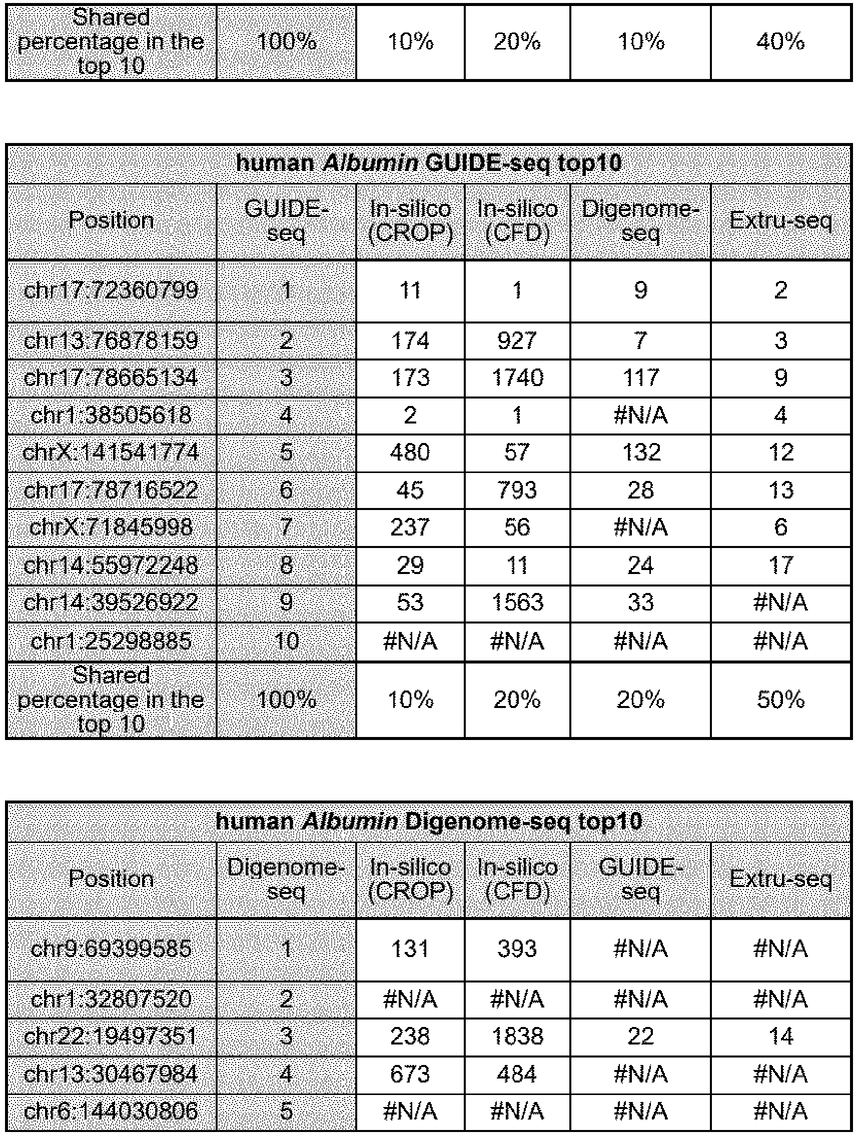

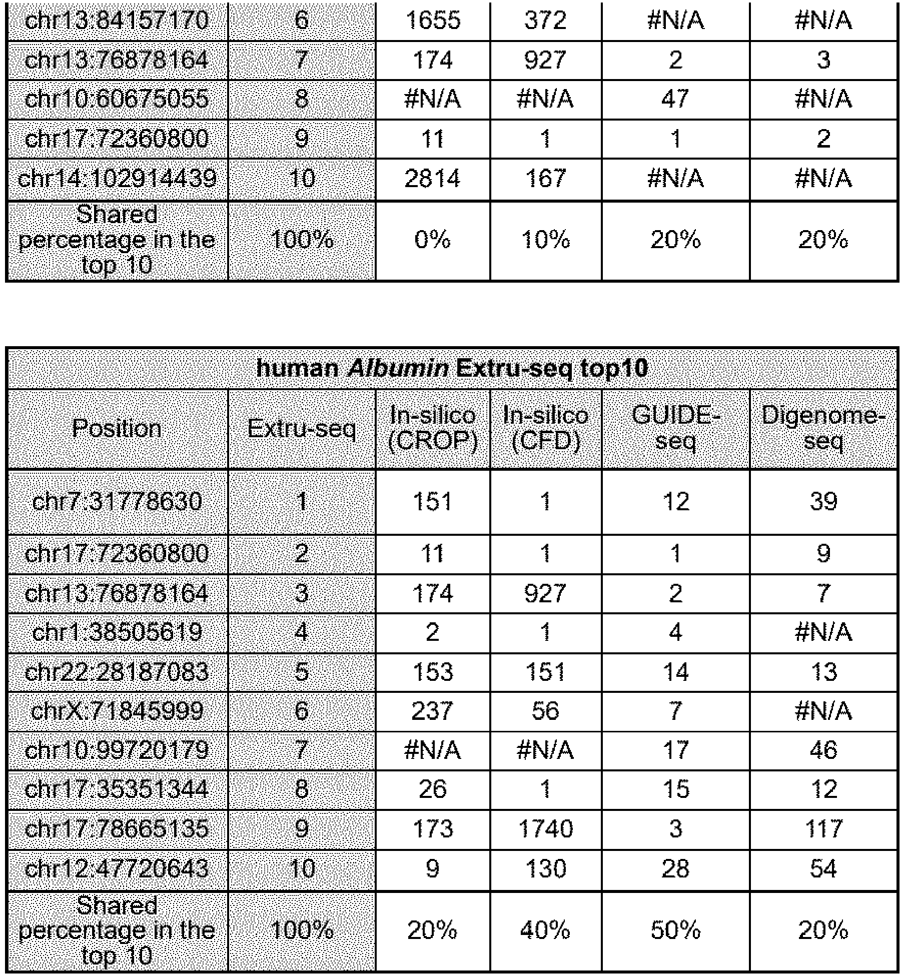

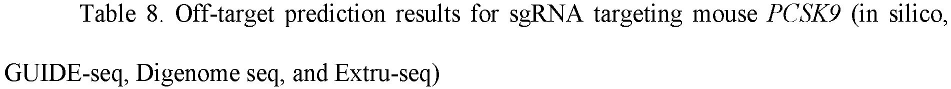

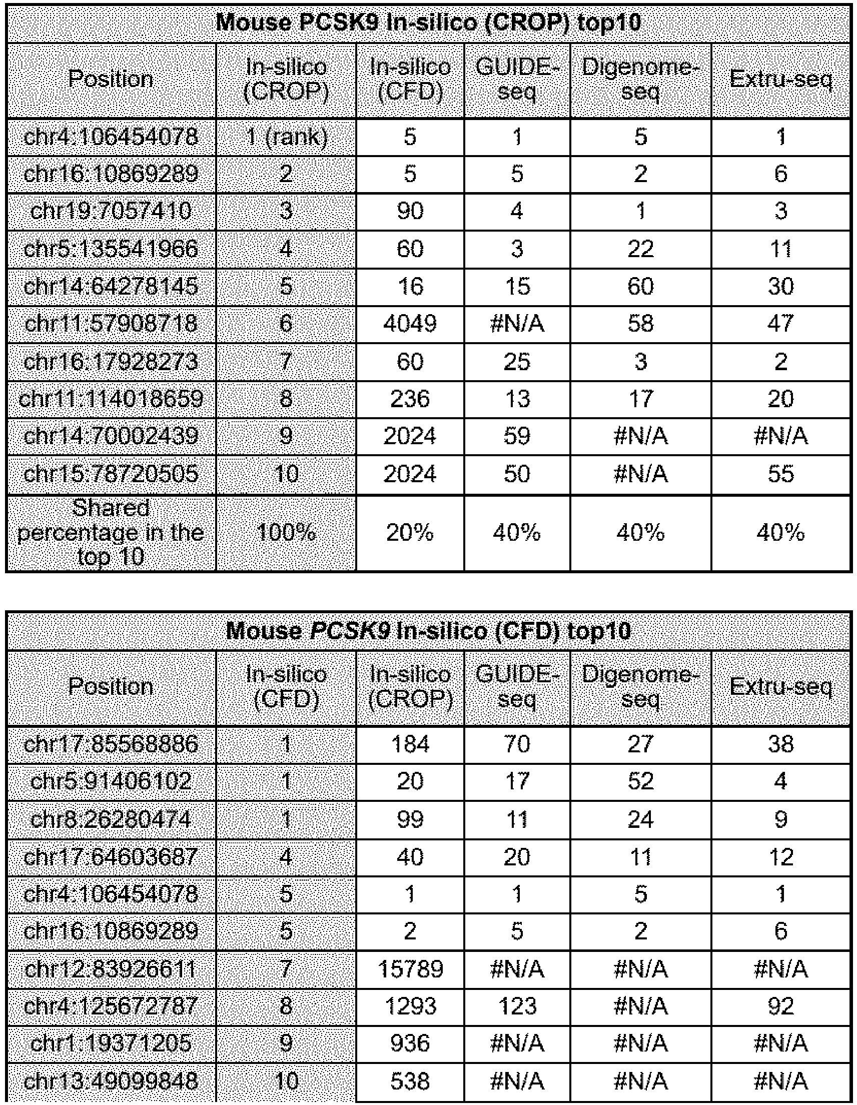

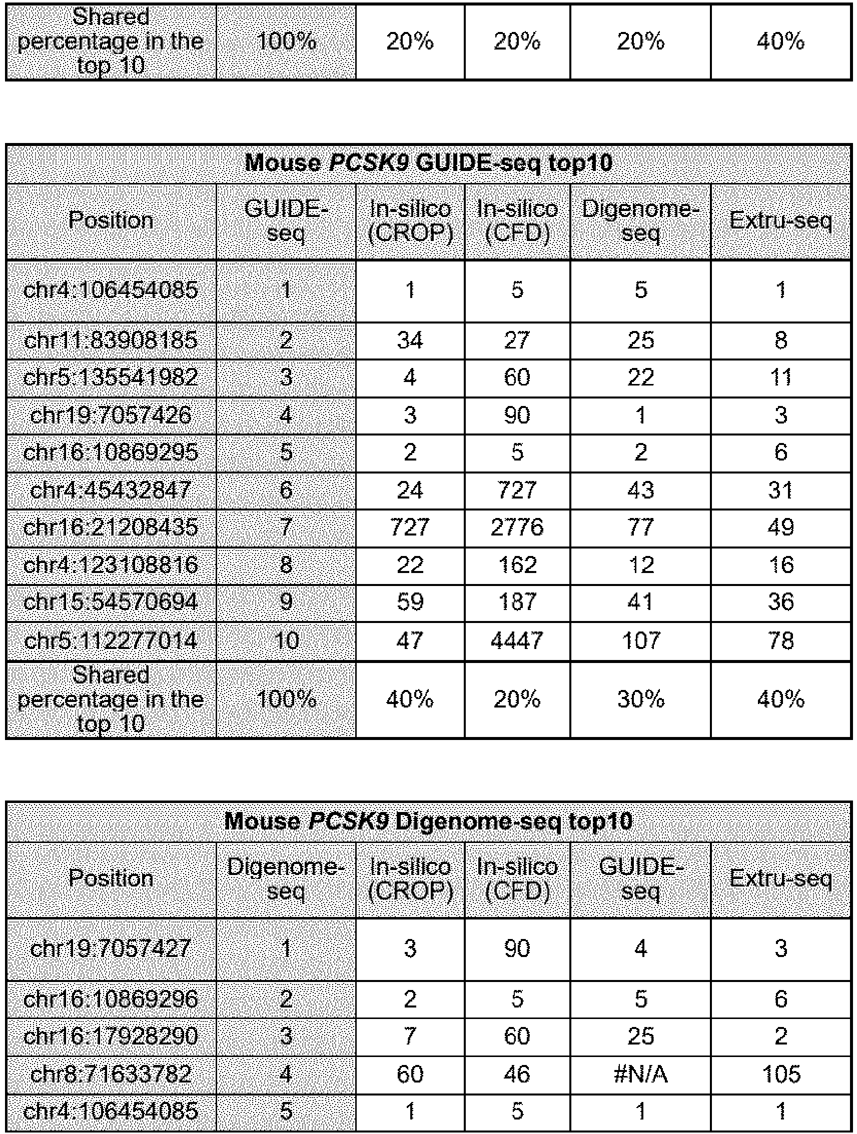

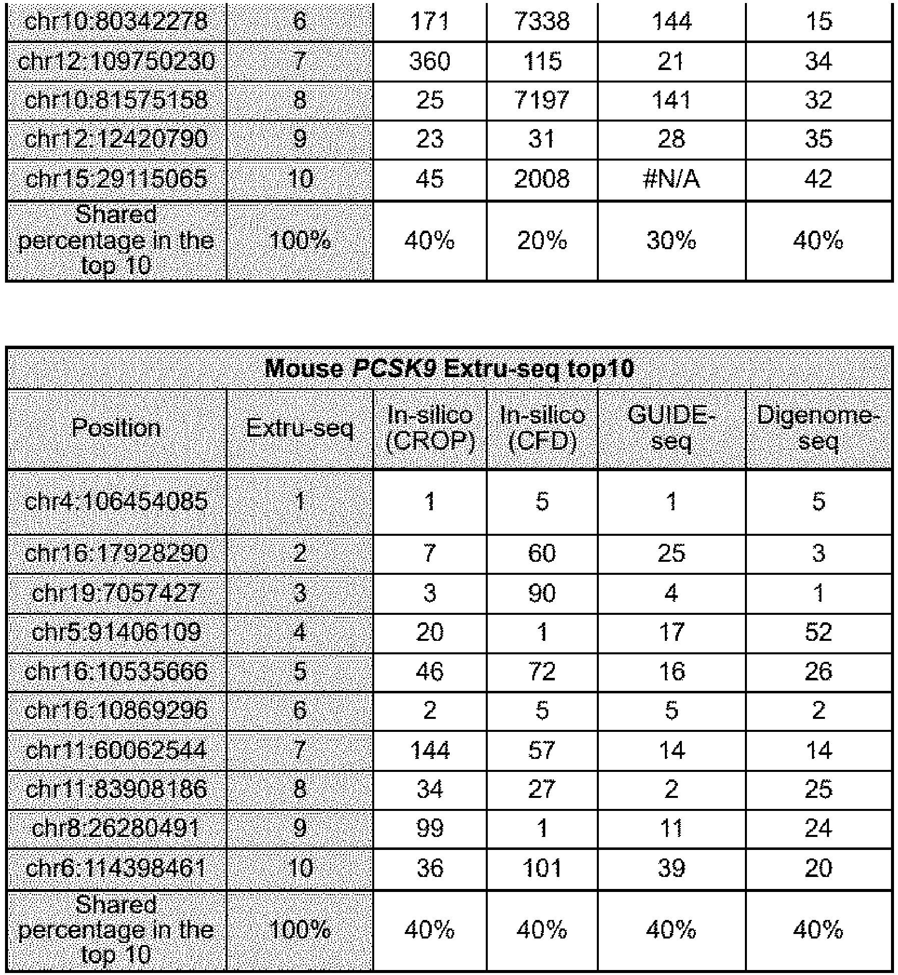

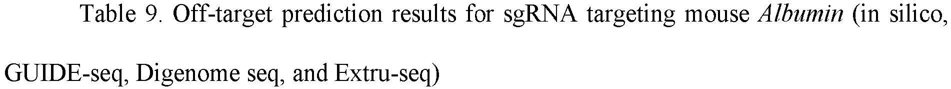

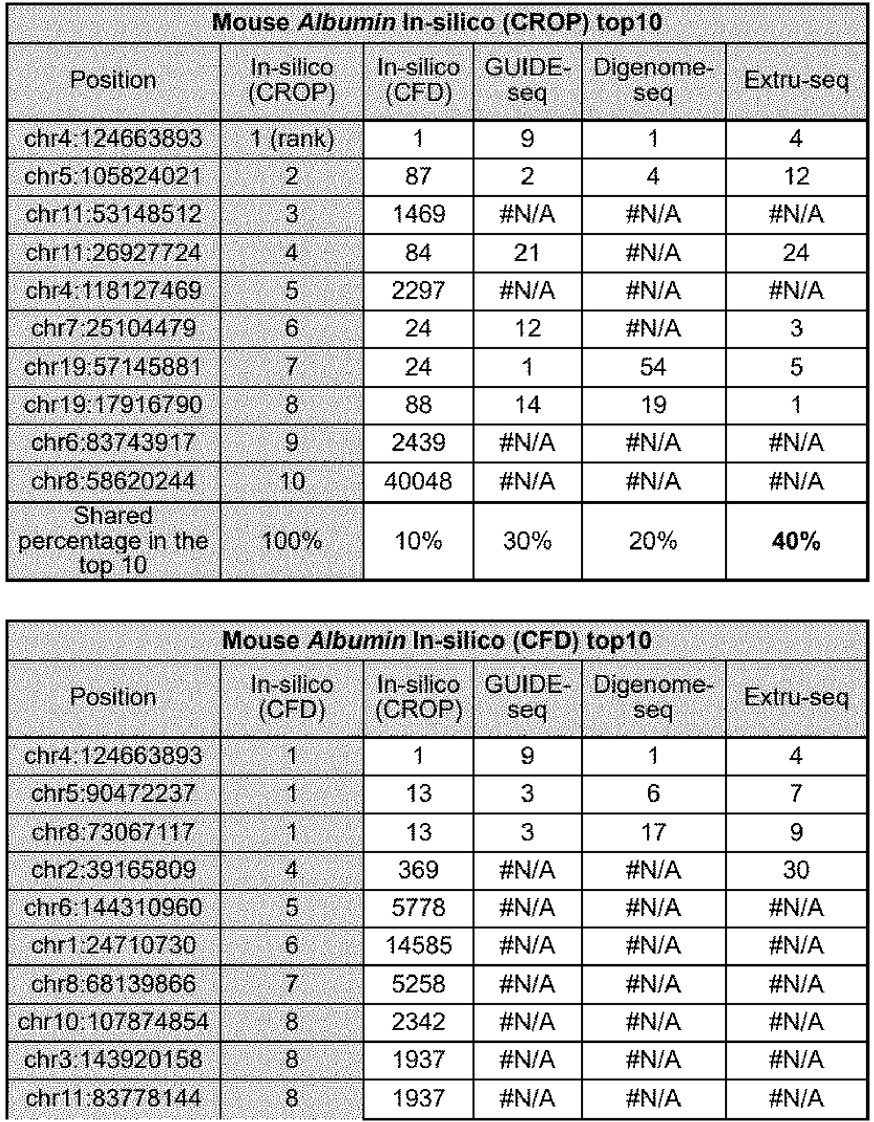

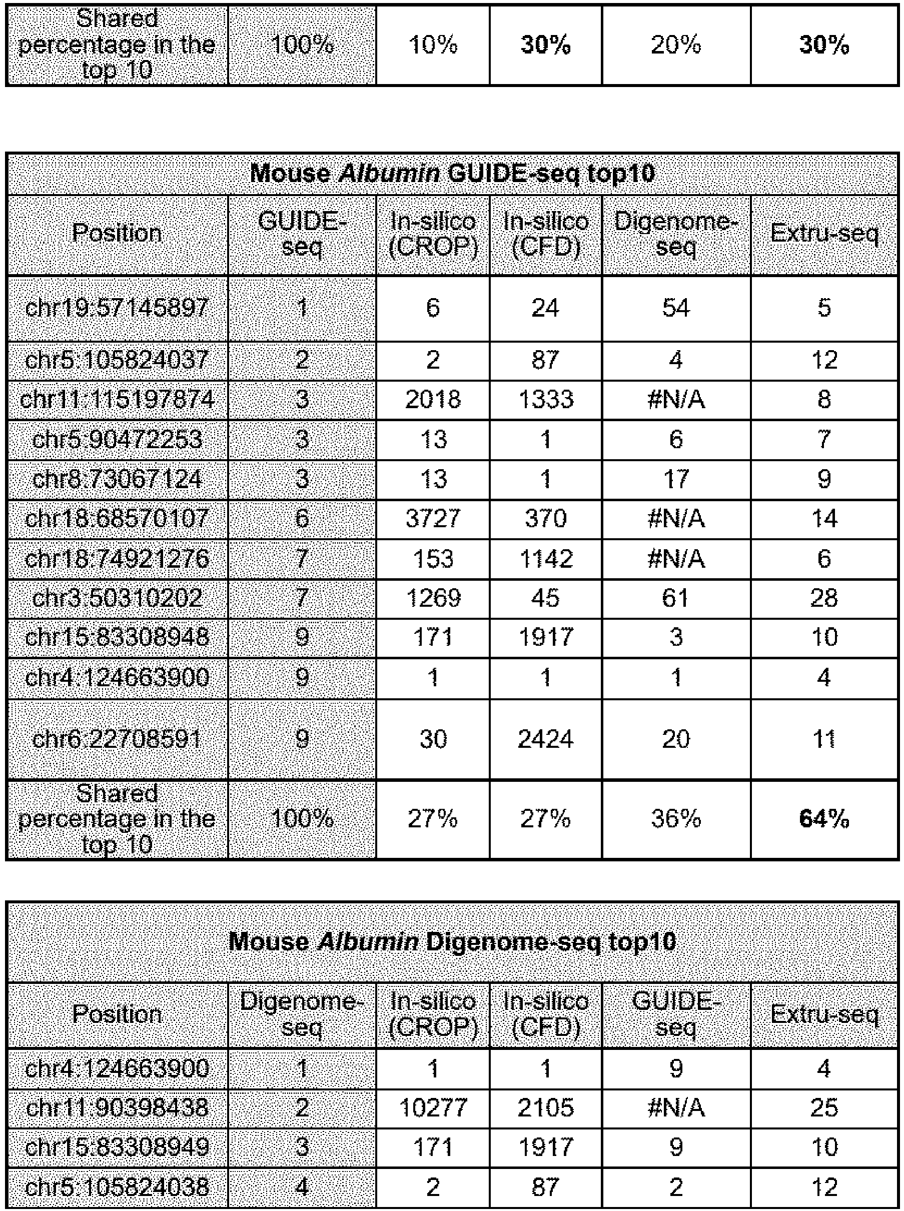

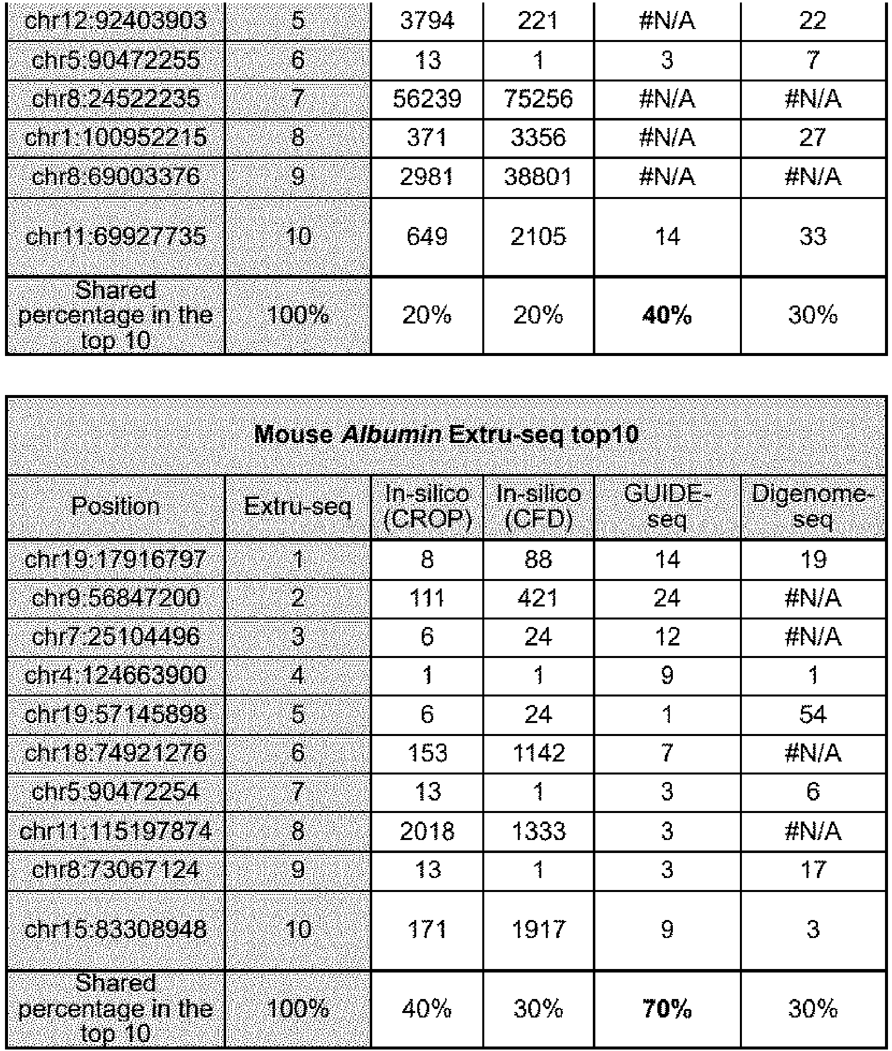

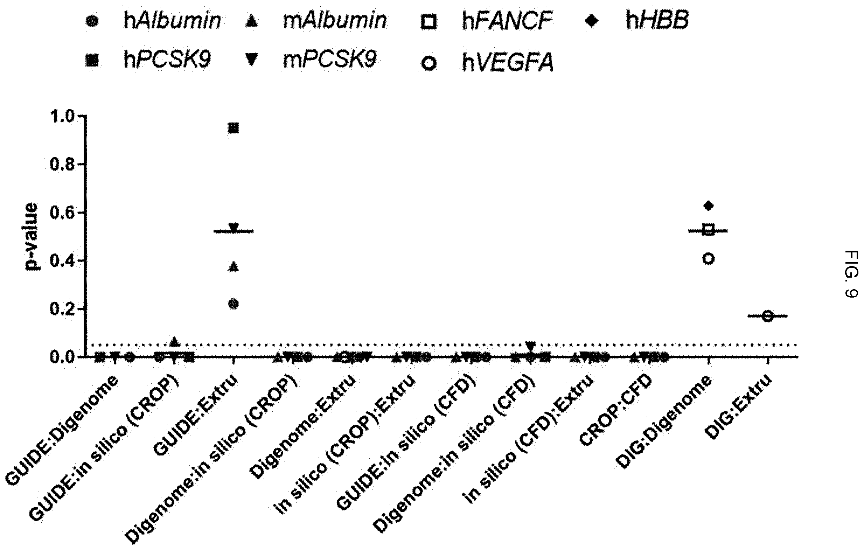

The comparison of ranks may also be performed with all off-target sites, comprising unvalidated sites. The Venn diagrams (FIGS. 3, 4, 6, and 7) show that there are statistically significant numbers of candidate off-target sites in the intersection to be analyzed. To check the equality of the medians for the scores of loci in the intersections of the results from two methods, score/read counts were min-max normalized and the Wilcoxon rank sum test was performed. Analysis results are shown in FIG. 9. In FIG. 9, the dotted line is p=0.05. Since at least 16 sample sizes were required for using asymptotic nonparametric Wilcoxon rank tests (refer to the documents [MUNDRY, ROGER, and JULIA FISCHER. "Use of statistical programs for nonparametric tests of small samples often leads to incorrect P values: examples from animal behavior." Animal behavior 56.1 (1998): 256-259.; and Dwivedi, Alok Kumar, Indika Mallawaarachchi, and Luis A. Alvarado. "Analysis of small sample size studies using nonparametric bootstrap test with pooled resampling method." Statistics in medicine 36.14 (2017): 2187-2205.]), an intersection with less than 16 samples was not included in this analysis. Table 10 below is referred. In the tests, a low p-value indicates that the scores of loci from the intersection between two populations are differently distributed. Except for the distributions of GUIDE-seq:Extru-seq and DIG-seq:Digenome-seq pairs, none of the pairs were not similarly distributed, and had high p values at n ≥ 3. In FIG. 9, the p-value was obtained from a normalized rank sum test for each off-target prediction method pair. In terms of sgRNAs, sgRNAs targeting *FANCF, VEGFA,* and *HBB* were used in HeLa cells, and sgRNAs targeting *PCSK9* and *Albumin* were used in human cells and mouse cells (n ≥ 16 was selected for analysis).

Regarding to FIG. 9, Table 10 below is shown. Table 10 shows the number of samples found in the intersection of Venn diagram. The tables show the number of samples for each sgRNA (sgRNAs targeting human *PCSK9,* human *Albumin,* mouse *PCSK9,* mouse *Albumin,* human *FANCF,* human *VEGFA,* and human *HBB*)*.* Cases with n >= 16 (16 is the minimum number of samples required for the asymptotic nonparametric Wilcoxon rank tests) are underlined.

**Table 10. Number of predicted off-target candidates found in overlapping regions in FIGS. 3 and 4, and FIGS. 6 and 7 (compared for each method)**

| | *hPCSK9* | h*Albumin* | mPCSK9 | m*Albumin* |
|---|---|---|---|---|
| GUIDE-seq : Digenome-seq | 137 | 43 | 70 | 12 |
| GUIDE-seq : *in silica* | 119 | 55 | 126 | 25 |
| GUIDE-seq : Extru-seq | 128 | 28 | 93 | 22 |
| Digenome-seq : *in silica* | 3311 | 222 | 128 | 54 |
| Digenome-seq : Extru-seq | 1288 | 30 | 110 | 17 |
| *in silico*: Extru-seq | 1133 | 35 | 189 | 54 |

| | h*HBB* | h*VEGFA* | h*FANCF* |
|---|---|---|---|
| DIG-seq: Digenome-seq | 17 | 29 | 24 |
| DIG-seq : Extru-seq | 12 | 28 | 11 |
| DIG-seq : GUIDE-seq | 4 | 10 | 3 |
| Digenome-seq : Extru-seq | 10 | 38 | 10 |
| Digenome-seq : GUIDE-seq | 4 | 11 | 3 |
| GUIDE-seq : Extru-seq | 4 | 10 | 2 |

It is estimated that the disagreement between the result of GUIDE-seq or Extru-seq and the result of Digenome-seq or in silico prediction is due to a low validation rate of Digenome-seq caused by the large number of false positives and a low validation rate of the in silico prediction caused by the difference between machine learning-based prediction scores and real-world experimental values. Further, since the DIG-seq:Digenome-seq pair consistently showed high p values, unlike the Digenome-seq:Extru-seq pair showing low p values, the result obtained from DIG-seq is analyzed to be similar to the result obtained from the in vitro prediction method (herein denoted as Digenome-seq). However, the result obtained from Extru-seq was analyzed to be more similar to the cell-based prediction method (herein denoted as GUIDE-seq) and different from the result obtained from the in vitro prediction method such as Digenome-seq. This is because, as described above, the result for the GUIDE-seq:Extru-seq pair exhibited a high p value, and the result for the Extru-seq:Digenome-seq pair exhibited a low p value. In this regard, Extru-seq is distinct from DIG-seq (still showing similarity to Digenome-seq) in that it has lost similarity to the in vitro Digenome-seq. While all the experimental processes of Digenome-seq, DIG-seq and the Extru-seq used in this experiment comprise WGS, the analysis through GUIDE-seq is performed based on PCR, so such results showing the similarity between Extru-seq and GUIDE-seq are somewhat surprising. This shows that the conditions for treating genomic DNA with Cas9 are more important than an analysis procedure.

### Result 9. Comparison in miss rate of Extru-seq and GUIDE-seq

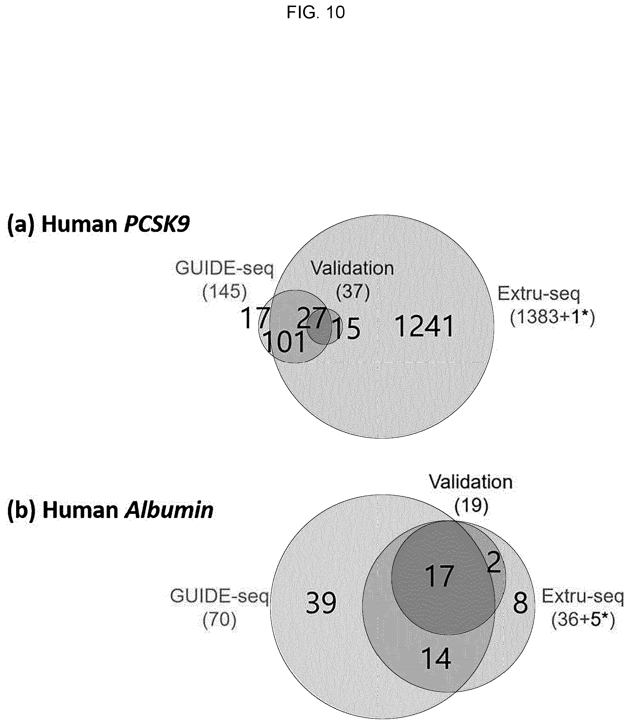

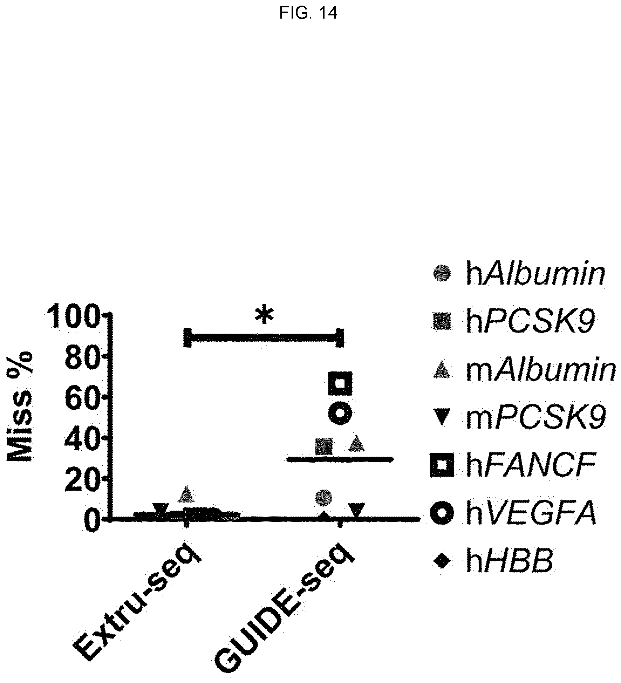

Cell-based methods, comprising GUIDE-seq, are known to sometimes miss a bona-fide off-target candidate. The inventors of the present application calculated a miss rate (or a false negative rate calculated by the following equation: (number of false negatives)/(number of false negatives + number of true positives)) using Venn diagrams showing the overlap between the prediction of Extru-seq and GUIDE-seq and validated targets in samples analyzed by deep sequencing. As a result of the investigation, the average miss rate of Extru-seq was confirmed to be 2.3%, and the average miss rate of GUIDE-seq was confirmed to be 29% (refer to FIGS. 10 to 14, and Tables 11 to 17). The summarizing result for the miss rates of Extru-seq and GUIDE-seq is shown in the graph of FIG. 14.

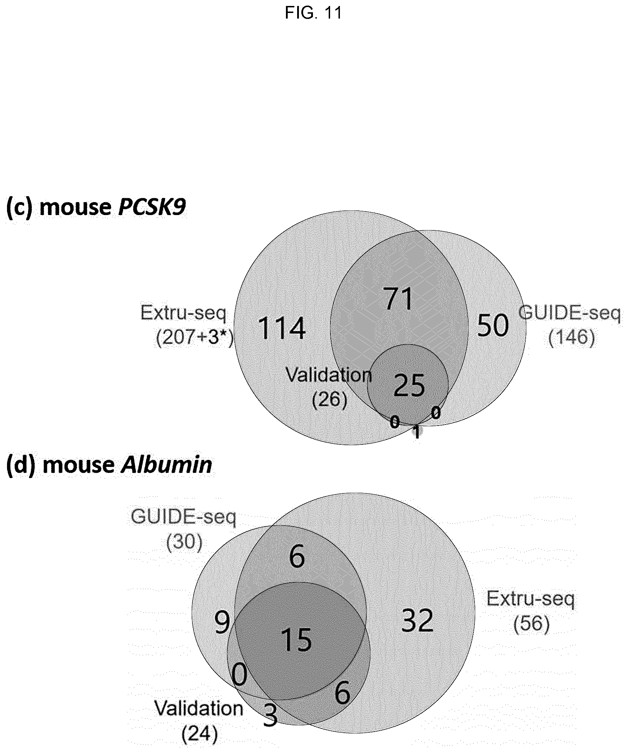

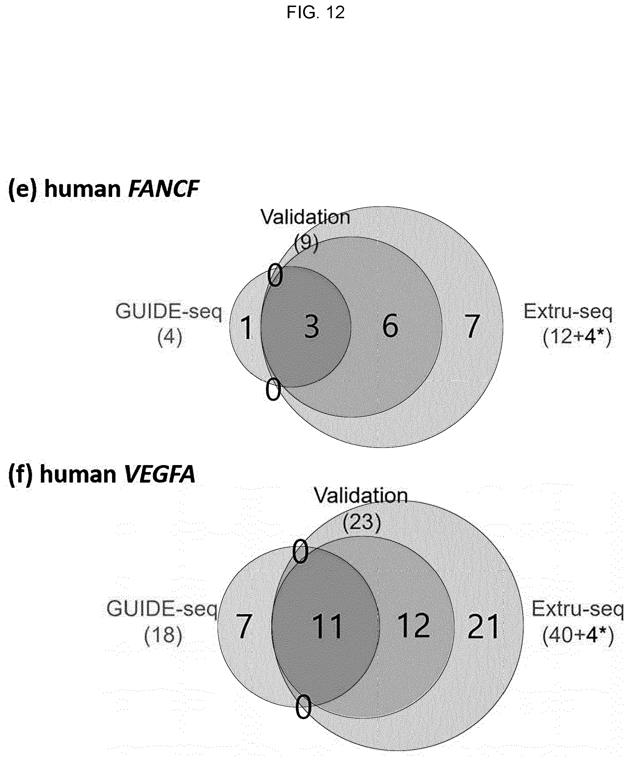

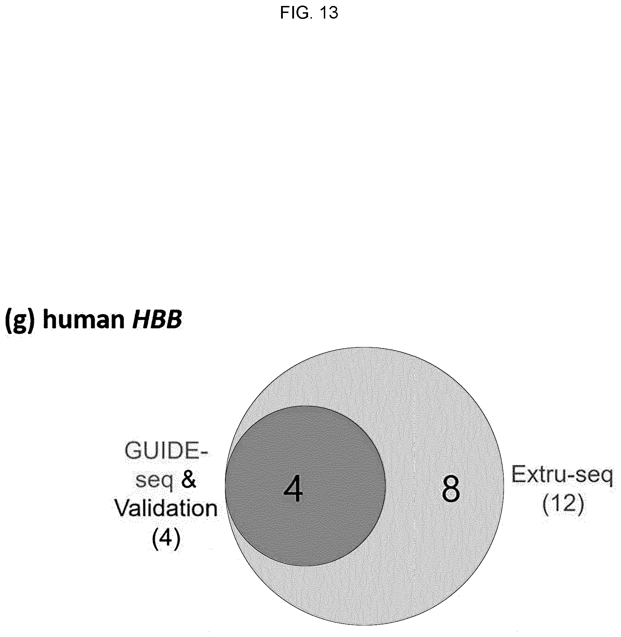

Specifically, FIGS. 10 to 13 show Venn diagrams used to confirm the miss rates. Specifically, the Venn diagrams shown in in FIGS. 10 to 13 show the comparison results for the off-target candidates predicted by Extru-seq and GUIDE-seq, and the validated off-targets (denoted as validation). The off-target prediction and validation were performed using (a) sgRNA targeting human *PCSK9* (FIG. 10), (b) sgRNA targeting human *Albumin* (FIG. 10), (c) sgRNA targeting mouse *PCSK9* (FIG. 11), (d) sgRNA targeting mouse *Albumin* (FIG. 11), (e) sgRNA targeting human *FANCF* (FIG. 12), (f) sgRNA targeting human *VEGFA* (FIG. 12), and (g) sgRNA targeting human *HBB* (FIG. 13). Validation represents targets (off-targets and on-targets) validated by target deep sequencing. In FIGS. 10 to 13, * denotes the number of manually confirmed off-target sites.

FIG. 14 graphically shows the miss rates investigated for Extru-seq and GUIDE-seq. The miss rates are disclosed for each method. In addition, the miss rates are disclosed for each sgRNA (sgRNA targeting human *PCSK9,* sgRNA targeting human *Albumin,* sgRNA targeting mouse *PCSK9,* sgRNA targeting mouse *Albumin,* sgRNA targeting human *FANCF,* sgRNA targeting human *VEGFA,* and sgRNA targeting human *HBB*) (**:* P<0.05 in two-sided unpaired Mann-Whitney test).

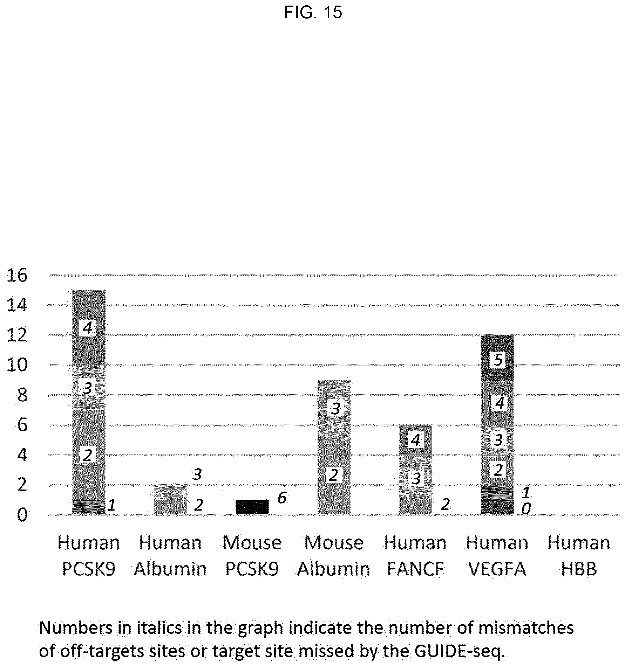

FIG. 15 shows the distribution of numbers of mismatches for off-targets missed in GUIDE-seq.

Tables 11 to 17 show the off-target analysis result predicted by each method (Extru-seq or GUIDE-seq) and the actual off-target analysis result confirmed through deep sequencing (related to FIGS. 10 to 14). That is, the results for the off-target candidates predicted by Extru-seq or GUIDE-seq and the off-target sites validated by deep sequencing are compared. Each of (a) sgRNA targeting human *PCSK9,* (b) sgRNA targeting human *Albumin,* (c) sgRNA targeting mouse *PCSK9,* (d) sgRNA targeting mouse *Albumin,* (e) sgRNA targeting human *FANCF,* (f) sgRNA targeting human *VEGFA,* and (g) sgRNA targeting human *HBB* was investigated. For validation, the indel frequency at an off-target site must be higher than 0.1%, and the equation '((indel frequency at the off-target locus)/(indel frequency in the control) > 2)' must be satisfied.

In Tables 11 to 17, the Target and Position columns show information about targets validated through deep sequencing. + shows that a target (the validated on-target or off-target) was predicted by the denoted off-target prediction method. A blank box indicates that a target (on-target or off-target) was not predicted by a denoted off-target prediction method (i.e., the denoted off-target prediction method missed a validated target). A miss rate was calculated by the equation '(number of blank boxes)/(number of entire boxes)'. * indicates that a target was manually confirmed (refer to FIGS. 77 to 116).

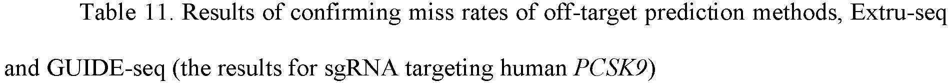

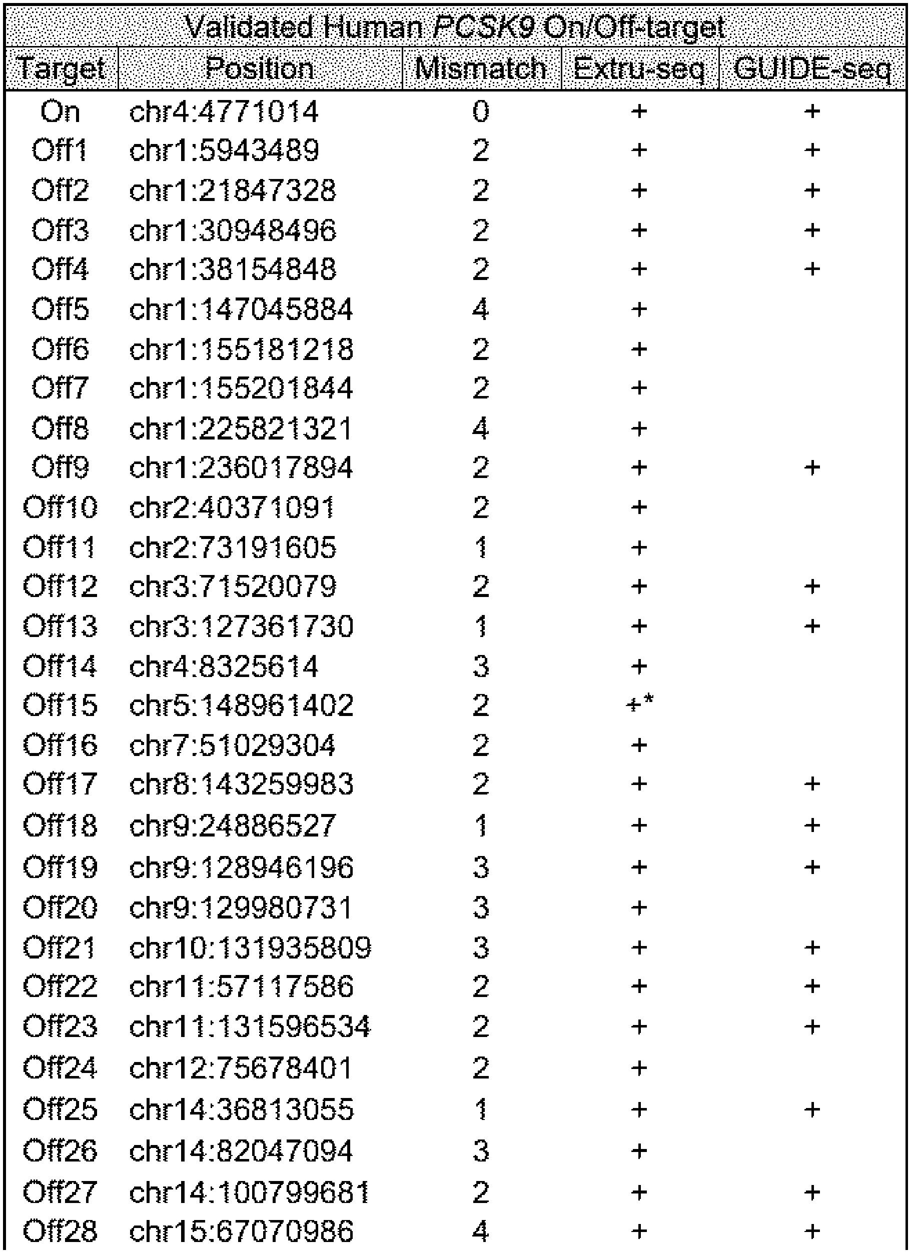

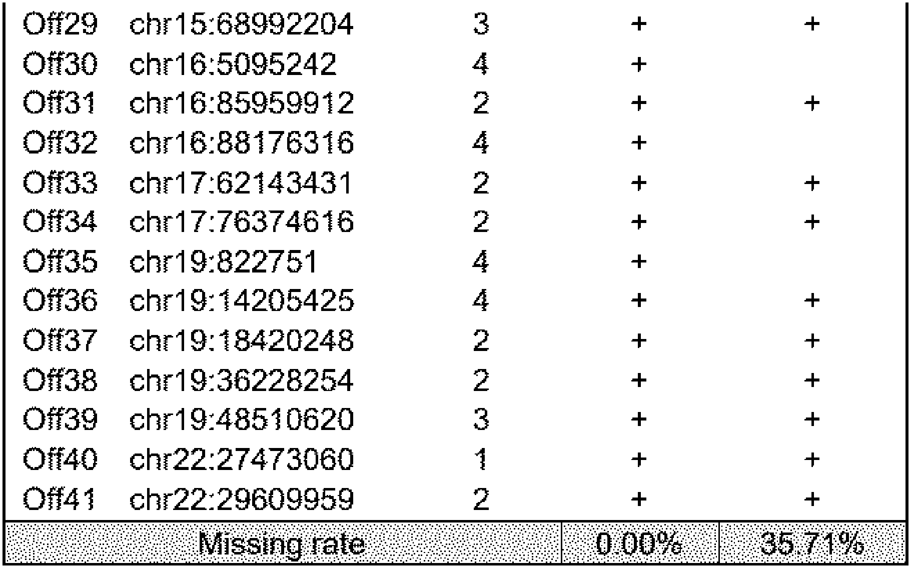

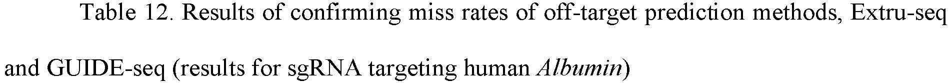

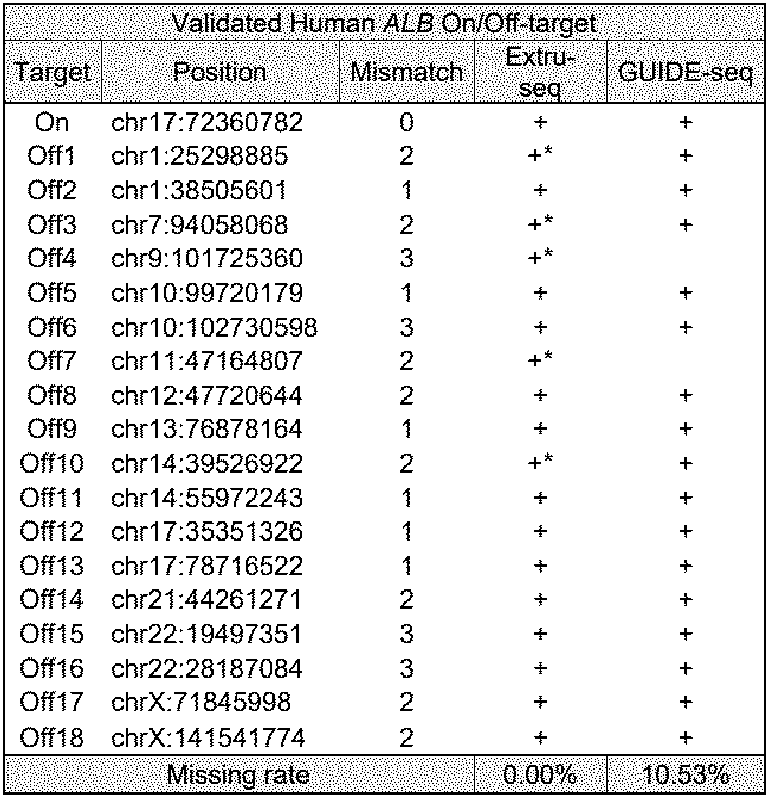

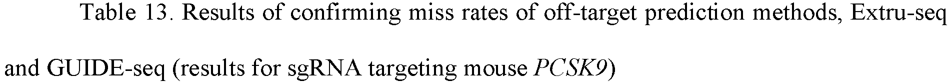

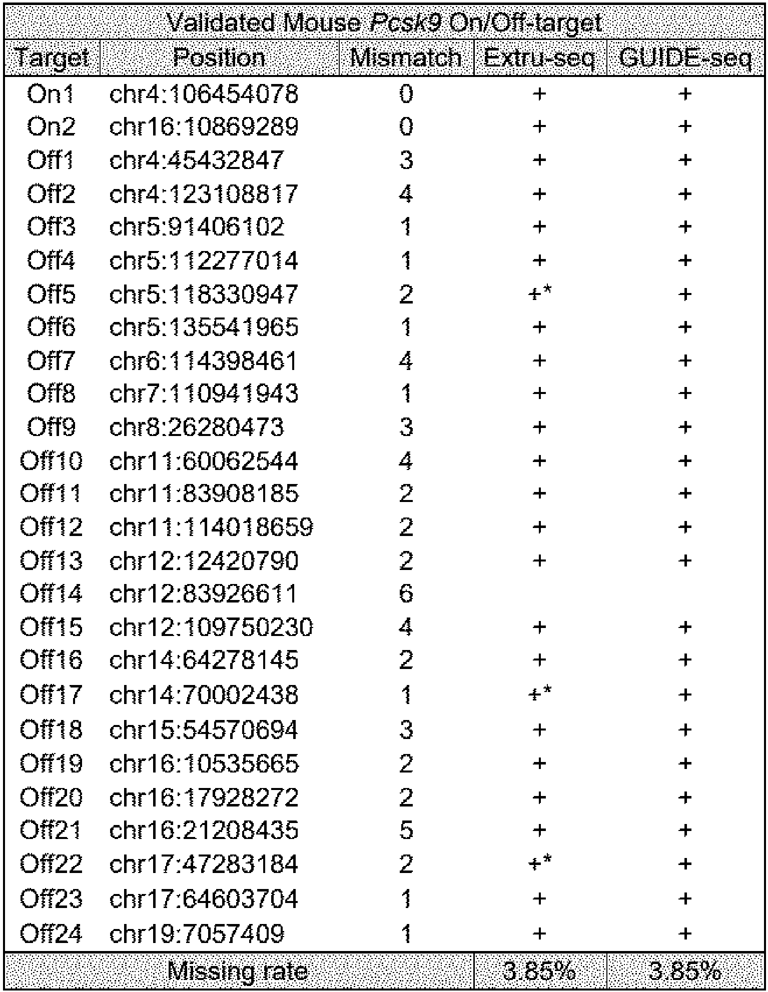

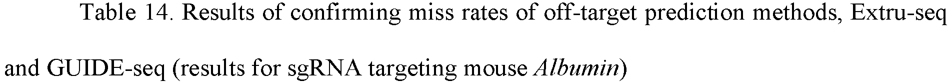

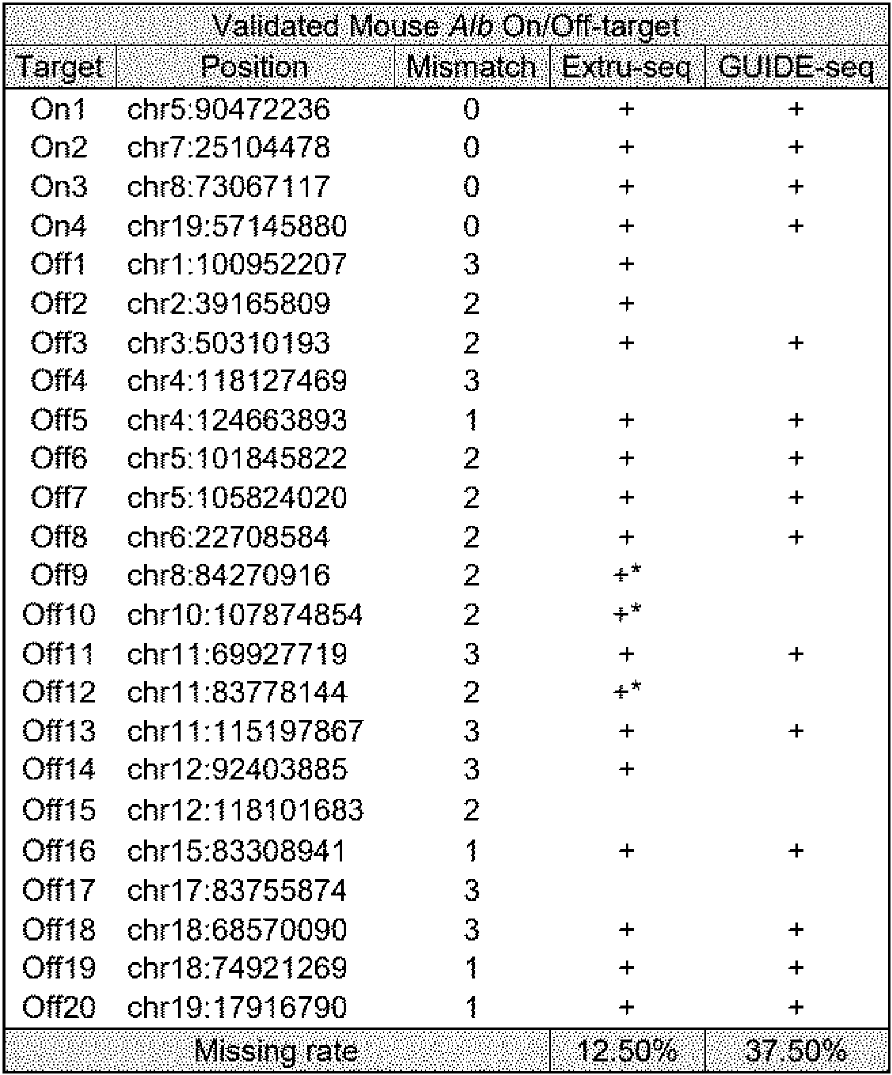

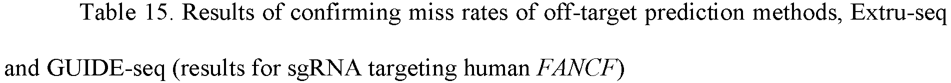

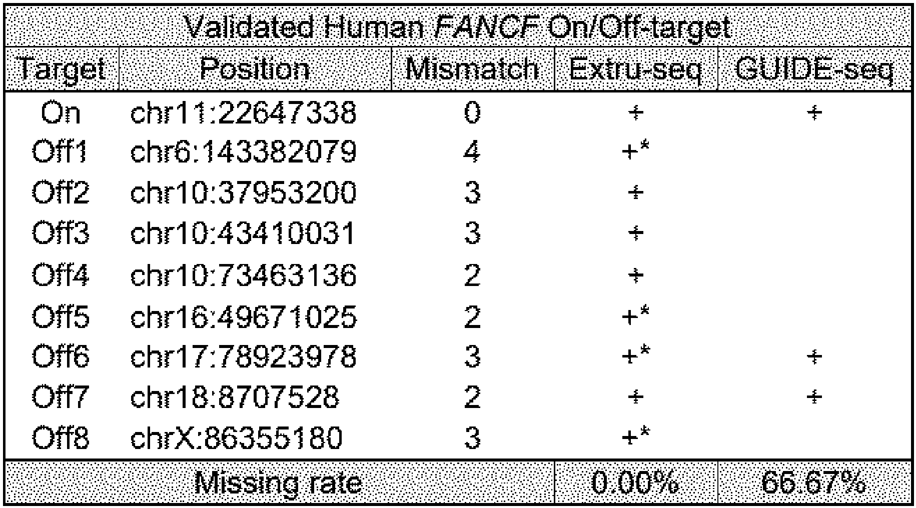

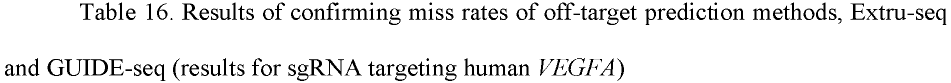

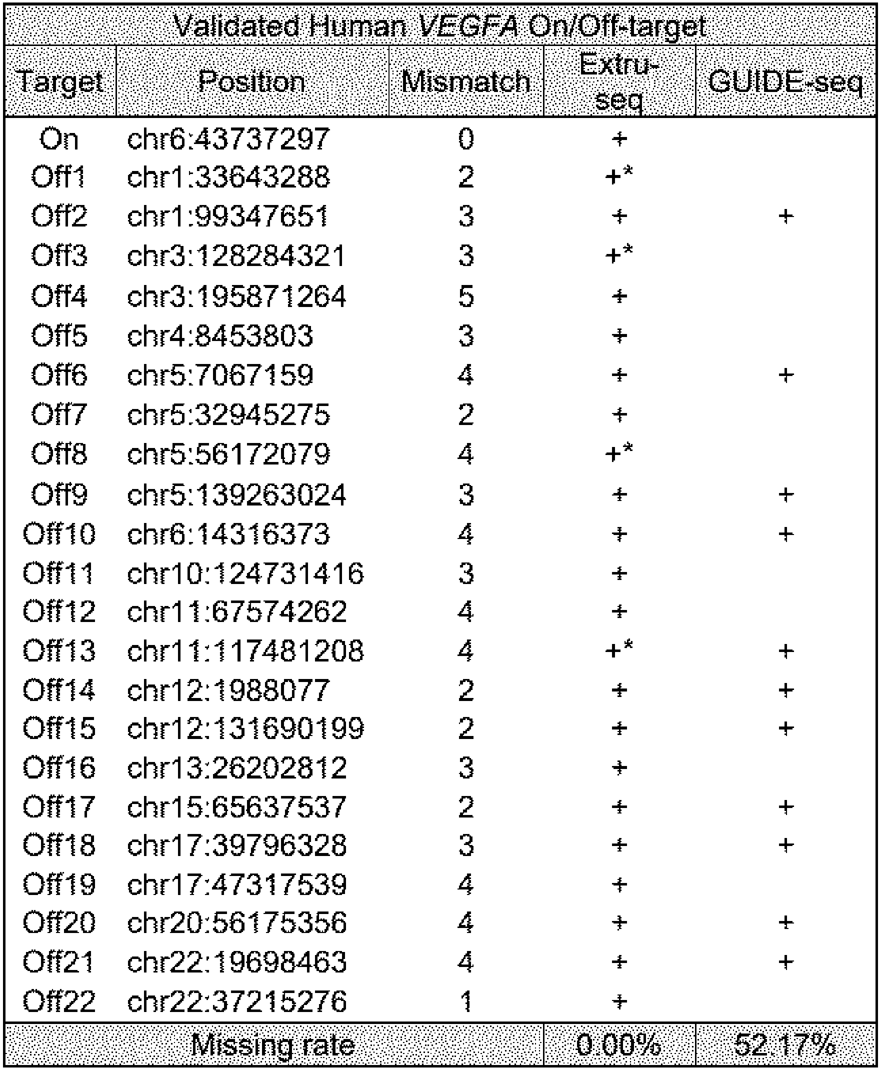

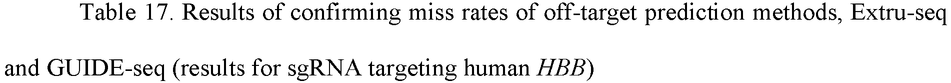

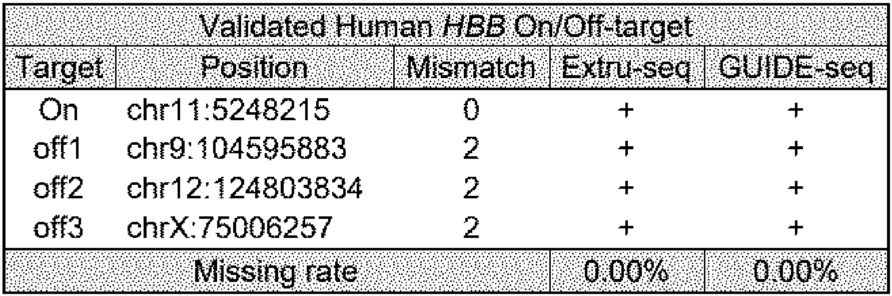

The results related to the miss rates in FIGS. 10 to 15, and Tables 11 to 17 show that the sensitivity of Extru-seq is much higher than that of the cell-based GUIDE-seq method, and Extru-seq rarely misses an actual off-target site.

From the results disclosed in the present application, it shows that GUIDE-seq overlooked validated off-target sites comprising 1 to 6 mismatches. Therefore, the only GUIDE-seq-dependent IND study (refer to the document [Stadtmauer, Edward A., et al. "CRISPR-engineered T cells in patients with refractory cancer." Science 367.6481 (2020): eaba7365.]) is at risk of overlooking valid off-target candidates. For CTX001, an in silico method was used to complement GUIDE-seq (refer to the document [Frangoul, Haydar, et al. "CRISPR-Cas9 gene editing for sickle cell disease and β-thalassemia." New England Journal of Medicine 384.3 (2021): 252-260.]). However, only genomic sites with three or less mismatches, or two or less mismatches, and a single DNA or RNA bulge are computationally identified, and valid off-target sites with three or more mismatches are still at the risk of being overlooked.

### Result 10. Receiver operating characteristic (ROC) curve of Extru-seq

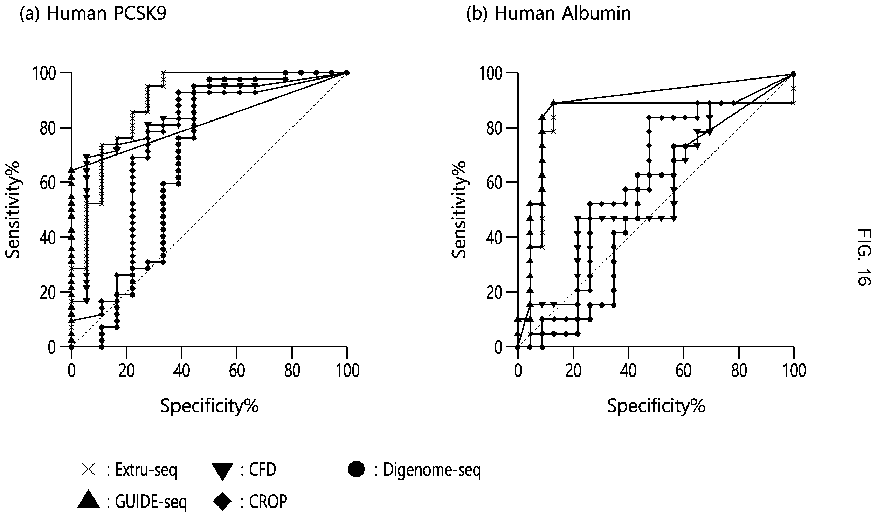

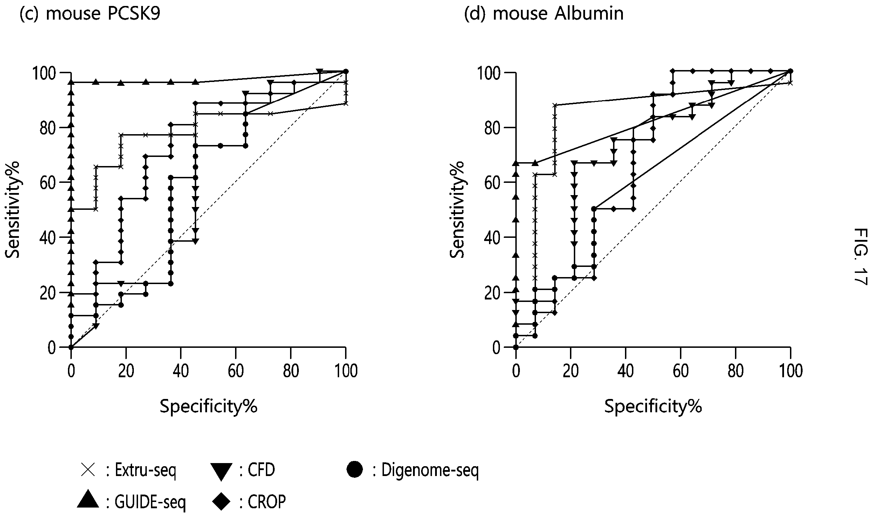

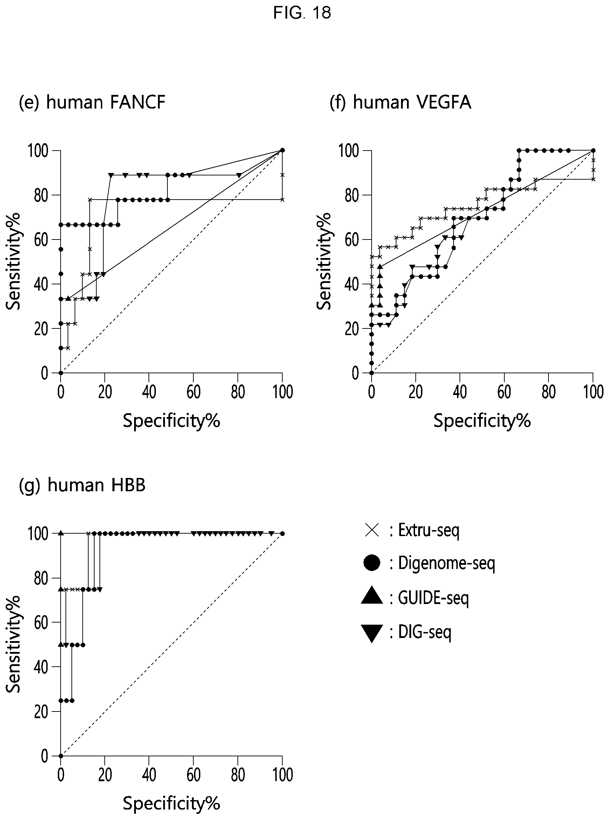

One of the powerful tools for evaluating prediction models is an ROC curve. The ROC curve shows sensitivity and specificity on the y- and x-axes, respectively. By metric for predicting the validation result through binary classification, ROC curves were plotted using sequence read counts (GUIDE-seq), DNA cleavage scores (Digenome-seq, DIG-seq, and Extru-seq), CDF scores (CDF), or CROP scores (CROP). The ROC curves for different prediction methods are shown in FIGS. 16 to 18. Specifically, FIGS. 16 to 18 show the ROC curves of GUIDE-seq, Digenome-seq, Extru-seq, CROP, and CFD prediction methods. (a) of FIG. 16 shows the result for a prediction method performed using sgRNA targeting human *PCSK9.* (b) of FIG. 16 shows the result for a prediction method performed using sgRNA targeting human *Albumin,* (c) of FIG. 17 shows the result for a prediction method performed using sgRNA targeting mouse *PCSK9.* (d) of FIG. 17 shows the result for a prediction method performed using sgRNA targeting mouse *Albumin,* (e) of FIG. 18 shows the result for a prediction method performed using sgRNA targeting human *FANCF.* (f) of FIG. 18 shows the result for a prediction method performed using sgRNA targeting human *VEGFA.* (g) of FIG. 18 shows the result for a prediction method performed using sgRNA targeting human *HBB.*

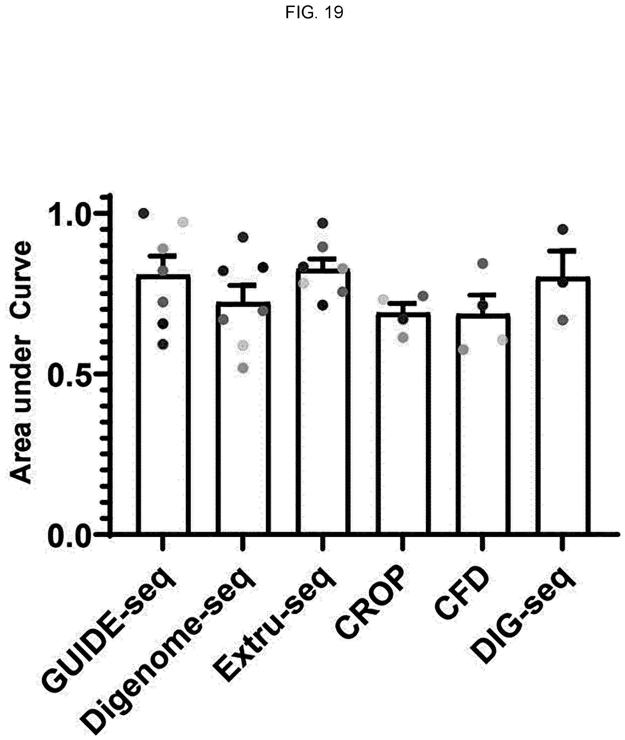

FIG. 19 shows the areas under curves of different methods, calculated from the results (ROC curve results) disclosed in FIGS. 16 to 18. Extru-seq showed an area under curve value of 0.83, GUIDE-seq showed an area under curve value of 0.81, DIG-seq showed an area under curve value of 0.80, Digenome-seq showed an area under curve value of 0.72, CROP showed an area under curve value of 0.69, and CFD showed an area under curve value of 0.68. Error bars indicate standard deviation.

As described above, the areas under the ROC curves were calculated, and Extru-seq showed the highest value of area. Specifically, Extru-seq showed an area under the ROC curve of 0.83, GUIDE-seq showed an area under the ROC curve of 0.81, DIG-seq showed an area under the ROC curve of 0.80, Digenome-seq showed an area under the ROC curve of 0.72, CROP showed an area under the ROC curve of 0.69, and CFD showed an area under the ROC curve of 0.68. The closer the area under the ROC curve is to 1, the better the model is in the prediction of the validation result. The highest area under ROC curve of Extru-seq suggests high performance in the DNA cleavage score of Extru-seq. In addition, the use of different thresholds or cutoff values may affect the predicted number of off-target sites. A high area under ROC curve indicates that the possibility of finding a significant threshold for Extru-seq is higher than other methods.

### Result 11. Use of Extru-seq in primary cells

The inventors of the present application confirmed that Extru-seq can be applied to primary cells with less optimization. The GUIDE-seq method requires a high insertion rate of double-stranded oligodeoxynucleotides (dsODNs) at double strand break (DSB) sites, which may be difficult to be experimentally achieved in some cell types and experimental conditions. For example, the inventors of the present application may not obtain the high insertion rate of dsODNs in primary mesenchymal stem cells (MSCs) derived from bone marrow. In contrast, Extru-seq does not require the insertion of dsODN. Considering these advantages of Extru-seq, the inventors of the present application performed Extru-seq in MSCs using the above-mentioned promiscuous sgRNAs targeting human *PCSK9* and *Albumin.*

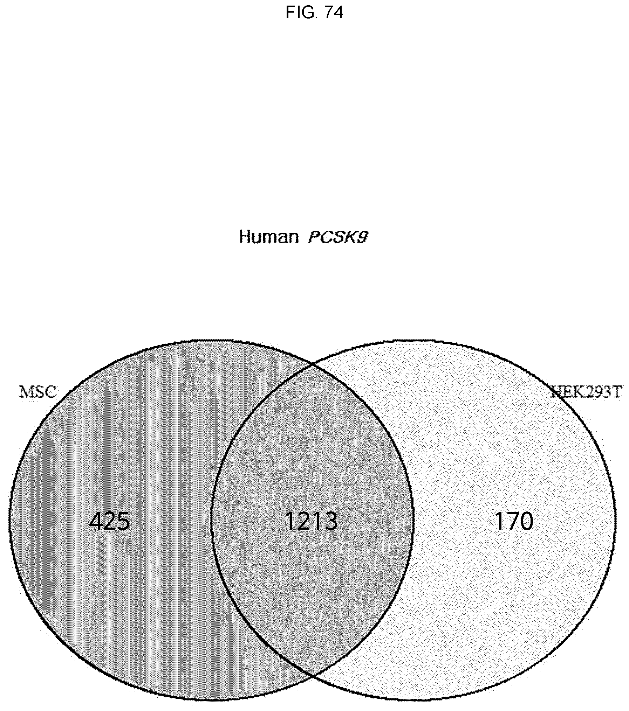

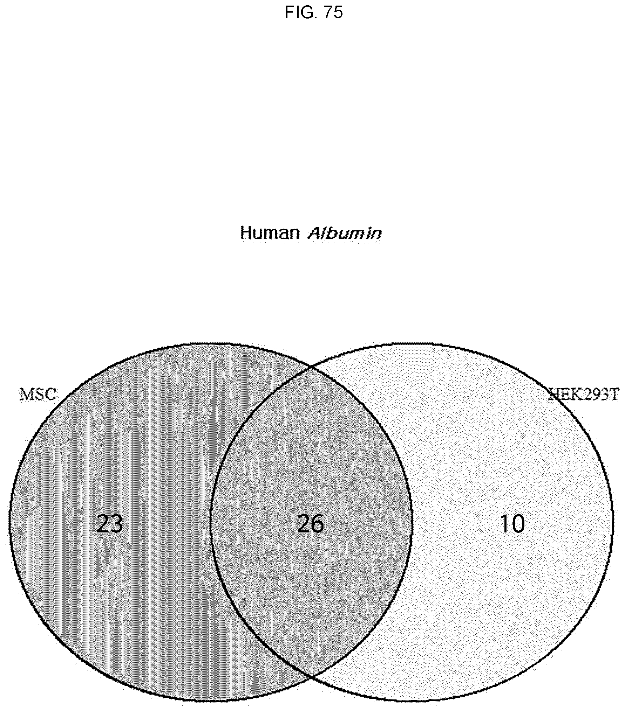

The Venn diagrams shown in FIGS. 74 and 75 show that there is a difference between the Extru-seq result obtained in MSCs and the Extru-seq result obtained in HEK293T cells.

Specifically, FIG. 74 shows the comparison result of predicted off-target sites for sgRNA targeting human *PCSK9.* It shows that only 1213 off-target sites among off-target sites predicted through Extru-seq performed on MSCs and the off-target sites predicted through Extru-seq performed on HEK293T cells overlap (showing some differences depending on the type of cells).

FIG. 75 shows the comparison results for the off-target candidates predicted using sgRNA targeting human *Albumin.* It shows that only 26 off-target sites among the off-target sites predicted by Extru-seq performed with MSCs and the off-target sites predicted by Extru-seq performed with HEK293T cells overlap (showing some differences depending on cell type).

In MSCs and HEK293T cells, the examination results for each prediction method are further shown in Tables 18 and 19. As sgRNAs, the above-mentioned sgRNAs targeting *PCSK9* and *Albumin* were used. Specifically, Table 18 shows the top 10 candidates from the off-target loci predicted by Extru-seq, performed in MSCs, for sgRNA targeting human *PCSK9.* Further, the ranks of the top 10 loci from Extru-seq, predicted by other prediction methods, are shown. The top 10 loci from Extru-seq were derived based on DNA cleavage scores.

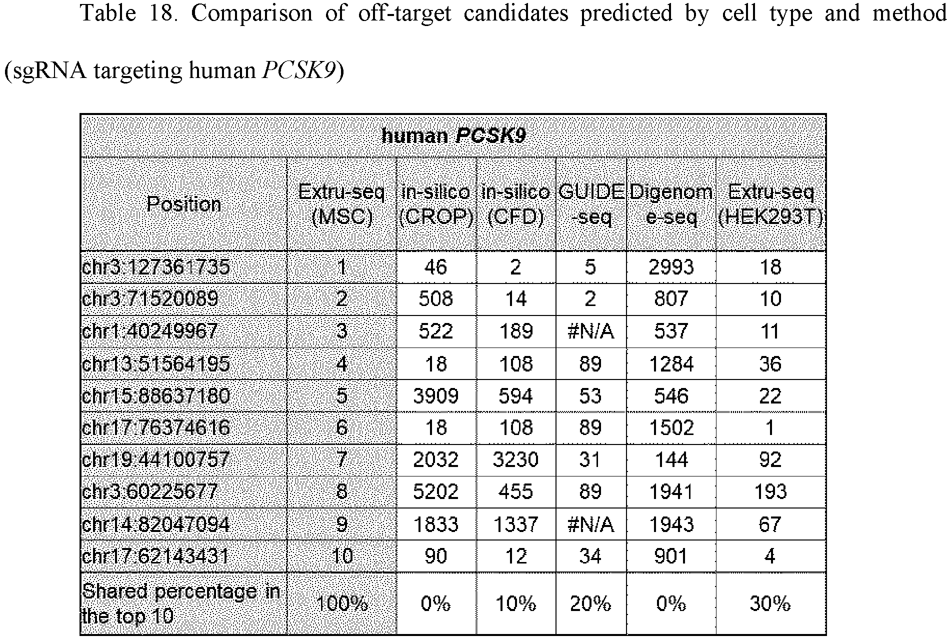

Table 19 shows the top 10 candidates of off-target loci, predicted by Extru-seq, for sgRNA targeting human *Albumin* in HEK293T cells. Further, Table 19 shows the ranks of the top10 loci from Extru-seq, predicted by other prediction methods. The top 10 loci from Extru-seq were derived based on DNA cleavage scores.

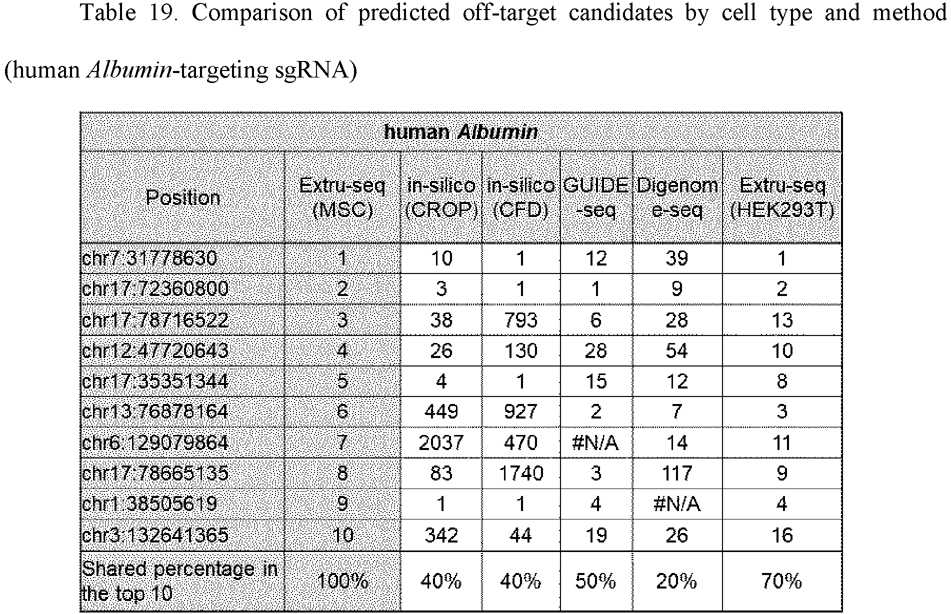

As shown in Tables 18 and 19, in comparison based on Extru-seq top 10 in MSCs, from the results associated with sgRNA targeting human *PCSK9,* it was confirmed that Extru-seq top 10 (MSC) and Extru-seq top 10 (HEK293T) were 30% identical.

In comparison based on the Extru-seq top 10 in MSCs, from the results associated with sgRNA targeting human *Albumin,* it was confirmed that Extru-seq top 10 (MSC) and Extru-seq top 10 (HEK293T) were 70% identical. These results indicate that genome-wide off-target loci predicted by Extru-seq vary depending on cell type.

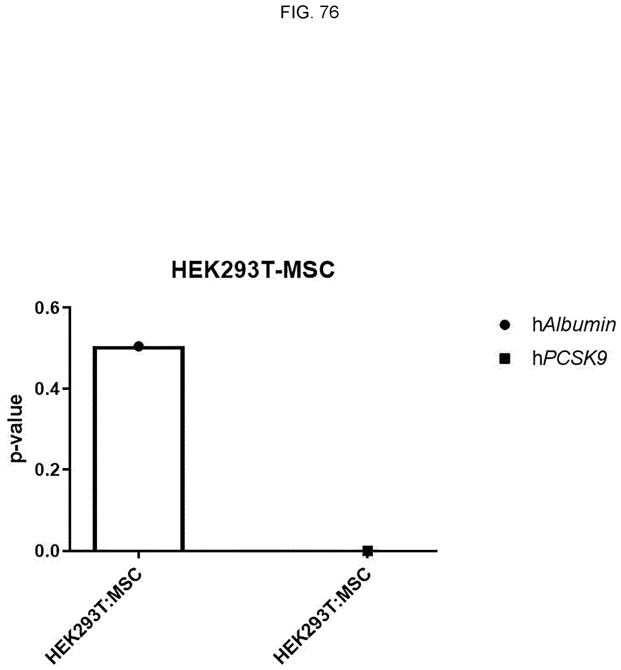

As a result of analyzing the intersections of Venn diagrams using a normalized rank sum test, a high p-value was observed in the test for sgRNA targeting *Albumin.* On the other hand, a low p-value was observed in a test for sgRNA targeting *PCSK9* (refer to FIG. 76).

Specifically, FIG. 76 shows a p-value, obtained from a normalized rank sum test with each pair, of off-target prediction method for promiscuous sgRNAs targeting *PCSK9* and *Albumin* in MSCs and HEK293T cells. These results indicate that off-target ranks may or may not change depending on cell type.

A cell-based method such as GUIDE-seq is known to miss more valid off-target candidates than in vitro and in silico methods. The inventors of the present application confirmed that the miss rate of Extru-seq (2.33%) is 12.6-fold lower than that of GUIDE-seq (29.5%). Further, similar to other in vitro methods, it was confirmed that Extru-seq can be universally applied to various cell types of different origins. This is because, unlike GUIDE-seq requiring the insertion of dsODN into DSB sites, Extru-seq does not need dsODN insertion. Extru-seq overcame key limitations of a cell-based (high miss rates and the need for optimization for different cell types) and key limitations of an in vitro method (low validation rates and the loss of cell type-specific information). In addition, the intensive performance of Extru-seq as the binary classifier of the validation results was supported by area under ROC curves for Extru-seq. Accordingly, Extru-seq is expected to be a strong candidate as a balanced method for obtaining a comprehensive list of off-target sites in various cell types and patient-specific clinical safety tests.

Most of the cell-based methods use "surrogate" cell lines to predict genome-wide off-target sites for human clinical samples. However, as can be seen by comparing the results of Extru-seq for HEK293T cells and MSCs, there may be differences in chromatin and epigenetic status between a dividing in vitro cell line and most non-dividing in vivo cells. Therefore, it is preferable that off-target prediction is performed by Extru-seq with clinically more relevant cells, not a surrogate cell line. Recently, two cell-based methods, such as DISCOVER-seq (refer to the document [Wienert, Beeke, et al. "Unbiased detection of CRISPR off-targets in vivo using DISCOVER-Seq." Science 364.6437 (2019): 286-289.]) and GUIDE-tag (refer to the document [Liang, Shun-Qing, et al. "Genome-wide detection of CRISPR editing in vivo using GUIDE-tag." Nature communications 13.1 (2022): 1-14.]), were directly performed in vivo in mouse models. However, for preclinical research for human treatments, performing these methods directly in human organs is almost impossible. Extru-seq has the advantage of being performed in primary human cells isolated from a specific patient or organ. In the experimental examples of the present application, whole genome sequencing (WGS) was used as a genome analysis method after the extrusion of Extru-seq. The present application is not limited to the methods disclosed in the experimental examples of the present application, and it is strongly predicted that, for genome analysis after extrusion (e.g., analysis at DNA cleavage positions), different methods such as the PCR-based amplification protocol (e.g., PCR-based amplification protocol used in SITE-seq, refer to the document [Cameron, Peter, et al. "Mapping the genomic landscape of CRISPR-Cas9 cleavage." Nature methods 14.6 (2017): 600-606.]) may also be used. Further, it is strongly predicted that, for Extru-seq optimization, the optimization of algorithms used (e.g., optimization of algorithms for increasing analysis sensitivity), optimization of extruder (e.g., optimization of the size, cost, and throughput etc., of an extruder) may be performed. Inventions that succeed the inventive idea of Extru-seq disclosed in the present application, developed after the filing date of the present application, will be comprised in the scope of the present application. Further, Extru-seq may be used in combination with a tool such as recently developed CAST-seq (refer to the document [Turchiano G, Andrieux G, Klermund J, Blattner G, Pennucci V, El Gaz M, Monaco G, Poddar S, Mussolino C, Cornu TI et al: Quantitative evaluation of chromosomal rearrangements in gene-edited human stem cells by CAST-Seq. Cell Stem Cell 2021, 28(6):1136-1147 e1135.]) to detect Cas9-mediated large-scale deletion, chromosome depletion and translocation.

Hereinafter, some of the reference documents referred in this specification are disclosed. The documents referenced in this specification may or may not be mentioned in the paragraphs relevant to the corresponding reference.

### References

1. Mullard A: Gene-editing pipeline takes off. Nat Rev Drug Discov 2020, 19(6):367-372.
2. Tsai SQ, Zheng Z, Nguyen NT, Liebers M, Topkar VV, Thapar V, Wyvekens N, Khayter C, Iafrate AJ, Le LP et al: GUIDE-seq enables genome-wide profiling of off-target cleavage by CRISPR-Cas nucleases. Nat Biotechnol 2015, 33(2):187-197.
3. Liang SQ, Liu P, Smith JL, Mintzer E, Maitland S, Dong X, Yang Q, Lee J, Haynes CM, Zhu LJ et al: Genome-wide detection of CRISPR editing in vivo using GUIDE-tag. Nat Commun 2022, 13(1):437.
4. Wienert B, Wyman SK, Richardson CD, Yeh CD, Akcakaya P, Porritt MJ, Morlock M, Vu JT, Kazane KR, Watry HL et al: Unbiased detection of CRISPR off-targets in vivo using DISCOVER-Seq. Science 2019, 364(6437):286-289.
5. Yan WX, Mirzazadeh R, Garnerone S, Scott D, Schneider MW, Kallas T, Custodio J, Wernersson E, Li Y, Gao L et al: BLISS is a versatile and quantitative method for genome-wide profiling of DNA double-strand breaks. Nat Commun 2017, 8:15058.
6. Crosetto N, Mitra A, Silva MJ, Bienko M, Dojer N, Wang Q, Karaca E, Chiarle R, Skrzypczak M, Ginalski K et al: Nucleotide-resolution DNA double-strand break mapping by next-generation sequencing. Nat Methods 2013, 10(4):361-365.
7. Wang X, Wang Y, Wu X, Wang J, Qiu Z, Chang T, Huang H, Lin RJ, Yee JK: Unbiased detection of off-target cleavage by CRISPR-Cas9 and TALENs using integrase-defective lentiviral vectors. Nat Biotechnol 2015, 33(2):175-178.
8. Chiarle R, Zhang Y, Frock RL, Lewis SM, Molinie B, Ho YJ, Myers DR, Choi VW, Compagno M, Malkin DJ et al: Genome-wide translocation sequencing reveals mechanisms of chromosome breaks and rearrangements in B cells. Cell 2011, 147(1):107-119.
9. Petri K, Kim DY, Sasaki KE, Canver MC, Wang X, Shah H, Lee H, Horng JE, Clement K, Iyer S et al: Global-scale CRISPR gene editor specificity profiling by ONE-seq identifies population-specific, variant off-target effects. bioRxiv 2021:2021.2004.2005.438458.
10. Kim HS, Hwang GH, Lee HK, Bae T, Park SH, Kim YJ, Lee S, Park JH, Bae S, Hur JK: CReVIS-Seq: A highly accurate and multiplexable method for genome-wide mapping of lentiviral integration sites. Mol Ther Methods Clin Dev 2021, 20:792-800.
11. Breton C, Clark PM, Wang L, Greig JA, Wilson JM: ITR-Seq, a next-generation sequencing assay, identifies genome-wide DNA editing sites in vivo following adeno-associated viral vector-mediated genome editing. BMC Genomics 2020, 21(1):239.
12. Huang H, Hu Y, Huang G, Ma S, Feng J, Wang D, Lin Y, Zhou J, Rong Z: Tag-seq: a convenient and scalable method for genome-wide specificity assessment of CRISPR/Cas nucleases. Commun Biol 2021, 4(1):830.
13. Kim D, Bae S, Park J, Kim E, Kim S, Yu HR, Hwang J, Kim JI, Kim JS: Digenome-seq: genome-wide profiling of CRISPR-Cas9 off-target effects in human cells. Nat Methods 2015, 12(3):237-243, 231 p following 243.
14. Kim D, Kim JS: DIG-seq: a genome-wide CRISPR off-target profiling method using chromatin DNA. Genome Res 2018, 28(12):1894-1900.
15. Cameron P, Fuller CK, Donohoue PD, Jones BN, Thompson MS, Carter MM, Gradia S, Vidal B, Garner E, Slorach EM et al: Mapping the genomic landscape of CRISPR-Cas9 cleavage. Nat Methods 2017, 14(6):600-606.
16. Tsai SQ, Nguyen NT, Malagon-Lopez J, Topkar VV, Aryee MJ, Joung JK: CIRCLE-seq: a highly sensitive in vitro screen for genome-wide CRISPR-Cas9 nuclease off-targets. Nat Methods 2017, 14(6):607-614.
17. Lazzarotto CR, Malinin NL, Li Y, Zhang R, Yang Y, Lee G, Cowley E, He Y, Lan X, Jividen K et al: CHANGE-seq reveals genetic and epigenetic effects on CRISPR-Cas9 genome-wide activity. Nat Biotechnol 2020, 38(11):1317-1327.
18. Bae S, Park J, Kim JS: Cas-OFFinder: a fast and versatile algorithm that searches for potential off-target sites of Cas9 RNA-guided endonucleases. Bioinformatics 2014, 30(10):1473-1475.
19. Montague TG, Cruz JM, Gagnon JA, Church GM, Valen E: CHOPCHOP: a CRISPR/Cas9 and TALEN web tool for genome editing. Nucleic Acids Res 2014, 42(Web Server issue): W401-407.
20. Concordet JP, Haeussler M: CRISPOR: intuitive guide selection for CRISPR/Cas9 genome editing experiments and screens. Nucleic Acids Res 2018, 46(W1):W242-W245.
21. Shapiro J, Iancu O, Jacobi AM, McNeill MS, Turk R, Rettig GR, Amit I, Tovin-Recht A, Yakhini Z, Behlke MA et al: Increasing CRISPR Efficiency and Measuring Its Specificity in HSPCs Using a Clinically Relevant System. Mol Ther Methods Clin Dev 2020, 17:1097-1107.
22. Gillmore JD, Gane E, Taubel J, Kao J, Fontana M, Maitland ML, Seitzer J, O'Connell D, Walsh KR, Wood K et al: CRISPR-Cas9 In Vivo Gene Editing for Transthyretin Amyloidosis. N Engl J Med 2021, 385(6):493-502.
23. Maeder ML, Stefanidakis M, Wilson CJ, Baral R, Barrera LA, Bounoutas GS, Bumcrot D, Chao H, Ciulla DM, DaSilva JA et al: Development of a gene-editing approach to restore vision loss in Leber congenital amaurosis type 10. Nat Med 2019, 25(2):229-233.
24. Poirot L, Philip B, Schiffer-Mannioui C, Le Clerre D, Chion-Sotinel I, Derniame S, Potrel P, Bas C, Lemaire L, Galetto R et al: Multiplex Genome-Edited T-cell Manufacturing Platform for "Off-the-Shelf" Adoptive T-cell Immunotherapies. Cancer Res 2015, 75(18):3853-3864.
25. MacLeod DT, Antony J, Martin AJ, Moser RJ, Hekele A, Wetzel KJ, Brown AE, Triggiano MA, Hux JA, Pham CD et al: Integration of a CD19 CAR into the TCR Alpha Chain Locus Streamlines Production of Allogeneic Gene-Edited CAR T Cells. Mol Ther 2017, 25(4):949-961.
26. Stadtmauer EA, Fraietta JA, Davis MM, Cohen AD, Weber KL, Lancaster E, Mangan PA, Kulikovskaya I, Gupta M, Chen F et al: CRISPR-engineered T cells in patients with refractory cancer. Science 2020, 367(6481).
27. Goh WJ, Zou S, Ong WY, Torta F, Alexandra AF, Schiffelers RM, Storm G, Wang JW, Czarny B, Pastorin G: Bioinspired Cell-Derived Nanovesicles versus Exosomes as Drug Delivery Systems: a Cost-Effective Alternative. Sci Rep 2017, 7(1):14322.
28. Kim D, Kim S, Park J, Kim JS: Genome-wide target specificities of CRISPR-Cas9 nucleases revealed by multiplex Digenome-seq. Genome Res 2016, 26(3):406-415.
29. Chu VT, Weber T, Wefers B, Wurst W, Sander S, Rajewsky K, Kuhn R: Increasing the efficiency of homology-directed repair for CRISPR-Cas9-induced precise gene editing in mammalian cells. Nat Biotechnol 2015, 33(5):543-548.
30. Akcakaya P, Bobbin ML, Guo JA, Malagon-Lopez J, Clement K, Garcia SP, Fellows MD, Porritt MJ, Firth MA, Carreras A et al: In vivo CRISPR editing with no detectable genome-wide off-target mutations. Nature 2018, 561(7723):416-419.
31. Liu Q, Cheng X, Liu G, Li B, Liu X: Deep learning improves the ability of sgRNA off-target propensity prediction. BMC Bioinformatics 2020, 21(1):51.
32. Doench JG, Fusi N, Sullender M, Hegde M, Vaimberg EW, Donovan KF, Smith I, Tothova Z, Wilen C, Orchard R et al: Optimized sgRNA design to maximize activity and minimize off-target effects of CRISPR-Cas9. Nat Biotechnol 2016, 34(2): 184-191.
33. Mundry R, Fischer J: Use of statistical programs for nonparametric tests of small samples often leads to incorrect P values: examples from animal behaviour.. Animal Behaviour 1998, 56:256-259.
34. Dwivedi AK, Mallawaarachchi I, Alvarado LA: Analysis of small sample size studies using nonparametric bootstrap test with pooled resampling method. Stat Med 2017, 36(14):2187-2205.
35. Frangoul H, Altshuler D, Cappellini MD, Chen YS, Domm J, Eustace BK, Foell J, de la Fuente J, Grupp S, Handgretinger R et al: CRISPR-Cas9 Gene Editing for Sickle Cell Disease and beta-Thalassemia. N Engl J Med 2020, 384(3):252-260.
36. Turchiano G, Andrieux G, Klermund J, Blattner G, Pennucci V, El Gaz M, Monaco G, Poddar S, Mussolino C, Cornu TI et al: Quantitative evaluation of chromosomal rearrangements in gene-edited human stem cells by CAST-Seq. Cell Stem Cell 2021, 28(6):1136-1147 e1135.
37. Park J, Bae S, Kim JS: Cas-Designer: a web-based tool for choice of CRISPR-Cas9 target sites. Bioinformatics 2015, 31(24):4014-4016.
38. Cho SW, Kim S, Kim JM, Kim JS: Targeted genome engineering in human cells with the Cas9 RNA-guided endonuclease. Nat Biotechnol 2013, 31(3):230-232.
39. Kim E, Koo T, Park SW, Kim D, Kim K, Cho HY, Song DW, Lee KJ, Jung MH, Kim S et al: In vivo genome editing with a small Cas9 orthologue derived from Campylobacter jejuni. Nat Commun 2017, 8:14500.
40. Kim D, Kang BC, Kim JS: Identifying genome-wide off-target sites of CRISPR RNA-guided nucleases and deaminases with Digenome-seq. Nat Protoc 2021, 16(2):1170-1192.
41. Park J, Childs L, Kim D, Hwang GH, Kim S, Kim ST, Kim JS, Bae S: Digenome-seq web tool for profiling CRISPR specificity. Nat Methods 2017, 14(6):548-549.
42. DiGiusto DL, Cannon PM, Holmes MC, Li L, Rao A, Wang J, Lee G, Gregory PD, Kim KA, Hayward SB et al: Preclinical development and qualification of ZFN-mediated CCR5 disruption in human hematopoietic stem/progenitor cells. Mol Ther Methods Clin Dev 2016, 3:16067.
43. A Safety and Efficacy Study Evaluating CTX110 in Subjects With Relapsed or Refractory B-Cell Malignancies (CARBON). https://clinicaltrials.gov/ct2/show/NCT04035434. Accessed 15 Dec 2022.
44. Safety, Tolerability, and PK of LBP-EC01 in Patients With Lower Urinary Tract Colonization Caused by E. Coli. https://clinicaltrials.gov/ct2/show/NCT04191148. Accessed 15 Dec 2022.
45. Miller JC, Paschon D, Rebar EJ: METHODS AND COMPOSITIONS FOR TREATING HEMOPHILIA. World Intellectual Property Organization 2015, WO:2015/089046.
46. Kwon J, Kim M, Lee J: Extru-seq: A method for predicting genome-wide off-target sites with high sensitivity. NCBI Bioproject 2022, PRJNA796642. https://www.ncbi.nlm.nih.gov/bioproj ect/?term=PRJNA796642

## Claims

1. A method for identifying information about off-targets occurring in a genome editing process using a CRISPR/Cas genome editing system, comprising:
(i) preparing a starting composition comprising a Cas protein, a guide RNA, and a cell;
(ii) obtaining an analyte composition through physically disrupting the cell,
wherein, through the physically disrupting the cell, a genome DNA contacts with a Cas/gRNA complex formed by the Cas protein and the guide RNA, whereby the genome DNA is cleaved at one or more cleavage sites; and
(iii) analyzing the analyte composition to obtain information about one or more cleavage sites.

2. The method of claim 1,
wherein the physically disrupting the cell comprises passing the cell through a filter having pores, wherein an average diameter of the pores of the filter is smaller than the size of the cell.

3. The method of claim 2,
wherein a force that causes the cell to pass through the filter is pressure.

4. The method of claim 2,
wherein the average diameter of the pores of the filter is 5 to 15µm.

5. The method of claim 1,
wherein the physically disrupting the cell is achieved through a use of an extruder comprising a filter with pores.

6. The method of claim 5,
wherein an average diameter of the pores of the filter comprised in the extruder is smaller than a size of the cell.

7. The method of claim 5,
wherein an average diameter of the pores of the filter is 5 to 15µm.

8. The method of claim 1,
wherein the information about the cleavage site comprises one or more of the following:
a genomic DNA location of each cleavage site for the one or more cleavage sites;
a cleavage score of each cleavage site for the one or more cleavage sites; and
the number of cleavage sites.

9. The method of claim 1,
wherein the method further comprises:
(iv) identifying an information about off-target candidate from the information about cleavage site obtained from (iii).

10. The method of claim 9,
wherein the information about off-target candidate comprises one or more of the following:
a genomic DNA location of each off-target candidate for one or more off-target candidates;
an off-target prediction score of each off-target candidate for one or more off-target candidates; and
the number of predicted off-target candidates.

11. The method of claim 1,
wherein the analyzing the analyte composition comprises analyzing a cleaved genome DNA contained in the analyte composition through a sequencing.

12. The method of claim 1,
wherein the analyzing the analyte composition comprises analyzing a cleaved genome DNA contained in the analyte composition through a PCR-based method.

13. The method of claim 1,
wherein, through the physically disrupting the cell, a membrane structure comprising a cell membrane is disrupted, whereby an environment in which the Cas/gRNA complex is able to contact the genome DNA from the cell is created.

14. The method of claim 1,
wherein, through the physically disrupting the cell, a membrane structure comprising a nuclear membrane of the cell is disrupted, whereby an environment in which the Cas/gRNA complex is able to contact the genome DNA from the cell is created.

15. The method of claim 1,
wherein the method further comprises:
identifying a predetermined CRISPR/Cas genome editing system, wherein the identifying predetermined CRISPR/Cas genome editing system is performed prior to (i).

16. The method of claim 15,
wherein the predetermined CRISPR/Cas genome editing system comprises a use of a predetermined guide RNA having a predetermined guide sequence,
wherein the predetermined guide sequence and a guide sequence of the guide RNA are same.

17. The method of claim 15,
wherein the predetermined CRISPR/Cas genome editing system comprises a use of a predetermined cell, wherein the predetermined cell and the cell are same.

18. The method of claim 1,
wherein the analyte composition comprises a cleaved genome DNA in which the genome DNA from physically disrupted cell is cleaved by the Cas/gRNA complex.

19. The method of claim 1,
wherein a concentration of the Cas protein contained in the starting composition is 4000nM to 6000nM.

20. The method of claim 1,
wherein a concentration of the guide RNA contained in the starting composition is 4000nM to 6000nM.

21. The method of claim 1,
wherein a concentration of the Cas/gRNA complex contained in the starting composition is 4000nM to 6000nM.

22. The method of claim 1,
wherein a concentration of the cell contained in the starting composition is 1×10⁷ cells/mL.

23. The method of claim 1,
wherein the obtaining an analyte composition further comprises: incubating a composition obtained through the disrupting the cell.

24. The method of claim 1,
wherein the obtaining an analyte composition further comprises:
removing RNAs from a composition obtained through the disrupting the cell.

25. The method of claim 1,
wherein the obtaining an analyte composition further comprises:
purifying DNAs from a composition obtained through the disrupting the cell.

26. A method for identifying information about off-targets occurring in a genome editing process using a CRISPR/Cas genome editing system, comprising:
(i) loading a starting composition comprising a Cas protein, a guide RNA and a cell into a first container of an extruder;
(ii) performing an extrusion process comprising the following step, using the extruder to obtain an analyte composition:
applying pressure to the first container to move components of the starting composition from the first container of the extruder to a second container of the extruder,
wherein the components of the starting composition pass through a filter with pores, which is located between the first container and the second container by the applied pressure, whereby a mixture is loaded to the second container;
wherein the cell, which is a component larger in size than a diameter of pore of the filter, is disrupted and passes through the pores of the filter, by the applied pressure,
wherein through physically disrupting the cell, an environment in which a genome DNA is able to contact the Cas protein and the guide RNA is created,
whereby the genome DNA contacts with a Cas/gRNA complex,
whereby the genome DNA is cleaved at one or more cleavage sites; and
(iii) analyzing the analyte composition to obtain information of the cleavage site.

27. The method of claim 26,
wherein the pressure applied to the first container is generated through a process of pushing a piston designed for applying the pressure to the first container in a direction to the first container and the filter.

28. A method for identifying information about off-target occurring in a genome editing process using a CRISPR/Cas genome editing system, comprising:
(i) loading a starting composition comprising a Cas protein, a guide RNA and a cell into a first container of an extruder;
(ii) performing an extrusion process comprising the following steps, using the extruder to obtain an analyte composition:
(a) applying pressure to the first container to move components of the starting composition from the first container of the extruder to a second container of the extruder,
wherein the components of the starting composition pass through a filter with pores, which is located between the first container and the second container by the applied pressure, whereby a mixture is loaded to the second container,
(b) applying pressure to the second container to move components of the mixture from the second container to the first container,
wherein the components of the mixture pass through the filter with pores, which is located between the first container and the second container by the applied pressure, whereby a mixture moved from the second container by passing through the filter is loaded to the first container, and
(c) repeating processes (a) and (b) a predetermined number of times,
wherein the predetermined number of times is counted in increments of 0.5, wherein 0.5 represents a performance of a single process of (a) or (b),
wherein the cell, which is a component larger in size than a diameter of pore of the filter, is disrupted and passes through the pores of the filter, by the applied pressure,
wherein through physically disrupting the cell, an environment in which the genome DNA is able to contact the Cas protein and the guide RNA is created,
whereby the genome DNA contacts with a Cas/gRNA complex,
whereby the genome DNA is cleaved at one or more cleavage sites; and
(iii) analyzing the analyte composition to obtain information of the cleavage site.

29. The method of claim 28,
wherein the pressure applied to the first container is generated through a process of pushing a piston designed for applying the pressure to the first container in a direction to the first container and the filter,
wherein the pressure applied to the second container is generated through a process of pushing a piston designed for applying pressure to the second container in a direction to the second container and the filter.
